# EUROPEAN PATENT APPLICATION

(11) **EP 3 239 302 A1**
(43) Date of publication of application: **01.11.2017**
(21) Application number: 14908826.2
(22) Date of filing: 26.12.2014
(51) Int. Cl.: C12Q 1/68

(54) **METHOD FOR DETECTING DIFFERENTIALLY METHYLATED CPG ISLANDS ASSOCIATED WITH ABNORMAL STATE OF HUMAN BODY**

(71) Applicant: Peking University, Beijing 100871 (CN); Beijing Shijitan Hospital Capital Medical Universty, Haidian District, Beijing 100038 (CN)
(72) Inventor: WEN, Lu, Beijing 100871 (CN); PENG, Jirun, Beijing 100038 (CN); HUANG, Yanyi, Beijing 100871 (CN); TANG, Fuchou, Beijing 100871 (CN); LIU, Xiaomeng, Beijing 100871 (CN); LI, Jingyi, Beijing 100871 (CN); GUO, Huahu, Beijing 100038 (CN)
(74) Representative: Haseltine Lake LLP
(86) International application number: PCT/CN2014/095120
(87) International publication number: WO 2016/101258

(57) **Abstract**

Disclosed is a method for detecting differentially methylated CpG islands associated with an abnormal state of a human body, characterized by detecting very minute amounts of methylated CpG short tandem nucleic acid sequences in highly fragmented DNA samples with genome scale, identifying differentially methylated CpG islands associated with abnormal state of human body and determining the corresponding abnormal state of human body. Sequencing libraries are constructed by using CpG short tandem sequences as primers to perform three steps of PCR reactions on DNAs which are conversed by nodifiers, and detections of very minute amounts of methylated CpG short tandem nucleic acid sequences are implemented with high throughput sequencing technology. A group of genome sequences and methylation patterns of differentially methylated CpG islands which are associated with hepatocellular carcinoma are also disclosed; they may be used for distinguishing between hepatacellular carcinoma and non-cancerous state.

## Description

### Field of the Invention

The present invention relates to the field of biomedicine. Especially, the present invention is a method and a system to determine a human abnormal state such as cancer *via* DNA methylation information.

### Background Art

DNA methylation is the modification of the cytosine (C) to the 5'-methylated-cytosine (5mC) by adding a methyl group to the C5 position of the cytosine, mainly occuring at the CpG site (CpG indicates dinucleotide of which the guanine (G) base immediately follows the cytosine base along the DNA strand). Most of the CpG sites were diffused distributed in the human genome and highly methylated. However, in some region of the genome, CpG dinucleotides show the expected or even higher frequency and these regions are referred to as CpG islands (about 30,000 in the human genome), with most CpG islands being demethylated in the normal physical state. CpG islands enrich in gene promoters, with more than 60% of promoters contain a CpG island. Demethylation of the CpG islands promises the activity of gene transcription. And methylation of the promoter CpG islands leads to silencing of gene expression by recruiting methylation binding proteins. This mechanism participates in many physical biological processes, including X-chromosome inactivation and genomic imprinting (Jones and Baylin, Nat Rev Genet, 2002).

Aberrant methylation of CpG islands is common in human cancers. It not only occurs in nearly all common human cancerss, such as hepatocellular carcinoma, colon cancer, lung cancer, gastric cancer, breast cancer, prostatic cancer et al., but also occurs in the early stage of caner or even precancerous lesion, and relates to a great many genes (for reviews see Baylin and Jones, Nat Rev Cancer, 2011; Feinberg and Tycko, Nat Rev Cancer, 2004). In recent years, a lot of genomic studies show that thousands of CpG islands could be aberrantly hypermethylated in various types of tumors, and it has been estimated that on average hundreds of CpG islands can be aberrantly hypermethylated at the early stage of a cancer (Ammerpohl et al., Int J Cancer 2012; Costello et al., Nat Genet 2000; Hinoue et al., Genome Res 2012; Keshet et al., Nat Genet 2006). Aberrant hypermethylation of some CpG islands is closely related to prognosis and drug resistance of cancer. In addition, aberrant CGI hypermethylation is related to other human abnormal states such as systemic lupus erythematosus (Robertson. Nat Rev Genet, 2005). The human body fluid (including blood, urine, saliva and excretion, ect) contains the cell-free DNA. Detecting cell-free DNA in the human body fluid is a noninvasive way to obtain information about the human state, minimizing the hurt to the body that could occur when sampling the tissue. (Schwarzenbach et al., Nat Rev Cancer, 2011). Studies have shown that tumor DNA can be release to blood at the early cancer stage. Therefore, detecting the cell-free tumor DNA (ctDNA) shows great promises for detecting, screening and monitoring cancers in a non-invasive manner. Comparing with the mutation of tumor DNA, aberrant CpG island hypermethyaltion occurs more frequently and thus have more advantages in cancer screening (Heyn and Esteller, Nat Rev Genet, 2012; Schwarzenbach et al., 2011).

However, since ctDNA is scare in amount (at the level of pilgrims) and makes up only a minority of the total ccfDNA (0.1%-1%) and is highly fragmented (70-200 base pairs), dection of ctDNA is a technical challenge. (Schwarzenbach et al., Nat Rev Cancer, 201). Current reported techniques for detecting CpG island methylation in ctDNA, including restriction enzyme digestion, restriction enzyme digestion and PCR, methylation-specific PCR (Herman, et al., PNAS, 1999), pyrosequencing (Korshunova, et al., Genome Research), Methylight (Eads et al., Nucleic Acids Res, 2000), MethylBeaming (Li et al., Nat Biotechnol, 2009), have relatively high sensitivity and are suitable for detecting highly-fragmented DNAs. However, they are able to detect single or a small number of sites. On the other side, genome-wide DNA methylation methods (Laird, Nat Rev Genet, 2010), such as the Infinium methylation array (Bibikova et al., Genome Research, 2006) and reduced representation bisulfite sequencing (RRBS) (Meissner et al., Nature, 2008), have been successfully used for cell and tissue samples. Their applications to ctDNA, however, are hampered by technical limitations. For example, the Infinium methylation array requires more than 1 microgram DNA (Bibikova et al., Genome Research, 2006). RRBS is able to detect DNA of pilgrims, but includes a size selection step that is not suitable for severely fragmented ccfDNA (Gu et al., Nature Protocol, 2011). Direct detection of the ccfDNA by whole genome bisulfite sequencing has been reported, which is able to detect the more wide-spreading hypomethylation changes. However, as the depth of sequencing is relatively low (1x coverage per CpG island), it is hard to analysis CpG island hypermethylation (Chan, et al., PNAS, 2013).

Therefore, techniques for high-efficient detection of the human abnormal states related methylated CpG islands from extremely small amount of ccfDNA remain limited.

### Summary of the Invention

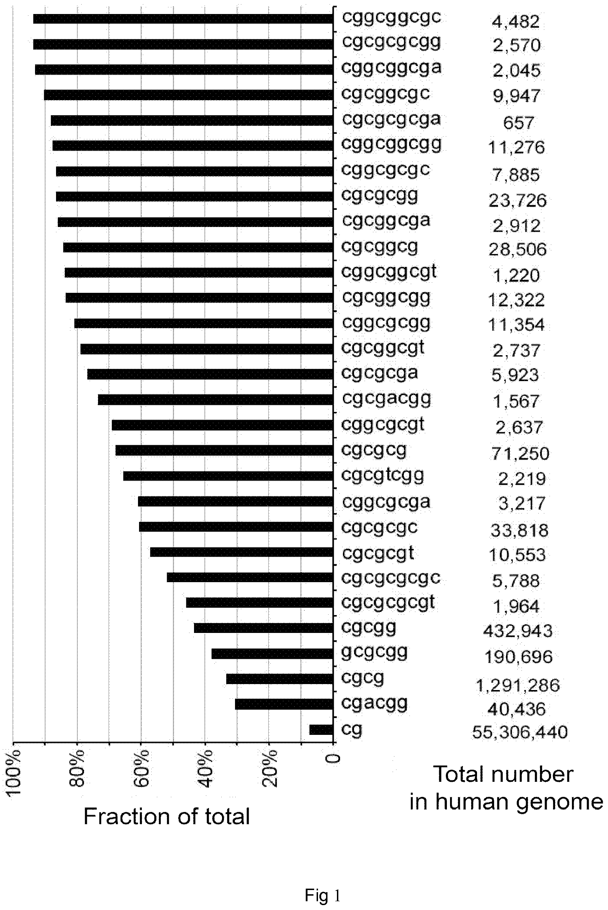

In a first aspect, the present invention provides a method for detecting differentially methylated genomic sites relating to human abnormal states, comprising the following steps:
(1) Obtaining human DNA samples.
   The DNA sample can come from human cells, tissues, blood, body fluid, urine, excrement or their combination. Preferably, the DNA sample is the cell-free DNA of human plasma or urine. Cell-free DNA in peripheral blood mainly comes from blood cells; comparing with serum, there is less DNA released from blood cells in plasma, which could intensively reduce the noise comeing from the blood cell DNA. A large amount of ccfDNA could be flushed out by urine, so we could detect ccfDNA from urine. Comparing with plasma, urine analysis is a more non-invasive means for detection of ccfDNA.
(2) Genome-scale detection of methylation of the short CpG tandem sequences of the referred DNA samples
   Genome-scale detection refers to detecting more than 100 CpG islands simultaneously. Comparing with the existing method of detecting CpG islands which can only detect one single or a small number of sites, our method can detect thousands of CpG islands. Thus, the present invention has a significant improvement over the prior art. The key to the improvement of present invention lies in the setting of short CpG tandem as the target. Short CpG tandem refers to the short DNA sequence (7-9 base pairs) which contains three or more than three CpG nucleotide pairs. These short CpG tandems have three characteristics: a) There is a large number of copies in the human genome. So they could be the targets for genome-scale detection. b) These short CpG tandem sequences are highly enriched in the CpG islands, so they contain important methylation information that can be used to determine the abnormal state of the human body. c) These short CpG tandem sequences have a relatively high melting temperature and can therefore be efficiently amplified. Taking CGCGCGG as an example (Fig1), out of all 23,726 CGCGCGG sequences used in this study, 20,525 (86.5%) are located within the 9,373 (34.2% of all 27,435) CGIs in the human genome. Its melting temperature is 43 degrees Celsius, which is close to the conventional PCR reaction annealing temperature. Therefore, CGCGCGG is a good target site for genome-scale detection of methylation level. For there is a large amount of CGCGCGG in human genome and it is enriched in the CpG islands, CGCGCGG is one of the most preferred short CpG tandem for genome-scale detection of methylation level. In addition to CGCGCGG, there are other good choices for target sites, such as CGGCGGCGG (87.6% of the 11,276 CGGCGGCGG are located within CpG islands), CGCGGCGG (83.6% of the 12,322 CGCGGCGG are located within CpG islands), CGGCGCGG (80.8% of the 11,354 CGGCGCGG are located within CpG islands), CGCGGCGC (90.3% of the 9,947 CGCGGCGC are located within CpG islands), CGGCGCGC (86.6% of the 7,885 CGGCGCGC are located within CpG islands), CGCGCGC (60.7% of the 33,818 CGCGCGC are located within CpG islands), CGCGCGA (76.8% of the 5,923 CGCGCGA are located within CpG islands) and CGCGCGT (57% of the 10,553 CGCGCGT are located within CpG islands). Targeting several DNA short tandem simultaneously could further improve the detection range and the detection sensitivity. The guanines outside of CpG in the DNA short tandems improve the melting temperature and amplification efficiency. Professional staff in this field know that the specificity of hybridization and amplification of nucleic acid sequences is mainly dependent on its 3 'end sequence, while the effect of 5'-end CpG nucleotide pair is gradually reduced. For short CpG tandem sequences, the first 3 nucleotides at 3'-end are the most important for hybridization, followed by 4 to 6 nucleotides, and then 7 to 9 nucleotides. There are eight preferred sequences for the 3'-end of primer, CGCGG, CGCGA, CGCGT, CGCGC, CGGCGG, CGGCGA, CGGCGT and GGCGC, in which CGCGG and CGGCGG are prefered, and CGCGG is the most prefered. Atypical short CpG tandem sequence such as GGCGCGG, which has less CpG nucleotides (2 CpG) than the typical one, could also be used for genome-scale detection of methylated CpG islands with lower detection efficiency. Longer short CpG tandem sequence (more than 9 base pairs) and more CpG nucleotides will further increase the melting temperature, however, with less targeting sites. As described above, CpG nucleotide at 5'-end of the primer has less effect on the primer specificity compared with nucleotide at 3'-end.
(3) Comparing the DNA methylation level of short CpG tandems described above in DNA samples mentioned above with those in DNA samples of normal individuals, we could identify the CpG islands with aberrant hypermethylation level in abnormal human body and,
(4) using the differential methylated CpG islands, we could diagnose the corresponding human abnormalities.

On one hand, the present invention can be applied to clinical research, large-scale analysis and identification of differential methylation CpG islands associated with abnormalities of the human body; on the other hand, it can be applied to clinical molecular testing, by identifying the differential methylated CpG island to predict if an individual has an abnormal state.

The existing DNA methylation assay, such as the Infinium methylation chip and RRBS, can be used to analyze the differential methylated CpG islands associated with abnormal human status in tissue or cell DNA samples, but the results can not be directly converted into clinical application of free DNA in clinical molecular testing. The advantage of the method of the present invention is that it is possible to directly identify a large amount of differential methylated CpG islands associated with the abnormal state of the human body in the cell free DNA sample, and thus the results can be directly applied in clinical testing.

Tumor is one of the important applications of the present invention. According to one example of the present invention, methylation level of 132 CpG islands were identified to be significantly differentiated between hepatocellular patients and non-tumor individuals by analysis of the cell free DNA of hepatocellular patients and non-tumor individuals. The sequences of 132 CpG are as below (1∼132). Aberrantly methylated CpG islands suggest that these individuals suffer from hepatocellular carcinoma. 132 aberrantly methylated CpG islands can be divided into two groups: the first is type I markers (68, no.1∼68), the second is type II markers (64, no. 69∼132). The type I markers are differential methylated between patients and normal people in both tissue and plasma, however, type II markers are only differential methylated in plasma.

The correlation between some of the type I markers and hepatocellular carcinoma has been reported (Shen, et al., 2012, Hepatology; Ammerpohl, et al., 2012, Int J Cancer; Song, et al, 2013, PLoS One). However, the existing method cannot tell which of them also showed differential methylation between hepatocellular carcinoma patients and normal individuals in cell-free DNA samples. The type I CpG islands markers disclosed by the present invention correlates the cell-free DNA methylation detection and whether the individual is suffering from hepatocellular carcinoma directly.

The relationship between differential methylated CpG islands (type II markers) in plasma and hepatocellular carcinoma was not previously revealed, since the difference in methylation level of type II markers between hepatocellular carcinoma patients and non-cancerous individual lie only in plasma instead of tissues. This is because that Type II markers are hypermethylated in normal liver tissues, but hypomethylated in white blood cells as well as in cell-free DNA from plasma of healthy individuals. These hypermethylated loci appear to be contained in liver cells under normal circumstances but are released into the blood when malignance occurs together with the necrosis and apoptosis of tumor cells, thus these loci in the cell-free plasma sample are detected as hypermethylated ones. Besides HCC, it could be possible to detect hypermethylated type II markers in cell-free DNA under other circumstances of liver damage. Therefore, the result that hypermethylation of the type II differentially methylated CpG islands in cell-free DNA from plasma disclosed in the present invention may imply that the individual in the testing is likely to suffer from HCC or other aberrant condition of liver damage.

A group of CpG islands (no. 133∼187) with low methylation level in noncancerous individuals are included in this invention. This group of CpG islands (belong to tissue differential methylation CpG island), although are not differentially methylated between plasmas from hepatocellular carcinoma patients and noncancerous individuals, they are aberrantly hypermethylated in tumor tissue with certain frequency. It has a low methylation background (uMePM < 0.1 in non-cancerous individuals) and high methylation detection efficiency (mean of MePM in FMG > 75), making it widely used to detect early neoplastic diseases.

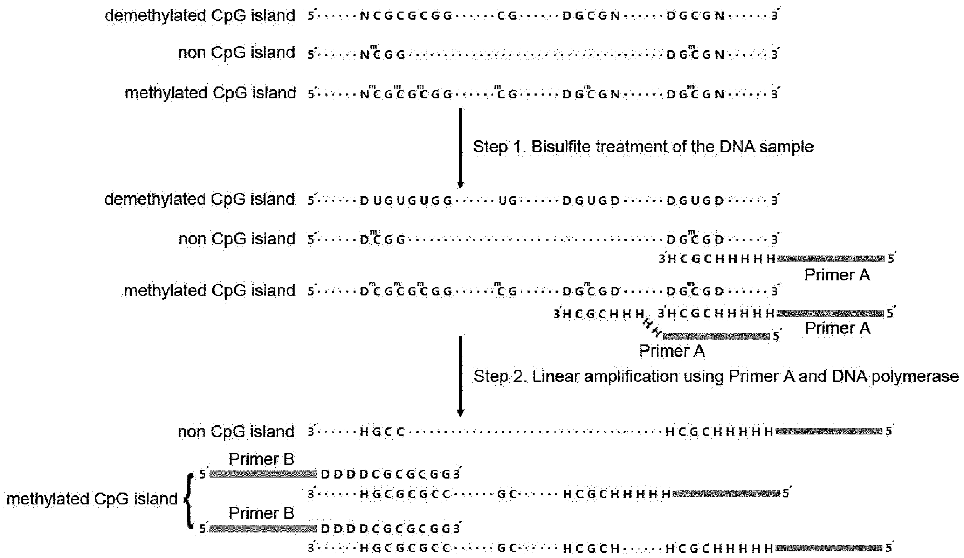

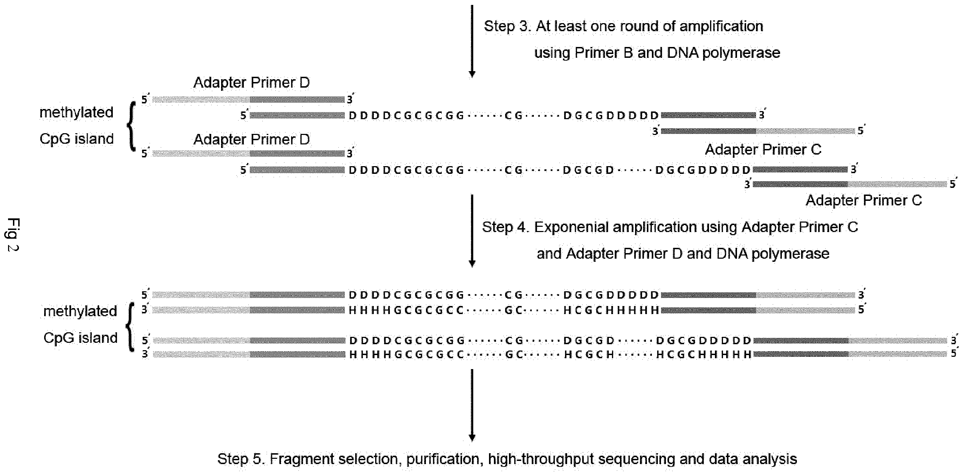

Secondly, this invention provides a method for detecting the level of methylation of the short CpG tandem nucleic acid sequence on a genomic scale, which is Methylated CpG Tandems Amplification and Sequencing (MCTA-Seq). As shown in Figure 2, it includes the following steps:
Step one, treating a DNA sample with a modifying agent to form the modified DNA wherein cytosine bases instead of 5'-methyl-cytosine bases of the DNA sample are modified to uracil bases.

The agent for treating the DNA sample modifies cytosine bases but not 5'-methyl-cytosine bases followed by the formation of single-stranded DNA. The modifying agent can be selected from bisulfite, acetate or citrate. Preferably, the agent is bisulfite. Optionally, bisulfite treatment of the DNA sample can be achieved by using commercial kits such as MethylCode Bisulfite Conversion Kit (Invitrogen), EZ DNA methylation-Gold Kit (ZYMO) or EpiTect Bisulfite Kit (Qiagen).

Step two, providing Primer A and DNA polymerase to the modified DNA to allow for at least one round of linear amplification to form the semi-amplicon capable of anchoring Adapter Primer C at one end.

The primer A is composed of two portions: a 3' end and a 5' end. The 3' end is used for binding and amplifying the converted DNA fragments, which is characteristic for a range of random sequences capable of binding the converted DNA fragments. Preferably, the 3' end contains only C, A and T except the CpG dinucleotide. Since the majority of C are converted to U after treatment of modifying agent, this design may improve the binding efficiency. Preferably, the first 7 nucleotides at 3' end contains at least 1 nucleotide pair. On one hand, due to the conversion of CpG in non-methylated CpG island to UpG by the modifying agent, this design allows preliminary enrichment of the methylated CpG islands. On the other hand, CpG may increase the melting temperature of primers and improve the amplifying efficiency. More preferably, the second nucleotide at the 3' terminal is C. On one hand this may increase the melting temperature of primers and improve the amplifying efficiency. On the other hand since the 3' end of the primers contains only C, A and T, except CpG, the the C as the second nucleotide at the 3' terminal may limit the formation of dimers. Preferably, the portion between the 3' and 5' end of the primer A contains a unique molecular identifier. This sequence add a tag to each amplicon before the exponential amplification in step four, allowing for the identifying and reducing the product of PCR over-amplification. Preferably, the sequence of the unique molecular identifier is HHHHH (H = A, T or C), identifying 243 different molucules.

The 5' end of primer A is used to anchor Adapter Primer C; it allows Adapter Primer C to bind to its reverse complementary sequence for PCR amplification. The word "anchor" describes the function of the 5' end to join Adapter Primer C to the amplicon *via* PCR.

The DNA polymerase can be any suitable polymerase, such as Taq polymerase, ExTaq polymerase, LATaq polymerase, AmpliTaq polymerase, Amplitaq Gold polymerase, Titanium Taq polymerase, KlenTaq polymerase, Platinum Taq polymerase, Accuprime Taq polymerase, Pyrobest DNA polymerase, Pfu polymerase, Pfu polymerase turbo, Phusion polymerase, Pwo polymerase, Vent polymerase, Vent Exo- polymerase, Sequenase TM polymerase, 9° Nm DNA polymerase, Therminator DNA polymerase, Expand DNA polymerase, rTth DNA polymerase, DyNazymeTM EXT polymerase, DNA polymerase I, T7 DNA polymerase, T4 DNA polymerase, Bst DNA polymerase, phi-29 DNA polymerase, and Klenow fragment.

Preferably, the DNA polymerase is capable of strand displacement. Since one single DNA template may bind several primer A, especially in CpG island regions with high CpG density, the DNA polymerase capable of strand displacement may enable the primer at 5' end to extend, resulting in linear amplification even with one-time amplification. It can be any suitable polymerase with the strand displacement activity, including but not restricted to DNA polymerase I (Klenow) large fragment (New England Biolabs (NEB) Cat. No.: M0210S), Klenow fragment (exo-) (NEB Cat. No.: M0212S), Bst DNA polymerase large fragment (NEB Cat. No.: M0275S), Vent(exo-) (NEB Cat. No.: M0257S), Deep Vent (exo-) (NEB Cat. No.: M0259S), M-MulV reverse transcriptase (NEB Cat. No.: M0253S), 9.Nm DNA polymerase (NEB Cat. No.: M0260S) and Phi-29 DNA polymerase (NEB Cat. No.: M0269S). Preferably, the DNA polymerase is deficient of exonuclease activity. In one preferred embodiment, the DNA polymerase is Klenow fragment (exo-).

Linear amplification refers to that the amount of amplified products increase in a linear instead of an exponential relationship to the amplification times. As mentioned above, the DNA polymerase capable of strand displacement may enable linear amplification even with one-time amplification. 2∼30 rounds of linear amplification are alternative. Many DNA polymerase with activity of chain replacement such as Klenow fragment are inactivated during DNA denaturation and need to be re-added.

Step three, amplifying the semi-amplicon using Primer B and DNA polymerase to form the full-amplicon enriched with methylated CpG islands and capable of anchoring Adapter Primer C and D separately at both ends;
Primer B is composed of a 3' end and a 5' end. The 3' end allows for selective amplification of the methylated CpG tandem sequences, which is the stated Short CpG tandem sequences. Technical personnel in this field knows that the CpG or CpG tandem sequence located at the 3 'end of the primer is easy to form a primer dimer. In order to avoid the formation of primer dimers, the first nucleic acid at the 3 'end of the short CpG tandem nucleic acid sequence primer is designed as G, A or T. Preferably, the 3 'and 5' end portions contain a random sequence of 2 to 10 nucleotides in length. Preferably, the 3' portion of Primer B contains only G, A and T. Random sequence can help increase the melting temperature and it only needs to contain G, A, T, for most of C has been converted to U after treated with modifying agent and in the complementary strand, most G have been converted into A. Take CGCGCGG as an example, its melting temperature increase from 43 °C to 53 °C (AAACGCGCGG or TTTCGCGCGG) or 66 °C (GGGCGCGCGG) when adding 4 D (D=G/A/T) at 5' end of primer, thus it is able to effectively amplify at conventional annealing temperatures. The 5' end of Primer B is used to anchor Primer D. It allows Adapter Primer D to bind to its reverse complementary sequence for PCR amplification.

The polymerase can be any suitable polymerase mentioned above. Preferably, the polymerase is hot-start. Technical personnel in this field can understand that hot-start DNA polymerase increases the specificity of PCR reaction. Especially, it prevents the formation of dimers in primers with the short CpG tandems before amplification.

Step four, amplifying the full-amplicon exponentially using Adapter Primer C, Adapter Primer D and DNA polymerase to form the final-amplicon via PCR.

The word "Adapter Primer" herein refers to that the function of the primer is similar to the "adapter" used in conventional methods for constructing the high-throughput sequencing library (such as Illumnia's TruSeq DNA Sample Prep Kit and Applied Biosystems (ABI)'s The SOLiD™ Fragment Library Construction Kit), which allows for binding of the DNA fragments to the flow cell for subsequent amplification and sequencing. Different from the usage of ligation reaction to add "adapter" in conventional methods for establishing high-throughput sequencing library, the present invention adds "adapters" to each end of the amplicons by means of PCR reaction *via* the anchor sequences at the 5' end of Primers A and B. Technical personnel in this field can understand that either Primer C or Primer D can contain "barcode" sequences, which facilitate to simultaneously sequence multiple libraries in one flow cell. The Primer C and D correspond to the adapter sequences of a given high-throughput sequencing platform, which includes, but not limited to, the Illumina's Genome Analyzer IIx, HiSeq and MiSeq platforms, ABI's SoLiD, 5500 W Series Genetic Analyzer, Ion Torrent PGM platforms, Roche454's GS Junior and GS FLX+ platforms.

Step five, separating and purifying the final-amplicon to form the library for high-throughput sequencing, then sequencing the library and analyzing the data.

The approach to separate and purify the final amplicons can be any suitable method, including but not restricted to magnetic beads-based, column-based and gel electrophoresis-based purification. Preferably, the purification method is able to achieve size-selection for the amplicons. Preferably, the amplicons are separated using 3%-4% agarose gel electrophoresis and the fragments between 160 and 250 bp are excised and then purified.

The high-throughput sequencing platform for analysis includes, but not limited to, the Illumina's Genome Analyzer IIx, HiSeq and MiSeq platforms, ABI's SoLiD, 5500 W Series Genetic Analyzer, Ion Torrent PGM platforms, Roche454's GS Junior and GS FLX+ platforms.

The method for data analysis is not limited and can be any suitable software for data analysis and sequence alignment, which includes, but not limited to Bismark, BSMAP, Bowtie, SOAP and R packages.

Thirdly, the present invention provides a kit for detecting methylated CpG islands using high-throughput sequencing. The kit comprises a set of primers including the Primer A, Primer B, Adapter Primer C, Adapter Primer D, and the DNA polymerases, as well as instructions for the kit.

Fourthly, the present invention provides a method for detecting hepatocellular carcinoma including the following steps: 1) The DNA sample can derive from human cells, tissues, blood, body fluid, urine, saliva, excrement or their combination. Preferably, the DNA sample is the cell-free DNA of human plasma or urine. 2) Nucleic acid sequence no. 1∼68 is the type I markers hypermethylated in the plasma of hepatocellular carcinoma and nucleic acid no. 69∼132 is the type II markers hypermethylated in the plasma of hepatocellular carcinoma. If aberrant methylation of type I marker or type II marker or both of them is detected in the plasma of an individual, it indicates that the individual may have hepatocellular carcinoma. The methods for detecting methylation level include, but are not limited to MCTA-Seq, methylation-specific PCR, restriction enzyme digestion and PCR, pyrosequencing, Methylight and MethylBeaming et.al.

### Brief Description of the drawings

FIG 1. The number of various CpG tandems in human genome and the proportion in CpG islands were shown.
FIG 2. Schematic of the MCTA-Seq for detecting methylated CpG islands based on the high-throughput sequencing. The underscore part of primer A indicates the unique molecular recognition tag. ①, ② and ③ indicate different barcode sequences. Note that different molecules have different amplification sites.

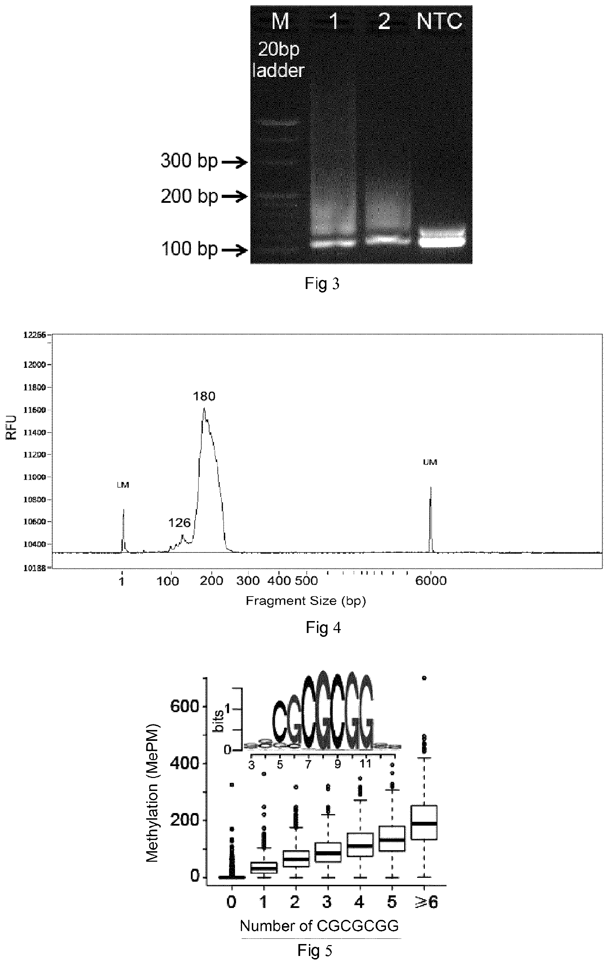

FIG 3. Gel results of MCTA-Seq. The MCTA-Seq results of the genomic DNA extracted from WBCs (lane1) and the DNA sonicated to an average size of 200 bp (lane 2) were shown. M:marker, 20bp ladder, TAKARA. bp: base pair. NTC: no template control.
FIG 4. Fragment Analyzer result of the sequencing library constructed by MCTA-Seq.
FIG 5. Correlation between short CpG tandem and MCTA-Seq sequencing results. X axis indicates CpG island with different number of CGCGCGG tandems, Y axis indicate the methylation detection values of human fully methylated genome (MePM: methylated alleles per million mapped reads, refers to average methylated alleles per million mapped reads). The sequence logos of the starting 3^{rd}∼13^{th} nucleotide of read2 are shown (black box indicates the CGCGCGG sequence, the 5^{th}∼11^{th} nucleotide). Bits: degree of enrichment of the sequence.

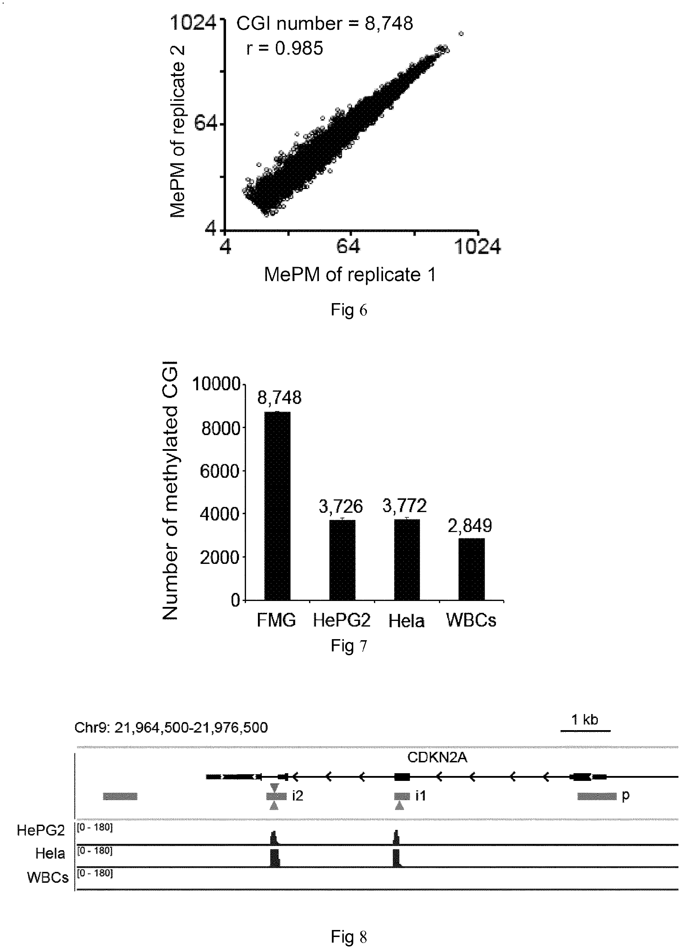

FIG 6. The throughput and reproducibility of MCTA-Seq. The MCTA-seq result of two technical duplicates (X axis and Y axis) of fully methylated human genome standard sample is shown in the figure. The total number of CGIs detected in FMGs (average MePM> 8 in two replicates) and Pearson correlation coefficient (r) between two replicates are shown.
FIG 7. The number of CpG islands detected in different kind of samples. FMG: fully methylated human genomic DNA; HePG2: human liver cancer cell line; Hela: Human cervical cancer cell line; WBC: human white blood cells. The error bars represent means ± s.d
FIG 8. MCTA-seq result of CDKN2A (CYCLIN-DEPENDENT KINASE INHIBITOR 2A) gene region. The genomic region of the CDKN2A gene contains 4 CpG islands, including one promoter CGI (p), two intragenic CGIs (i1 and i2) and CGCGCGG tandems showed with triangles. The intragenic CGI i2 contains two CGCGCGG tandems (triangles above and below the rectangle indicate the CGCGCGG tandems positioned at the forward and the reverse strands, respectively). The scale is MePM, indicating all MCTA-seq reads of HePG2, Hela and WBC.

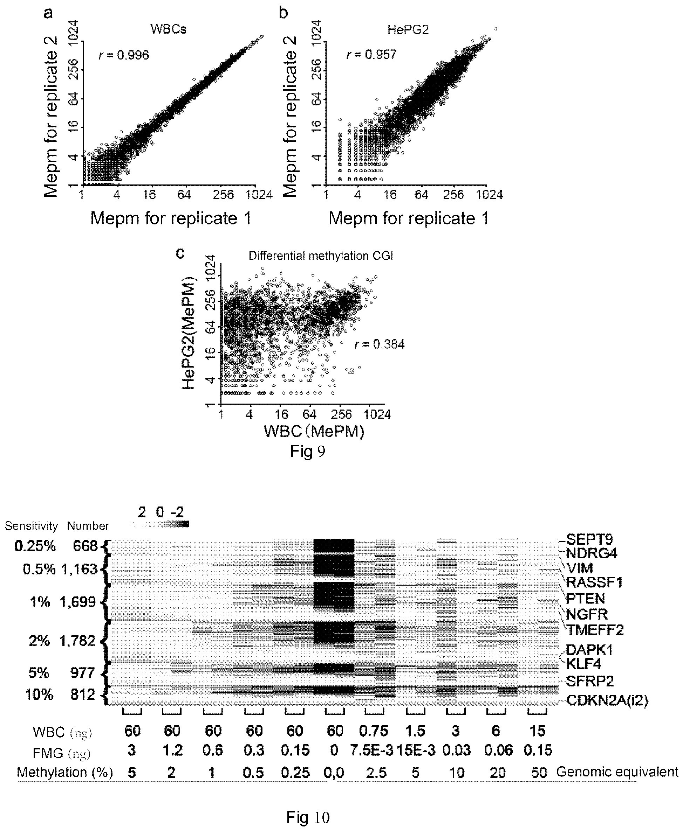

FIG 9. Reproducibility of MCTA-Seq and differential methylation detection. Comparison of methylation profiles between technical duplicates of WBCs (a) and HePG2 cells (b) shows high reproducibility of MCTA-Seq. (c) Comparison of methylation profiles between WBCs and HePG2 cells shows differential methylation between two cell types, mainly the hypermethylation sites in HePG2.
FIG 10. Heatmap showing the sensitivity of MCTA-Seq. The figure below shows different amounts of FMG diluted into the WBC. Each experiment has two replicates. Right of the figure shows the promoter or intragenic CGI (CDKN2A(i2)) In the heat map, black color indicates low and white indicates high DNA methylation values [Log2(MePM)].

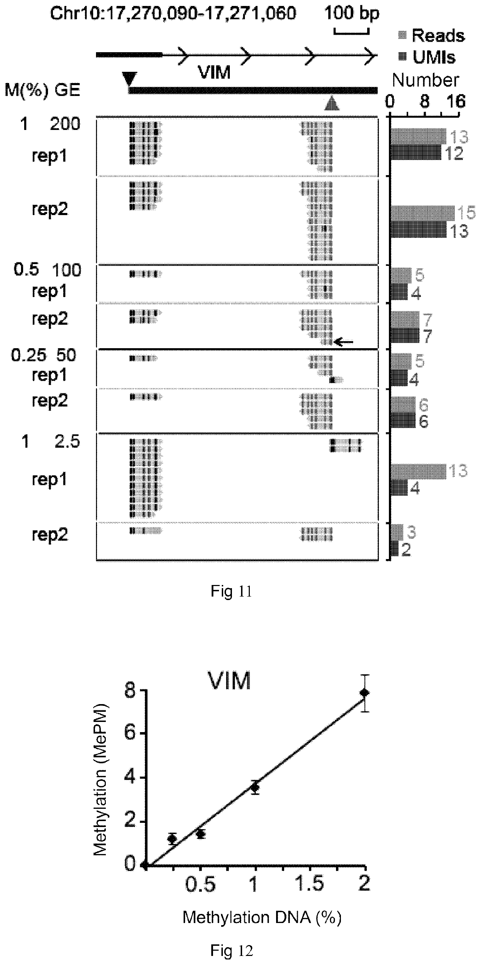

FIG 11. MCTA-seq result of VIM (VIMENTIN) gene region. The grey box indicates the promoter CGI of the VIM (no.2). The triangles above and below indicate two CGCGCGG sequences positioned at the forward and the reverse strand, respectively. M(%): the frequency of methylation, GE: the haploid genomic equivalent. Rep1: duplicate 1; Rep2: duplicate 2; Reads: sequencing reads; UMIs: unique molecular identifier. The arrow indicates the shortest amplicon (30 bp).
FIG 12. The MePM scores of the promoter CGI of VIM were plotted against the varying percentage of FMG to WBC; a linear fit was observed. The error bars represent means ±s.d.

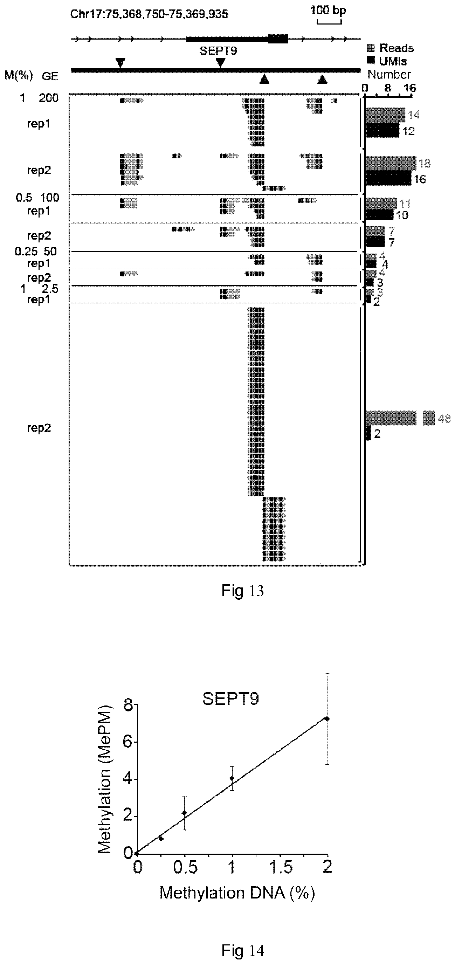

FIG 13. MCTA-seq result of SEPT9 (SEPTIN9) gene region. The grey box indicates the promoter CGI of the SEPT9 gene. The triangles above and below indicate two CGCGCGG sequences positioned at the forward and the reverse strand, respectively. M(%): frequency of methylation; GE: genomic equivalent; rep1: duplicate 1; rep2: duplicate 2; Reads: sequencing reads; UMIs: unique molecular identifier.
FIG 14. The MePM scores of the promoter CGI of SEPT9 were plotted against the varying percentage of FMG to WBC; a linear fit was observed. The error bars represent means ±s.d.

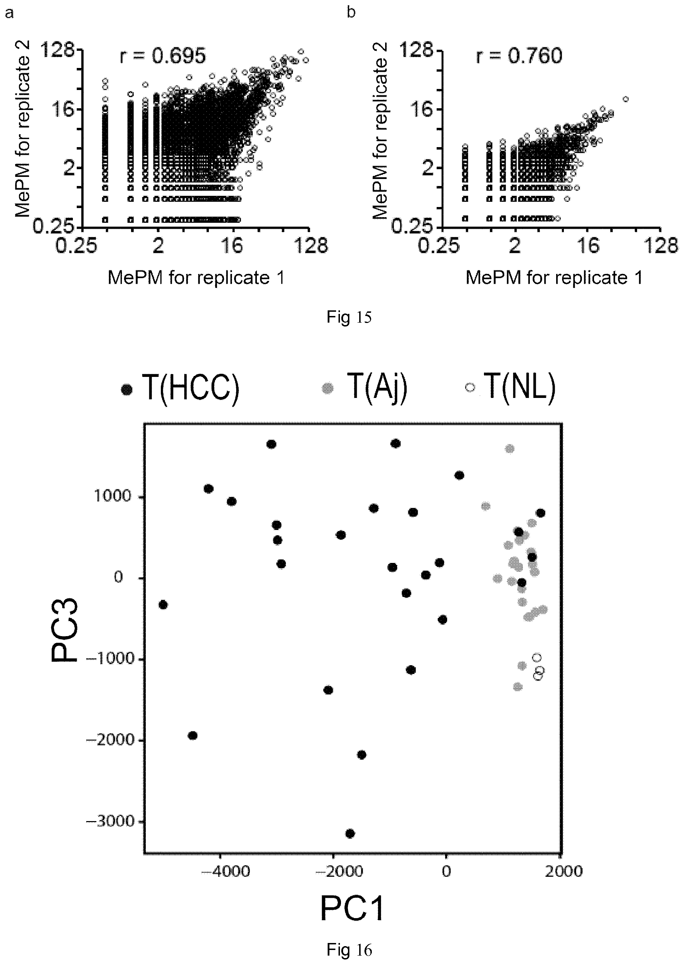

FIG 15. Unique molecular identifiers reduce amplification bias. Scatterplots showing the replicates of the 7.5 pg experiments using read counts (MePM, a) and UMI-adjusted counts (uMePM, b). uMePM: unique methylated alleles per million mapped reads.
FIG 16. Principal component analysis of HCC tissues.

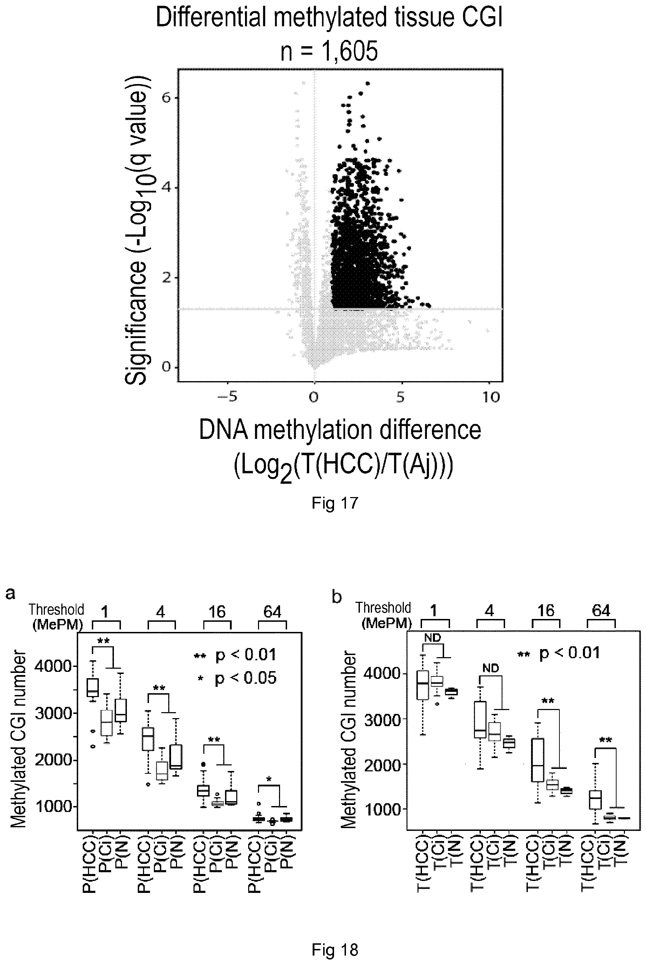

FIG 17. Differentially methylated CGIs in tissue samples. Volcano plot for differentially methylated CGIs between the HCCs and the adjacent noncancerous liver tissues. The x axis shows the fold changes of the average methylation value between the HCCs (n=27) and the adjacent tissues (n=27), and the y axis shows the q-value as the FDR analogue of the P value (-Log(q-value)) for a two-tailed t-test of differences between two groups. A total of 1,605 CGIs (tdmCGIs) were differentially hypermethylated in HCC tissues (q<0.05, HCC vs adjacent tissues > 2).
FIG 18. Number of methylated CpG islands. Boxplot (left) show the number of CpG islands under different threshold in HCC plasma (P(HCC), n=17), cirrhosis plasma (P(Ci), n=11) and healthy individual plasma (P(N), n=9). And the boxplot (right) show the number of CpG islands under different threshold in HCC tissues (T(HCC), n=27), adjacent tissues (T(Aj), n=27) and normal liver tissues (T(NL), 3). Two-tailed MWW.

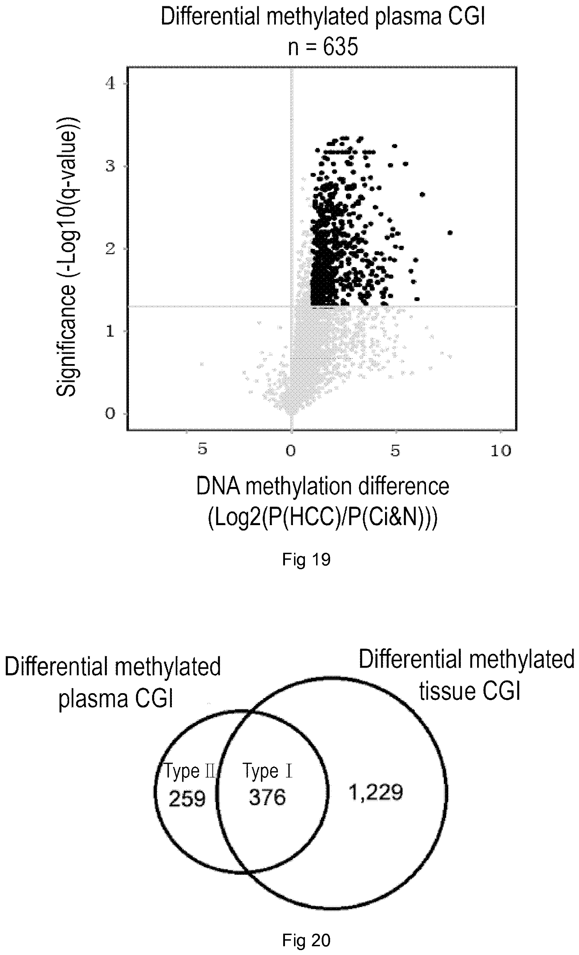

FIG 19. Differential methylated CpG islands in plasma. Volcano plot showing CGIs in plasma that are differentially methylated between the HCC patients with small tumors (n = 13, the largest tumor diameter < 5 cm) and cancer-free individuals (n = 20, including cirrhosis patients and healthy controls). The x axis shows the fold changes of the average methylation between the HCC patients and the cancer-free individuals, and the y axis shows the q-value as the FDR analogue of the P value (-Log10(q-value)) for a two-tailed t-test of differences between two groups. A total of 635 differentially methylated plasma CGIs are identified (HCC patients vs cancer-free individuals > 2, q<0.05).
FIG 20. Venn plot view comparing plasma differentiated CGIs and tissue differentiated CGIs. Type I markers (376) are CpG islands that show differential methylated level between cancer patients and cancer-free individuals both in plasma and tissues. Type II markers (259) are CpG islands that show differentially methylated level only in plasma.

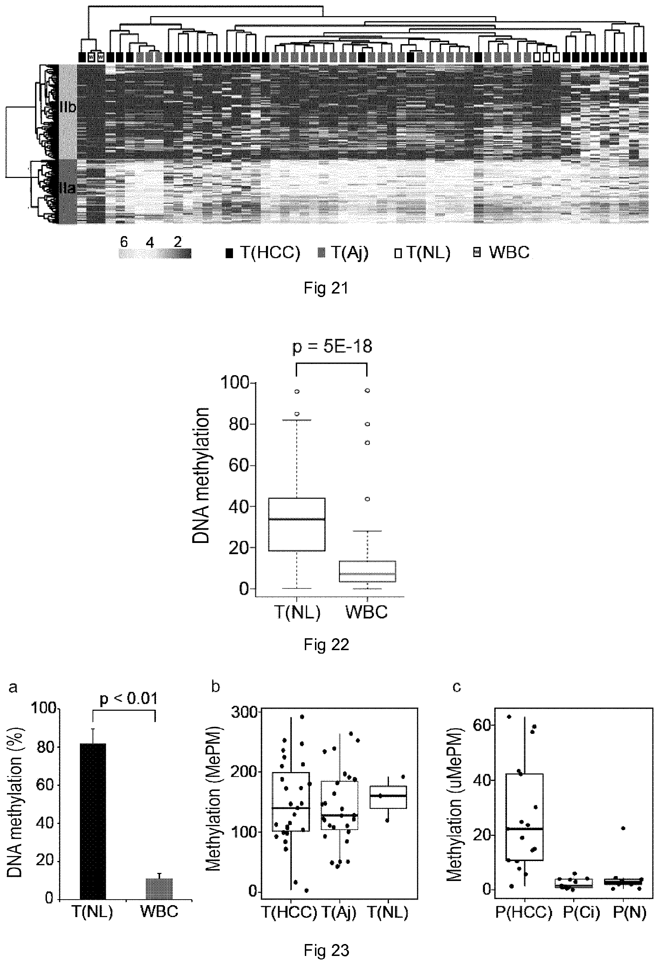

FIG 21. Classification of type II markers. The clustering shows that 102 CpG islands, called type IIa markers, show high methylation level both in adjacent tumor tissues and normal liver tissues but low methylation level in WBC. The rest of the type II markers are type IIb markers. White color indicates high methylation level, black color indicates low methylation level. Due to color limitation only part of the CpG islands are shown.
FIG 22. Differential methylaion level of type IIa markers. Results of RRBS show that 94 IIa markers could be detected by RRBS and they show hypermethylation in normal tissues (T(NL)) and hypomethylation in WBC. Two-tailed MWW.
FIG 23. Results of CpG island in SHANK2 (i2). Take the second CpG islands in SHANK2 gene (SHANK2 (i2), no.79), a typical type IIa marker, as an example. (a) Results of RRBS, T (NL): normal liver tissues, two-tailed t-test, the error bars represent means ± s.d. (b) Results of MCTA-Seq, T(HCC): tumor tissue from hepatocellular patients; T(Aj): tumor adjacent tissues. (c) Results of MCTA-Seq in plasma samples, P(HCC): plasma from hepatocellular patients; P(Ci): plasma from cirrhosis patients; P(N): plasma from healthy individuals.

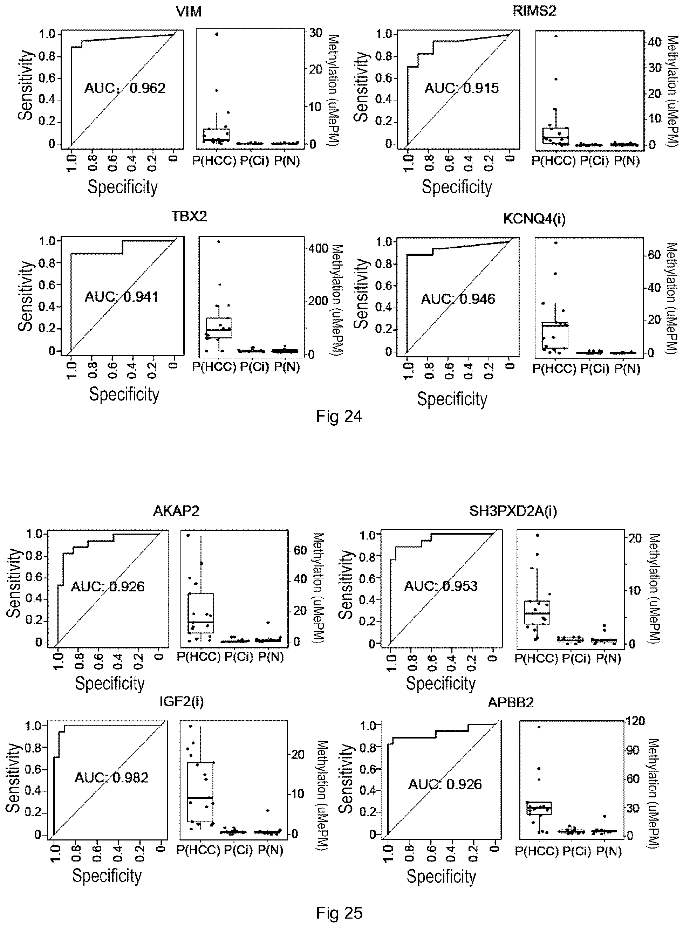

FIG 24. Example of high-performance type I markers. Boxplots and roc curve show four representative type I markers out of 68 type I markers (AUC > 0.9) including CpG island located in the promoter of VIM (no. 2), CpG island located in the promoter of RIMS (no. 64), CpG island located in the promoter of TBX2 (no. 25), CpG island located in the intragenic region of KCNQ4 (no.38).
FIG 25. Example of high-performance type II markers. Boxplots and roc curve show four representative type II markers out of 64 type II markers (AUC > 0.9) including CpG island located in the promoter of APAK2 (no.130), CpG island located in the SH3PXD2A (no. 70), CpG island in the IGF2 (no. 75) and CpG island located in the promoter of APBB2 (no. 116).

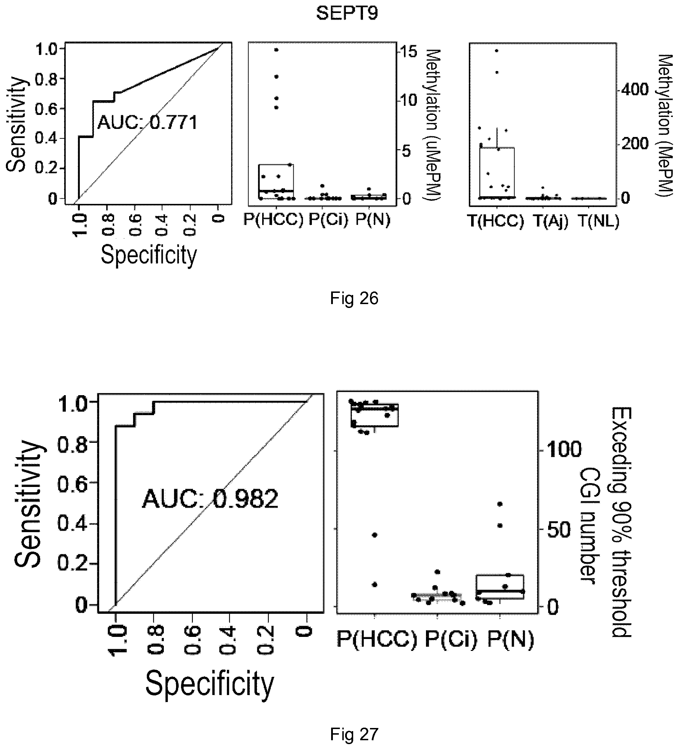

FIG 26. Roc curve of CpG island in SEPT9 promoter and boxplot showing the methylation value in plasma and tissue samples.
FIG 27. Cancer detection by combination of 132 high-performance CpG islands. Boxplot showing the number of positive CpG islands with MePM exceeding the 90% percentile of the normal people. Roc curve shows the specificity and sensitivity of the detection method.

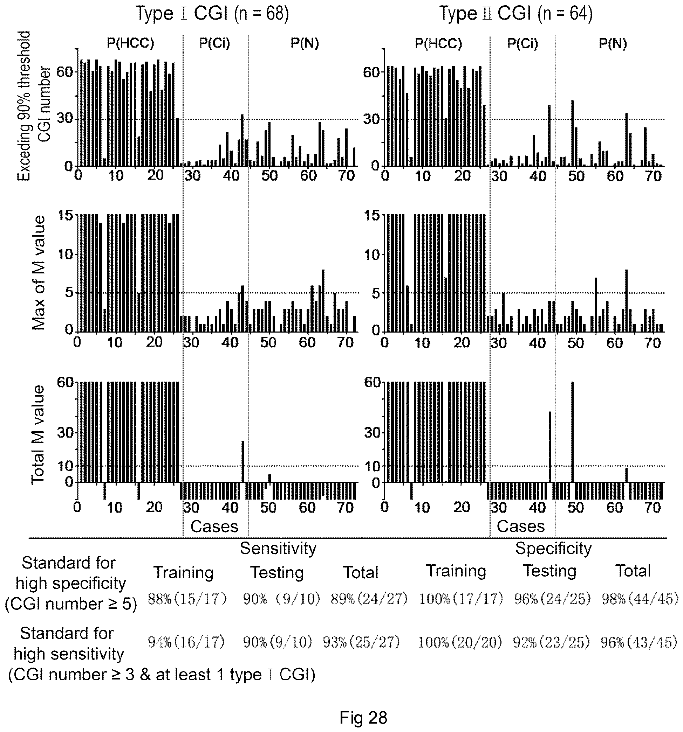

FIG 28. Multiple sites detection of hepatocellular carcinoma. There are 3 indicators: 1) the number of loci passing the 90% specificity threshold (upper panel), 2) the uppermost number of standard deviations (SDs) above the 90% specificity threshold (refer to Top M-score, middle panel), and 3) the total number of SDs above the 90% specificity threshold (refer to Total M-score, bottom panel),. Those indicators were detected in 68 type I markers (left) and 64 type II markers (right). A threshold was set (dashed lines for each indicators which could distinguish hepatocellular carcinoma patients and cancer-free individuals. "Positive" was defined as methylation level exceeding threshold. To get high specificity, 5 or 6 of the 6 indicators are positive. To get high sensitivity, 2 or 3 of the 6 indicators are positive, in which as least one type I is positive. P(HCC): plasma of hepatocellular carcinoma patients, case 1∼17 are training group, sorted according to tumor size: 1(P50), 2(P29), 3(P41), 4(P3), 5(P37), 6(P57), 7(P62), 8(P77), 9(P10), 10(P39), 11(P60), 12(P36), 13(P44), 14(P59), 15(P74), 16(P58), 17(P51); case 18∼27 are testing group, sorted according to tumor size: 18(P92), 19(P119), 20(P109), 21(P101), 22(P114), 23(P85), 24(P115), 25(P80), 26(P81), 27(P113). P(ci): plasma of cirrhosis patients, cases 28∼38 are training group, cases 39∼44 are testing group. P(N): plasma of healthy individuals, cases 45∼53 are training group, 54∼72 are testing group.

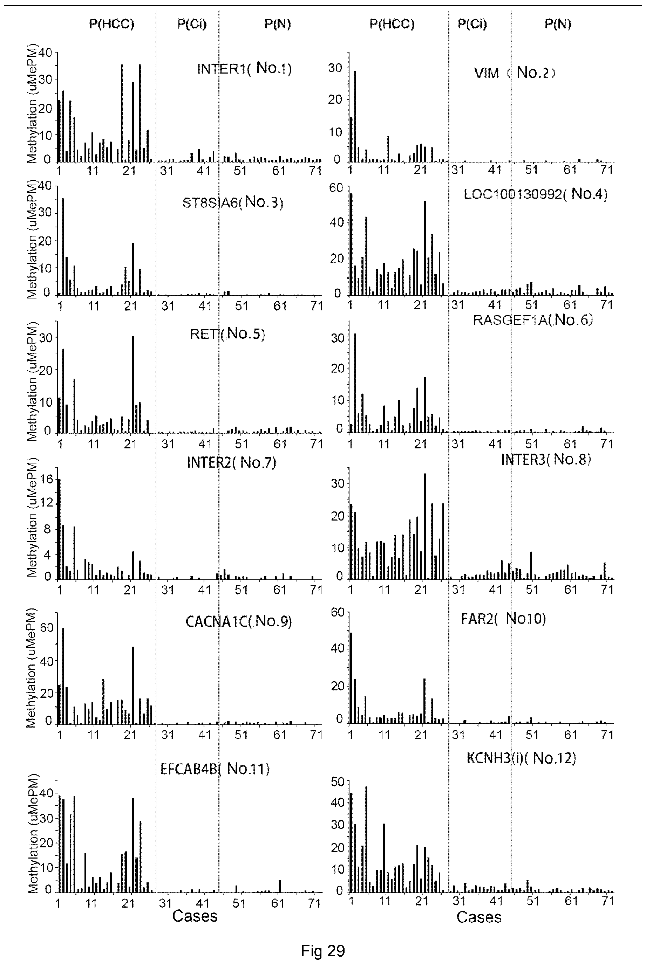

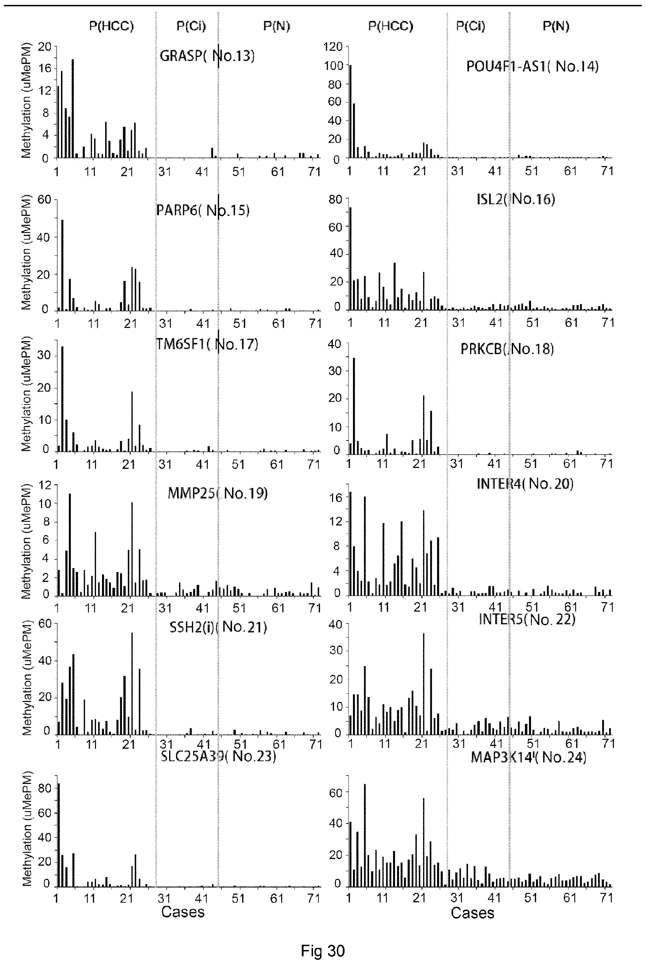

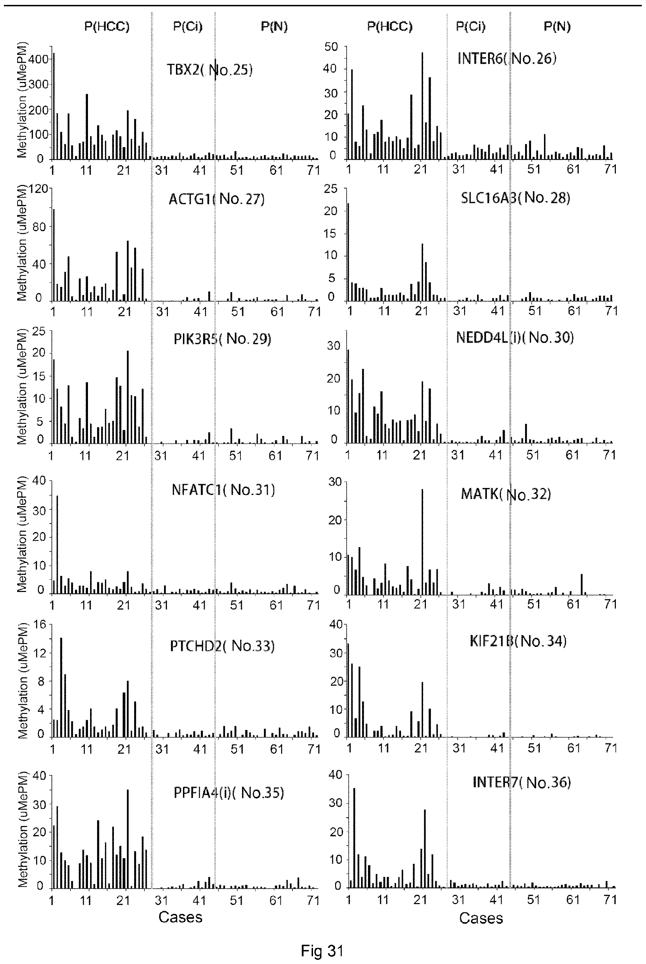

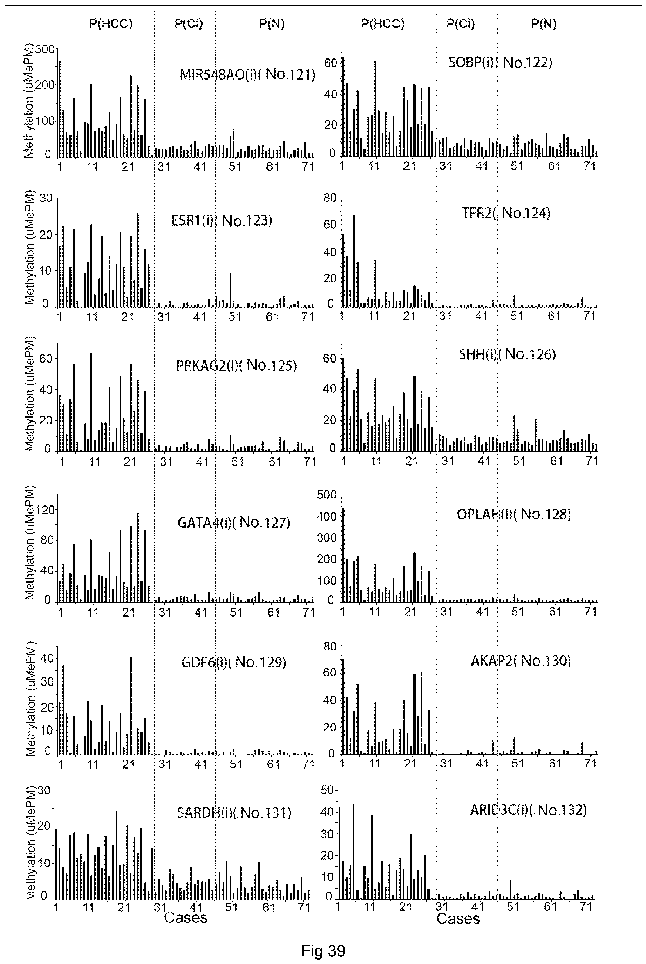

FIG 29∼FIG 39 MCTA-Seq results of 132 high performance type I and type II CpG island markers in 72 studied plasma cases. Ranking of study objects is the same as in FIG 28.

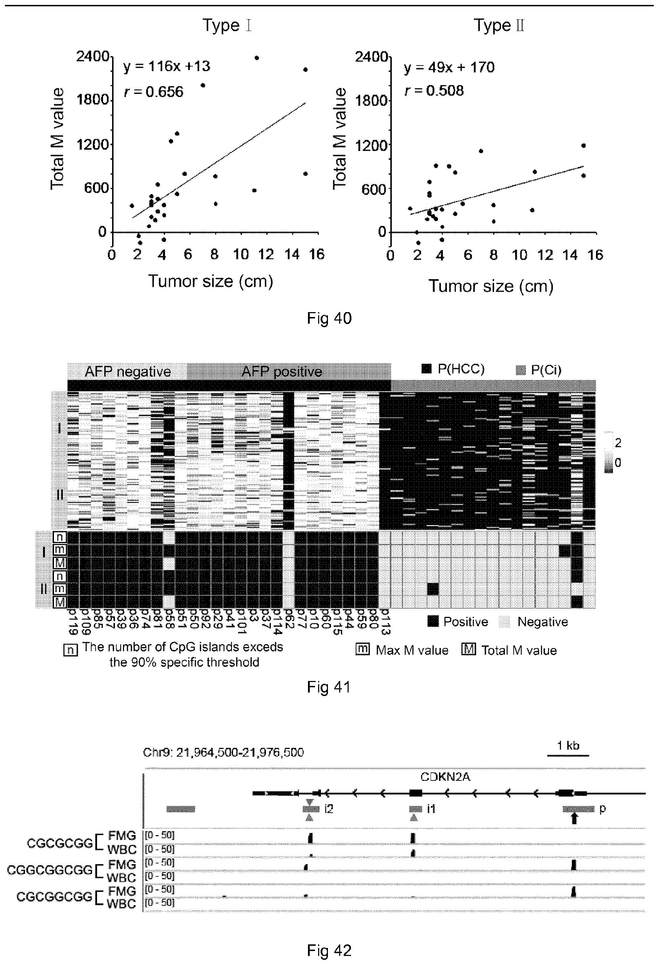

FIG 40 Total M score has a linear relationship with the tumor size. Total M score of type I (left) and type II (right) markers have linear relationships with maximum diameter of tumor (formula and correlation were shown).
FIG 41 Comparing the performance of ccfDNA methylation classifiers and AFP. Heatmap shows MCTA-Seq results from plasma of hepatocellular carcinoma patients (n=27) and cirrhosis patients (n=17). The heatmap above shows M score of 68 type I markers and 64 type II markers. The heatmap below shows 3 indicators of type I and type II markers.
FIG 42. The genomic region of the CDKN2A could be detected by different short CpG tandems. p: CpG island in promoter of CDKN2A; i1 and i2: CpG island in CDKN2A; triangle: CGCGCGG short tandem sequence; arrow: CGGCGGCGG short tandem sequence.

### Detailed Description Of Implementions

In order that the present invention may be more clearly understood, preferred forms will described with reference to the following examples and drawings. It will be appreciated by persons skilled in the art that numerous variations and/or modification may be made to the invention as shown in the specific embodiments without deportioning from the spirit or scope of the invention as broadly described.

### Example 1: The methylated CpG tandems amplification and sequencing (MCTA-Seq) according to the present invention operates in the following manners (FIG 2).

1. Bisulfite conversion of the DNA sample
The bisulfite conversion is performed by using the MethylCode™ Bisulfite Conversion Kit (Invitrogen) according to the protocol provided by the manufacturer. Detailed steps are as follows:
1.1 Preparing the CT conversion reagent: Add 900 µl of sterile distilled water, 50 µl of resuspension buffer, and 300 µl of dilution buffer directly to one tube of CT conversion reagent; mix by shaking or intermittent brief vortexing for 10 minutes for dissolvation; keep protected from light at room temperature;
1.2 Add 20 µl of the DNA sample ranging from 500 pg to 500 ng to a PCR tube;
1.3 Add 130 µl of CT conversion reagent to the DNA sample, and mix by flicking the tube or pipetting up and down;
1.4 Place the tube in a thermal cycler and run the following program: 98°C for 10 minutes, 64°C for 2.5 hours and 4°C storage for no more than 20 hours;
1.5 Place a spin column in a collection tube and add 600 µl of binding buffer to the column.
1.6 Add the sample from step 1.4 to the binding buffer in the column; close the cap and mix by inverting several times.
1.7 Centrifuge at full speed (≥ 10,000 × g) for 30 seconds, then discard the flow-through;
1.8 Add 100 µl of wash buffer prepared with ethanol to the column, and centrifuge at full speed for 30 seconds; discard the flow-through;
1.9 Add 200 µl of desulphonation buffer to the column and let the column stand at room temperature for 15-20 minutes;
1.10 Centrifuge at full speed for 30 seconds; discard the flow-through;
1.11 Add 100 µl of wash buffer with ethanol to the column and centrifuge at full speed for 30 seconds; discard the flow-through;
1.12 Repeat the wash in step 1.11 one more time, then transfer the spin column to a new, clean 1.5-ml microcentrifuge tube;
1.13 Add 10 µl of elution buffer directly to the column matrix; centrifuge at full speed for 30 seconds to elute the DNA.

2. Lineal amplification by means of Primer A and DNA polymerase.
2.1 Prepare the following reaction mixture in a PCR tube for the DNA sample obtained from step 1.13:

| Contents | Volume |
|---|---|
| The DNA sample (from step 1.13) and water | 10.5 µl |
| NEBuffer 2 | 1.5 µl |
| dNTP (2.5 mM) | 1.5 µl |
| Primer A (5 uM)* | 1 µl |
| Klenow (exo-) ** | 0.5 µl |
| Total | 15 µl |

| | |
|---|---|
| *: Primer A: An equimolar mixture of the following four primers. The total concentration is 5 µM: 5' -TTTCCCTACACGACGCTCTTCCGATCTHHHHHHHHCGCH-3' ; 5'-TTTCCCTACACGACGCTCTTCCGATCTHHHHHHHCGHCH-3' 5'-TTTCCCTACACGACGCTCTTCCGATCTHHHHHHCGHHCH-3', 5'-TTTCCCTACACGACGCTCTTCCGATCTHHHHHCGHHHCH-3'; (H = A/T/C), the 3' portion of Primer A randem sequence is underlined by a wave line, the 5' portion of anchor sequence is underlined by a straight line, and the unique molecular identifier is underlined by a double line. **: add at step 2.3 | |

2.2 Place the tube in a thermal cycler and run the following program: 95°C for 2 minutes and 4°C for pause;
2.3 Centrifuge at 13000 rpm for a minute in 4°C ;
2.4 Add 0.5 µl of Klenow Fragment (exo-) (NEB catalog: M0212S), mix and briefly centrifuge;
2.5 Centrifuge at 13000 rpm for a minute in 4°C ;
2.6 Place the tube in a thermal cycler and run the following program: 4°C for 50 seconds, 10°C for a minute, 20°C for 4 minutes, 30°C for 4 minutes, 37°C for 4 minutes;
2.7 Run the following program to inactivate the Klenow Fragment: 75°C for 20 minutes and 4°C for pause;
2.8 Centrifuge at 13000 rpm for a minute in 4°C ;
3. Amplification by means of Primer B and DNA polymerase.
3.1 Prepare the following reaction mixture in a new PCR tube:

| Contents | Volume |
|---|---|
| Ex Taq Buffer | 0.5 µl |
| Primer B (10 uM) * | 0.5 µl |
| HS Ex Taq (Takara catalog: DRR006B) | 0.3 µl |
| Water | 3.7 µl |
| Total | 5 µl |

| | |
|---|---|
| *: Primer B: (5'- GTGACTGGAGTTCAGACGTGTGCTCTTCCGATCTDDDDCGCGCGG-3', (D=A/T/G); the 3' portion of short CpG tandem sequence is underlined by a wave line, the 5' portion of anchor sequnce is underlined by a straight line and the random sequence is underlined by a double line. | |

3.2 Add the mixture above to the products (the semi-amplicons) from step 2.8, vortexing.
3.3 Centrifuge at 13000 rpm for a minute in 4°C ;
3.4 Place the tube in a thermal cycler and run the following program: 95°C for 3 minutes, 50°C for 2 minutes, 72°C for 1 minute, 4°C for pause;
3.5 Centrifuge at 13000 rpm for a minute in 4°C ;
4. Exponential amplification using Adapter Primer C, Adapter Primer D and DNA polymerase.
4.1 Prepare the following reaction mixture in a new PCR tube:

| Contents | Volume |
|---|---|
| Ex Taq Buffer | 3 µl |
| Adapter Primer C (100 uM) * | 0.6 µl |
| Adapter Primer D (100 uM) ** | 0.6 µl |
| dNTP (2.5 mM) | 3 µl |
| HS Ex Taq (Takara catalog: DRR006B) | 0.3 µl |
| Water | 22.5 µl |
| Total | 30 µl |

| | |
|---|---|
| *: Adapter Primer C: 5'-AATGATACGGCGACCACCGAGATCTACACTCTTTCCCTACACGACGCTCTTCCGATCT-3'; the underlined oligonucleotide sequence is the same as the 5' portion of Primer A; **: Adapter Primer D: 5'-CAAGCAGAAGACGGCATACGAGATCTGATCGTGACTGGAGTTCAGACGTGTGCT-3' , the underlined (by a straight line) oligonucleotide sequence is the same as the 5' portion of Primer B and the barcode sequence (corresponding to the Illumina index 9) is underlined by a double-line; | |

4.2 Add the mixture to the product (full-amplicons) from step 3.5, vortexing;
4.3 Centrifuge at 13000 rpm for a minute in 4°C ;
4.4 Place the tube in a thermal cycler and run the following program: 95°C for 3 minutes;
4.5 Run the following program: 17 cycles of 95°C for 30 seconds, 65°C for 30 seconds, 72°C for 1 minute, and then 4°C for storage.
5. Size-selection, purification, high throughput sequencing and data analysis.
5.1 Prepare 4% agarose gel, add 1×SYBR Safe (Invitrogen);
5.2 Run the products (final-amplicons) from step 4.5 in the agarose gel by electrophosis;
5.3 Imagine the gel (FIG 3, sonicated DNA sample and MCTA-Seq DNA samples without sonication have the same amplification efficiency indicated that MCTA-Seq is highly efficient for amplification of heavily fragmented DNA samples.);
5.4 Excise the gel for DNA fragments ranging from 180 to 250 bp;
5.5 Purify the DNA from the gel by using the QIAquick Gel Extraction Kit (Qiagen) according to the manufacturer's protocol, obtaining the library for high throughput sequencing;
5.6 Analyze the size distribution of the library by using the Fragment Analyzer (Agilent) (FIG 4, results show that size of DNA fragments in sequencing library range from 180-250bp) and quantify the concentration of the library by using QPCR;
5.7 Sequence the library on an Illumina HiSeq2000/2500 sequencer at pair-end 100bp reads to obtain about 10,000,000 raw paired reads;
5.8 Data analysis: firstly discard whole or any subsets of adapter sequences and low quality reads, then map the reads to the human genome reference (hg19), and then perform subsequent bioinformatics analysis.

### Example 2: The throughput, replicability, sensitivity and unique molecular identifiers of MCTA-Seq.

We validated the MCTA-Seq method by applying it to the fully methylated human genomic DNA (FMG, Chemicon/Millipore S7821, CpGenome Universal Methylated DNA), genomic DNA extracted from human white blood cells (WBCs) and two cancer cell lines (HepG2 and HeLa cells). For these and subsequent samples, the MCTA-Seq libraries were sequenced using the Illumina HiSeq2000/2500 system, obtaining on average of 7.5 million pair-end raw reads per library. Lambda DNA was spiked into the samples for assessing the conversion rate (average 98.7%, 97.53∼99.31%, table 1).

The results demonstrate that the aligned reads predominantly started from genomic CGCGCGG sequences (FIG 5). The data of FMG show that, out of all 9,393 CGIs containing one or more CGCGCGG sequences, 8,748 (93.3%) were efficiently detected (average methylated alleles per million mapped reads (MePM) > 8, FIG 6). The detection efficiency of a CGI is positively correlated with the number of CGCGCGG sequences within the CGI (FIG 5). MCTA-Seq detected on average 2,849, 3,726 and 3,773 methylated CGIs in WBCs, HepG2 and HeLa cells, respectively (FIG 7). The methylation profiles were highly reproducible between technical replicates with average correlation of 0.974 (FIG 6 and FIG 9). In addition, the differentially methylated CGIs were clearly revealed when comparing the results of the cancer cell lines with those of WBCs (FIG 8 and FIG 9). FIG 8 shows the result of CDKN2A by MCTA-Seq: 1) Almost all of the reads amplified initiate from three CGCGCGG short tandems in CDKN2A indicating the high specificity of MCTA-Seq amplification; 2) HePG2 and Hela cell lines have strong signal in two of the three CGCGCGG tandems while WBC has no signals which indicates that the CpG island is differential methylated and was shown by MCTA-Seq.

To analyze the sensitivity of MCTA-Seq, we spiked FMG into WBC gDNA in serial ratios of 1:10, 1:20, 1:50, 1:100, 1:200 and 1:400. MCTA-Seq can detect a methylated allele at a frequency as low as 0.25% (FIG 10). In addition, the sensitivity should depend on the low detection limit (LoD) of the method, and could be further increased with the increase in sequencing depth. To determine the LoD of MCTA-Seq, we performed another dilution experiment in which the absolute amounts of FMG were serially decreased to 150, 60, 30, 15 and 7.5 pg while maintained the FMG:WBC = 1:100. The result demonstrated that MCTA-Seq can detect as little as 7.5 pg (equivalent of ∼2.5 haploid genome) of methylated DNA. 84% (3,005 of 3,579) of the CGIs that were not detected in WBCs were detectable in at least one 7.5 pg replicate; 47% (1,687 of 3,579) of these were detectable in both 7.5 pg replicates (FIG 10). The detection ratios substantially increased when the amount of methylated DNA rose to 15 pg, which is consistent with high sampling stochasticity in the 7.5 pg groups.

Taking CpG islands of promoter of VIM (VIMENTIN, no. 2) and SEPT9 (SEPTIN9) as an example, the amplicons predominantly initiated from two and four CGCGCGG sequences located within the CGI, respectively; the shortest amplicon was only 30 bp (FIG 11 and FIG 13), and the methylation value was linearly correlated with the lowest dilution range (FIG 12 and FIG 14). And the data shows that the increase in reproducibility was evident in the 7.5 pg experiments after normalization with UMIs, which were PCR over-amplified to the most extent (FIG 15, FIG 11 and FIG 13).

### Example 3: MCTA-Seq of HCC tissue samples

We performed MCTA-Seq in 27 pairs of HCCs and matched adjacent noncancerous liver samples and three normal liver samples obtained from patients with hepatic hemangioma during surgery (Table 2). Sample collection and further analysis were approved by patients themselves with signatures and the research was approved by ethics committee of Capital Medical University Affiliated Beijing Shi Ji Tan hospital. The principal component analysis (PCA) of tissue samples demonstrate that MCTA-Seq can successfully distinguish most cancerous tissues (23 of 27) from noncancerous tissues (FIG 16). A total of 1,605 hypermethylated CGIs in HCC tissues were identified (referred to as tissue dmCGIs or tdmCGIs, two-tailed t-test, FDR < 0.05, average methylation fold changes > 2, FIG 17).

### Example 4: MCTA-Seq of HCC plasma samples

We performed MCTA-Seq in hepatocellular carcinoma patients' plasma (17 training group, 10 testing group, clinical information in Table 3), cirrhosis patients' plasma (11 training group, 6 testing group) and healthy individuals' plasma (9 training group, 19 testing group). Sample collection and further analysis were approved by patients themselves with signatures and the research was approved by ethics committee of Capital Medical University Affiliated Beijing Shi Ji Tan hospital. To prepare plasma, 5 ml peripheral blood was collected using EDTA anticoagulant tubes and the plasma samples were prepared within 6 h by centrifuging the blood tube at 1 350x g for 12 min at room temperature, and transferring the plasma to a 15-ml tube, and re-centrifuging at 1,350× g for 12 min, and transferring to 1.5- or 2-ml tubes, and re-centrifuging at >10,000× g for 5 min and transferring to a new tube. The prepared plasma samples (about 2 ml) were then stored at -80 °C immediately. The plasma cell-free DNAs were extracted using the QIAamp DNA Blood Midi Kit (Qiagen) according to the manufacturer's protocol.

The overall number of methylated CGIs in HCC patients was significantly higher than that in cancer-free individuals (MePM > 1, HCC vs non-cancerous individuals: median 3,465 vs 2,837, P < 0.01, two-tailed MWW test, FIG 18). The difference between plasma hepatocellular carcinoma patients and non-cancerous individuals gradually decreased with the increase of MePM threshold while the difference between hepatocellular carcinoma and adjacent tissues increased with the increase of MePM threshold (FIG 18). This suggest that tumor-related methylation changes in cell free DNA predominantly occurs in low methylation range, significantly lower than the changes in tumor tissues. We found 635 aberrantly hypermethylated CpG islands in plasma of hepatocellular carcinoma patients (referred to plasma dmCGIs or pdmCGIs, FDR < 0.05, average methylation fold changes > 2, two-tailed t-test, FIG 19). Among them, 376 CpG islands are hypermethylated in tumor tissues, which were referred to type I markers and the rest of 259 CpG islands were referred to type II markers (FIG 20). Clustering heatmap shows that type II markers could be divided into two groups. One group is type IIa markers (n=102) which are hypermethylated in tumor tissues, tumor adjacent tissues and normal liver tissues while hypomethylated in WBC showing tissue specific characteristics (FIG 21, Table 4). We performed RRBS which confirmed that they were significantly more methylated in the normal liver tissues than the WBCs (median 32.8% vs 8%, P = 5E-18, two tailed MWW test, FIG 22, Table 4). We found that the type IIa markers are likely to be intragenic CGIs (53%, 54 of 102 markers), which is in agreement with previous reports that tissue-specifically methylated CGIs are often intragenic. CpG islands in SHANK2 is a typical intragenic type IIa CpG islands (no. 79, FIG 23).

Then, we applied the receiver operating characteristic (ROC) curve analysis to find markers with best diagnostic performance. Out of 635 differentially methylated CGIs, a total of 132 top HCC-detecting markers were identified using an area under the curve (AUC) cutoff as > 0.9. Among them 68 markers are type I markers, 64 markers are type II markers and most of the type II markers are type IIa markers (n=56). Detailed information and data of these two types of markers are provided in FIG 24, FIG 25 and Table 4.

In addition, SEPT9, for example, which showed good ability to distinguish between other carcinoma patients (colon cancer) and normal people had poor AUC (SEPT9 0.771) in hepatocellular carcinoma, suggesting that different tumors have different methylation patterns. It is possible to distinguish different types of tumors by methylation patterns of genome-scale detection of differential methylated CpG islands.

### Example 5: MCTA-Seq diagnoses of hepatocellular carcinoma patients by detecting ccfDNA methylation.

For each plasma differential methylated CpG island with high AUC value, a threshold corresponding to 90% specificity was set (i.e., for 90% of tumor-free individuals, the methylation level of the CpG island in cell free DNA was below the threshold) to analyze the number of CpG islands exceeding the threshold in 132 high-performance CpG islands. The results show that DNA from 132 high-performance CpG islands could be detected simultaneously in most of the the hepatocellular carcinoma patients while in most of the non-cancerous individuals. The correlation between the 132 CpG islands was weak. Counting the number of CpG islands exceeding the threshold corresponding to 90% specificity increases the specificity from 90% to 100% with AUC value 0.982 (95% CI: 0.952-1, FIG 28).

We next sought to add quantitative information and established the mutilple sites detection mode for HCC (Fig. 29). The number of standard deviations (SDs) above the 90% specificity threshold was calculated, named as the M-score (Fig. 6a). We measured two types of M-scores, the top M-score and the total M-score. The top M-score provides information of the uppermost hypermethylated locus and is expected to maximize the sensitivity of the assay because it uses the extreme marker to make a positive call. The total M-score represents an overall hypermethylation evaluation. In total, there were three types of parameters: the number of positive loci, the top M-score and the total M-score. For each parameter, we typically set a threshold just above the uppermost value in the cancer-free subjects to define a positive call (the number of positive loci ≥30, the top M-score ≥5, and the total M-score ≥10). We independently calculated these parameters for the type I and type II markers, resulting in a total of 6 values in the final diagnostic panel. Further, we established two types of criterions with the high specificity criterion requiring 5 or 6 positive calls and the high sensitivity criterion requiring 3 or 4 positive calls with at least 1 from the type I markers.

The performance of the diagnostic panel in the training group was examined. The high specificity criterion had a sensitivity of 88% (15/17) and a specificity of 100% (1/20), and the high-sensitivity criterion had a sensitivity of 94% (16/17) and a specificity of 95% (1/20). To test our results, we analyzed a new set of 35 plasma samples, including those from 10 HCC patients, 6 cirrhosis patients and 19 healthy subjects, and performed the experiments and analysis in a double-blind manner (Table 2,3). Both criterions identified 9 out of 10 HCC patients (90% sensitivity), whereas 1 cirrhosis patient showed positive detection in the high-specificity criterion (96% specificity) and additional 1 healthy subject showed positive detection in the high-sensitivity criterion (92% specificity). The overall sensitivity of the training and testing groups was 93% in the high sensitivity criterion and 89% in the high-specificity criterion, and the overall specificity was 96% in the high sensitivity criterion and 98% in the high specificity criterion. FIG 29∼FIG39 show the MCTA-Seq results of 132 type I and type II markers among the 72 individuals plasma samples.

We further associated the MCTA-Seq results with the tumor size. The total M-scores of both CGI types were positively correlated with the tumor size (type I: r = 0.656; type II: r = 0.508, FIG 40). Then, we compared the MCTA-Seq results with those of the AFP serum assay. In our study, 37% (10 of 27) of the HCC patients exhibited false-negative results in the AFP assay (<20 ng/mL, FIG 41, Table 3). Importantly, 90% (9 of 10) of these AFP-negative patients were positively detected via MCTA-Seq using the high-specificity criterion, and all were positively detected using the high sensitivity criterion (FIG 42).

### Example 6: Target other short CpG tandem sequences except for CGCGCGG in methylation detection by MCTA-Seq.

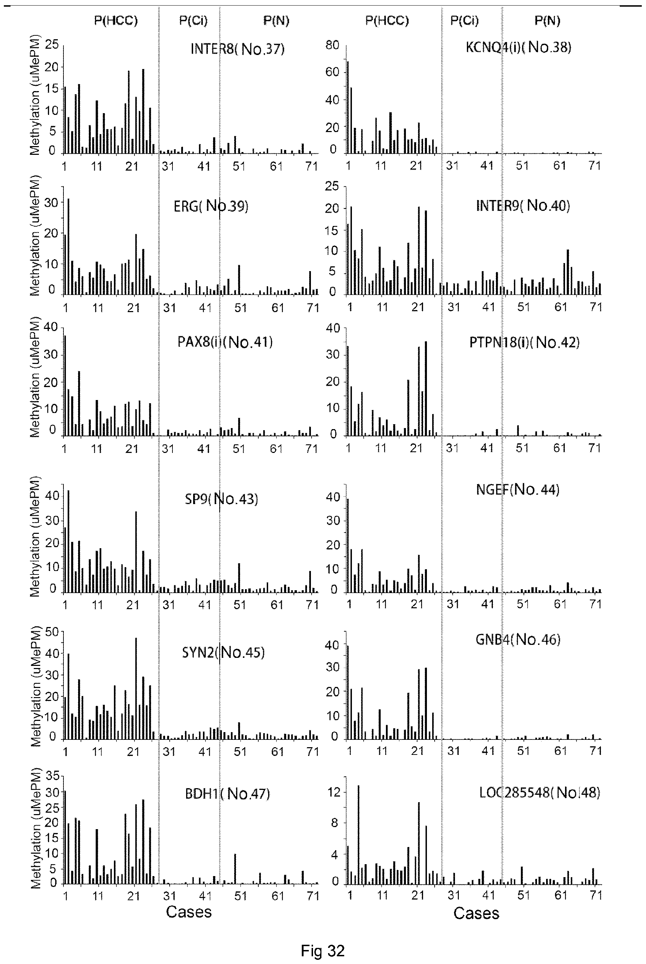

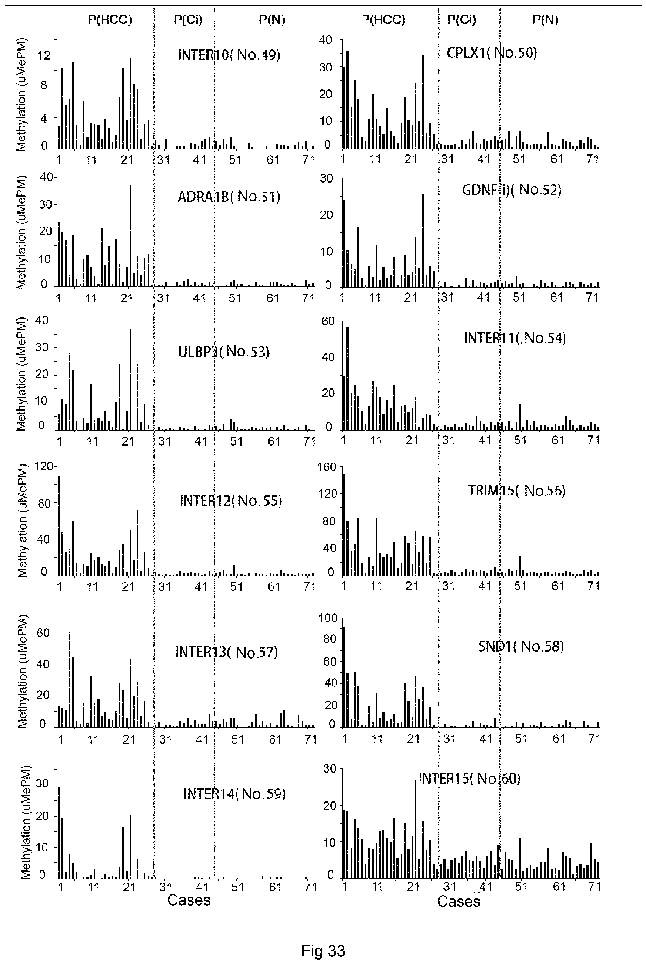

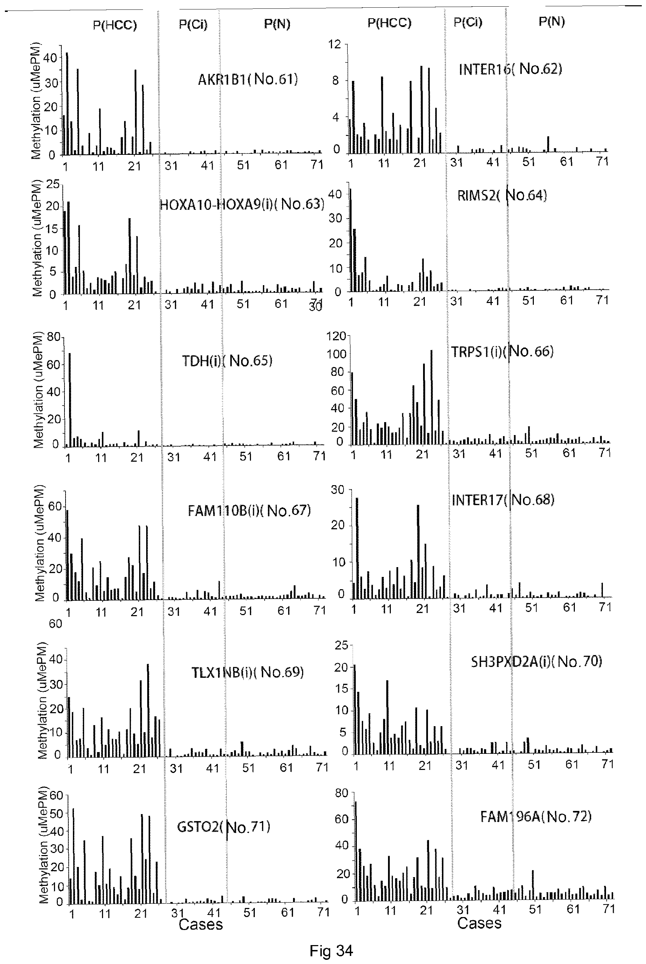

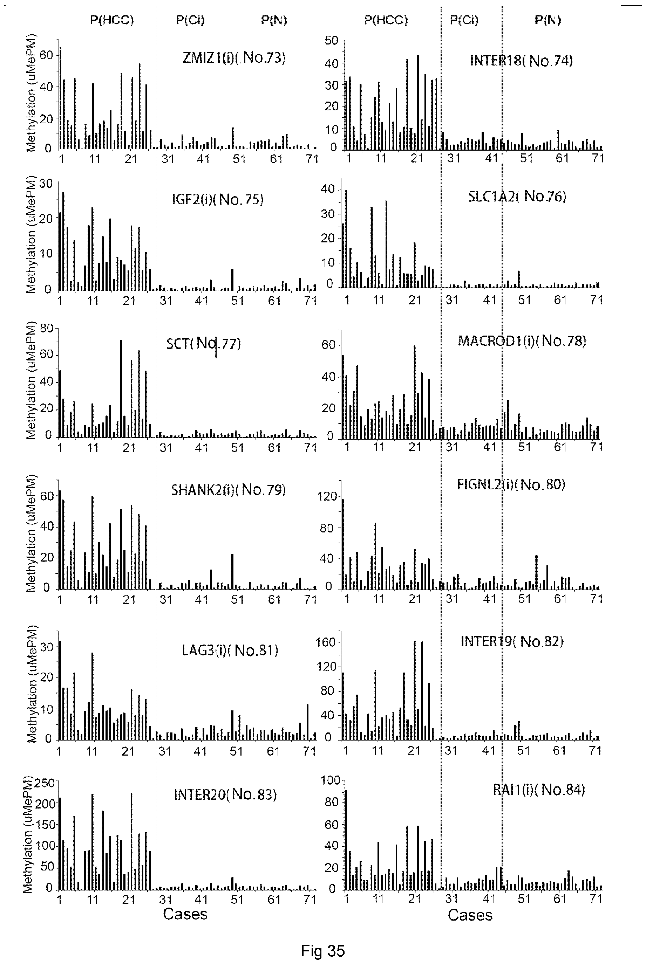

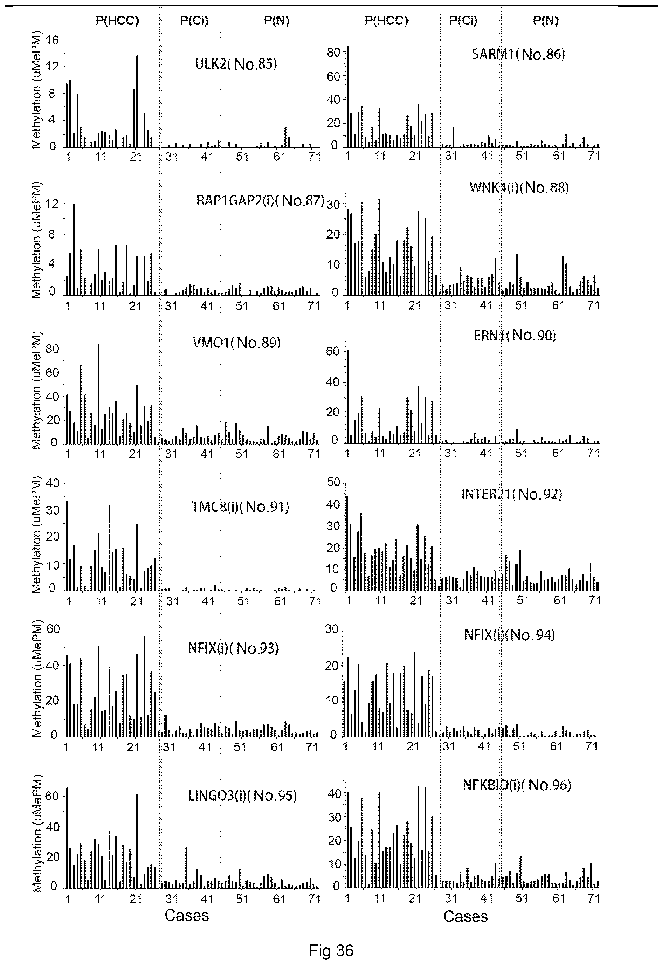

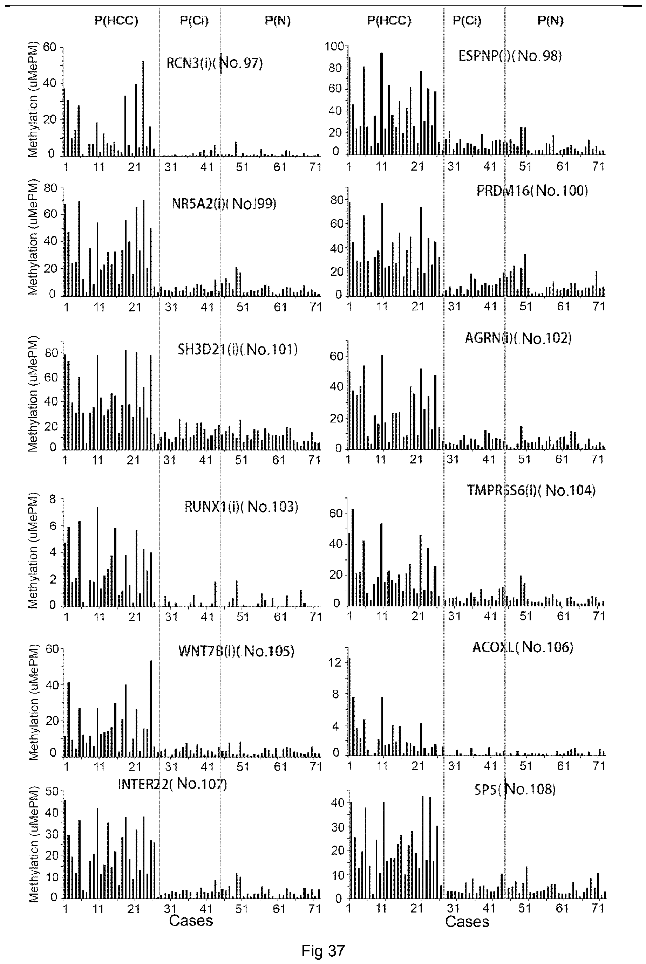

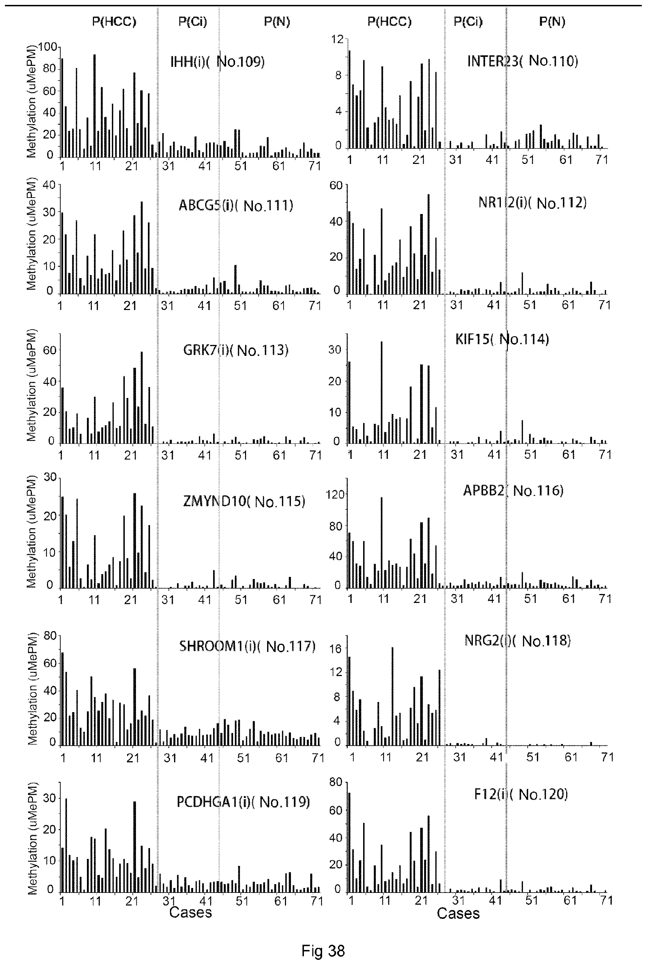

Taking CDKN2A as an example, it is possible to further increase the detection range by simultaneously targeting several types of CpG tandems (FIG 32). CDKN2A promoter CpG island does not contain CGCGCGG short tandem sequence, so CDKN2A could not be detected using CGCGCGG as the primer of MCTA-Seq. But it contains a CGGCGGCGG site. Using CGGCGGCGG or CGCGGCGG as the primer, CDKN2A promoter CpG island can be detected with high efficiency.

**TABLE 1. Sequencing information**

| Name | Groups | Sample number | Raw reads (pair end, millions Mean±SD) | Uniquely mapping reads (millions Mean±SD) | Bisulfite transformation (%, Mean±SD) |
|---|---|---|---|---|---|
| | FMG, WBCs, HePG2, Hela and dilution experiments | 30 | 7.9 ± 2.8 | 3.2 ± 1.2 | 98.86 ± 0.19 |
| T(HCC) | Tissue | 27 | 6.3 ± 2.3 | 2.1 ± 1.0 | 98.80 ± 0.18 |
| T(Aj) | Tissue | 27 | 6.7 ± 3.0 | 2.2 ± 0.8 | 98.63 ± 0.47 |
| T(NL) | Tissue | 3 | 5.3 ± 0.8 | 2.8 ± 0.3 | 98.90 ± 0.16 |
| P(HCC)-tr | Plasma, training group | 17 | 8.4 ± 2.4 | 3.0 ± 0.7 | 98.70 ± 0.33 |
| P(HCC)-te | 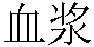 , testing | 10 | 9.6 ± 3.1 | 3.8 ± 1.1 | 98.53 ± 0.42 |
| P(Ci)-tr | Plasma, training group | 11 | 7.1 ± 0.8 | 2.6 ± 0.3 | 98.34 ± 0.24 |
| P(Ci)-te | Plasma, testing group | 5 | 9.4 ± 1.4 | 3.5 ± 0.7 | 98.79 ± 0.30 |
| P(N)-tr | Plasma, training group | 9 | 7.4 ± 3.1 | 2.9 ± 1.0 | 98.65 ± 0.54 |
| P(N)-te | Plasma, testing group | 20 | 8.9 ± 2.1 | 3.1 ± 0.8 | 98.68 ± 0.40 |

T(HCC): HCC cancer tissue; T(Aj): adjacent tissues of HCC; T(NL): normal liver tissue; P(HCC): plasma from HCC patients; P(Ci): plasma from cirrhosis patients; P(N): plasma from healthy individuals; tr: training group; te: testing group.

**Table 2. Clinicle information of HCC patients and healthy individuals.**

| | Groups | Age (years, Mean±SD) | Sex | | Tumor size (cm) | | |
|---|---|---|---|---|---|---|---|
| | | | Male (samples/p ercentage) | Female (samples/percentage) | > 5 | 3∼5 | ≤ 3 |
| T(HCC) | Tissue | 59 ± 9 | 22(81%) | 5(19%) | 10(37%) | 6(22%) | 11(41%) |
| T(Aj) | Tissue | 59 ± 9 | 22(81%) | 5(19%) | 10(37%) | 6(22%) | 11(41%) |
| T(NL) | Tissue | 55 ± 8 | 1(33%) | 2(67%) | | | |
| P(HCC)-tr | Plasma, training group | 61 ± 10 | 15(88%) | 2(12%) | 4(24%) | 7(41%) | 6(35%) |
| P(HCC)-te | Plasma, testing group | 63 ± 11 | 8(80%) | 2(20%) | 4(40%) | 3(30%) | 3(30%) |
| P(Ci)-tr | Plasma, training group | 56 ± 11 | 8(73%) | 3(27%) | | | |
| P(Ci)-te | Plasma, testing group | 50 ± 8 | 4(75%) | 1(25%) | | | |
| P(N)-tr | Plasma, training group | 62 ± 15 | 5(56%) | 4(44%) | | | |
| P(N)-te | Plasma, testing group | 55 ± 17 | 16(80%) | 4(20%) | | | |

**Table 3. Clinical and pathological data of HCC patients.**

| Numb er | Group | Age | Sex | HBV infection | Nodle types | Tumor size | Vascular invasion | TNM stages | AFP (ng/mL) |
|---|---|---|---|---|---|---|---|---|---|
| p50 | training | 68 | male | Yes | Satellites | 15 | yes | T3aN0M0 | >1210 |
| p92 | testing | 77 | male | No | Satellites | 15 | yes | T3bN0M0 | >1210 |
| p29 | training | 56 | male | No | Unique | 11.2 | yes | T4N0M0 | >1210 |
| p119 | testing | 50 | male | Yes | Unique | 11 | yes | T3bN0M0 | 4.41 |
| p41 | training | 57 | male | No | Satellites | 8 | No | T3aN0M0 | 59 |
| p109 | testing | 74 | male | No | Unique | 8 | No | T1N0M0 | 2.61 |
| p101 | testing | 76 | fem ale | Yes | Unique | 7 | yes | T2N0M0 | >1210 |
| p3 | training | 42 | male | Yes | Satellites | 5.6 | No | T3aN0M0 | 78.5 |
| p37 | training | 64 | male | Yes | Unique | 5 | No | T1N0M0 | 675.9 |
| p114 | testing | 39 | male | Yes | Satellites | 5 | yes | T4N0M0 | >1210 |
| p85 | testing | 63 | male | Yes | Unique | 4.5 | yes | T2N0M0 | 16.2 |
| p57 | training | 76 | fem ale | No | Unique | 4 | yes | T2N0M0 | 2.29 |
| P62 | training | 51 | male | Yes | Unique | 4 | yes | T4N0M0 | 20.23 |
| P77 | training | 61 | male | Yes | Unique | 4 | yes | T4N0M0 | >1210 |
| p10 | training | 56 | male | Yes | Satellites | 3.5 | No | T2N0M0 | 768.5 |
| p39 | training | 72 | male | Yes | Satellites | 3.5 | No | T2N0M0 | 3.65 |
| p60 | training | 70 | male | Yes | Unique | 3.5 | yes | T3bN0M0 | 446.5 |
| p115 | testing | 60 | male | Yes | Unique | 3.3 | No | T1N0M0 | 27.4 |
| p36 | training | 44 | male | Yes | Unique | 3 | No | T1N0M0 | 5.74 |
| p44 | training | 63 | male | Yes | Satellites | 3 | yes | T4N0M1 | >1210 |
| p59 | training | 73 | male | No | Unique | 3 | No | T1N0M0 | >1210 |
| P74 | training | 68 | fem ale | No | Unique | 3 | yes | T2N0M0 | 12.6 |
| p80 | testing | 70 | male | Yes | Unique | 3 | No | T1N0M0 | 36.8 |
| p81 | testing | 61 | fem ale | No | Unique | 2.8 | No | T1N0M0 | 8.23 |
| p113 | testing | 63 | male | No | Unique | 2.1 | yes | T4N0M0 | 126.1 |
| p58 | training | 60 | male | No | Unique | 2 | No | T1N0M0 | 3.64 |
| p51 | training | 51 | male | Yes | Unique | 1.5 | No | T1N0M0 | 5.12 |

**Table 4. MCTA-Seq data of type I and type II plasma differentially methylated CpG islands.**

| Seri al nu mb er | Gene name | Ty pe | AUC | T(HCC) MePM | T(Aj) MePM | T(NL) MePM | T(W BC) Me PM | P(HCC) uMeP M | P(Co n) uMe PM | NL RRBS (%) | WBC RRBS (%) |
|---|---|---|---|---|---|---|---|---|---|---|---|
| 1 | INTER1 | I | 0.94 | 95(106) | 32(17) | 34(13) | 1(1) | 10(11) | 1(1) | 41(16) | 31(7) |
| 2 | VIM | I | 0.96 | 52(47) | 4(6) | 6(3) | 0(0) | 3(6) | 0(0) | 3(1) | 1(0) |
| 3 | ST8SIA6 | I | 0.96 | 88(111) | 5(6) | 2(0) | 0(0) | 5(8) | 0(0) | 4(1) | 1(0) |
| 4 | LOC 100130992 | I | 0.92 | 113(93) | 33(15) | 22(7) | 1(0) | 17(14) | 2(2) | 4(2) | 1(0) |
| 5 | RET(i) | I | 0.93 | 56(72) | 8(5) | 11(3) | 0(0) | 6(8) | 1(1) | 4(1) | 1(0) |
| 6 | RASGEF1A(i) | I | 0.96 | 75(83) | 11(9) | 9(5) | 1(0) | 6(7) | 0(0) | 5(3) | 1(0) |
| 7 | INTER2 | I | 0.91 | 21(33) | 3(2) | 1(1) | 0(0) | 2(3) | 0(0) | 1(2) | 3(2) |
| 8 | INTER3 | I | 0.92 | 43(36) | 16(7) | 16(8) | 1(1) | 12(8) | 2(2) | 21(13) | 10(3) |
| 9 | CACNA1C | I | 0.92 | 104(102) | 18(9) | 30(17) | 0(0) | 14(14) | 1(1) | 10(6) | 1(1) |
| 10 | FAR2 | I | 0.93 | 46(48) | 4(2) | 3(1) | 0(0) | 7(10) | 1(1) | 7(1) | 2(1) |
| 11 | EFCAB4B | I | 0.95 | 124(112) | 13(16) | 14(3) | 0(0) | 12(14) | 0(1) | 30(1) | 1(1) |
| 12 | KCNH3(i) | I | 0.96 | 100(65) | 43(17) | 24(6) | 1(0) | 14(12) | 2(1) | 29(4) | 9(8) |
| 13 | GRASP | I | 0.92 | 36(35) | 8(12) | 1(0) | 0(0) | 4(5) | 0(0) | 8(2) | 1(0) |
| 14 | POU4F1-AS1 | I | 0.9 | 135(115) | 11(7) | 4(1) | 1(0) | 10(21) | 1(1) | 8(1) | 3(1) |
| 15 | PARP6 | I | 0.91 | 75(94) | 5(6) | 7(5) | 0(0) | 7(11) | 0(0) | 9(3) | 0(0) |
| 16 | ISL2(i) | I | 0.93 | 66(55) | 20(11) | 23(7) | 1(0) | 14(15) | 2(2) | 13(2) | 3(0) |
| 17 | TM6SF1 | I | 0.92 | 23(25) | 2(1) | 0(0) | 0(0) | 4(7) | 0(0) | 8(6) | 2(0) |
| 18 | PRKCB | I | 0.94 | 72(81) | 5(5) | 2(1) | 0(0) | 4(8) | 0(0) | 3(1) | 0(0) |
| 19 | MMP25 | I | 0.91 | 32(53) | 3(3) | 2(1) | 0(0) | 3(3) | 1(0) | 4(2) | 2(1) |
| 20 | INTER4 | I | 0.97 | 46(35) | 22(13) | 6(5) | 1(0) | 5(5) | 1(0) | 48(13) | 3(0) |
| 21 | SSH2(i) | I | 0.93 | 162(187) | 23(33) | 25(11) | 0(0) | 13(15) | 1(1) | 15(9) | 0(0) |
| 22 | INTER5 | I | 0.9 | 61(70) | 14(7) | 13(2) | 2(0) | 10(8) | 3(2) | 19(12) | 6(1) |
| 23 | SLC25A39 | I | 0.92 | 109(143) | 14(11) | 28(3) | 0(1) | 9(17) | 0(1) | 11(1) | 1(0) |
| 24 | MAP3K14(i) | I | 0.93 | 67(94) | 18(10) | 23(1) | 9(6) | 21(14) | 6(3) | 1(1) | 1(0) |
| 25 | TBX2 | I | 0.94 | 577(487) | 181(50) | 117(22 ) | 7(1) | 107(86 ) | 14(6 ) | 19(5) | 6(1) |
| 26 | INTER6 | I | 0.93 | 61(57) | 30(17) | 29(6) | 1(0) | 14(11) | 4(2) | 43(28) | ND |
| 27 | ACTG1 | I | 0.96 | 141(144) | 64(45) | 110(33 ) | 1(1) | 22(24) | 2(3) | 6(1) | 1(0) |
| 28 | SLC16A3 | I | 0.91 | 42(55) | 9(6) | 5(3) | 1(0) | 3(4) | 1(1) | 12(0) | 5(1) |
| 29 | PIK3R5 | I | 0.98 | 41(36) | 19(9) | 12(5) | 0(0) | 7(6) | 1(1) | 10(2) | 1(0) |
| 30 | NEDD4L(i) | I | 0.96 | 82(68) | 23(22) | 26(4) | 0(0) | 9(7) | 1(1) | 24(13) | 3(3) |
| 31 | NFATC1 | I | 0.91 | 33(43) | 10(3) | 4(1) | 3(2) | 4(6) | 1(1) | 23(1) | 20(10 ) |
| 32 | MATK | I | 0.94 | 53(53) | 7(5) | 6(6) | 0(0) | 5(6) | 1(1) | 5(1) | 2(0) |
| 33 | PTCHD2 | I | 0.9 | 36(52) | 5(4) | 3(1) | 1(0) | 3(3) | 1(0) | 3(1) | 2(0) |
| 34 | KIF21B | I | 0.95 | 60(76) | 6(5) | 8(2) | 0(0) | 6(9) | 0(0) | 10(5) | 1(0) |
| 35 | PPFIA4(i) | I | 0.95 | 53(40) | 22(10) | 15(5) | 0(0) | 12(9) | 1(1) | 35(2) | 3(1) |
| 36 | INTER7 | I | 0.91 | 61(103) | 5(6) | 2(1) | 1(0) | 6(8) | 1(1) | 5(1) | 2(2) |
| 37 | INTER8 | I | 0.98 | 94(77) | 47(23) | 87(32) | 0(0) | 8(6) | 1(1) | 18(9) | 3(1) |
| 38 | KCNQ4(i) | I | 0.95 | 60(46) | 27(13) | 17(5) | 0(0) | 14(15) | 0(0) | 35(10) | 14(13 ) |
| 39 | ERG | I | 0.91 | 57(40) | 19(7) | 16(2) | 1(1) | 9(7) | 2(2) | 7(2) | 5(1) |
| 40 | INTER9 | I | 0.91 | 72(48) | 28(15) | 16(3) | 0(1) | 8(6) | 3(2) | 9(1) | 7(2) |
| 41 | PAX8(i) | I | 0.9 | 147(99) | 32(15) | 10(4) | 1(0) | 9(8) | 1(1) | 27(4) | 10(2) |
| 42 | PTPN18(i) | I | 0.97 | 119(104) | 26(31) | 9(5) | 0(0) | 9(11) | 1(1) | 12(3) | 2(0) |
| 43 | SP9 | I | 0.94 | 145(141) | 32(11) | 35(11) | 3(0) | 13(10) | 3(2) | 9(2) | 3(0) |
| 44 | NGEF | I | 0.93 | 62(49) | 26(22) | 7(1) | 1(0) | 7(8) | 1(1) | 33(7) | 10(1) |
| 45 | SYN2 | I | 0.94 | 75(56) | 31(13) | 32(9) | 2(0) | 16(11) | 3(2) | 7(0) | 6(2) |
| 46 | GNB4 | I | 0.91 | 61(44) | 17(10) | 15(3) | 0(0) | 9(10) | 0(1) | 11(15) | 1(1) |
| 47 | BDH1 | I | 0.92 | 95(69) | 43(25) | 92(33) | 0(0) | 10(10) | 1(2) | 26(6) | 1(0) |
| 48 | LOC285548 | I | 0.93 | 29(42) | 5(4) | 2(1) | 0(0) | 3(3) | 1(1) | 6(2) | 3(1) |
| 49 | INTER10 | I | 0.96 | 52(36) | 7(6) | 7(3) | 0(0) | 4(3) | 0(0) | 19(13) | 7(8) |
| 50 | CPLX1(i) | I | 0.92 | 113(69) | 41(34) | 62(18) | 2(0) | 13(10) | 3(2) | 57(2) | 33(8) |
| 51 | ADRA1B(i) | I | 0.93 | 63(63) | 21(9) | 21(9) | 0(0) | 10(9) | 1(1) | 43(26) | 5(0) |
| 52 | GDNF(i) | I | 0.92 | 47(38) | 16(12) | 16(7) | 1(1) | 7(6) | 1(1) | 26(1) | 5(1) |
| 53 | ULBP3 | I | 0.92 | 51(55) | 17(16) | 18(10) | 0(0) | 9(10) | 1(1) | 1(1) | 1(0) |
| 54 | INTER11 | I | 0.95 | 65(65) | 19(9) | 14(6) | 3(0) | 15(11) | 3(2) | 12(1) | 6(1) |
| 55 | INTER12 | I | 0.94 | 215(127) | 67(33) | 24(17) | 1(1) | 24(25) | 2(2) | 18(1) | 3(1) |
| 56 | TRIM15(i) | I | 0.93 | 234(176) | 116(47) | 95(13) | 4(1) | 40(32) | 6(4) | 43(4) | 12(1) |
| 57 | INTER13 | I | 0.91 | 209(163) | 77(43) | 51(31) | 2(1) | 16(15) | 3(3) | 17(4) | 9(2) |
| 58 | SND1 | I | 0.95 | 249(175) | 76(50) | 55(14) | 0(1) | 20(21) | 2(2) | 55(2) | 2(0) |
| 59 | INTER14 | I | 0.92 | 38(58) | 1(1) | 1(1) | 0(0) | 5(7) | 0(0) | 6(0) | 2(1) |
| 60 | INTER15 | I | 0.92 | 75(55) | 23(10) | 25(5) | 4(2) | 11(5) | 5(2) | 12(3) | 7(4) |
| 61 | AKR1B1 | I | 0.94 | 136(125) | 12(10) | 17(2) | 0(1) | 9(12) | 0(0) | 5(1) | 0(0) |
| 62 | INTER16 | I | 0.91 | 28(36) | 7(4) | 11(1) | 0(0) | 3(3) | 0(0) | 9(0) | 1(0) |
| 63 | HOXA10 -HOXA9(i) | I | 0.9 | 61(66) | 7(4) | 3(0) | 0(0) | 6(6) | 1(1) | 9(0) | 2(1) |
| 64 | RIMS2 | I | 0.91 | 146(159) | 7(4) | 3(1) | 0(0) | 6(9) | 0(0) | 7(3) | 3(0) |
| 65 | TDH(i) | I | 0.91 | 39(53) | 4(3) | 2(1) | 0(0) | 5(13) | 1(1) | 8(1) | 2(1) |
| 66 | TRPS1(i) | I | 0.92 | 314(230) | 84(40) | 133(23 ) | 4(1) | 31(26) | 5(3) | 21(29) | 2(3) |
| 67 | FAM110B(i) | I | 0.92 | 171(134) | 32(21) | 29(8) | 1(0) | 17(16) | 2(2) | 96(0) | 92(1) |
| 68 | INTER17 | I | 0.91 | 33(46) | 6(3) | 6(3) | 0(1) | 6(7) | 1(1) | 5(2) | 3(1) |
| 69 | TLX1NB(i) | IIa | 0.92 | 76(39) | 48(28) | 57(4) | 0(0) | 12(9) | 2(1) | 34(2) | 4(2) |
| 70 | SH3PXD2A(i) | IIa | 0.95 | 40(20) | 33(18) | 27(7) | 0(0) | 6(5) | 1(1) | 68(7) | 1(0) |
| 71 | GSTO2 | IIa | 0.98 | 118(93) | 78(45) | 92(35) | 0(0) | 17(16) | 1(1) | 28(2) | 0(0) |
| 72 | FAM196A | IIa | 0.92 | 136(106) | 81(26) | 69(13) | 2(1) | 21(15) | 6(3) | 10(2) | 5(2) |
| 73 | ZMIZ1(i) | IIa | 0.91 | 151(84) | 129(59) | 97(41) | 0(0) | 22(18) | 4(3) | 33(6) | 3(0) |
| 74 | INTER18 | IIa | 0.91 | 106(87) | 111(54) | 49(11) | 2(2) | 19(13) | 4(2) | 51(2) | 10(0) |
| 75 | IGF2(i) | IIa | 0.98 | 40(29) | 45(21) | 72(16) | 0(0) | 10(7) | 1(1) | 49(14) | 4(3) |
| 76 | SLC1A2 | IIb | 0.92 | 41(39) | 23(8) | 16(7) | 1(0) | 11(11) | 1(1) | 11(2) | 3(1) |
| 77 | SCT | IIa | 0.98 | 47(35) | 43(18) | 31(5) | 1(1) | 21(19) | 2(2) | 36(14) | 11(5) |
| 78 | MACROD1(i) | IIa | 0.91 | 156(127) | 79(35) | 48(19) | 7(0) | 24(15) | 8(4) | 34(3) | 11(1) |
| 79 | SHANK2(i) | IIa | 0.94 | 147(71) | 140(63) | 158(37 ) | 1(0) | 26(19) | 3(4) | 82(7) | 11(3) |
| 80 | FIGNL2(i) | IIa | 0.91 | 99(83) | 72(30) | 60(12) | 4(1) | 31(25) | 10(8 ) | 32(6) | 4(0) |
| 81 | LAG3(i) | IIa | 0.92 | 65(36) | 59(22) | 53(9) | 2(1) | 11(7) | 3(2) | 55(33) | 8(6) |
| 82 | INTER19 | IIa | 0.93 | 284(245) | 176(80) | 183(65 ) | 2(0) | 52(45) | 8(5) | 37(52) | ND |
| 83 | INTER20 | IIa | 0.94 | 315(197) | 245(73) | 179(37) | 2(1) | 93(65) | 8(5) | 0(0) | 0(0) |
| 84 | RAI1(i) | IIa | 0.92 | 127(91) | 99(55) | 157(39 ) | 4(0) | 26(20) | 9(4) | 54(1) | 28(1) |
| 85 | ULK2 | IIb | 0.92 | 39(80) | 7(8) | 3(3) | 0(0) | 3(4) | 0(1) | 9(4) | 8(0) |
| 86 | SARM1 | IIa | 0.96 | 139(100) | 86(40) | 68(17) | 1(0) | 18(17) | 3(3) | 14(3) | 1(0) |
| 87 | RAP1GAP2(i) | IIb | 0.9 | 11(19) | 8(4) | 2(0) | 0(0) | 3(3) | 1(0) | 6(1) | 2(0) |
| 88 | WNK4(i) | IIa | 0.96 | 70(47) | 65(30) | 57(9) | 7(4) | 15(9) | 5(3) | ND | 3(4) |
| 89 | VMO1 | IIa | 0.91 | 80(54) | 110(39) | 118(31 ) | 7(2) | 26(18) | 6(4) | 87(19) | ND |
| 90 | ERN1 | IIa | 0.93 | 153(188) | 71(46) | 115(21 ) | 2(0) | 14(14) | 2(2) | 2(0) | 1(0) |
| 91 | TMC8(i) | IIb | 0.97 | 17(22) | 22(8) | 16(6) | 0(0) | 11(9) | 0(0) | 21(3) | 1(0) |
| 92 | INTER21 | IIa | 0.91 | 120(91) | 101(33) | 106(13 ) | 13( 2) | 18(10) | 7(4) | 35(11) | 8(1) |
| 93 | NFIX(i) | IIa | 0.97 | 68(38) | 72(31) | 99(21) | 3(1) | 24(16) | 4(2) | 55(10) | 25(11 ) |
| 94 | NFIX(i) | IIa | 0.94 | 49(54) | 62(37) | 49(30) | 0(0) | 12(7) | 2(1) | 48(7) | 18(1) |
| 95 | LINGO3(i) | IIa | 0.92 | 50(60) | 32(14) | 49(5) | 9(0) | 21(16) | 5(4) | 9(2) | 7(3) |
| 96 | NFKBID(i) | IIa | 0.96 | 75(54) | 43(28) | 73(21) | 0(0) | 12(11) | 1(1) | ND | ND |
| 97 | RCN3(i) | IIa | 0.91 | 127(90) | 73(50) | 55(12) | 0(0) | 13(14) | 1(2) | 38(11) | 1(1) |
| 98 | ESPNP(i) | IIa | 0.94 | 283(172) | 159(105) | 151(50 ) | 2(0) | 34(32) | 6(4) | 30(1) | 15(3) |
| 99 | NR5A2(i) | IIa | 0.91 | 165(94) | 140(74) | 292(88 ) | 7(1) | 32(21) | 6(4) | 38(4) | 11(0) |
| 100 | PRDM16 | IIa | 0.91 | 150(80) | 152(35) | 93(19) | 6(1) | 36(21) | 10(7 ) | 18(1) | 13(2) |
| 101 | SH3D21(i) | IIa | 0.92 | 132(88) | 71(21) | 54(17) | 17( 2) | 42(23) | 13(6 ) | 42(6) | 44(8) |
| 102 | AGRN(i) | IIa | 0.93 | 149(132) | 92(41) | 30(8) | 1(1) | 26(17) | 5(3) | 42(5) | 18(3) |
| 103 | RUNX1(i) | IIb | 0.92 | 11(10) | 11(4) | 14(6) | 0(0) | 3(2) | 0(0) | 47(13) | 0(0) |
| 104 | TM PRSS6(i) | IIa | 0.92 | 185(101) | 173(62) | 171(32 ) | 8(3) | 22(16) | 6(4) | 50(4) | 11(3) |
| 105 | WNT7B(i) | IIa | 0.93 | 36(47) | 51(30) | 9(7) | 4(1) | 16(13) | 3(2) | 13(2) | 13(0) |
| 106 | ACOXL | IIb | 0.93 | 20(12) | 11(5) | 2(2) | 0(0) | 3(3) | 0(0) | 7(0) | 1(0) |
| 107 | INTER22 | IIa | 0.94 | 105(58) | 99(56) | 97(18) | 2(1) | 21(13) | 3(2) | 63(10) | 13(4) |
| 108 | SP5 | IIa | 0.93 | 113(53) | 82(36) | 98(12) | 4(1) | 21(12) | 5(3) | 14(4) | 5(2) |
| 109 | IHH(i) | IIa | 0.91 | 234(110) | 184(64) | 236(65 ) | 7(3) | 39(26) | 9(6) | 31(3) | 5(1) |
| 110 | INTER23 | IIb | 0.94 | 44(57) | 21(12) | 17(15) | 1(0) | 4(3) | 1(1) | 9(8) | 1(0) |
| 111 | ABCG5(i) | IIa | 0.96 | 113(63) | 98(39) | 119(22 ) | 1(0) | 14(9) | 2(2) | 29(12) | 3(3) |
| 112 | NR1I2(i) | IIa | 0.94 | 192(96) | 145(102) | 125(33 ) | 0(0) | 21(15) | 2(2) | 64(51) | 0(0) |
| 113 | GRK7(i) | IIa | 0.94 | 95(60) | 71(28) | 59(16) | 0(0) | 19(15) | 2(2) | ND | ND |
| 114 | KIF15 | IIb | 0.92 | 17(28) | 44(31) | 94(28) | 0(0) | 8(9) | 1(1) | 13(1) | 1(0) |
| 115 | ZMYND10 | IIa | 0.94 | 82(48) | 42(23) | 31(10) | 0(0) | 9(8) | 1(1) | 26(19) | 2(0) |
| 116 | APBB2 | IIa | 0.93 | 163(99) | 113(72) | 123(12 ) | 1(1) | 37(28) | 6(4) | 43(13) | 0(0) |
| 117 | SHROOM1(i) | IIa | 0.9 | 126(53) | 109(36) | 196(31 ) | 10( 1) | 28(16) | 9(4) | 68(3) | 17(0) |
| 118 | NRG2(i) | IIb | 0.96 | 17(23) | 7(5) | 2(1) | 0(0) | 5(4) | 0(0) | 4(4) | 1(0) |
| 119 | PCDHGA1(i) | IIa | 0.91 | 53(35) | 34(11) | 31(10) | 0(1) | 11(7) | 3(2) | 42(9) | 13(3) |
| 120 | F12(i) | IIa | 0.96 | 165(111) | 114(56) | 122(21 ) | 0(0) | 21(19) | 2(2) | 41(22) | 1(1) |
| 121 | MIR548AO(i) | IIa | 0.93 | 543(256) | 324(100) | 343(61 ) | 15( 1) | 101(65 ) | 27(1 2) | 44(2) | 12(5) |
| 122 | SOBP(i) | IIa | 0.9 | 127(64) | 105(33) | 76(5) | 4(2) | 28(16) | 8(3) | 51(8) | 17(0) |
| 123 | ESR1(i) | IIa | 0.91 | 69(42) | 55(32) | 97(25) | 1(1) | 11(8) | 1(2) | 47(1) | 2(0) |
| 124 | TFR2(i) | IIa | 0.96 | 82(48) | 64(29) | 40(13) | 1(0) | 14(17) | 2(2) | 34(9) | 1(1) |
| 125 | PRKAG2(i) | IIa | 0.94 | 160(145) | 102(51) | 75(3) | 1(0) | 24(18) | 3(2) | 35(39) | 2(3) |
| 126 | SHH(i) | IIa | 0.91 | 261(122) | 175(66) | 137(33 ) | 4(2) | 27(15) | 8(4) | 39(2) | 9(2) |
| 127 | GATA4(i) | IIa | 0.96 | 124(109) | 165(60) | 234(43 ) | 2(1) | 40(31) | 6(3) | 33(8) | 11(4) |
| 128 | OPLAH(i) | IIa | 0.97 | 919(691) | 479(202) | 404 (145) | 9(2) | 105(93 ) | 13(6 ) | 27(3) | 6(2) |
| 129 | GDF6(i) | IIb | 0.94 | 29(45) | 21(12) | 29(16) | 0(0) | 11(10) | 1(1) | 25(1) | 7(1) |
| 130 | AKAP2 | IIa | 0.93 | 125(87) | 65(48) | 82(4) | 0(0) | 22(21) | 2(3) | 56(15) | 1(1) |
| 131 | SARDH(i) | IIa | 0.92 | 163(119) | 144(76) | 227(44 ) | 7(1) | 13(5) | 5(3) | 34(8) | 6(2) |
| 132 | ARID3C(i) | IIa | 0.92 | 63(58) | 44(24) | 56(12) | 0(0) | 14(12) | 2(2) | 46(26) | 5(6) |

| | | | | | | | | | | | |
|---|---|---|---|---|---|---|---|---|---|---|---|
| Note: MePM and uMePM values: Mean (SD). T(HCC): HCC cancer tissue; T(Aj): adjacent tissues of HCC; T(NL): normal liver tissue; P(HCC): plasma from HCC patients; P(Con): plasma from cancer-free individuals including cirrhosis patients and healthy individuals. | | | | | | | | | | | |

The above description of exemplary embodiments of the invention has been presented for the purposes of illustration and description. It is not intended to be exhaustive or to limit the invention to the precise form described, and many modifications and variations are possible in light of the teaching above. The embodiments were chosen and described in order to best explain the principles of the invention and its practical applications to thereby enable others skilled in the art to best utilize the invention in various embodiments and with various modifications as are suited to the particular use contemplated.

## Claims

1. A method for genome-scale detection of differentially methylated CpG island relating to human abnormal states comprises: 1) Obtaining human DNA samples; 2) Detecting the methylation level of short CpG tandem sequences of the referred DNA samples in a genome-scale manner; 3) Comparing the methylation level of the referred short CpG tandem sequences between the referred DNA samples and DNA samples from normal population to identify the differentially methylated CpG islands relating to human abnormal states.
The referred DNA samples are derived from human cells, tissues, blood, body fluid, urine, excrement or their combination; preferably, the referred DNA samples are derived from from plasma or urine cell-free DNA.
The genome-scale detection refers to simultaneously detecting more than 100 CpG islands.

2. The method according to claim 1, wherein the differentiatial methylated CpG islands are used to determine the human abnormal state.

3. The method according to claim 1 or 2, wherein the short CpG tandem sequence is a nucleitide sequence of 7 nucleitides in length containing 3 CpG dinucleotides.

4. The method according to claim 1 or 2, wherein the short CpG tandem sequence is a nucleitide sequence of 8 nucleitides in length containing 3 or more CpG dinucleotides.

5. The method according to claim 1 or 2, wherein the short CpG tandem sequence is a nucleitide sequence of 9 nucleitides in length containing 3 or more CpG dinucleotides.

6. The method according to claim 1 or 2, wherein the short CpG tandem sequence is one the following sequences or a combination of the following sequences: CGCGCGG, CGCGCGA, CGCGCGT, CGCGCGC, CGGCGCGG, CGGCGCGA, CGGCGCGT, CGGCGCGC, CGCGGCGG, CGCGGCGA, CGCGGCGT, CGCGGCGC, CGGCGGCGG, CGGCGGCGA, CGGCGGCGT, CGGCGGCGC; preferably at lease one sequence is CGCGCGG.

7. The method according to any one of claims 1∼6, wherein the human abnormal state is cancer.

8. The method according to any one of claims 1∼6, wherein the referred DNA sample is derived from human plasma or urine cell-free DNA; wherein the differentially methylated CpG islands are nucleic acid sequences no. 1∼68; aberrent hypermethylation of those sequences indicates hepatocellular carcinoma.

9. The method according to any one of claims 1∼6, wherein the DNA sample is derived from human plasma or urine cell-free DNA; wherein the differentially methylated CpG islands are nucleic acid sequences no.69∼132; aberrent hypermethylation of those sequences indicates hepatocellular carcinoma or abnormal liver tissue injury.

10. The method according to any one of claims 1 to 9, wherein the methylated CpG tandems amplification and sequencing include the following steps:
Step 1, treating a DNA sample with a modifying agent to form the modified DNA wherein cytosine bases but not 5'-methyl-cytosine bases of the DNA sample are modified to uracil bases.
Step 2, providing Primer A and DNA polymerase to the modified DNA to allow at least one round of linear amplification to form the semi-amplicon capable of anchoring Adapter Primer C at one end, wherein Primer A is composed of a 3' portion and a 5' portion, wherein the 3' portion contains 4 or more nucleotides capable of binding to the modified DNA and allowing amplification, wherein the 5' portion allows Adapter Primer C to bind to its reverse complementary sequence for PCR amplification. Preferably, the 3' portion contains only C, A and T except for the CpG dinucleotide.
Step 3, amplifying the semi-amplicon by using Primer B and DNA polymerase to form the full-amplicon enriched with methylated CpG islands and capable of anchoring Adapter Primer C at one end and Adapter Primer D at the other end, wherein Primer B is composed of a 3' portion and a 5' portion, wherein the 3' portion contains the referred short CpG tandem allowing amplification and enrichment of the methylated CpG islands, wherein the 5' portion allows Adapter Primer D to bind to its reverse complementary sequence for PCR amplification.
Step 4, amplifying the full-amplicon by using Adapter Primer C, Adapter Primer D and DNA polymerase to form the final-amplicon via PCR exponential amplification.
Step 5, separating and purifying the final-amplicon to form the library for high-throughput sequencing, sequencing the library and analyzing the data.

11. The method according to claim 10, wherein a modifying agent is bisulfite.

12. The method according to claim 10 or 11, wherein the 3'-terminal 7 nucleotides of Primer A contains 1 CpG dinucleotide.

13. The method according to any one of claims 10∼12, wherein the second nucleotide at the 3' terminal of Primer A is C.

14. The method according to any one of claims 10∼13 wherein a unique molecular identifier tag sequence is located between the 3' portion and and 5' portion of Primer A.

15. The method according to any one of claims 10∼14, wherein DNA polymerase in Step2 is capable of strand displacement.

16. The method according to any one of claims 10∼15, wherein the DNA polymerase described in Step 2 is deficient of exonuclease activity

17. The method according to any one of claims 10∼16, wherein 2∼30 rounds of linear amplification are performed in Step 2.

18. The method according to any one of claims 10∼17, wherein the 3 'and 5' end portions contain a random sequence of 2 to 10 nucleotides in length; preferably, the random sequence contains only G, A and T.

19. The method according to any one of claims 10∼18, wherein the DNA polymerase described in Step 3 is hot-start.

20. A kit for the high-throughput sequencing method for detecting methylated CpG islands by the methylated CpG tandems amplification and sequencing method comprising the Primer A, Primer B, Adapter Primer C, Adapter Primer D and DNA polymerase according claim 10∼19, as well as an instruction for the kit.

21. A method for detection of hepatocellular carcinoma comprising the following steps: 1) Obtaining human DNA samples; 2) Determining whether one of the nucleic acid sequences no. 1∼68 or no.69∼132, or a combination of the sequences, is methylated; methylation of those nucleic acid sequences indicates the hepatocellular carcinoma; the referred DNA samples are derive from human cells, tissues, blood, body fluid, urine, excrement or their combination; preferably, the referred DNA samples are derive from human plasma or urine cell-free DNA.


### SEQUENCE LISTING

<110> Peking University et al
<120> Method for Dectecting Differentially Methylated CPG Islands Associated with Abnormal State of Human Body
   <130> JSP14PCT1598E
<140> 14908826.2
   <141> 26.12.2014
<160> 132
<170> PatentIn version 3.3
<210> 1
   <211> 1062
   <212> DNA
   <213> Homo sapiens
<400> 1
   cgggagtgga gtccggccgc ggacggcgtc ccttccaggg ccggccgcca gagggcgagc      60
   gcgccttccc gggcggggcg gggacgggac ggggacgggg aggggacggg acggggacgg     120
   ggaggggacg gggacacgga cacgggaggc ttccgctcgc ccgcgggtgg cgtctctgct     180
   cccactagag cgggccaggc ctctgggttc cgtttctcaa gcacacaggg agggtggccg     240
   cgggccaggg gcggccgcga gactccacgt ccacgtcccg ggcagtccct ccggaggttg     300
   ggccgcgcgg cccggggagc ggagcgctgc gcttctctgc gagcaccccg gggcctttag     360
   gcgcccccgt ttcctcgtca gtggggggcg cggtgccgct tctccgcggg ctaaactgct     420
   gaggaagggg cgagttcctg cggcccctgc ctgtcccggc cacgccccac ctccgccccg     480
   ccgcgttgcg cgttcccgcg gggccccggg cacccctggg gccccgggga tctctgacgc     540
   ccccttccct ccaacccccg ctgcgatccc acaggtgggg tgaacagcgc ccctgctgtc     600
   ccacggggcg tcttcgcggg tgatgcccac actctcagcc tgaagagcag gacctgtccg     660
   cgggtggggc ccacccccag gcttccggtg cccgcgcggc ctccagggcg cctcccccgg     720
   gcgcatcctc cgggcctggc agggagcgca gcctgggagt caggggcgac tggagcccac     780
   ctgctcagcg cccaccgcgg cggccctgcc ctttcccccg ggagcagcag ggacagcctt     840
   ggagcagcag ctcccgcagg gggaggaatg gcgcagtggc ccgaggctct ggagctggcc     900
   ccctgctcgc ccggggctac ctcggggaag cgcagaattc gcacctttct tctccagctc     960
   cacgcacccc tcatggaacc ttagcgaggc ccagggcagg ggatgctgcc aggccgctgc    1020
   caggctctga cgcggttgcg gatgcctcgc ccggtctctc cg                       1062
<210> 2
   <211> 2187
   <212> DNA
   <213> Homo sapiens
<400> 2
   cgcgcgcgga gcccgctgag acttgaatca atctggtcta acggtttccc ctaaaccgct 60
   aggagccctc aatcggcggg acagcagggc gcggtgagtc accgccggtg actaagcgac 120
   cccacccctc tccctcgggc tttcctctgc caccgccgtc tcgcaactcc cgccgtccga 180
   agctggactg agcccgttag gtccctcgac agaacctccc ctccccccaa catctctccg 240
   ccaaggcaag tcgatggaca gaggcgcggg ccggagcagc ccccctttcc aagcgggcgg 300
   cgcgcgaggc tgcggcgagg cctgagccct gcgttcctgc gctgtgcgcg cccccacccc 360
   gcgttccaat ctcaggcgct ctttgtttct ttctccgcga cttcagatct gagggattcc 420
   ttactctttc ctcttcccgc tcctttgccc gcgggtctcc ccgcctgacc gcagccccga 480
   gaccgccgcg cacctcctcc cacgcccctt tggcgtggtg ccaccggacc cctctggttc 540
   agtcccaggc ggaccccccc ctcaccgcgc gaccccgcct ttttcagcac cccagggtga 600
   gcccagctca gactatcatc cggaaagccc ccaaaagtcc cagcccagcg ctgaagtaac 660
   gggaccatgc ccagtcccag gccccggagc aggaaggctc gagggcgccc ccaccccacc 720
   cgcccaccct ccccgcttct cgctaggtcc ctattggctg gcgcgctccg cggctgggat 780
   ggcagtggga ggggaccctc tttcctaacg gggttataaa aacagcgccc tcggcggggt 840
   ccagtcctct gccactctcg ctccgaggtc cccgcgccag agacgcagcc gcgctcccac 900
   cacccacacc caccgcgccc tcgttcgcct cttctccggg agccagtccg cgccaccgcc 960
   gccgcccagg ccatcgccac cctccgcagc catgtccacc aggtccgtgt cctcgtcctc 1020
   ctaccgcagg atgttcggcg gcccgggcac cgcgagccgg ccgagctcca gccggagcta 1080
   cgtgactacg tccacccgca cctacagcct gggcagcgcg ctgcgcccca gcaccagccg 1140
   cagcctctac gcctcgtccc cgggcggcgt gtatgccacg cgctcctctg ccgtgcgcct 1200
   gcggagcagc gtgcccgggg tgcggctcct gcaggactcg gtggacttct cgctggccga 1260
   cgccatcaac accgagttca agaacacccg caccaacgag aaggtggagc tgcaggagct 1320
   gaatgaccgc ttcgccaact acatcgacaa ggtgcgcttc ctggagcagc agaataagat 1380
   cctgctggcc gagctcgagc agctcaaggg ccaaggcaag tcgcgcctgg gggacctcta 1440
   cgaggaggag atgcgggagc tgcgccggca ggtggaccag ctaaccaacg acaaagcccg 1500
   cgtcgaggtg gagcgcgaca acctggccga ggacatcatg cgcctccggg agaagtaagg 1560
   ctgcgcccat gcaagtagct gggcctcggg agggggctgg agggagaggg gaacgccccc 1620
   ccggcccccg cgagagctgc cacgcccttg gggatgtggc cggggggagg cctgccaggg 1680
   agacagcgga gagcggggct gtggctgtgg tggcgcagcc ccgcccagaa cccagacctt 1740
   gcagttcgca tttcctcctc tgtccccaca cattgcccaa ggacgctccg tttcaagtta 1800
   cagatttctt aaaactacca ctttgtgtgc agttgaaggc ccttgggcac aatgagagcc 1860
   agtcctccaa actttcagaa agtttcctgc cccttctggc aggctgccaa tcaccgggcg 1920
   ggagaaggaa ggaggggaag gcggtggagg gagcgagaca aagggatggt ccctcggggg 1980
   cggggatggc ggggctgtcc tgtaggtctg tgcggccacc gtgattgccc ctctgcgcgg 2040
   tgcccgaagt cccgctgaaa cctgccgagg gcagcaggtc tgaaagctgc aggcgctagt 2100
   tgcgcggagg tggcgcagct gctctggagg cgcagagcga atacgtggtg tttgggtgtg 2160
   gccgccccgc ccctggcggt ttcctcg 2187
<210> 3
   <211> 1314
   <212> DNA
   <213> Homo sapiens
<400> 3
   cgcgccctct tcaaacacac gcacactctt acactcactc gcgccgtccc agcctgccta      60
   ctcccaacga gtccaagccg gtgcagccgc gccccatgcc cggctcgcag ctcccgagcc     120
   ccagtccgcc cgcgctgggc gccgctgtac ctgttagtgg cgcgcggtac cgcggtcgcc     180
   gggctccgga gcgtcctcag cgctgcgggg gtgccgtggg tggcctccct gctttcctcc     240
   accagaatcc tgcacagccg gggagaaaac ttaagttgct acaccccgcc gggagctgtt     300
   ggctgaaagg ctgggagtcg cgctccttgc ctggcaggga gatctcgcac tccccggctc     360
   ccaggccagg tccccagccc cagagttgga agagccttag ggcgggaagg aagagacagc     420
   aaggaccaga atggggagca tgagatcctg atgcggaacc cgacggcagg tcccgctcca     480
   cagctgcctc gcccacagcg atgcgcgcta gagcccccgc agccctgacc ggtccctctg     540
   ctccccagcc gccgtccacc ggtcggaggg tcgcctccct gcgaccccct cccccactcc     600
   actctcaggc cgtcgccgcc cgtgggctgc tccccgccgc ggccaaaagt ctccgcgccc     660
   gaggggaagc gatgggcggc ttgggggtcc gggggcgcca ggcggggcgc gcggcgcggg     720
   gcgcggccgg cgggtgggtg ggttacctgg cgcggccggg cgcgtctgcc gggcaccaga     780
   gcaggcgcag cagcagcagc agcagcaggc tggcgagcag ggcgagcagt gcgccccccg    840
   gccgcatcct gagccacagg cgccgccgcc accgctgctg ccggggcgaa gcacagcccg     900
   ggcggccccg actcgcggct cccgccgccg ccgccaccgc cgccgtggcc gcagccccgc     960
   gcacccacag cgttcacagg cggcagcgag gggcgcccgc agcccacccg gcagagtctc    1020
   cgcggcgggc ggagaagaag ccgcgttcgg gctcgccccg ggctcccggc cccggcccgc    1080
   ggagcgccgc gatcggctgg cagatgacga ttcgccgagc tcgccgcctg tcctcccgga    1140
   ggcgctcgga gctgctgcgg gctccggtcg ccgctgccac tgctgctgcc gccgccgccg    1200
   cgcggggtca cctgaaggtt ggggcgcgga agctcaactc cgtgctgatt gggctccaag    1260
   ttttctgcgc cctcgcctcg tcccgagtgc ccgcgaatcc cccggacgcc cacg          1314
<210> 4
   <211> 2032
   <212> DNA
   <213> Homo sapiens
<400> 4
   cgcttctgcg gagaacccga gcctcctcct gggcgggcga cgctcggctg atgggaccgg 60
   gccgcgcaag cctcggatgc agccgggtgg ggggcaccag gcgctaggcg aaggagcgca 120
   ggaccaaagg agatgcttgg ctcctaagat aacaccttta aaaagaagaa gaagaaagaa 180
   agaaaaacaa acaaacaaac tcgatactta gcctcacgat ccttgccacc ccctccctgc 240
   tccgccaccc acacacaaaa taaagtgagc cgggcggggg agtgcgcgcc ctgaaaggga 300
   acaagaagca aggcgcctgt gacacaaaat ggagaaagag gaaccgggag tggaaaggaa 360
   gaaggaaaac cgagtaaagg aggttattca taagaacagg aaaggggccg aggggtagag 420
   aacacccgaa ggaaagcgca ggaggccctc gtgagagggc aagggggaaa agatcgacga 480
   agtaagagcg aaaagaggaa aggaaagaaa gactttctag atgcggagtc tccattttca 540
   gcagcgccat attcgctttt cctctcgccg cttttggctg ggttttgtgt gcggcggtgg 600
   acaggcgcca gcacatttct gcagcttcag cagctctgct cagacacctg gggagggccg 660
   cagggcccgg gcgggggacg cgcgctcttg gcccaggccc gggcgcggcc ccctccccgg 720
   cctggcggtt cctgcgcagc gccagccccg cgctcctcta ggctgcaaag ggggcctccg 780
   actcgggccc ggcccggagg ggacacctca ctcggggccc cgcggcagcc cccaggggac 840
   gcgggccctc tagtttcgcc cacggtgtgg ctgggcatgc gtgccaaggg gatgcgtaac 900
   cgagaaccga aaaagacgaa cttaaccttc ggggaggagg agagggcgca cgctgcgggt 960
   accggggctt gggggcgctc ctggaggtga cggggtcacc tccacctgcg cggggccact 1020
   ctctgggagc cggcgatcga tgcccgccgc ggcgcctggc ccaggccccc aggcctctcg 1080
   cggctcccgg aaaaacgtcc gaggtcgggg ctcggccggg agccccaagc gggtcccctg 1140
   gcctcgggac gccagtccgg gaggcggccg gaactccggt tttcgcggtt ctcagcgata 1200
   ttaggcgcgg ccagtgtctg aaagctcctc ggggttacgt cctggggcga ctggaggcgg 1260
   ctcacgactc agaaagtcgg ggcttctcgc cccggatccc caccgctggc gtctgtcgaa 1320
   tccccacctc ggtcctcggg tagaggcgca agcgcgcccc aagaccattc tcttcgcgcc 1380
   cacccccgtt attcttcact ttcacttgac atcgttcttc actcaaggtt aggtcctcgc 1440
   tagtgccaga gaagcccctg ggtccccagt gccctcagcc ccgcagcgta gggagcagcc 1500
   ggcccgggag ataaggaggc cacagccatg gaggccccag gcccggagtc cccgcgccgt 1560
   cgggttttga gccgcgcggc tggccaggac cgccggctcc ttctctgtcc cggtcctccg 1620
   ctcgcagccc cgcagaggtg acggtacctg gaggctggaa gcgacggttt cctcgggccg 1680
   gtggagggaa acatgtcagg gtcacagcga aggaaaccga gacccacagg ggtccccggg 1740
   cgtccgggac gcgtcgctgg ttttgctcct agaaggaaag gccttcccgc ttttgttcca 1800
   ccggctcacc aggtcctccc ttcctggcca actctaacct cagctttgtg cgtgtcaccc 1860
   ggccccggga taatcggatc ctcccagacc cgggaatcca tcagaagcgc tacccgtact 1920
   tggcgccctg aaagttcccc aagcgcagcg gccgcgccct cgagtgcatc cactccgcga 1980
   gccacgggcg gcgcctgccc acccgcggct cggctttgcg tgttgtaagc cg 2032
<210> 5
   <211> 1019
   <212> DNA
   <213> Homo sapiens
<400> 5
   cgagtgagga cgcagctgca gcaggacgta agcacagtca tcgctgcaaa ctgcaaactc 60
   gtaagcacag tcatcgctgc aaactgcaaa ctcgtgctcc gagcgctgcc ctcccctgtg 120
   gagcggagga ggggaggcct ggggccgcgg cggtgtgcgc cccgctctga ccgcagagcc 180
   cccttcccga ggaaagcggc tggcccggtc ccggctggtg atcacgcggg gcccctgtct 240
   gcttggtgcg caggtgaggg tctgcccttc cgctgcgccc cggacagcct ggaggtgagc 300
   acgcgctggg ccctggaccg cgagcagcgg gagaagtacg agctggtggc cgtgtgcacc 360
   gtgcacgccg gcgcgcgcga ggaggtggtg atggtgccct tcccggtgac cgtgtacgac 420
   gaggacgact cggcgcccac cttccccgcg ggcgtcgaca ccgccagcgc cgtggtggag 480
   ttcaagcgga aggaggtgct tgtccgcgcg tgctgtggtc tacccagtgt ctgtctccgg 540
   ccacagttcg tttctcggtc ggtttagtgt ccgtgtagcc acccaaccgt gtggccgacc 600
   attcgcgctt tcatttgtcc ttcgcctccg tctgcgccgt ctgtcctagg gggaggggaa 660
   gggggagtcc tgccagcacc cagctgggcc ttgcctcggg aggcaaggac caggacgagg 720
   cccgagggct cgcgtctggg gcatacttgt gccgctgcag gcgggcgcgg cgcgctgccc 780
   gggcggggag catctgccgg gagggcactc cctcccacca gcagttagcc cccaacggga 840
   gggcccttga gtgaccacga gcagagccgg ggattggaga aggacgggaa ggcggatcac 900
   ctccggcgcc gcccgccccg cccttctccg gctcgcgctg gtggagcgcg accgccacct 960
   gctgggcctc ggccttcctg cagccggccc acccagcagg ggccgtggga gagtgggcg 1019
<210> 6
   <211> 400
   <212> DNA
   <213> Homo sapiens
<400> 6
   cgacagcgct gaaaggggtg tgaccccgag gggaggcgac gcgctcccgc gccctagtcc 60
   acaggcatgc acgaccccac ccagcacagg gcctagggca tggggactgg ccctcttggc 120
   tgaaacgact ccgaccctct cggaagatgc ccgcgcggcc tctgcccccg gggagagggg 180
   actgtgcccg atgctcaggc gccgcgcccc catcctccaa tcccccagaa cgcaccacag 240
   gccaagcccc tcctgcgcca gccaagactc cgcgccagcc cacaccagcc ccactcagac 300
   cgctggaccc cgcccccggc ccccgcacca cccagcccgc aactctgcag ctttcttcgc 360
   ccacgccgca cagcctccaa accagcggat tccgcccacg 400
<210> 7
   <211> 609
   <212> DNA
   <213> Homo sapiens
<400> 7
   cgcggagagg gcgcttagtg cgtgagctac cgtcgttccc cgcgactcag ccgagcggca 60
   tccgcacacc ctgctagcct aggaagcgca tcgagggagc ccgctcaagc tgaagcgctg 120
   cacgagctgt gtcttccttc tctccgccgc gaatgcgggc aggatgcgag caaaagaccc 180
   ggcaaagagg gcgccgaggg ggcgcagccg gggcgctggg cggacccaga ggggacatgg 240
   agaggaacgc ggaaggtacg cggtagagac ggggaggtgg cgcgaggcgg gacatggtga 300
   gcgacccgag gggcgcgcgg actgggctcg cggacggggc tcgcggacgt agagcagtcg 360
   aggcgcggag ggggagcgcg cccccgtgtg gccaggcggg gaggcgccac ggccggggcc 420
   gtgggggcgg tgggaagcgc tccgcgtccg ggctctcagc gttccctggc ctgccccggc 480
   tcgctaggct ttgatggctg cttccgctcc ccataaaggc ctgtttcatg gccgacgccg 540
   gcatgggctt tacgtctgcc ctcgggctcc ctgcgggctg cagagggcag cggtgaaatc 600
   ccctgtccg 609
<210> 8
   <211> 724
   <212> DNA
   <213> Homo sapiens
<400> 8
   cgtacagtga atgcgtctgc gcacgtacag tcctcgtgca ccctttttct ctctctggac 60
   agaatttctt ggggatcctt cgccccccac ccccaccccc ggccgtattt ctccctaggc 120
   tcttattaaa accaggtgca cggaaggcgg cccattctgg acagaaaagc tttcgtttga 180
   cccaaagctc ccccttccgg cgtgttttct agagggaatt cgtgagctga gccgaggagc 240
   gagcaggcgg cttctcctga ccggggagac gcgggaggtt tgcaaacaga ccttggcctg 300
   ggctgcgcag ggacaaagtg ttcaagggca gcaggggagg ctgagggagt acacggctgg 360
   gaacaaggga cactgccgcc cccagccacg caggagcggt gtctgcacca ctgaaagtcc 420
   cccggggccc acagctctat ccaggcggaa gggaagcagc caggccgccg cggatctgaa 480
   cttgaatccc gggtggaccg gacgccccgg ccggcgctcc tgacgcgcgg aggagctggc 540
   gagaacctgc agatctcagg gctcgcccgc ggtccggcgc tcggttcagg acgtccggga 600
   accccgcagc gcttaccctc tgcagcaggc gtcgctggcg cctccaaggg caggggcggg 660
   tccggcgggt acaagtcgcg attgacgcga cggcccaggc aggcactgtg ggcttcaccg 720
   cccg 724
<210> 9
   <211> 923
   <212> DNA
   <213> Homo sapiens
<400> 9
   cgacgcctgc gacatgacca tagaggagat ggagagcgcg gccgacaaca tcctcagcgg 60
   gggcgcccca cagagcccca atggcgccct cttacccttt gtgaactgca gggacgcggg 120
   gcaggaccga gccgggggcg aagaggacgc gggctgtgtg cgcgcgcggg gtcgaccgag 180
   tgaggaggag ctccaggaca gcagggtcta cgtcagcagc ctgtagtggg cgctgccaga 240
   tgcgggcttt tttttatttg tttcaatgtt cctaatgggt tcgtttcaga agtgcctcac 300
   tgttctcgtg acctggagtt aaccggaaca gcgtcttcat tcatttctgt tgggaccaga 360
   cgcggagcct gggtgcgcga gccgccctcc gggaggaagg cgcccggctg cgtctgcaga 420
   ggcggggaga ggaggcggcg agggtcccgg ggcgcgagga aggcgcctgc cctctcccag 480
   ctcgcaggcc ccgggcccgg ccgcgcctcc gcggggagag caccccggct tcccgcgcgc 540
   cctcaccaaa aggaccctac agcaaacggg tgtctttcga ctctgcttgt agaaaccatt 600
   tgcacatatt ctgtacgagc ctcgctgtct ccctagagcc agggccctgc ggatttggag 660
   aagggagcgg ggcaggactt ccaggaggac cccaacccgg cccggagagg gaggaggagg 720
   cctccagggg cgcggagctc tggggatggg cgtcgggccg gcagtggtgc ggctcactcc 780
   gtccctgccc acctgcgacg ggatcccccg accggcacgg gccacgccga gctcccggcc 840
   agccgccggc ccgcaggcag cgcgagggag gagctgcgcc gccggctccg cccaaccagg 900
   tggtgctgag cttccgctga gcg 923
<210> 10
   <211> 920
   <212> DNA
   <213> Homo sapiens
<400> 10
   cgatgctgtc gcccccgcga cctgtagggc cttcgctggc ccctcaccct cctcccgccc 60
   cgggctgagg ctgccgccgg tttcgcgccc agtgccgagg cagcggcgga gccggggaag 120
   gtcggaggga gggtggtttc ctcccgcccc acacccagtc tccgagccgg atatatagag 180
   tgtcacgttt gggagccgaa agactggagc cgtttccttg tggctggagc gcttcccgta 240
   gcctcgggga aggagcagga tttagaggac cactagttgg accccatcct cgtgctggag 300
   gaacaggtat tccagggtcc ccagccctgg cgctcgcaga gcccacgcgc tccaaaagca 360
   gagaccggac tgggggtggc ccaggaggga gggctcggct gggttcgcag ggcggggcgg 420
   ttcccggcgg gacctcgagg agggtgggtg tgaggccggg tgggaggaga gaagcatttg 480
   cggaggctct gccccctgaa gctgcccccg gctgcacccc aatcgtcacc ggccgtttac 540
   gcgcggttgg cgtcggcgga gcccgccaag gtggggcgcg cgggggttgc aaagggcaga 600
   aagccgggaa ggacccagca aggttaatgt ggggggccgc gccttgggga ctggggaccc 660
   gggaagagag gcgcggggcc gagtcccacc caggcctcct gggcccttgg ggctattcct 720
   ggagagagcg cgaaaatgcc ggggccacgt gtgaaacggc agcggtccta ggtcccctcc 780
   cggtgcctgg gaccgccagg gcgcccagtc ctggtccccg gagcaggtcc tcgccagccc 840
   ccagccccac cccaggctgc gcacgtccct accctcccac ctgggcctcg ccggggtcgt 900
   ggctgacgcg cgctggggcg 920
<210> 11
   <211> 538
   <212> DNA
   <213> Homo sapiens
<400> 11
   cgagttccat gtgcggcaga cacgcgctca cacttcgcag ccggaggagc aggcaggaag 60
   cgccgccccc cggccgcccc ggccgcccac cctcaacctt cctccacgtt accttgggcg 120
   tgtaggcttc ccgtggacct gcccgcgccg cccgctcggc tctcagctgt caagcagccg 180
   cctgctccgc ccgactcttt ccgctccgag ccgcgcgggc tggaccagcc cctcagcgga 240
   gctgaacggg aagcggcgcc ccctggacgc cctccgggac gtgcgcgcgc caagcggtgg 300
   ggtccgggcc tgcaggcgcg acctgaggcc gccagggagg ctgggtggcg agggcggggc 360
   ggctgccgac ttagccggcc tgactggtcg ggcttccggg gtcccgctgc tccctaggcc 420
   ccttgccccg attcaggctc ccaaggtggg gcatcagtcc ggccctcgct ggctcactgg 480
   agaggtggtg tctgggagtg cacccccgaa aacccggcag cagcccggcg tcactccg 538
<210> 12
   <211> 649
   <212> DNA
   <213> Homo sapiens
<400> 12
   cgaggccaac gggacggggc tggagctgct gggcggcccg tcgctgcgca gcgcctacat 60
   cacctccctc tacttcgcac tcagcagcct caccagcgtg ggcttcggca acgtgtccgc 120
   caacacggac accgagaaga tcttctccat ctgcaccatg ctcatcggcg gtgagcccgg 180
   ccgcgcgcgt cttcccgggg ccactcccag acttctgccc gcaggcggcg ccgtcccact 240
   ccccggaggg cctgaggccg ggggctcttc cgctttaggt ggccgcggaa cccgaggccg 300
   ggggtggggg agacttcgcc cgcacagcgg ccttcaggcc ttgcctagga gcgtgcgggc 360
   cgaggacaga tgagcccagc gtggtgtcag gccgggccag gtcccctagg tgaccccctc 420
   tcgtcaccct cccccagccc tgatgcacgc ggtggtgttt gggaacgtga cggccatcat 480
   ccagcgcatg tacgcccgcc gctttctgta ccacagccgc acgcgcgacc tgcgcgacta 540
   catccgcatc caccgtatcc ccaagcccct caagcagcgc atgctggagt acttccaggc 600
   cacctgggcg gtgaacaatg gcatcgacac caccgaggtg cggcctccg 649
<210> 13
   <211> 1229
   <212> DNA
   <213> Homo sapiens
<400> 13
   cgcagccccg agccccgagc cccgccccgg gggcctcatc ccgccccttc gcgtccgcgg      60
   ctcgttttcc cccactgagc gcccagctcc cgcagtttcc ccggccgtcg agcgccgtgg 120
   gcggggctcc agggcggcgg cgcctcgcgg ggagggtcct ccgtgctggg ggcgaggcca 180
   cccgaggcag ctccccgccc gcccccaacc ccgccccgct ctcggagcct ataaagggag 240
   gcgacccgcg gcccgcccgg ctggcatccc ccagccgccg ccagccccgc cgaggggagc 300
   cagcgccgtc tctgaggggc gtccggcgcc ggagccatga ccctccgccg actcaggaag 360
   ctgcagcaga aggaggaggc ggcggccacc ccggaccccg ccgcccggac tcccgactcg 420
   gaagtcgcgc ccgccgctcc ggtcccgacc ccgggacccc ctgccgcagc cgccacccct 480
   gggcccccag cggacgagct gtacgcggcg ctggaggact atcaccctgc cgagctgtac 540
   cgcgcgctcg ccgtgtccgg gggcaccctg ccccgccgaa aggtgcgtcc cccgcccgcc 600
   ttcaggatct gctcagcccc tctccgactc cctacagggc ctgctgactc cgcagtgccc 660
   tctcctcggc gtccgcggag tcccccacct tcttccccgg cccgctgggt gcctcgactc 720
   cccgcgttcc ccgctgctgc gaaggccgtg gccctcgcct gcacaccgcg cccaggctcg 780
   gtggctctta actccgcgcc ccatgcacgc cccctctctc cctccttgac tcctcccagc     840
   accccccttc tcctacccgc tccatctggc tttctgcccc ccatgccccg cctccccgtg     900
   gccaggtgtc ctgggtcccc aggagcccct cgcccgaggg acagagacag ccccaggcaa 960
   gttgaaggtc cgagagcccc cggtgggaga agcgggccgg tggctgcgcc gcgtgcgttc 1020
   tcactctgag gaagtgcgtg gggagccgct gactccggat agcacaccct tccgagggga 1080
   ctccccgatt cctgggctgg gggcctgccg cctggcccca cgtctgacgt acggggcgcg 1140
   agggccactg ctccctggac ttctgtcgga accggacgca gtgggagggg tcgcagggcg 1200
   cccgcggggc aggaaggatg cgggccgcg 1229
<210> 14
   <211> 2375
   <212> DNA
   <213> Homo sapiens
<400> 14
   cgcaccacgt tctttttgag gtccagtttc tcggcgatgg cggcgatctt ctcggacgag 60
   ggccggggct gcacggcgaa gtaggcctcg agggagcgct tctcgggcgc ggcgatggaa 120
   gtccgcttgc gcttcttctc gccgccgttg aagagctcag gcttgttcat tttctcgcgc 180
   tgggcgccct cggcctcctc gagccacgcc tgcaggatgg gcttgagcgc gatcatgttg 240
   ttgtgcgaga gcgtgagcga ctcgaacctg cagatggtgc tctggctgag tgagcccacg 300
   cccgggatct tgaggttggc cagcgccgag cccacgtcgg cctgcgtcac gcccagcttg 360
   atgcgccgct gcttgaagcg ctccgcgaac gcctcgagct cgcgcgggtc cgtgtccgag 420
   tcgcagatgg acgccaggcc cgctgcgccc accacggccg ccgccgccga tgccgctgcc 480
   acctgcccgg ccgccgccgc cgccgctgcc gctgccgcgc cgtggtgcgc cgccgccgcc 540
   accagcccgg ggtgcggcag cccggacggc atgttcatgg cggccgccgc cgcggggtgc 600
   gacaggtggc ccaggctgtg catatgcggg tgagggtgcg cggagccgcc caggagcccg 660
   ccgcccgggc cgccgccgcc gccccccggg ccgccaccgc cgcctccccc ggggccgccg 720
   cccgggccgc cgccgccgcc cgggccgcca ccgccccccg ggccgtcgtg ggcgccgccg 780
   ccgccggccg ccgcgcccgc gccgcccgcg ccggccatga gcgcgagcga cggcgaggag 840
   atgtggtcca gcagatcgcc gggttcgagc gcctggtggt ggtggtggtg gtggtggtgg 900
   tgcgccagag gcaccgtgga agtggacgtg cacggcacgc tgttcatcgt gtggtacgtg 960
   gcgtccggct tgaaaggatg gctcttgccc tgggacacgg cgatgtccac ggccgccagc 1020
   gcctcggccc gcgccagcag cgtctcgtcc aggctggcga agaggttgct ctgcagctgc 1080
   aggcgacaca aaccaaacca aaaaaaccac aaaaccaaaa gagcaaaaca aaacaacaga 1140
   agaaacacac acacaggccg gaaagcacag catgcgaagg gcaaacacaa agcaaccaaa 1200
   ataacaacgg gtttgggggc agtggagagc gggaaagacg gagagggggc acattgacga 1260
   ccagggaggg ggcagacgag aagggatggg agcgtggaga gggggacaga agtagggaga 1320
   aagggggaca caagaacaca ttccggaaac gggcgtggga gacgaaaaag agggaaaaga 1380
   agaaatggaa atgtaactcg cagctgggga cccgtgtcac acacccgagc acgcacagag 1440
   actgcctttc tgaggcgtga aattaacaga gaaagtagag ggaagtcgag agtcctggcc 1500
   ccagcgctcc ctactccgat cgcaggcgcc tgacgcggag gcagaggcag acaggaacac 1560
   gacattaagg gccgccggac aaaacgtgcc tcgctgcggg actgctctac accccctgcc 1620
   cgcagttccc cgcaccggga cctgcacaca ccagcccccg acatgcagcg tttcggagac 1680
   ggggaggggc gggcgcgcgg gccggggccg cgggcgtggg gcgcttaccg gcggcgtggg 1740
   caggcaggcc cgccggatgg cctcggagct ggagtgcagc gacgggtact tgtgctcagg 1800
   gagggtggga tgcatggcaa agtgaggctg cttgctgttc atggacatca tcgtggcggc 1860
   ttggcatgta tatccacaaa cactccgaaa gtccgcggga aagtgcgtac gccggctcac 1920
   ccggcctccc ttcggaggct gcagccgcgg cggtcgcggc ggctggcggc ggccccgccg 1980
   cgggctgctg ctgctgctcc tgctgctgcc aggcgctccc tctgaccgcg cagagcgccg 2040
   cgcgccggcc tcgcggtccc gcttctccga cagctctagc cccgcgcgcc gacgggatgc 2100
   actcctctaa cacctgagcc ccacttctcg cggccgtccc ggggagctct cgcgagagct 2160
   cgcggcccca ccgcgctgac aggcatcagc tgtctccgtc tgtctgacgc gcgctctcct 2220
   tctcggcggc cccgtgcgtc tgcgcgcgcg cgcgctcgtc cggccgcgac cagcgcgtgg 2280
   gagccgctct tatagtgacc accagcaagg acagcgcagg tgatgcacct gtagctaccc 2340
   gggcatgcgc actgcccgcc tcacctttcc caccg 2375
<210> 15
   <211> 723
   <212> DNA
   <213> Homo sapiens
<400> 15
   cgactcccgc aggttagcgc tatctcgggc accccgcagg gagagaggac acggctgccc 60
   cgcgaaaaca ctctctggca cccgtgcttc caggacaggc cctgaggcct ctcccgggga 120
   agcagttaca cctgttctcc ggggctcccc tgaagacccc aaaaacaccc aacacctgcc 180
   tccccaggaa agacgctctg ggcccgatct atgccgcccg gccgccgggc ctgagtgaag 240
   cctgtccagc ctaggagggg ccctcagaac ctcccggtcc aagcctctca cttcgtccac 300
   ccctgcggag acggggcgag ccccgcaccc ttcccgtggc ccggccgcac cgccctgtgc 360
   ccgtggtggc ggccacacag gccagcctgg cgagtactca cggcgacccc gggccgcgcg 420
   cggccgcagc cgacgggacg agcggcccgg gacgccccgc gggggcggag cggcaggcca 480
   aggggcgggg gccgggcctg gcgggcgagg tcgcgcaccc cacgcctcgt cgttccgtca 540
   cgggcgcgct cagccactct gcgcacgctc gcgcggtgac acgatggcac gtggcccgct 600
   cctgaccaac agcctgacgg cctgctcctg ggcgcgcctc agcagccgac gccagccttg 660
   agacgcctgc gcggcggtcg ctagtcggga aagggtgcgg agtggggggt ggcgcctgcg 720
   ccg 723
<210> 16
   <211> 3253
   <212> DNA
   <213> Homo sapiens
<400> 16
   cgacttcttc cggagggctt cgctcgttcc gagggtcctg cgactggagg aggcacatcc 60
   ctgagcagcc actccctccc cgcagcggcc tgtcgccgag ctcccgcgcc ctgcttgagc 120
   tcgggagaga tcgctgcggg gcagtccggc ccgggagcga gagaaagaac gaaaatgcac 180
   agctctctcc gagtaaggcg aacagatgga gaggaagacg aacaattaaa cgaaacaaac 240
   agaataaaac agaaacgaaa tcgggccccg gggggctggg ccacccggag gttgggctcg 300
   gggcgggtgg gtggcggccc ctcgctaacc tctggcctca ccctgtcctg gcagacgctg 360
   ggtcgggccg gcagcccgcg ttgcgcccgc acgtgcacaa gcagacggag aagacgaccc 420
   gcgtgcggac tgtgctgaac gagaagcagc tgcacactct gcggacctgc tacgccgcca 480
   acccgcggcc cgacgctctc atgaaggagc agctggtgga gatgaccggc ctgagcccgc 540
   gggtcatccg cgtctggttc cagaacaagc gctgcaagga caagaagaaa tccattctca 600
   tgaagcagct gcagcagcag cagcacagcg acaagacggt gagcagccgc tgggccggag 660
   gctcgagtcg ggtgggggct gcgcttccgc cgcggtatct gcgtgccctt ttctgggcga 720
   gccctgggag atccagggag aactgggcgc tccagatggt gtatgtctgt accttcacag 780
   caaggcttcc cttggatttg aggcttccta ttttgtctgg gatcggggtt tctccttgtc 840
   ccagtggcag ccccgcgttg cgggttccgg gcgctgcgcg gagcccaagg ctgcatggca 900
   gtgtgcagcg cccgccagtc gggctggtgg gttgtgcact ccgtcggcag ctgcagaaag 960
   gtgggagtgc aggtcttgcc tttcctcacc gggcggttgg cttccagcac cgaggctgac 1020
   ctatcgtggc aagtttgcgg cccccgcaga tccccagtgg agaaagaggg ctcttccgat 1080
   gcgatcgagt gtgcgcctcc ccgcaaagca atgcagaccc taaatcactc aaggcctgga 1140
   gctccagtct caaaggtggc agaaaaggcc agacctaact cgagcaccta ctgccttctg 1200
   cttgccccgc agagccttca gggactgact gggacgcccc tggtggcggg cagtcccatc 1260
   cgccatgaga acgccgtgca gggcagcgca gtggaggtgc agacgtacca gccgccgtgg 1320
   aaggcgctca gcgagtttgc cctccagagc gacctggacc aacccgcctt ccaacagctg 1380
   gtgaggccct gccctacccg ccccgacctc gggactctgc gggttgggga tttagccact 1440
   tagcctggca gagaggggag ggggtggcct tgggctgagg ggctgggtac agccctaggc 1500
   ggtgggggag ggggaacagt ggcgggctct gaaacctcac ctcggcccat tacgcgccct 1560
   aaaccaggtc tccctggatt aaagtgctca caagagaggt cgcaggatta accaacccgc 1620
   tcccccgccc taatcccccc ctcgtgcgcc tggggacctg gcctccttct ccgcagggct 1680
   tgctctcagc tggcggccgg tccccaaggg acactttccg actcggagca cgcggccctg 1740
   gagcaccagc tcgcgtgcct cttcacctgc ctcttcccgg tgtttccgcc gccccaggtc 1800
   tccttctccg agtccggctc cctaggcaac tcctccggca gcgacgtgac ctccctgtcc 1860
   tcgcagctcc cggacacccc caacagtatg gtgccgagtc ccgtggagac gtgaggggga 1920
   cccctccctg ccagcccgcg gacctcgcat gctccctgca tgagactcac ccatgctcag 1980
   gccattccag ttccgaaagc tctctcgcct tcgtaattat tctattgtta tttatgagag 2040
   agtaccgaga gacacggtct ggacagccca aggcgccagg atgcaacctg ctttcaccag 2100
   actgcagacc cctgctccga ggactcttag tttttcaaaa ccagaatctg ggacttacca 2160
   gggttagctc tgccctctcc tctcctctct acgtggccgc cgctctgtct ctccacgccc 2220
   cacctgtgtc cccatctcgg ccggcccgga gctcgcccac gcggaccccc gccctgcccc 2280
   agctcagcgc tccctggcgg cttcgcccgg gctcctagcg gggaaaagga aggggataac 2340
   tcagaggaac agacactcaa actcccaaag cgcatgattg ctgggaaaca gtagaaacca 2400
   gacttgcctt gaaagtgttt aagttattcg acggaggaca gagtatgtga gcctttgccg 2460
   aacaaacaaa cgtaagttat tgttatttat tgtgagaaca gccagttcat agtgggactt 2520
   gtattttgat cttaataaaa aataataacc cggggcgacg ccactcctct gtgctgttgg 2580
   cgcggcggga gggccggcgg aggccagttc aggggtcagg ctggcgtcgg ctgccggggc 2640
   tccgcgtgct gcgggcgggg cgggcccggt ggggattggg cgcggccgag ggggtggggc 2700
   tgggcgcatg cgcgctgagg gccctcccag aagcaggagc gccccattgg cgacccctgg 2760
   gcacctggcg gcccaggccc gcgggcagga ggcgggaccc gtttgggcgg gaaacctgcg 2820
   gcacttgtgg cggttgagcg gcggactccg gcctagggtt atgcgggcga gggcgcgccc 2880
   cgctcccgga accagccttg gcctggcgcc tgccgagccc cgagccccac tctcgctcgg 2940
   cgccgcgggc cctcccggcg cttccttccc cagcggtccg cggccctggg tgaggctcga 3000
   agccccgcgc gctgcccctg gccctgcagc acagcagtga ggccaacccc ggccagagcg 3060
   ggcggccgtg acgggccgtg ccccgcgctg tccccggagt ggatgcgcga cgtgatcaat 3120
   ccgcggcatt tattaattcc acagtctaga cagaggcggg ggccgggcgg cggctgctta 3180
   tctgcgtttg gcatcacctg caagcgatac accgaggggc cgcaacgagc ccccgctagg 3240
   agcgcggctg ccg 3253
<210> 17
   <211> 1061
   <212> DNA
   <213> Homo sapiens
<400> 17
   cgccgatggc tactggagcg gagaggttcc gaggtttcag gggcattttg tcctttccca      60
   cttctgggcc gtgggctccc ccatagacga tcccgcgggc ccttcctcct ctgacaccct     120
   cggatctggt tccacctttc ccggaccgca gctcagcccc gcgcctctcc agctcttcga     180
   tgaccggttc ccggccgtgc cccgggccct ccctcggagc gcgcgccgcc aggggcacct     240
   gtccctgcgc gggggtgtcg ccgcccctcg gcgccgccgg gcgcttgccg ctgagccgtg     300
   gcgcccctgg caggagcact gcaaggacgc ctcccgggac tgcaccgcgg ccctgcgcac     360
   ttggggcgac ggggccaggc gcgggggacg gctgcgggct gggagggcgc gcggaggaga     420
   caccgctgag gggacgccgc tgagggcgcc acgcgggggg cgcggctgag gcgcctcgaa     480
   gggcaagcgc caagggggcg cgggcgccgc cggaagctgg ggcggggcgc ccagcgggat      540
   gcggtgaagg gcgagcggcg cggcggctgc gatgagtgcc tctgcggcca ccggggtctt     600
   cgtgctgtcc ctctcggcca tcccggtcac ctatgtcttc aaccacctgg cggcccagca     660
   tgagtgagtg agccggcgcg gcgggggtcg cgccgagggg cggcgggagt tggctcgccg     720
   cgacgggagc ctcgcaactt ttccgagggg gctgggaccg tccgccgcgg gacagaggtt     780
   cgtggccgca ggggctcccc gcgcctggcc agactagggg ggcgccccag gggtcgcacg     840
   ggccgggtct tggagccggg ccctgaggtg cccaggctgg cgcatttcgg gatgttggtg     900
   ccagcaccgc gccaggtgcc cggggtcaca ggcgtcaata cggccatgcc cctgccctgg     960
   aggggcccag gggccggtgg ggagcggaac aaacacggac actacgaacc tggccgtgtc    1020
   agctcgtttg tgccttgttc tttttgcatc tcacgggaac g                        1061
<210> 18
   <211> 1161
   <212> DNA
   <213> Homo sapiens
<400> 18
   cgcagaggcc ggggtggaat tcgctgcgcc ccaccccttc cacccgctcc ccttcgcccc      60
   gtaggtcttt ccactctcgc tcctcccctg ggcacatctc ctgaacgcag ccccgggggc     120
   cgaggacggg gtggggtggg gggcgaggct cgggtccgac gaccccgggc tgcggtcccg 180
   gcgctgcaga gctgcggctg tgcacgctta gccgcgaggc ccgcggtagc ccgggcgccg 240
   atatgtaaag cagctggcag cgctgggcgg ggcctgggcg cgatgcaaat gaggagggcg 300
   gggctggccc ggggctccgc ctccctcccc cgcagctggg gccagcggtg ccaagcgcag 360
   ctggacgagc ggcagcagct gggcgagtga cagccccggc tccgcgcgcc gcggccgcca 420
   gagccggcgc aggggaagcg cccgcggccc cgggtgcagc agcggccgcc gcctcccgcg 480
   cctccccggc ccgcagcccg cggtcccgcg gccccggggc cggcacctct cgggctccgg 540
   ctccccgcgc gcaagatggc tgacccggct gcggggccgc cgccgagcga gggcgaggag 600
   agcaccgtgc gcttcgcccg caaaggcgcc ctccggcaga agaacgtgca tgaggtcaag 660
   aaccacaaat tcaccgcccg cttcttcaag cagcccacct tctgcagcca ctgcaccgac 720
   ttcatctggt gagcgcgcgc gcgcagggca ccttcccggg cccccgaggg cagcgccgcg 780
   ccagggaccc cctctccgcg ccctctgcgc cctccgcgcc ctccgcaccc tgggaccccg 840
   cgtctccgga ctcccggctc cggaccctgc tgcccgggac tcccggatgg acagtcctgc 900
   cgttgccctg tccccaccct ggtcccagac gggccgccgc ggggcgcctc ctgccctctc 960
   ctgctctcag gcgcctctag agcgcccagg ggcggcgtcg cgggagcctt tgctccacct 1020
   gactaggagc gcgcggggtc tgtgcctgcc ctggagggca gcgcctcggg tgctctccga 1080
   cccggggttc cctatctctc cgcctgcttc cgggcgcgag gagccctcgc cccccacccc 1140
   ttgtttccgg ggggggcggc g 1161
<210> 19
   <211> 1433
   <212> DNA
   <213> Homo sapiens
<400> 19
   cgcgggatcg cagtgtttgc ctttgcatct ccagctccgc atcctgctcg ctgtttattt      60
   tgtgagcagc tccggcggct gcccctgaac tgctccgaga ctgagcagtt taactcacac     120
   tcgcccaccc gccctcccca ggaagcctcc cgcccgcttc ctccagttcc ttccccacct     180
   gtccggcctc ctagcccggt gctctcggcg cgggaggagg cggctcgcag acccccaggt     240
   ctcggcttcc cggatctccc ccgcctactc actccccggt ccccggctcc cggcactggc     300
   aggcgctgtg gcttccgcgc agtggctgca gcatttaaat gtccggcact tagggagagg     360
   ggtgcagtcg cggagcagcc gctgctcccg gcatctagag gggcccaaac ccccgcgtgc      420
   ccaggccgag ccccgcgcgc agcccacgtg ggctgaggga ctgagtattg gccctcgggc     480
   agctgcaacc gcgcacagct ggagctggcg gggcggaggg cgggccggga ccccgcgggg     540
   aaggaggctt ccttcccagc ctcctgctgc ccggaaatcc ccccggctct cagacaacct     600
   gccccacggg gtcctggggg gaaggagagg ggcgggatac agggatgggg agggctggct     660
   gagccgaagg cccgggccgc cctcccccag gccgcgcggc ttcgagaaag cagaagcgag     720
   ccctgccccg gggagtccca caggagcggg gatctgggaa gagaaacttg cggacgaggg     780
   agttagagcc gtggaccccc ggcccccgcc cgggcgtcct gcgtcggagg agggtctctg     840
   ggtcgggcag gggccacctc ttggcccgcc ccttgtcgct ggacgtccga agattgggcc     900
   atttcccttg ccgcgccctt accgcatatc ggggtgcgcc cggcccggcc cggcccgccc     960
   cacccagccc tccgctcgcg cccggagagg aggggccgct ggcgcagcgc cccgggaccc    1020
   cgagaggccg ccgcggcaca tccagacctc cgccgctccc gcgccctctc aaccatcctg    1080
   ggattcccgg gcccacccga cccagcggcg cgaccctggc cctccgggac cctccgctga    1140
   ctccaccgcg cacttcccgg gacccccaca cacatcccag ccctccggcc gatccctccc    1200
   tactcggtgc cgggtgcccc ccgccctctc caggcccgga tctcctcccc caggtccccg    1260
   gggcggcccc agccaggccc ccttcgaacc ccgccggcgg cccgggctgg ggcgcaccat    1320
   gcggctgcgg ctccggcttc tggcgctgct gcttctgctg ctggcaccgc ccgcgcgcgc    1380
   cccgaagccc tcggcgcagg acgtgagcct gggcgtggtg agcgcggggt ccg           1433
<210> 20
   <211> 1118
   <212> DNA
   <213> Homo sapiens
<400> 20
   cgagacacac gagaccactc ctgccgggga aaacccatga gggggtgggg gcggccgcgc 60
   cgcagggagg ggccctctgc tgtcccagcc aacgtcccat gacgattttg cacccccgcc 120
   agcagtgccc tcggctggac acacctgcgt tgtgggcagc cggcagcgcg tgacccactg 180
   tgcacctgcg ccttgatgta ggggggagac cctgcagaga gcgcttcctt ccgccctgtc 240
   cagtgccctc cctgcctcac ccctcttccc gcaggcccgc tcagggcgct gaagggctcc 300
   tgaccgtggg tccgtccttc gctgcgtctg gggggcgtcc cccgcgctgc ctctgacccg 360
   cccggccctg cgtttgtctg aagcccctcg ttccgcatct ccggaccagg gtccgggggc 420
   gtggtctgct gggggcgggc ccgggttgtg gtttgagagc cgctcggcct gtctgggcct 480
   ccgcctccgg gtcgccctcc agccttcctg ccgccgcttt tccagtcttg cccaacccgg 540
   tatttctgcg cttgcctcag ccacccgcag gagcgcaggc cgcccttcgt cctcccgtcc 600
   tctgcgccca caggaccccg ggccccgccc agtccgcagg tcccgcaggt ccccccgggc 660
   gtgtcttcct ggccctgcga cccccgggac agcgctgaga acacgcggaa ggtggggaga 720
   cgcgcggcgc tggccgggtc cctggcgccg ttctcgcggt gcagcctgtg cctgggagtc 780
   cggccgtccc acctcagaac cagagcaaag cgggagatgg ggcggcggcc gctggggaga 840
   aggcagcccc gggtcggagg aggaaggagg cgggggcgga gcggagtccg cggtggggct 900
   gccttcttcc atcctcccct gggcggggcg caggatgcgg gggcgcccag ccgagggagg 960
   gaggccgggg aggacactcc tgtgcccgag ggctccggct tcttctgctc actccctcct 1020
   gccgaacttt tccactcggc catttacctc ctcaggcact ggctgccctc atcaggccga 1080
   ccaggtcctg cccgagggaa cggcgtgtgt gacccacg 1118
<210> 21
   <211> 580
   <212> DNA
   <213> Homo sapiens
<400> 21
   cgcacggaga cctccaggca agcaggatgc gcgctcgccc tcgcttgatt gtttgctccc 60
   caggatgctg gggaaagcgg ctgctgaaag agcaaacttt ctgagcgttc gcggaggcgg 120
   agggcgcgcg gcggactcac agctggttga tggcgttctg cgagatgacc gcgttggcgg 180
   agaagtaagg aatcatgtcg gggccaccga ggctgcaggt gcaagcgagg ggcgccttcc 240
   tcttgcgggc caacgtgctc agggtcatgg ggccggctca aagtgcacaa ctccgcatcc 300
   tggcctctgc tctgctccgg cacgaggcgc aggctgcgcg tgcacccggc ggcggaggcg 360
   ggcccgccgg gtcgcgcgga ggcagccgcc gaagccccag ggctcggcgg tagagtccgg 420
   actgacgggc gtcgccagca gttcgccccc acccgcgtcc ggggcgggac gggcgggtcc 480
   tgagagcgga gagggtaggc ccggccggcg ccccgcccca gatctcggga cgcccgcgcg 540
   gatcccgcag cgcgcgtcac agcctttagg gcatccggcg 580
<210> 22
   <211> 370
   <212> DNA
   <213> Homo sapiens
<400> 22
   cggggaggct gggcgtgcca agctggaccg gtccggccag gctggaagcc cagctgcccg 60
   ctttttctca gtgcctggag accgcccgcc ttttcagcac cacggtcagc ggccttttcg 120
   caggcgcgcg ccccagcccc gccccgggtt gggctggcat ccagcctccc ggcccttctg 180
   cgtgtccctc ggcccaccgg catcccgggc tggaggtgtg ggtggggcag gcgaccgggg 240
   tatgagaggc ggtggggtgc ctcctccgcg cgtctctgcg agtatctgtc tcccccatgc 300
   gtccgctgct cctcatagag gcgtcgggag agtgtgttcg cgactttctc gcacagcggc 360
   tgcgcccgcg 370
<210> 23
   <211> 479
   <212> DNA
   <213> Homo sapiens
<400> 23
   cgccttctgg acggcgaggg tgacgcactg gccctcttcc tcggtgtcct caggtgcgtg 60
   gcggcggcag tgagaatgag ttcttcgaga tcgccgggag accacacaat aacgacgaag 120
   ttacttgccg ggctacccgc cgagacttga aaacttcgcg gcgacttctc tcgccggcct 180
   tagcatcgcc cccatttttc aggttacagg acaggcctgc aactgcaaaa cgctttgtaa 240
   accacagggc cccgcgcggg tgtgcgccac tgggttagct gagctgcagc ccgggccggg 300
   cgggctggga cgtgagcaag ccgggggaga cgggagagcc atggggagcc agatcccacc 360
   ttcgcgtgac cttgggcaaa tcacaacctc gcctaaggcc cggaatgact ctgcgcggag 420
   aggaagagca ctgagtgcgg gagggcatcg acgttttggt gttggttgct ctcgtatcg 479
<210> 24
   <211> 708
   <212> DNA
   <213> Homo sapiens
<400> 24
   cgcgcatgcg tgtccgtgag agcacatggg tgtgcaccgc gcaggggctg aggctgggag      60
   ccgtagtcag gggcaccggg tgggctttgg cggcgggtcg acctgccttt caggtgccgg     120
   gggtttggga gcaggggaac gggccctggg cggattcctg accgccccct tcgcgcccct     180
   ccccctctca ctttcgcttt ccccgcctgc cccaaccctc gcccccgggc gctcggccgc     240
   tcccacctgt cacccccatg cagaccgagg ctctgtcctg gaggcgggga gcgggctggt     300
   ggatgctgcc tctccgcctc cagtgtgctc tttggctgag ggactggtgg gcgctggaaa     360
   tattagaggc gccggtgcct gtgatgtcgc cgcctctgtg acgtccccgc cgcgacccca    420
   caggcaggga gccgcattta ggtttcgggc gggcggacgg gggacggccc gagtgaggcg     480
   tcctccgaag gctgcgggcc cgaagccaga actccttcct ctccgaagac ctcgggggtg     540
   gggctcgcgc ttccgcagcg ggcctagagc gagaggcagg cggcaggtaa gcctggctgt     600
   gctggacagg acttgcaccc gctctgcacg ccagcggctc agggctgccg cccttttcgt     660
   ggtcccaggg cccttccaag aaccggctaa accaacccaa gccgcgcg                  708
<210> 25
   <211> 10206
   <212> DNA
   <213> Homo sapiens
<400> 25
   cggaggcggc acagctggag cccggattgt ggcacgccgt caccgtgctg ctccggggaa     60
   tcccgacccg ctccctgcga aagcgtttcc gaacgcgaac ccagagcctg tgaacgcgcc    120
   ggcaagcccc ccactccccc accgccgccc gtcgcaggtg ggcccgtcct aggggtcctt    180
   cctgcgctct accccgtctc tcaagtcact cagtcgatcg ccccgttcac gctcccgtga    240
   tcccagacat ccataaccac gatctcgcct ccatgcacat ccaacgcacg acggtgcaca    300
   gacgtgcacc tgacttctgc ggaccaggtg tctcaagcgt acagcggcca cccgcggaac    360
   cgcggcccgg ggccagtgag tcgtctgcag ctccccgggt taggggattc cccagaactc    420
   cgggaaagtc acccgaagtc catccgggat gcggccttgg ttctcggccg cgtgatctcg    480
   cccttaggtg cagaacgacg cccttccagg gcccacagct gccaggctgg gccttgccct    540
   cgcatccccc gggaagacca gggacggggc cacacaggcc gtggctgcgg agacgcttcc    600
   ccgggccacc ccgcgaccag gagggagtcg agccgcccgc tctccccgcg tcccccgccc    660
   catcccagcc agttggcccc accctgcgcg ggataattgg gacgggaggg aggcgacggg    720
   agggcggcgg ctccagagag actgcgcgcc tgtcagcggc aatttgttta agtggacggg    780
   acgggccggg ccgctgcggg ctggggtcac cgaggccgcg gcccccaccc caaccagacc    840
   tgggaccgcg ggggagcccg gtccggccgc taaaccgggc tggctggcgc cagggctccg    900
   ggaggtgcgg tccggcgggg aagccgtgat gggaagcgac tctgtccagg gagtgtcctt 960
   caccaccaca ctcctcacgt ccaggcagtg atcgacggcc tggcggcacc ctcacagcgg 1020
   gcccatagca cggggccaca cacgtcccct gagcttagcc tgggcacatt cgtctgccac 1080
   cgagggctta agccagtctg cagcccgcgc cccgtcactc ggacgcaagt ccgtcgtccg 1140
   ctctgccacg cggccgcaca gcccgagctt cctgctgccc actgcccgcg gggtcacgca 1200
   accccggccc tgcacacaga ggaggaagcg gcggggaccc cctgccacct ggaggctcaa 1260
   cccggggaag gtcccagcag tgccccttaa ccctgcgaga ggtgacgggg ccggaggacg 1320
   gaagggcagc cacgccaggc tcaggaccgc ggccccaccc ggcccaggcc gtccccgctc 1380
   ccgccccatt tcactgttgc gggaagcgcg gggcgcacag accgcgcgcc accagcccaa 1440
   gcccgcccga cgccctggcc ggagaccgcc tgcgagaggg aggggcggcc gagcgcgcgt 1500
   cccccgcctg tctgcgggac ctggccgatg cgaagtgaca ggggccggag ctttgttgtg 1560
   gagcctcggc cgcctggcgc cagccgcccc cgcccgcgcc ctccccctcc agccccgggc 1620
   ggcccgcgag gcgccccccg gggcctctta aaggaacacg cacactcggc ccccggggct 1680
   ccgccagggg agggggttcc tgggaagggg gagggggccg gcctaggtgg gggaggggcc 1740
   cagcggaggc ctcacggcgc cgctccaggg cctcccaggc ctccccagcg ccagctttcg 1800
   gtgagcctcg ggcattccta gctggagagt ttggctgggg gtcgctacag actcttcaga 1860
   gcggctgcag caggacctca gggtgatcgc gggaccgcgg ggcggggcga gtggtgggat 1920
   cctggcccgg acagagcccc ggaaagccag agggaaggtc ggtgccactg taggtggggt 1980
   cgccgtcccc agatgcagcc gctgcactgt cgccgccagg tttctcggtc aaataaccca 2040
   ggcctgagtg tttcaaactc ttcaggcctc ggacccctgg accagctatg gatggactaa 2100
   tgaaatggac taatgtctaa aacgcacaca tttagatgta cacatactct tacaaggaca 2160
   aaagtatgtg tctacacaca cacacacaca cacacacaca cacacgcgcg cgcgcgcgcg 2220
   cgcgactttg cgaatgtatt cacggattcc taggccggta cctgggttcc cggctaagga 2280
   aagctgccct ggttattatc tggtcacctc accggctgcg caaacgtgtc cacaacgggt 2340
   ccctccccga gaggccacat ctcgcctaag gtggagccag caggtatttg cctgtggaaa 2400
   actgcagtgg atcctgcccc gtctgcgtag actgcgcagc tcggagtcaa agattcgttc 2460
   tggccagaga ggaggtacta agagaggtgc gacctagcca agaacgaggt cgtggagaaa 2520
   accctgtggg ggcggcgctc acatatgcgg tgatgaggag ttgtgagggc cctggggtcg 2580
   gccccgaaaa gtagggataa cggtcgcaga ggccatactg cttccgccgg tcccgaatcc 2640
   gcactgccag gagagatccg gccttcaaac gctgctccca acgttcaaca acgaagtgcc 2700
   ctgcgaccgg tgtgcgcagt gggcgggcgc ctgcatgggt caatgttgga tgtacgcgcc 2760
   caccacgcgg ttgtacgggt gggaatgtca cccgtggcta gtgcatgctc tgtcttttcc 2820
   ttcagaaaac gaccttcagg aatcagcctg agtgttcgcg cccgagcccg attggaagca 2880
   ggtgcgtggt cgcttcactc tccccgtgca caccttgagt tatagctctc gctgcgcaca 2940
   gagggcacca cacggggccc gacacacaca cacacacaca cacacacaca cacacacaca 3000
   cacacaccat cacttcatgg gggggaaact gaggctgccg gaagacagaa aacgaagact 3060
   gatcacagaa gagggggctt caatcacagg tgctcgcaga caccccaact ccctcacgcg 3120
   ttactcagca ctccttaact taggagacgt gcgcttccga gagatgagaa gaaactaaaa 3180
   aagtggttgg cagggcgaag agaggaagcc ggtatttcgg aagaaggaga ctttcgtctc 3240
   cggtaccgga tagggcgaaa attcgttggg tcgcgggtgg agaatccggg gtccgcccgc 3300
   gcggtgccgc cctgtgctca gcgaaaggtg cgctcacctt cctggccccg aggatccgct 3360
   cctcccggcc gctggaaagc tcagggcggg gcccgggcct gcggagaact ccaggttcct 3420
   tccgagtctt ccggtgaagg cgggcgcggg tagggggtcc ccgcgtttcc ccagcgcaag 3480
   gccgcgtccc gctcccgccc gcgccggccc cagcagggct cggccatggc tccactcacc 3540
   agctcccgcc acctgggatc cgctcccgga cctcggccgc ccgagcctcc cgactcgccc 3600
   gcccaccggc ctcgctttcc agtgcctgct gtctctttcc ggggcgcagg gacccccagg 3660
   cggcgccacg acccggagct gggtcggagg ccaggctggg gctcgggcca gagcggccgg 3720
   gcctctgggc gcgggagaag gagggcgccc cctctccgct cggggtcgtg tcaatgcttt 3780
   gcacttgggg ccggcgtgcg gctgcgggtc cttccccaag gccccgggac ccgggctccc 3840
   ctcgcctcag gccctttcgg cgggtcaggt cggccctccg cgctctccag tccggcgcag 3900
   gcagccgcag gcgcgggcgg gcggtggggg ccggggcagg aggaggggtc tcggggcccg 3960
   gcggcccgtc attggttaat attttattct gttgacatgt tttcttactg ctgaggcttc 4020
   cgacaccttc tcccaggccc cccctcccgg ccggagcttg gcctgagctg tcaaaacccc 4080
   gcccccggag acccacaatt ggtccaaaaa gcgtaaaatc agcaatcaag gggggcctgg 4140
   ctcgttagcg caggggatcc gagctgggca ggacatgtga gatagtcaca gttttccaga 4200
   gatcacgaca agatctaacc agtcgcgcgt ggtccccggc gccggagcgg gccagctcag 4260
   cccggcccag cccggccccg cgcagagccc ccgccgcccc cgcgcacaga gccgggtgcc 4320
   ccttgcggtg cgccggacgg gaagccccga ggagcagctg ctgcgcccgc cacccgggtc 4380
   gtccgtccac cgcgcgcgcc gccgcccggg ccgggggtcc gagccgcgcg cccccggccc 4440
   cggccccggc ccccgggcgc ctgggccgga tgtcccgatg agagagccgg cgctggcggc 4500
   cagcgccatg gcttaccacc cgttccacgc gccacggccc gccgacttcc ccatgtccgc 4560
   ctttctggcg gcggcgcagc cctccttctt cccggcactc gcgctgccgc ccggcgcgct 4620
   ggccaagccg ctgcccgacc cgggcctggc gggggcggcg gccgcggcgg cggcggcggc 4680
   agcagcggcc gaggcggggc tgcacgtctc ggcactgggc ccgcacccgc ccgccgcgca 4740
   tctgcgctcc ctcaagagcc tggagcccga ggacgaggtg gaggacgacc ccaaggtgac 4800
   gctggaggcc aaggagctgt gggaccagtt ccacaagcta ggcacggaga tggtcatcac 4860
   caagtccggg aggtagggct gccggccggc tggaaggcgc gcgggcgggc gggcgggctg 4920
   gggcacggga ctgcacggat cagagcagag ctggggactc ccggctcccg gctcccggct 4980
   ccaggttctg gccctgacgc cacgcttcgc tcccacggac aaccaagttg acttttctcg 5040
   tttggcaccg gagcgatttt tttttaaaac aaaaacgcta aaatcctccg aatgaaataa 5100
   aacgaaaaca tactgctaaa gaatagcccc aaccgttctg agtcccagcg cagaggaggc 5160
   cccgcggctt ggccctggcg gtctcctccg ccccgcgctc tcgctcttct gcgtccgggt 5220
   ccgtctccga gctcggggga aattcagcct ctctcagact ctgctccgac cccgaagccc 5280
   ctagtgggac ctgggcccag ctaaccccac gctggttgct cggttcctgg cagcgagccc 5340
   cgggtcagcc gagccctccg cctcaccggg ctgggagccc tttctccacc gcgggcatcc 5400
   gagccctgga cgcatccgcc gccccaggcc ttaaagcctg aagaaagcca cagccccggc 5460
   ccctccaccc ttcgactcag ccaccgagaa tccaggcctc gggttcaccc tcttcccccg 5520
   aactgcacgg ccaggatgtc ttatcaggct ctgtagccca gttcccaata caaacaccct 5580
   ccagatttat ttctgggagc ttccgtccca agtgggtatt tcccctgaac gaatttcggg 5640
   gagattaagg agaagcgaga atatttctag agggcctaga cttctctcct gggcatcagc 5700
   tttcatctca gatcagggag aaagagggtc cccagatctg agcacaaggc ctaaaggagg 5760
   ctgtttagga gagggtctga caggcagaaa tgggatctcc tgggagcaac aaccacaggt 5820
   ggggtcgtcc gggcagtgat gagagggcag agcagccgac cacaggggaa acagccaggc 5880
   ggcagcggtg tgcgcaacga ggagggataa ataaaggagg agtggggtcc tggaacctag 5940
   aacagccggt tccaatggga tctcctccct tccctccctc ccaggcggat gttccccccc 6000
   ttcaaggtgc gagtcagcgg cctggacaag aaggccaagt atatcctgct gatggacatt 6060
   gtagccgctg acgattgccg ctataagttc cacaactcgc gctggatggt ggcgggcaag 6120
   gccgaccctg agatgcccaa acgcatgtac atccacccag acagcccagc cacgggggag 6180
   cagtggatgg ctaagcctgt ggccttccac aagctgaagc tgaccaacaa catctctgac 6240
   aagcacggct tcgtgagtgt tggggcaggg tggggacggt gcaggagctt gttgacccag 6300
   cactgcagct gagcaggaga gcagggcggc aggatctccc agggggaagc gctgggcaaa 6360
   cccccagagt gcccctgccc gggtcactgc cctgtggtct acgtgggctg ggcctgggcc 6420
   tgggccccag cgctctcctt tgcaagcctg ggaaatggtg gggtcagtgg ctggcagctc 6480
   acaatctcag ttcctcaggc cgtggagggt cccttgtacc gtcaataggg aggcaggtgc 6540
   tgccaggact gggtctgcag tgctggacca cggaggtcac cctgctcttg gcctgtaggt 6600
   gccgagcttg gaagacctgc ccatgacctc aacttgtcca tcacccttct gccaagaccc 6660
   gcgtccctgg ccctccccca agactagggg atttaaaagt taaaccaccc tccccaccca 6720
   cccccccagc ctccctaaga cctttccctc ctcccacagg cagatgccca actaggagga 6780
   gagggtttgg gggttctctc catttatgcc tgagcaaggc cccagcgaac gggggtggtg 6840
   tttcataggg taaaaggaac tgcccattct gaggtagggc agttggtgag cccccgaagg 6900
   ttggaatctc ctctttgtgg ggttaggggg tttaccagtg ccagaaagga agagagtgtt 6960
   aagagtggaa ttgtagggct ctgttttgtt ttattttgtt tgggtttctt ttgtggggag 7020
   gggttgtttt ttggttggtt tttttgtttt gttttttgtt ttgttttgtt tttgctctgt 7080
   ctcatttctg tttccaactg ggaaattttt tttaatggca agagaaaaag aagctgtgaa 7140
   aagagaaaag cgagaggaaa agtagaagaa aaagaaataa gggtgagaaa agagcagaga 7200
   caggagaaaa tggggaagag gaagaaccga tagagagaga gagagagaga gagagagaga 7260
   gagagagaga gaaagtggag aggaagaggt caggtaccca aagaatagaa aagctcgggc 7320
   cggggctggt ggctgccggc tgacccccac cctccccgca gaccatccta aactccatgc 7380
   acaagtacca gccgcgcttc cacatagtgc gagccaacga catcctgaag ctgccttaca 7440
   gcaccttccg cacctacgtg ttcccggaga ccgacttcat cgccgtcact gcctaccaga 7500
   atgacaaggt gcgcgcggcg ggcggtgggc taagcccctg cactgacgcc cctcaacacg 7560
   tgcaggccca accgtccgtt catcgcccct cgaagccccc tgcacgggat ccgcgctctt 7620
   gcggcccgcc cccgagcacc gagcctcgca tccatacgcg cagcactcac ggaagtcctc 7680
   aaggcgccct ctcaatccca ccgcgcgcac acacaggctc ccctggggcg agcgaacagc 7740
   tgggccgtcg ttctgatgga gatgtttact taacaattag ctgagactcc gggcccgcgc 7800
   tgacattttt acattttatt cgtttatttc ttggctcagg aatgagaatg taagtgaaga 7860
   gagggcgccc gaggcaatct gcccaggctc taccgatgct cagaacccgg ggcccagttt 7920
   tcactctctc ggggggagcc agagggtggg acaggtgaca cagcccccca aagcactcct 7980
   ggctactgct ctgggtcagt ctaggcccca gaccccttgg gaggcgctta gagaattacc 8040
   ttccaatcaa agggcttttt ctgcttgcgg cttctccacc cttgttctag tggggcagag 8100
   catcacccct ctaggcctgt gcccttgtgc gccactgtcg agtgaaagag agggtacgag 8160
   tgtccgtgcc ggtcgttgtg gctttgtgaa ccaaggtgta cgctgggctg tgcgtgcccc 8220
   gtttggggtg tgttggatac ctgtggaggc atgtgaattc gtttggttcc gtaggcgttt 8280
   gttatgtctg tgcacacttg cgtgtgtgcg agcccgtgag tgtgatttgg ctttgtgtgt 8340
   acctgcagcc ttgcgtgtga actgcagtct ctgttcgcgc ctgctcgggt gcggagtcgt 8400
   ttaacttgcg caagcccgcg ggcgggtgtg catgcttggg ctggttccga cactgtggca 8460
   ctgctttagg cctgtgtggg gctgtctagg cctgtctagg gataattcct cgaatgaagc 8520
   ttttggagga cagaccccgt cgctttccgc gaggccctcc caccggcctc agctcggggc 8580
   gtcccatccc aggcgggtgg gtaccaagtg gacgctcccc gggtcttccc tctgcggcca 8640
   gcacccgctg acctcggggt gccaccgacc acgctggaag agccacggcc tgacttagcg 8700
   ccgccccctt ggtccccgca gatcacacag ctgaagatcg acaacaaccc gtttgccaag 8760
   ggcttccggg acaccgggaa cggccggcgg gagaaaaggt gagaggccga ggacagcagc 8820
   cctgtggcgg gtgcccgcgg gagcagcgag caggagggat ggagggatgg gctccccgct 8880
   gacctggttc atccgctcat ccccaggaag cagctgacgc tgccgtctct acgcttgtac 8940
   gaggagcact gcaaacccga gcgcgatggc gcggagtcag acgcctcgtc gtgcgaccct 9000
   ccccccgcgc gggaaccacc cacctccccg ggcgcagcgc ccagtccgct gcgcctgcac 9060
   cgggcccgag gtgagggtcg gaccggagga gggacaggga ggtggcggcg gggggtcctc 9120
   aggtcgctgg gctggtcttt tgctgagcca cccgctaacc tgaaaggcca ggaaggaaac 9180
   gtcggcgagt gtctgggatg gggtttccgt cccgggactc ccctacgagg gcggtccccg 9240
   gtagccagaa gatccggccg gactccgagc ctggcccctt gggcgccgtg taattctata 9300
   gctgtagggt ttcctctcca gggctgggtt ccttccactg taaatctggg gtcttggatt 9360
   aggtgacctg cagctgccct ttgcccgact ccagcagctc ctccgactcg gcctccccga 9420
   gagcagccct tcccgagtgt ccctgatcct gctcgtcggc ctccgcccag gcccctgtaa 9480
   tccgcgcgcc ctctccccgc agctgaggag aagtcgtgcg ccgcggacag cgacccggag 9540
   cctgagcggt tgagcgagga gcgtgcgggg gcgccgctag gccgcagccc ggctccagac 9600
   agcgccagcc ccactcgctt gaccgaaccc gagcgcgccc gggagcggcg tagtcccgag 9660
   aggggcaagg agccggccga gagcggcggg gacggcccgt tcggcctgag gagcctggag 9720
   aaggagcgcg ccgaagctcg gaggaaggac gaggggcgca aggaggcggc cgagggcaag 9780
   gagcagggcc tggcgccgct ggtggtgcag acagacagtg cgtcccccct gggcgccgga 9840
   cacctgcccg gcctggcctt ttccagccac ttgcacgggc agcagttctt tgggccgctg 9900
   ggagccggcc agccgctctt cctgcaccct ggacagttca ccatgggccc tggcgccttc 9960
   tccgccatgg gcatgggtca cctactggcc tcggtggcag gcggcggcaa cggcggaggt 10020
   ggcgggcctg ggaccgccgc ggggctggac gcaggcgggc tgggtcccgc ggccagcgca 10080
   gcaagcaccg ccgcgccctt cccgttccac ctctcccagc acatgctggc atctcaggta 10140
   aggcctgtga ccccgcggca gcgccagcga gggagaaggc ccagggagct ggcggcgagg 10200
   ccagcg 10206
<210> 26
   <211> 609
   <212> DNA
   <213> Homo sapiens
<400> 26
   cgcctcgcca ggtggcaagg ccttcgctcc tggtccctga gcgccgctcg cttccctccc     60
   gcgtgggcag ggggcagccg cgacgccggc ccagcgcctc cgagccggga accgcacagc     120
   acccggcgat ggccccgagc cccgcgcgcc cgaagccacg gcgccgctcg ctgggggcct    180
   cgggacggca gggacggctg ggggtggcgc ggaccgcgtc cccgaggcgc gtagcgcgcg    240
   atcgaaccac gcgccccgcc cgctgcggtc ctctaacagc ccctcgcgcc gccccacgtg    300
   gggccgcacg ggctggcggc cccctcaccc gattggccgc cgcacagcca atcagagagc    360
   ggaggcgata gcggccgcgc cgattggctg cggcgggccg gcccggggga gggggcggga    420
   ccccttttgt tctctccgca cctccgggtt cgcgcgactg tgagcgcccc cgccccggag     480
   caggggtccg cgctgggggt ccggagggcg ggggcgcggc ccgacgcccc acccccagct     540
   tgttcactgc cgccagggcc cagcgcctga agcgcgggtc cttcgctggg acgagcgctt    600
   tcatctccg                                                            609
<210> 27
   <211> 4191
   <212> DNA
   <213> Homo sapiens
<400> 27
   cgcctccgga caccggaacc gctcattgcc aatggtgatg acctggccat cgggcagctc     60
   gtagctcttc tccagagaag aggaggatgc ggcggtggcc atctcctgct cgaagtccag    120
   ggcgacgtag cacagcttct ccttgatgtc gcgcacgatt tcccgctcgg ccgtggtggt    180
   gaagctgtag cctcgctcag tgaggatctt catgaggtag tcggtcaggt cccggccagc    240
   caggtccaga cgcaggatgg cgtgggggag ggcgtagccc tcgtagatgg gcaccgtgtg    300
   ggtgaccccg tctccagagt ccatgacaat gccagtggtg cgcccagagg cgtagaggga    360
   cagcacggcc tggatggcca cgtacatggc cggggtgttg aaggtctcaa acataatctg    420
   agaagggaca aggggcggct tagtcaggga cagagaccca cggccacccc atgctcacac    480
   gccacaacat gctgcatgcc agtgtgatgt gtggagaaaa gaaaagaacg caggcagaaa    540
   ccaaatgaga aacctggagg cttcagggag gaaatgccgg gagaggaaca gagcctggaa    600
   cagcgaaaga aacacttaaa tgtcagaaat caagccgggc agaaaatgac tggggaaagg    660
   acgggaggag cacgggcgtc ggccgagcct cacctgagtc atcttctctc tgttggcctt    720
   ggggttcagg ggggcctcgg tcagcagcac tgggtgctcc tccggggcca cgcgcagctc    780
   gttgtagaag gtgtggtgcc agatcttctc catgtcgtcc cagttggtga cgatgccatg    840
   ctcaatgggg tacttcaggg tcaggatgcc acgcttgctc tgggcctcgt cgcccacgta    900
   ggagtccttc tggcccatgc ccaccatgac gccctgcagg ggacgacccg tcagcctcgc 960
   cggcgacacc gaacccaccc cgcaacgcag aacccaggag ccccgcggcg ccatccactc 1020
   acctggtgtc tggggcgccc gacgatggaa ggaaacacgg ctcggggagc gtcgtcccca 1080
   gcaaaaccag ctttgcacat gccggagcca ttgtcaatga ccagcgcggc gatctcttct 1140
   tccattgcga cctgcccgga aaaggatgga ctcaggcggg cgcgtctgta acacggtccc 1200
   ctccccacag ccacctaatg ccctcccgcg gggaagcctc ggccctgccc caaccccagc 1260
   ggccgtggcc tccaagatcg caaccgcctg gaaccgaagg ccgggccttt tacgtaacgt 1320
   ccacggctcg gaagtctgca ctgcggccgg gccccgccct ggacccccgg cgccccccca 1380
   gccccgtccg cctgacccgg cccaccccgc cttttgttcc cggggaaggc gcgacgaggc 1440
   ctcggcagct ggaagcgggg ccagccgggg tcggggggcg caggcctgcg acgtccagct 1500
   caggccccgg ggcggggcgc tcaccggcag agaaacgcga cggcggagcg gcggaagaac 1560
   agagtgcgag agctggcagc ggcgactgag accgaccgcg gcctcccccg ccgttattta 1620
   agcggaagcg gcgcgcccgc gcggcccggc ggcgcgcgcg gccccagaac atgtccatat 1680
   atggcgatct ttccgaaagc cgggcaccca ttggcccgcc gggcagggac acgtggggcc 1740
   ccggcccgcc ccgcccccag cccgcaccgc ctcccaacgg ccccgcccgc gccacccccg 1800
   cgcgctcgcg accctgcgcg ggccggcggg cgggtctgag ttggggcgcc ctccgagggt 1860
   cgccgggagg gccgaagggc tgacgggcct ggcccctccc cgggactgcc gcgccgtggg 1920
   gagggccctg ctgcgccccg aaactgcctg acccggggcg gggccgcgcc ggagctgggg 1980
   tgggtccccg agtccccggc cacgctgcgg ggctttgctc cctgggacgt cccttgcaat 2040
   ctttcccctc gggctccacg aggatctcgg gcgccagtgc cgggctgagg aaggggcgcg 2100
   gcgggggtcc gcgacgctct gcgtccctgg gcgcggggga gctctcagga gccctcacct 2160
   cgccccggtc accataggaa acagcccgcc cgtccccaag cgcagccacc accatgcagg 2220
   tccctccacg gggcgccctg ccccggatct ccgttcgttt gaagaccgag gcccctttct 2280
   acccgacccg acacatccct gcccctcaaa tcacaggcct gattgtcccc aggcgcgcgc   2340
   tccgggttgg ggctaccccg cccccttatg tgctgagaag gtggtgtgga gtgaggcctt   2400
   tgtttcattc tggtcacctg tagacattat agggaaactg aggtagacaa tgggagccct 2460
   gacttttttg aaggtcggct ccccagcttc gggcggtcgg ggggataccc tcttctgttt 2520
   acgatgctag ttaagagcag cgatcagggc tggcgtgggt ttagccacag gcttgtccag 2580
   ggcgctcact cctcgccagg agcggcgccg gtgctttggt tacaccccga gaggcgcccg 2640
   gcactacggt ttccttgtgc gctgctggaa ttttcagggt tggagcgccg gccgcgtagg 2700
   ggtggcagtg aggccgcgca gtgaggggac ggactcgccc gaggtcgcag agcgttcaga 2760
   ttgactcgac aggctggggg gcggggccgg ggtccccacg cggagtccgg ggccctgcaa 2820
   cgttagctcg cgaccccgtg tttgggagtg acaacagcga gtcagggcaa ggcggccggc 2880
   ccagctcacc tgcgggctgc agtcagcccg gacgccgaga cccgtgccta aggcccccgt 2940
   gccccaggcc tcccaggggt gctgagcgcc cccaggccca gaatctccgg gggcccgggc 3000
   aaggctgtca ggtatgttcc ctcccgtggc gaagccgggg ctgggcctcc aacagaccca 3060
   cccggactcg cgtccctggg cagaaccaca cagcctgctc gacccccggg tgaggacttc 3120
   cacttggggg gtcccaagat gcgacgtcac acaccgaggg acacgaggcg ggtggggaat 3180
   agccacaagc ccctccatcc tgcgttcacc ccacactacg ttctgctccg ggccggccgg 3240
   ggaccctgcc cctccgggtc tctggtctcc tccgaagctt ctttgtctag gaagtgcggg 3300
   gaggtgccgg ccccgggcgc gcgtccccgt cccccacctt tcttccccac gttgcggacc 3360
   cgaggccgcc agggtcgtgg gggcgggggc gaggctggcg cctcgagacc cccgggttcc 3420
   ccgccacccg cagggcctga gccgcgggga ggggcgggga ggggacgccg ccgcgggccg 3480
   ggggtggaga gcgacttcct cctccgcggc cgcgtcacgc ggcaggggtc aaggtgtggc 3540
   ccaggtacgc ggcggggggc ggtggcgcgg ccatatttgg cgacttcggc gccgccggag 3600
   ccccgcccgc ctccgccggg aagcccggcc cggggcagcg ccatccccgc ccgccgccgg 3660
   gcctgagtca cggcgggcgg ggggcggaag taacggacgg ggggcgcccg cccggcgctc 3720
   ctggccttat ttagtcaagg aggccgccga ttggcgcgcg ggacagaggg gcggcggccg 3780
   ggcagcctcg gggcgtagga gtggcccggc gcctccgagg ggtcggggcg ggagccgggc 3840
   gtggggggcc tcggagcctg gggagtcccg ggaccgacgg cggcggggat gcgggtctgc 3900
   ctgtccctgg gatgcgggcg ggcgggcagg gcgaggcctg gagcccgcgg ggcgcagggg 3960
   gctggaccgc gcgctgtgat ttttctgttt agcagcgggg cctcgcccaa ggtcgcgcgg 4020
   ggcctgtcct ggatatgctc tgggaagcca gtggcgcagg gcagccgcca cccttctgga 4080
   accagtgccg gagagggccg ggcagctggg cctggggagg ggaccgcatg gatggagctg 4140
   ggggtagctg ggcggcctcc ggagccggga gagtcggcag ctgcacttcc g 4191
<210> 28
   <211> 3855
   <212> DNA
   <213> Homo sapiens
<400> 28
   cgaggtccag cgggtgggca gccctcgcct ccccgaaggt gggagccgcc ccggcctccg 60
   cgtcgggttc tgggccgcga gaggggcggg gcaggggcgc agggctggag tcggtggctt 120
   tgtagggccc cgcagggcgc ggagccacct ggggaaacgg aagttcaggg agggcgaggc 180
   cgtgccccac atccaggcag cgcaggccgc gggccgctcc cctagccagg aagagccgcc 240
   gtccgcaccg ggaccggaga ggaagcggag gtctgaaggg ggacaggtcg gacgtggcct     300
   cagagaggga ggatgtgggc cccggagctg agccaggcgg ccggaacccc acgggacccg     360
   cgcgcacctc cccaccaaag gggccccggc gttggggggc gaggggtcgc tgccctggag      420
   gacgttggga cctgccgcgc tgcgctccga ggcggggaaa cggagggacg gcggggcgga     480
   ggggcgaagc gagcctggac tccgggggag gcgaggccgg gaagacccag tcccgaggag     540
   cagagggggc gggccagctc cgggccgggg gcgccgacgg ggccggttcc ggttgggggg      600
   gtccctgggc ccggcggggt tgcgggacgc gccgtcgggg agctgcggcc cgggagcccg     660
   tccggaggcg gcgcggggtc cgggcggggc gcgcaggggc ggcgccaggc ggtgacgtgc     720
   gcgggggacg tgccgcgcca gcgacccgag cacttaaagt cgcccccggc gcggagcgga      780
   ccggcagagg cgggcagagg cggcgagagg cggcgagagg cgggctgagg cggcccagcg     840
   gcggcaggta ggcgcggccg cgatgctcgc gcgcgggccg gggcccagga gctcccccgt     900
   gccgggcaag ggggcgacct cggggtccgc gctctcaccc ccaccccgcc cactttgtcc     960
   atcaggtccg ggggcgcccg gggcatggac tagccttctc gaccccgagc gggccccacc    1020
   gagggctccc gagagccagc cgcgcccctc cgcgccctcg catacctggc tgcccagtct    1080
   cgtctccccc ggtccggccc ggcgcccacc cgtcgggctg ccctcagggg ctgggctcgg    1140
   gccaggagat ggctgcgccg gcggccaagc ccggtccggg tataaatagc tcggtccgcg    1200
   gtggcggggg gcgcgggcta tatttggtgg ggctgctgca gccctccctg tatgggtggc    1260
   ctccccgagg gagtggggag cacttgcgct cgggccgtgg cgctttcccc tgttcgcaac    1320
   accctgaaca ctccccgacc cacacccgcc cttgggcaat gactaacagc ccgagaccgt    1380
   gcagggaagt gggggaccct ctccagagcc aggctgggct ccctgggttc gccctacctc    1440
   cacccctggc ccccagctcg tgggaaaacc aggcgcagcc tttggtagcc tcccacttcc    1500
   taacccacgc ctcagtttcc ctatacgaaa agtgggttgg tcaggagcct ccctgggtgt    1560
   tgggccagtc aagagacagg agccgcagag tggaagggaa gcccttagct ctggccgggg    1620
   cctccagggg tggctggagt cggtgaggga cgatgggggg agccgagggg cggctgtcct    1680
   cctctctgtc ccagaggcat gggctgaaag ggtccctctc ctggtgatgg ccagagtcag    1740
   gcctgctggg cgggaaatgg cagttggccc accccaaggc ccaggggctt gtccatgctg    1800
   agcacaaggg tgggaggtct tgtgtctgtt ggggccgggg agtccaaggt cccagcttca    1860
   ctgcacaggc ctggaagtca ggtgggtgct ggcctgaccc tgctgaagga gggtctattc    1920
   cgccagcgtg gtgtggaggc ggctgggctc gactccctca tggagggaag gaactgagga    1980
   ggtgctagga ggccgtgggg gggtgtctgc ccccagagag gctttgagtg catgggggct    2040
   ctgccgtggc acgtgcagcg tctgtccccc agggccccat ggtgtggaca gagtctgggg    2100
   tgggcacctg gaggcctggt ctcaaggcag gcttggtaac gaccccatgt gaccttgggc    2160
   agggcccgcc atccaggcca cccagaaagc catgtggcct gcacgatcag gccagacccc 2220
   actgactgga gcggggcact ttactttgga ggggcagcta ccagagggct ctatggatcc 2280
   cagcccagcc gtcaggccgg acgggtcccc cctactggcc tgcagtgacc cttatgccag 2340
   gctcggctgg ctcagcggtg ggtcctgggg tgacctggcc acgtgggaca gaagggcctg 2400
   tgccgctccc tccagtgagg tgccaaatgt gggccacact ctgggcccca gcgggggcct 2460
   tccgggcagt ggcgcagagc cattggaggt gcccgggccc tggggcaggg gcggggcggg 2520
   agcagacaga gctcagggcg tcctcctagc gtcacctgcc cgagtcaggc ccatctgtga 2580
   aatgggccac gccgggcagc cgcacgcaac ggaaccgaac cgccgggctc ctgctccgag 2640
   acccgcagtt cacgagtcga cgcccccgag tcccgagccg cgcccactgg tcccacctct 2700
   gcagcaagtg gagtcggccc cgccctgtgg cttcgaggcc ccgacgcctg gcgcggctcc 2760
   cgggctccgc tgaccccggt ggacccgcgc gccggcaatt acggtagcgt ccagtagggc 2820
   gcctactgcg ggcaatacgg gggctcctcc catggcgcgg tcacgtcccc cacgtcccgc 2880
   ggctggcggg gcggggccgg gcggagcatc cttccctccc cctcccccgg gtgggccccg 2940
   cccagcctcg accacccgcg ccgacccccg cccgcagagc cgggaagccg gaacccgcag 3000
   gcgccctcca gccgtccctc cggcccgggc ggggattgcg ccgaggaggg gcctctcctg 3060
   gcgagggtgg agttttgggt tccccccttc gccccgcacc tcccgccttc ctgcctcctc 3120
   ccggtcttca ccttcccccc cgggtcccca cgacgcccgc tgggcggcgg tggggtaggg 3180
   ggcgcctcct gacccgactc cccttccagc cttggccttc aggcactggc tttagggtgg 3240
   aggggcggcc ttgcggggtc caggaggcct cggaggtggg ggacggcggc tctggaccag 3300
   ctccggcccc caccacctcc cggccccagg gcggtgacag ccttgcggag aaggggtctt 3360
   caaagctctg agaccccgca gtgccagctc aggattgaag cctgcggcgg cacatgcagc 3420
   cttcctttcc caaaggggac ttgcgcccag ccccacttgg ggcaccagtg ggggctcctg 3480
   gccctggccc tgggtgggag tggccaatcc gcaaatgagg cctgtgtgtc tgccccgccg 3540
   gcccctgccg tcgtgatgta agcggacaca gagcggcagg gcacgggctg cgcgaggggt 3600
   ctgcgggggt cagaggtggg gcgcagggcc caggcctggg gcagcccctt gcgcagcgtc 3660
   caccccctgc aagctcggcc ccgacacctt tgcaggcgcc tggggcgggg ctgccgggag 3720
   ttgctcactg ctccttcctt gtttgcacaa caggggctgc gggggaagaa gggaactgag 3780
   ggaggcccct cccccggtgc agcgccctcc cgccctcggc acccctcggc agccaggcgc 3840
   tggccacaga ggccg 3855
<210> 29
   <211> 903
   <212> DNA
   <213> Homo sapiens
<400> 29
   cgacggaggg cgccttacgc gggcacttcc atgactccca ggttctgacg gcacaagagc      60
   cctgatgccc acgctcacgg acagttcccc gctggagcgg gcaggggacc tgaccctacg     120
   acacagggcg tcctgtgtca gaggcaggag gctgaggaca cagctgcgca gagccgccca     180
   gcagaggtta cccggataac gataagtggt ccccgcgacg ccccttaagt ctctggggtt    240
   atctggagcg caggtttgca gacccgctgc ccccgcggtg ggccaggtcc acggggccgg     300
   aagatagcag gatctgtcca gccctcgggc tgcggcggct gctcgcggag cccactctcc     360
   ccgacggtcc ccagcttgct ccagctctgc ctggagctcc catgcgtgcc ctccgcatcc     420
   gccctggccg cccagtcaca cacctgcagt ccccgccggg cgccgagccc ctctccagcg     480
   gcgactccct gcgattcggg tgaccgtcgg gaccaccgcg ccccagctgg agccgcgcgg     540
   cgagccgcag catctgccca gcctgggctc ctccggcggg cggggcggag cctgtcaggg     600
   ggcggggcct aatggggcgg ggctgaccgc ggagccggcc tctccagcca cctggcggcc     660
   cggcagcgtc agccaggtgc gcgcccggcc ccacatcctc tgggtcacgc ccccgaggcc     720
   gcaaggcgtc tgagaagcag ccccggctct gacccgccag ccctcggaag aagcagaccc     780
   tggccggtcc atggcagtgg cccgaggggc accagcgcgg agcaccagcg acccccaagg     840
   agtaagggga ggttaggccc ccgcgtcgga agcgcggctt tggccccgca ggagggctgg     900
   gcg                                                                   903
<210> 30
   <211> 220
   <212> DNA
   <213> Homo sapiens
<400> 30
   cgattcgaga gaggcaggca tccgcggcag cagcttccaa cccagacagg cttaagccgc      60
   gcgagggtgc ccgggtgggt ggctgcgcag ggccctacct gggcgggagc ggctgcagag     120
   ccctgtccac gcggtgcctc cccagcacgg cacctcccac tccaggctgt tggttacctg     180
   gccgggagct ggcggagacc aggatttctc ctcgccgccg                           220
<210> 31
   <211> 7467
   <212> DNA
   <213> Homo sapiens
<400> 31
   cgcggcaccg cgtgtgccga gcgcccctga ggatggaaag ccccacgcgg ggcaggtggc     60
   acccaccctc cgaagacggg acgggatgga gcgttgagct tcggggcagc tccggcccgg    120
   cccgcgctgg agacgcccgc atctgccagg atggcgtctc atagccctgg tgctcacaca    180
   tgacgccagg aagccccagc aacagtgacc gcccaggctc tagaaaatat tggacggggt 240
   ggatgaacac ccaagtgcgc tccaggagaa gggatttggc accccaaggg gcttttaaaa 300
   cggtaagctt ctaggggtgt ctttgccccc aataatccat agaaacaaca gtcatctaaa 360
   aatagtcttg ttttctgtcc taagctcctt ttaactttgt tagtcatcac caatcctaaa 420
   ataaaacccg tgtaacgtct cccctagtag cggctataaa caaacctacg aggaggcagg 480
   aggaagagga aaggggcgca gggcgctcgg ggagcagagc cgggggcccg cggtggccgc 540
   agaggccggg ccggggcgca gaggccgggc gagctggccg cgctctgggc cgccgcctcc 600
   ggaactccct gcgcctggcg cgcggccacc gtggtcccgg caacggcatt aaacagaggg 660
   aaacagaccc gggattccgt cacccgggcg gggggataag gacggctttg agagcagaca 720
   ggaaaaggga gcttttctgc atggggtgaa aaaattattt attgaaggag gaggaggcgg 780
   cagcggagga aggggagggg cgggaggagg aggaagagcc ggccgccccc gccccggccc 840
   cggctcctca ggagccaagg gcagcctcgc caggtcggtc ccgggctcga ggaccgcggc 900
   tggggtcgag gggctcagtc tcccacgtga ccggctgggc gcgccccgcc agacccggcc 960
   tcgggattcc ctcctcccgg cgagtctccg cccgccccgt cctggaggtg gggagaagga 1020
   gggcggggcg ggggggacgg aaactctccc cgccaaatcc tggccccagg cctggggaca 1080
   ctcgcggcgg gaagatttgg aggggagggg agggggaggg gcgtgggggc gcggcctcgc 1140
   tggagtcccc ctgacccccc gacccccgcc caccggcctg ggcgtcctcc cgcggcccct 1200
   cctcccctcc cggcgcccgg tgctctgggg cgcgtgccac gcctggctcg gcgccgtagg 1260
   ggcccccgca ggtagagacc cctggaaatg gcctcgacgc cgcaggagcg aggcggccac 1320
   caccccgcta atccgggcac gtctctccag gccgaggcct gcggtggaaa agccggggtt 1380
   ccatttgtgc tgagtcgggg cggccgaatg gagccaggcc tcgggacgcg ggacggacgg 1440
   gctctggccg cgcaccttcg cgggctctgc agcgcccgac cgcctccccc ggcagggagg 1500
   aggcgcttgt ggggggcacc cacggggcac agtgatccct gggggtctgc ggacctcctg 1560
   ggccccgcag cagacacgag tttagccttt gggtttagtt taaatcacat aagggtgtcg 1620
   tgcaatcgat ttatggtttc tacacaccag acactttaac ctccaacccc ccccatccaa 1680
   agccaacaag aaaatgcggt gccgtgttgg cagctgagct gcgcccgaag agacgcaggg 1740
   agacgtaaga gaggaaagtg tgagtggccg gggggcctcc ccccgtcaga agtcgcgcag 1800
   tcgcgcccat aaaacgcccc ctccgggcgg ctagggcagg tgagcgcgtc cccgggcctc 1860
   cccacgccgg cccctgccac aggccgtcta ggtcgagcag atatttacag aataaaaatg 1920
   acaataactc gacgtcccgg gacggccacg caatctgtta gtaatttagc gggatgggaa 1980
   tttcctttct agggcctgcc agtgaagcgc ttttccaaat ttccacagcg ggggaagcct 2040
   gcgattttac ataatgactt cagcatgccg ggctttctcg acacccctcc ccggcccccg 2100
   gcccccgccc cccgcccctt ttccagcagg gccgggctcc ctccggacac ccgcgtggac 2160
   tcaggcgtcc cgtctggccc gttcgccccc gtttcccccg ccagccccag cgcccccctg 2220
   cccggccccc ggattccccg ttcccgcccc tacgccccca tcccctcccc gtgcgcccct 2280
   ccccgtgcgc ccccctcccc gtgcgccccc cctccccgtg cgcccccctc cccgtgcgcc 2340
   ccccctcccc gggcgccccc ctccccgggc gccccccctc cccgtgcgcc cccccctccc 2400
   cgtgcgcccc ccctccccgt gcgcgccccg cctcttgcgc ccctgccccc aggcgagcgg 2460
   ctgccgcggc gcggggaggg gcgggcgctc ggcgactcgt ccccggggcc ccgcgcgggc 2520
   ccgggcagca ggggcgtgat gtcacggcag ggagggggcg cgggagccgc cgggccggcg 2580
   gggaggcggg ggaggtgttt tccagcttta aaaaggcagg aggcagagcg cggccctgcg 2640
   tcagagcgag actcagaggc tccgaactcg ccggcggagt cgccgcgcca gatcccagca 2700
   gcagggcgcg ggcaccgggg cgcgggcagg gctcggagcc accgcgcagg tcctagggcc 2760
   gcggccgggc cccgccacgc gcgcacacgc ccctcgatga ctttcctccg gggcgcgcgg 2820
   cgctgagccc ggggcgaggg ctgtcttccc ggagacccga ccccggcagc gcggggcggc 2880
   cgcttctcct gtgcctccgc ccgccgctcc actccccgcc gccgccgcgc ggatgccaag 2940
   caccagcttt ccagtccctt ccaagtttcc acttggccct gcggctgcgg tcttcgggag 3000
   aggagaaact ttggggcccg cgccgcgcgc cggcggcacc atgaagtcag cggaggaagg 3060
   taagccccgc gcggcctccg cccccggacc cctgcgcccc ccacggcccg ggccgcgccc 3120
   cccgaccccg cccccgtcgc ccgcacggag gggtccgggc cagagaacga ggtcggccgg 3180
   gtctgtgcgc ccagggaggg gcgcggcgcg gaccgggaac ggcgtccccc agctcccagc 3240
   agcccggttc cgagcgcgcc ttctgccaat aggtgtctcc tctgccccag cgctgagggc 3300
   gcgaggcgag gccgccgcgg gtcccgccga ggaagcggct cgcaggccgg cgatttgggg 3360
   acgggcgagg ctgggccgtc aggaccacag agggcacgtg ggagggagga agggaggctt 3420
   ccttgggagt gaaggatgcc ccgtgtcttc ctgagcgagg cagagccacc ctctggcgcc 3480
   aagatcgcgg tttgggggtt ctgcgccccc cacccccacc ccgagctcgt ggcggcgtct 3540
   ccctcgcgcc gcgcccctga attccccagg gccgagcccg tcacgccggc tcctggacct 3600
   gcaggaccaa aagatccaaa gcgcggccgc agcacagccc cgaatttaag ccattttgaa 3660
   atcctatagg caggagttcc agccagagaa gatgtttcag gcaagtttga aaaggaaccg 3720
   ccaagtttcg agctgcttct ttatagtgaa aaaaagatgg cacagtgtgg gtccctgaaa 3780
   cccggtgtgg ggggcgctga gacccccctg cggcgcatct cacattccag tcccccacct 3840
   tagcccgtcc ccggcacaca cccctggcga tgccgggtgg gctcctgcaa gtggctttga 3900
   aaataccctt gcagaggtgt gagcatacga gttagggatt agggattgca atgctaatgg 3960
   aaattggaag acgcttttcc aaattcctta ccagctctga aatattttat ataatttatt 4020
   taaagagcgc cagcacacct gccaacatat gtacttcata agggcagacg tccttccgag 4080
   cgctgccgct tcctgcgcga ccctgataac tgcggggctt gcttgagaac cgggctcccc 4140
   cagctctgga aaactcgctg gtcttagcag agctgagcac agggccgtga gctccacgga 4200
   ggggggtccg tgtggtcggt gccctgacgc ttcacgggac gggaggtaga caccacttca 4260
   tcccaatgtt agaaaatgtt gacatgttta aaccatgtcc cgtgtcagtg ttttacacaa 4320
   tgtgctcttg ctagtagcag agtttaaaaa attttttttt gttggagttt cggttttaaa 4380
   tcctgggaca cttacgccgc agtgttcatt atcactccct ttagaaaggc aggtctttcc 4440
   aagttcagat ctctgtacct gaagagccgg tggccgacct gtagtaagga gcccctttct 4500
   tttacgcaag gaaaaaacac gacggggcag tggctagttt tcgtgtttcc tactgaagaa 4560
   aataagctcg aaaaggtccc tgctgcaagg cagtgcgccg ggcagcgacg cccgaccgac 4620
   atgggtgtat tttatgaggg ctgcagaact gggccagccg gaaagtgtga tctagtcctt 4680
   gatacacaca ggccggcagc atggggacac gcgtggtttc cacaccagcc atcgctgaga 4740
   ccaccgggaa gaactctgag tccttctgag taggccccac gaggccattc ctggcccatc 4800
   tgaggacctg ggcgcctccc ctgcgtccct cgggtttcgg gaccatctgg gtggaagctc 4860
   attaggcgtc gtggttcctg aaggccccgg gcttgcttcc tcatggagaa cgcagagaag 4920
   tacaggcctg taagtagcca cccagtcaga agtcgctcca gacacaatgc cccacgggct 4980
   ggggccaaac aaagctcacc ctgttctctg ccacttaact tcacagcgtt gtttcccagc 5040
   tcagattaga aaatggcagg gcgctggcac cccgcttcat cacacccagc ggggcttctg 5100
   tctccgcaga catccccagc ctctgggagg atgcgtccaa gctaaacatt tccatggaga 5160
   cacccagtgt ctgtggaacg ggcagctttg aatctggacc aatagctctg aaccatttgc 5220
   aggaaggaag cccagcactg gggcggggga agggggggct cctaggagca gcaggggagc 5280
   cgccccaccg accccttctc tgggatggag accagtgcag tcctaggaca gcagcagtga 5340
   gcctccatcc ctgcagggcc cgcggcctgg ggccaccagc aggcgggaca gtttagcaaa 5400
   aagttttagt ctaaacagat gataagggct gtcatttgtg tataatttta tggtgggggt 5460
   aaaaaggtgg ctggagtttt aaaaacaatg tacaggccgg gcgccgcggc tcacgcctgt 5520
   aatcccagca ctttgggagg ccgaggcaag cggatcacga ggtcaagaga tcgagaccat 5580
   cctggtcaac atggtgaaac cccgtctcta ctaaaactac aaaaattagc tgagagtggt 5640
   ggcgggtgcc tgtagtccca gctactctgg aggctgaggc aggagaatcg cttgaactcg 5700
   gaaggcggag gttgcagtga gcagagattg caccactgca ctccagcctg ggtgacagag 5760
   cgagactccg tctcaaacaa acaaaaacct gtacaatgga aagagaatga aacgttgtgg 5820
   cttctcaaca gaaaacagaa cttccacttg cccaaggtcg gcaaaagcag caaaactaca 5880
   acaaaacaac gctccaacat ccatcccaaa gtcagaagta ttattagaaa ctaaatcagg 5940
   ccacacggaa cgatggctta tcactggaga aaaccagcca gtgaaagggt cgcgggagaa 6000
   gcccggggac gaccctggga ctggagggtt tctcgcctct ggaaaaggca gtgcccgcgg 6060
   ggcaggccag agggagcgct ccgaggagct ttggggttgc cagccttgac acgcgcaccc 6120
   ctccgcccgg gccggctccc ctccgccctc agactcccac catcctccta ctattccaca 6180
   tgtcgggtgt atatggtgcg gagagcccgg gggaagttag aacacgcggc gggagaggca 6240
   ggcccagggc ggcctcagct aagcagcccg gctttccgga tccccgccgc gcacaggctc 6300
   gggccgccta gggtgggacc cgaactcgcc ttcccgcccc gctcaaggag gtcatctttg 6360
   gaggtgcccc tagaaggacc ggcgggtttc ccgcggggcg ggggcgcgca gggcgggtgt 6420
   ttggaggctg gggactcgca ggagaggccg gtgggtgccg ggcctcctgc agggggttgc 6480
   aggccgaggg gcgcgccacg aacccagcgg gaatccctgc gcttcccggc accgtcgggc 6540
   tgaaatcggg gttcctagcg ttggcggcct cgggctggga cctccccact cccggggctc 6600
   cgcagggcgc gcacgtggcg ctcgggggtc accgttgcag ccgctttccc gaggaggccc 6660
   ggattgaaaa cttctttaaa aaaaaaactt aaaaaaaaaa ttccgtcgcc ctgggcgcag 6720
   ccttaggtac ctggtgccgc tcgtggggtc ccgacgcccc tacccccggg tccggaggag 6780
   cctgcgccgg gccgggaccc ccagggcaca agaggccggg ggacccctgc ggtcgcgcgc 6840
   gcgcgagggg gcgggggcgg gggggacacg agtttattta aaaactcgtg tccggggagt 6900
   ttatttaaaa ctcggaagcc ggcggccgcg agccggttgt ttatgtaaac ccggaaacgc 6960
   cccggggggc gggggcgggg cggggacggg gggaggggcg ggggtcacgt tacgcggagg 7020
   acgcgcgggc agcgccggga gaaccgaacc cctggcggcc gcgaccccgg ctcccgcccc 7080
   ggccccggcc cgacccgcca tgacggggct ggaggaccag gagttcgact tcgagttcct 7140
   cttcgagttt aaccagcgcg acgagggcgc cgccgcggcc gccccaggtg ggtcagtccc 7200
   ggagggcgcg gggggcgcgg ggccaggtcg gggtttgggg gcgcccaggc cccctccgcg 7260
   tcctcgtcgc tccccgggga ccccaggagt ccccggcgcg cccgggaccg aggggctacg 7320
   gcgggggacg ctgcagtgcg gggcgtcctg ggctcggatg cggttcctcc gagcgctcgc 7380
   ggcggggtcc tgggggaaga gcgcagcccg gacacgggcg tggacgaagt ggtcccggac 7440
   agagggctgg tggcgctgcc agaggcg 7467
<210> 32
   <211> 1142
   <212> DNA
   <213> Homo sapiens
<400> 32
   cgaccgctag tggctgggaa gtgggcggtg ctggggggtg agtctcggcc cctccaagat      60
   gcgcctccca tgaaatcctc cagtcccacg tgtactcaca cgcatacgga tgcatgcaca    120
   cgcacagaaa cacacagggc tcggacacag ctgcgcgcag acaagcaaag gaccacgtcc    180
   acgtggaaac aggtgtgcgc acacgtcccc gcgcatccgc gcgcacaagc gctccacacc    240
   tgggaacgtg cgcacctgtc ccccgccccc ggcacacaca cagccgcccc acactggaac    300
   acctgggcgc cagcgccggg ccactcgggc acgcagggcg ctacgcaggg ccgcgcacac    360
   gcttttcggc cacacgcgca cagccctcca cgtggacgcg cgggtacaca cgcccggggc    420
   tcgtagccgc gcacacccgc acccaggaaa ccccagccac caccacgcgg ggtccgatcg    480
   cctctgagca ccctccgccc cagccgcgca cccgctgcgc gcccgggacg cgcacactcg    540
   cgcacacacc ggcccttcct gcgcacgtcc cgggcccacc cccgcctaga gccctcgggg    600
   tttcccccca ccccggcctc ggggtctctc cacgtctccc cgccgacgtg ctcacctgct    660
   cagggggcgc ccccgagccg cgccccgcgc ccgcccccag gagggcctcc gcgagccggc    720
   tgcacacccc gaggcggtcc cggctgcaca acttggagcg agttgctccg tttcctcatt    780
   ttgggggcga agaagggtcg gggagtgggg gaaagcggga ggcgccgcgg cctgggaggc    840
   ccccgcgggt cctagcgccg gccgcactgc ggtctcctcc gcgcgccgcc gccgccgccg    900
   gcccctcccc gccccgcctc cgcggccccc ggcgcctccc gcgcgccccg ggcggggtcc    960
   cggggcgcac gcgggctcca cgcctggccc tgacctggcc ctgtcgggcg tgcacccccc   1020
   acccaccctg ggaagcgggg ttcttggagc gtggaacggg cgccgccagt gcgcgcggcg   1080
   gggaccgggg gtgagggaga gtgagctgcc cgtccgggga gataagcaag gcgccaagcc   1140
   cg                                                                  1142
<210> 33
   <211> 1673
   <212> DNA
   <213> Homo sapiens
<400> 33
   cgaggaaggg acggcgccag gagcctgggc ttggcagctc cctccggagg gcgcccgggg      60
   atgcccgcga gtgtccagga ggctggtgac ctgccaaggc tgggcgggct ggctggagag     120
   gagcgggagc gcgggcccgg gagagacttg ggtatcagga aactcggagg cggggctggg     180
   gggtggtgcc tggcccggac cgtctgcagg ggcggggacc gacccccagg ggtcgctgcg     240
   ccttgcgctt cccccgcccg cgggcgccag ggccgggctg aaccaccgcg cggatcggca     300
   gggggagcgg cggcgaagac agcgccagac agcgggtccc agagaccgca gcgcgcggcg     360
   tggcgcaggg tctcgccggc cactgattca aggcgcggcg gcgcggagcc cgagaggcgc     420
   cagggattgg gcggcagcgc ggctccggag cctggctcgg acacccccgc gacccgtggt     480
   aggagcccgg acccaaacaa aggcgggcgg cgaataaaag gcggcggcgg agcggagtgc     540
   tcgggttgcg agctgaggac tgggattcgc gcgcagcttc ccgcggtctg cttgccctgg     600
   agcggagggg gagccccagc ctcctgcggc tccgagaagc ttccccctgc gacttccgcg     660
   aggagacgag tctgcgcagc gtggtggccg ccgccccccg accctctgcg cactctctcc     720
   cgcgccggcg gctcagccta gccccgttcg gccggccgag gtgagtgcac ggccgcgggg     780
   cgccatccgg gcttggtctc ggggctgggg gatgcattgc taggaggagg gggaagtcct     840
   ccgcgccgtc cgcaccccga cctgcttggc gccgcgcgcg aagttctctc gctctccagc     900
   cagcccggga tgcgggcctc ggagcccccc gggaaagacg gtcaggggtg gacagaaccc     960
   aagcttgccc tctacgaccg ggccccagct ccgcaccatc tccccgcggg actgcagcat    1020
   tcttggtcgg ccaaggggag ttggatgttg gatgtccagg aacggcaagg cttccgacct    1080
   ctggcccctg cttgtcccct ctgagggata tctgggtcca taaaagttga gcgtcagtct    1140
   gaagccgccc ttggatcggg ctctcgccca tgctagagcg gagggagggg acagagtaag    1200
   gctaatgacc ccctccaccc actccctacc tagcgtactc gtgctctcgg agggacattt    1260
   ccccgattaa cctggctgca ggtccggccg gggagggcgc gtgggggctt ggggcgggcc    1320
   gggccagcga tggatccgcg cgcgcgaggg tccccaggct ctgccgagaa cgcgaacgag    1380
   ggtggaggtg gagtgggcag ccgggcagtg gcggcgctga gggtcgggaa agattcccag    1440
   cgcaaggggc gccaagtccc gggagccaac cccaccgccg ggagaacttt tcccaactcc    1500
   ctggggaggc cggcagcagg gctgggaagg cgcctccttg ggggcttctc ttctcggcct    1560
   ctgctccacg gccggcgcca gcttcccgct gtcccccact cactgcgaga ctgcgcgcag    1620
   gtctccgtca gcccactccc cgggtcagca ccgcggacag cgcctcgcgg ccg            1673
<210> 34
   <211> 557
   <212> DNA
   <213> Homo sapiens
<400> 34
   cggcggggtc gccgctcccc tgcggcagac tggccagcgc gcggcgccct ccatcccgtc 60
   ccacgccggc ccctcctccg ggagtgcagg ctccagccca agcggtgctc gcgccccccg     120
   cccaaagccc acgcgagaca aagcccgagg cttctccgcg cgcccccttc cccgccccgg     180
   gtccctacct gacggccacc ttgacgcagc agtccccctg gccggccatg gccctctgga 240
   gctagggtct gggcgtggat cagaggcggg ggtctggggg ccaatgcccg aggcagcggc 300
   tgcggctgcg ggaggcgggg gcgcgggcgc ggctggcgga ggctgcggcg gcggcggctg 360
   gaggtgacat gctcgcgggc ggcaggccga ggggctcacc cgcggccggc cccccggggc 420
   tgggcccgcg cgcctcctcg cgccatcggg cggcggcgcg gagctagctg acagccggcg 480
   gcgcgacccg ccgctcccta cggcgcggca cacgcgcaca cacctcccac ccggcagcca 540
   cctcccgccg cagcgcg 557
<210> 35
   <211> 668
   <212> DNA
   <213> Homo sapiens
<400> 35
   cgccgcgcgc cctcctggag gaagcgcttc cggccgcggg agcaccacgg tcgcggcggc 60
   atgctcagcg cttccgcgga gaccctcccg gcgggcttcc gtgtgtccac cctggggacc 120
   ctgcagcccc caccggcccc gccaaagaag atcatgcctg aaggtgagta acaggcgggc 180
   tgggcatggc cgaggcccag ccgagcgcgg gcttcttcct ggcaccccag ggccgggccg 240
   ggtggagagg ggcgaggccg aggctggtgc cccgcgctcc tgcgctgcag ctgcactaac 300
   gctccgcggg gagcgtgtgt gcgcactaac ccgccgctct gtgtgttctc ccgcggctgc 360
   cgacttctcc cagctgggac ggcggggtcg cagagactgg agacctccac ggttcggacc 420
   tactcctgct gaccccccat cctcccgccc cgggtctgac gggggagtgc ccgtggctcg 480
   gggtaagtgg gccaggcccg gggacgcggg caccttgctg ctggccctcg gccccacgca 540
   cctgcccttg gctgccggcc tggccctgcc gctccagtcc cgcttaccag cactgcctgg 600
   cttgcccctt cctgcttcgt ctggcctggg gcgctttgcg cttggagcac gctgcgttgc 660
   cgctgtcg 668
<210> 36
   <211> 1727
   <212> DNA
   <213> Homo sapiens
<400> 36
   cgacctaggg cggcggctga cgcagcccgc cgccggtcag ctttcaggtg agtgcctgaa      60
   cccgcatccc gcccatgcct tagggacccc tcttcgccca gccaagcccg gggcggggag     120
   gaaagtcccc ctcagccgca ctcgggcccc caacccggcc aaggggtcgc caactcgcgg     180
   cactttctgc ctgtttgttt tcgctcagta ctcacagttt agatgctaag ccggctcctc     240
   ttgcactcac ttcctttgtg tctctctctc tctttctctc tctctccccc tcaccaatct     300
   ctttcttccc tccttgtccg attctctgct gtagcttcca gtggagaaaa ataattattc     360
   ttcaatgggt tagcacgggg cgcacgcaca cacgcgcgcg cacacacacc ccgccgaggt     420
   acccaatcaa tgcacaaatc ttcggggtcc ctttaacctc tgcggatgac tccgggagtt     480
   aattatgaag cctatttcgg cgcggcataa atatgcatta aggtaggaaa taatcgatac     540
   gctgaacttc tgggaactgg catttagacg gactccgtct cgccgccccc cactccctcc     600
   atggccccac gagccacctc cacagcctcg cgccttcccg gggtcgcaac gcctatcaca     660
   agcttctacc cgggtttcag ggggcccctg tagtggcgtg ggcgccgcgc cggtgccggg      720
   agaccccctc gtgcgccctc tgcggtcggg gcagaccctc agccggagct tcttcctccc     780
   ccacccctgc aggccgggcc caagctgcga cacccccagc acgggctcag cctgtgaaca     840
   aactccgagc tcagtactcg gaggaactgg cgagagcggg acggctacct gcccgcacgc     900
   ccgatttccg ggcacagcta gaaagaccgg gacgcgattc aactcccaat ccggtgaatc     960
   cgcctagcgc cttcgggtcc tgccgccccc ggccagtccc gacccgcaca catatgcagg    1020
   catttgcaca cacgcgcgca cacacatgca cgcacatgca cacacacata tgcacaccct    1080
   cgcgcacaca gacactcccg cgcgcgctca cgcccgcaca cactcagggt tataacaaag    1140
   gaagaggggc cagcgctgcc ttacttttgt ctctcttctc tcttttgcag ccccccagcc    1200
   ccggggccgg cgtgcccggc ccaccccgcc ctccgcgccc cccgacggcc gcggtcagcc    1260
   ggcattccgg agcagcgcgt cggagctgca acttcgcgcc taagcgcccg cacctcgcag    1320
   cccggcgggc gggagacaaa agctgcggcg ccgccgccgg aggccgcgcg gccaaggtgt    1380
   gttcgctaat tgtcaggaaa tgtgtgtgtg tgcggccggg agcccgcagg ggcgggggag    1440
   gggacggagg aagaggagac ccacccctcc tacccccgcc actcgccagc gccgggtacc    1500
   cgaaccggga ttagagaata aattatctgc tctcttctca ttgggctgtg gctgaggacg    1560
   ccgtcggcgg cgccgggctg ggagctctcc cggctctttg cattcaccgc acagctccgg    1620
   ctcgcctcgc ttcgtggagg ccgagagccc ggggcctcgg ctccagacat caggggacac    1680
   cagccggctg gccgcgccgc cccgcccctc ccctcccccg ccacccg                   1727
<210> 37
   <211> 765
   <212> DNA
   <213> Homo sapiens
<400> 37
   cgggcctccg caccgctcgg ccatggcgag tgaaagaccc ggggctccgg ccgtcggagc     60
   acctgaccgc cggggctacc cgcatccgct gcatttgcgg agaaatgcct cctccggccc    120
   gcccccctag caaacagcgg ccccacccgc agcccatgcg ggcggtgacc cgatccagat    180
   gctgcccgtg ccctggggca ctctccagga cccccaacct cattgttctc ctcggaccgc    240
   agaccccaga cgttcggcaa gagcccttgc ccccaccccg ggggcgtggt tctccgcccg    300
   gtccgcgcta catctttaag cccctggagg cgccagggcc ccccgcagcc ccaggtgagc    360
   tagcccgaga gcaagacctg catgaggccg gacagggaaa gtggcctggg gcgaacagag    420
   gcggggaggc ggggctgagc gagcgagggg gatcccccag gagcaggagg agctgggcaa    480
   ggggggcggt tgcggggagg ggtggatggt tcggaatggg cgtggtcttg ccgaatggga    540
   gggtctaacg cgagtggttg gcgtcccgcg cggggcccaa agtgtgacac gggtgggctt    600
   ggaaccggga ccttagctac tggtggggct gaagcccacc ctgccattgt tctgccacgg    660
   tcagtggggc ttgctcctcc ctatgggaag cacttacggc cgggagcggg aatcccgtca    720
   ccaccgcggt ggggaagccg cacggaaatc cacggggtgc ggacg                    765
<210> 38
   <211> 751
   <212> DNA
   <213> Homo sapiens
<400> 38
   cgtgatgatc gtggttttcg gcttggagta catcgtccgg gtctggtccg ccggatgctg 60
   ctgccgctac cgaggatggc agggtcgctt ccgctttgcc agaaagccct tctgtgtcat 120
   cggtaatgag gcgcgccccg cccgacctga cccctgaccc caggagccag ggtgacgcct 180
   ttactcccaa tcccgactct gaccctggag acccgcacag attcagcgga ccctccccct 240
   ccctccccag tcccctgcca catccccaga agtccccgcc tccgggtccg tgcgcggggt 300
   aggctggctg tgatctcgcc gcccccgccc ctgcagactt catcgtgttc gtggcctcgg 360
   tggccgtcat cgccgcgggt acccagggca acatcttcgc cacgtccgcg ctgcgcagca 420
   tgcgcttcct gcagatcctg cgcatggtgc gcatggaccg ccgcggcggc acctggaagc 480
   tgctgggctc agtggtctac gcgcatagca aggtgaggcc tgcaagccgc gcgcggagac 540
   ccgagggcgt gtctggggca cgaccagagc gggcagggcg gagagggcga ggtcagaggg 600
   gctgtctccg tgctgggaag gggtgtggcc tgcgggggtt ggagccctag caggaggcga 660
   ggtctaagcg ggtggggcga agtggattct gaattaggta ggggcgggcc taggagtccg 720
   gaccggatca gggggcgggg ccggagaggc g 751
<210> 39
   <211> 1422
   <212> DNA
   <213> Homo sapiens
<400> 39
   cgcacccgac tgcacgcgcc tccctgggac cccgcactgg cgagccgtcc ctcccggcgc 60
   tggacgcccc acctgtcccg gcgccgtcgg aagagccctg cagagtgagc ccctagggcc 120
   accccgctgc cttcctcccc gtccccgcag ccgctgtagc cagcgtggga gccgggcagg 180
   tcgcgtgggg cgctggactt accgagaggt tagcgccgcc gtcgggggag ggcgtgtccc 240
   gcacgtgtgt gtccgcgcgt gtccgcgccc gcgtgtgcca gcgcgcgtgc cttggccgtg 300
   cgcgccgagc cgggtcgcac taactccctc ggcgccgacg ggctcagccc atggtgtggg 360
   cgccgggttc agggaggctc cggctcccgc caatcgcgga gcagggcggc cgccccgacg 420
   cgcgacttgc gaacccggag tgcgcgccgg gggcggggca gggacgcgtg ggcgggaatg 480
   cacgcgcgtg gccaggtgcg cgtgggtgcg agcgcgcggg agtcttcgag tgcgcgcgtg 540
   tccgtgtgcg cgtgggtgtg ggtgtgctcg cgtgtccgtg cgcgcgccct cggggccaag 600
   agcgcgggcc gcgcgggcag gtttggacac tacgcgccct gctcgggttt aggacgcggc 660
   tggcggctcc gggaggaaga ggaggaggaa gaggaggagg cggaggagga ggaagaggag 720
   gagggaggcg gaggagcaga ggctgggacg gccggaggag agagtccggc cccagcagga 780
   aaggaaccca gcacgcgact actggagggg cgaggagcaa gggaaggacc agaggggagg 840
   agagggggtc aggggaggga aagtgtggga gggggagccg cgggggtgcg cgctcctcct 900
   aactcggggt cgcttttagc gagaagtgat atttttccag cctcattgaa aaccgttgca 960
   aacattgaaa atagtaaaag tgaaaacggt tgcgacacta agacgtttat aaaatgaccg 1020
   cggactgccc tcgatgctga tgagaactgg gacgacgtcg cgcagagcgt ccggagggag 1080
   aggcgcggtg cccgggacct cggtcgtggg gaggagccgg atacagccgc cgggacgcgg 1140
   ggatgccgag ggcaccgcgg cctggtctga ccgacgcacg cggtgacagc cgcccttggg 1200
   agacgacggc gtctgcaacc agcagcctcc aaagggtgca gccaggaggc tcagcttgtc 1260
   cgcctccggg gctcggggct aagccccttc cagaagtttt cgtttctacg cgaattccga 1320
   ccgcaaaatc cagcttacct gagcgccgct cctcttctct catgtccctc gggggccgtc 1380
   acgaccgtcc cggagcgatc tctaggaacc tgtcccgcag cg 1422
<210> 40
   <211> 2535
   <212> DNA
   <213> Homo sapiens
<400> 40
   cgaaaccgcc aagcgcgtcc aaagcacggg ttcgccaagc gccccccccg ccccactcca 60
   cattcccttc cccgccgact cagcctccgt agctcgcgga cggcccctcc tcacgccagc 120
   ccaggctttt tttttttttt tttcttctat tttaaggttg tcttttaatg acacaagcga 180
   catttggaga caaaaggaca catctcttcc tgacccacct ccaaccccag ctgacggccg 240
   ccctgagcct ggcgtagacg gcccggaacg ttccctgcgt gggttccgtc catcccgaac 300
   ccctgtcccc gcgccggctc cgggggtgct cggggggccg cgtggggtct gtgacgtcgc 360
   ctcgaggctg catcccggtg acccggcagc ccctggcgct cgcgggaggc gggcgggcgc 420
   ggaccccagg ctttagggcg cgattcctgc agctggctgc cggcccgagg ttctggggtg 480
   tctgaggtct cgggcggggc gaggacgttt ctccggctca gcccccccac ctcctgccct 540
   gccgcccccc acacccagct ccccacggac gccaagaggc gcctcccacc ccggcgagga 600
   cccgcgggga aacggggccc aggcgcggcg actgcggagg acgcgcctcg gccccagcgc 660
   cctggtcctc ggggcgtccg gctgcccttg cccgaggccg gggcgggcgc tcagcgccgc 720
   ggaagaaacg cccgggcggg gacgcacagc gaggcgggct ccgcgggaag taccgggaaa 780
   acggcgcgga gcggaacagc ccggagccag cccagagaaa gggcggcctc cccctgcagc 840
   ccccggggcc cagcaacgcc ccgccccagg ccacggcacg cgaacgccgg gcgggcggag 900
   gggacagggc ggcgggacca gcgcggggcg ggcagcgtct gggcacaggc tcacgagccg 960
   ctcggggccc ctgcccgccg ggcgctgcgg ccccagcctc cgggccgggg ccaggcccac 1020
   agagaacggg aagcgggcgc tgcaggggga ccttcggggt tgcggcacgg ctgggaattc 1080
   aacggccatt cccgtcccgt cgggcccacc cgcccgcccg ccaaatcccc gcccaccgcg 1140
   gatccgcccc gtccaccaag gtctcgctca ccaacggccc gccccgccca ccacaggcct 1200
   ggcccgccca cgaaggctcg ccccgcccat ccacggccct gcccaccaca ggcccgcccc 1260
   aagcctgggc cccgccccag caagggcact aatcccctaa cccccgagcc ccgcccactg 1320
   ctcttgccct tctcccgccc gccccgactt aggcctctcc ccggcctggc ctcccgtact 1380
   cagccccgcc cctgccccgc ccccacccca ccccaaatgt gacctgcccc tcccactgcc 1440
   cgtcccctcc ccgcttcact tcattaggcc cccgccccga ttcttgcccc gcccgctcaa 1500
   tgtcccgcct acgaccccgc ccactactag tgaccggccc gcccgttcga ggccccgcct 1560
   cctccccgcc caatgctcgg ccccgccccg cgcgtcattg ggccaggtcc ccgccccgcc 1620
   cccagcccca cccccgcccc tttgactgga gttggctttt tccagagaac tttccgggct 1680
   cggattggcc gcgccggttg atgttccgcc ctttcattgg cccgcggcgc ccgctttcgc 1740
   gagcctgccc ttgggcgggg gcggggccgg cggcggcgcg cggcgctaga ttggttagcg 1800
   gcgcagaggg gcgggcccgc gccgcgcgca gcgctcgggc tggagcgcct gaagccgcga 1860
   ccgccgcctc agccgcctca gccgcggtcg ccgccgcttc ggctgactta ggctccgccg 1920
   ccgcctcctc acccgcctcg ccccgacccc gcccgggcct cccggcctct ccccgccggc 1980
   ccgcgggctc gccgtccggc gcagggcagg tgagtgagcg gtggcgggcg gggtcggggt 2040
   cgggcgccct tgcgggttac ggggacccag tccccgacgg aggccgagtg acggggcgac 2100
   cccgggccgc ggcaaacttc gcccgagccg gggcgggcgg gggtcccggg ctgggtcgcg 2160
   ccgtccccga ccgccgccgg cgcctgcggc ggcccggggc agcgagggga ctcgcgctcc 2220
   ctcaaggtgc ggtcgccagg cctggcccgc ccgcctccgc cccctcccca aattcggaag 2280
   ttgcggcggg aagccgagaa cgtctcggat gtgggtcaga aaacgtctgg actacttgag 2340
   gcttcagttt caaaacggca caaatttcat ctcttgattg agaagagatc tggggaacac 2400
   cccggagcgg ccgcggcggc tggagggcgg cggggcgggg cgcggggcct cctccccctg 2460
   gacggccgcc gggagagcgc ggcctcaccc ctcggtgacc ctgcggagag ccgcgcgcgg 2520
   gcggtggggc gcgcg 2535
<210> 41
   <211> 1447
   <212> DNA
   <213> Homo sapiens
<400> 41
   cgctcgtggt tccaggacgc ggaccgtaaa gaggtgccag ggagctctgc tcggtcccag     60
   ggctccccaa ccccgccgcg ccggggaatg gctgcgaggt ctctcccctg aggaccttga    120
   agggaccccg cgccttctgg gggcagccgg acgattctgg cccaggtctc tgaggggaat    180
   gggggccggg agggagcatt cgtctgcagc tctagagaga acccgagtga gaaatcttcc    240
   ggcgccccag ggggttcccg ctcccctcct agttgggcca tggccctggg actggagact     300
   gcgccgcaga gcccgggccc ccgcagtgcg cttggctaaa tagagatctc agcctcgctg    360
   cgcgcccgat ccgaccctcc ttattataac aaatgtcccg tttgcagagc gggacatcat    420
   attcgcggag aattcattct gaaatattga tttcctctca cttcgccggc ccaaatcgat    480
   agcgatgcgc cctggcccca taaatcgcct tctctgcggc cggccgggct ccatccatca    540
   ctgcgggccg cgctgcgcct ccaccgctgt ccgcactcgc tgcgcgccgg gacggctgcg    600
   cccaccagcc ctgaccctgg agctccttcc cagcaaagac cctgcccagc cctggccggg    660
   aagggggaaa acaggcatcg cggcctggga gctcgagtgt gtttgaaggc cctgcttccg    720
   ggcgcaggtg gggagggggt tttgactccg cgagcggctt agtcctcacc agccggacgc     780
   cccgcgcgcg gcgcccggcc tgcccggtgc tgggccagga ggagtcggag agcacctgag    840
   tgcgctgccg gctcccgggc ccgggaccac cggctccatt gccgttggct tgagagatgc    900
   ggggagctca cagtccagcc aggggagtca ctgggaagga ctgcgcgcac ccgaggaaga    960
   ggacttagag gtcggggacg gggaaacggc aagtccagca ccggtgcagc ccagggacag   1020
   agcacgaaga atgtccacac taatgcgcca tgcaaggctg ggaatggagc tcaaaggaaa   1080
   ccccctctaa ctccacagag atcccctcac cgatccatgt ggtccctcga aaccgggcaa   1140
   gggcggctca gctggccggg tggaacccca gtgcccgaga acagcgaggc ggctccagcg   1200
   gagggccagg ccccaggcgc cccagctggg tccccacgcg ggtgggtccc gcgaatcccg   1260
   tgctgacgct ctcgagatcc aaccacccga gcgcacgcac ggacgctcag cggcgcgggg   1320
   gctcggggat cctgaccaca ccgcgttctt accagatctg atggagttgt gaggcatcgc   1380
   cggggagtcg ctcgcagccc gccgagggct cggggcttcc tcccgtaggt ccgggccgcg   1440
   cctgccg                                                             1447
<210> 42
   <211> 793
   <212> DNA
   <213> Homo sapiens
<400> 42
   cgcaccgcct cggcgggaag gaagcccgtg gtttgcgcgc tgagcagcct ccccactccc     60
   gccctgccca gcgccgccgt ttcacttcct cccggccctc cctgcctgca ggagcatctc    120
   tgtgcccggg tccccgggcc acgccatggc tgacacctac gcggtggtgc agaagcgcgg    180
   ggctccagcg ggcgccggga gtgggacgca gacggggacg gggacgggga cgggggcgcg    240
   cagcgcggag gaggcgccgc tctacagcaa ggtgacgccg cgcgcccagc gacccggggc    300
   gcacgcggag gacgcgaggg ggacgctgcc tggccgcggt gagtcgaggc ttgctccttc    360
   tcagggcatc atcctgctgt gatccgcagg gcggaaccat cctgcagtgg tccgtcaggc    420
   cctggcggcc gcggggaccc cagccgctag cctggccacg ccccgacgct gatgtccagg    480
   cgtccctggc cacgccccgg aagcgccccc tggcggtcgc agtcgcggtc caggggcagg    540
   gttcctggca atacttgtct tggccgcgcc caaaggctgg aggccacgtc cggggtgtgt    600
   gccttctccc gcccggcgcc ctcggctctc cccttcgggc ctccggggaa gcgtccccgc    660
   taggggtggg gtcttgggac tccctggggc ttccggagct gacccgtggg gggtctgctg    720
   ccctcagttc ctgctgacca aagtcctgcc ggatctggcg cctacgagga cgtggcgggt    780
   ggagctcaga ccg                                                       793
<210> 43
   <211> 3176
   <212> DNA
   <213> Homo sapiens
<400> 43
   cgccctcgcc ccccccccgc aagtatcccc cactactccc ccccaccccc gcggccctag     60
   ctagctgact tgactggcac gcgccgggag cccgggctcg ggcccctcgg agcgtctgat    120
   tggctgcggg gcagctccgg tctgctctgc ctgcgccctc attgggcgag aggcgcagcc    180
   agcggcactt caaagcgggt gctcctcgca cttaggctga gtttagccgg cgggagcctg    240
   gagtccgctc ggcacgagcg cggggacgcg ggagccgcgc gggacccaag cagtttttcc    300
   gagcagccgc caggctcagc cccgctccca gcctcgctgc gcagccagag acctgctatg    360
   gccacgtcta tactcggggt aagtcgcaag cgcggcaacg catttgcttg ttttaaccgg    420
   agtaattttt cgttatggct tctggggtct gcggctccgg agaaactgtt gctgctagac    480
   tgcagcttca gcttctatta gcactttcca catttctggg actgattttt ccccaagttt    540
   ttggaaccca gaaagatgcc tttgcaagaa aaggccttct taatcaagtc ctttttcatt    600
   tggtttcaaa aaaaagctag aggtttcttt tcttcccctc gtgatttata ccccatcccc    660
   cgcattgctt tggggatttt gttgcaatta tgcgacaatg gtgtttccag aaaacagtct    720
   taatcgtttt gcagtccatc tagtcacgct aataaacatt tacatttccg cggctcccca    780
   attcgcccat gactttgaaa tctgtctctg gccgcttatc ctcggattat tgttttcata    840
   atcgctgttt ttgtgtgtat gtaaacttta aaaccaaacg ttttctatta aaaaagaaaa    900
   gtcaagtcgt agccaccctg ggagagctgc aagtttcgcc cgtccctggc agctgtttcg 960
   gccgcctccg ccctcacccg gtgtttgttg tctctggtct ctgtgtttac cttcgcgctt 1020
   caccccgaag ggcgacttgg ggtcttctgg gagatgagcc ggtttacggc gacaacccaa 1080
   gtccggttgt aggcagggtt gcagcgggga acacggaggc atctttgatt tatagcctca 1140
   gcgaagtgca actttccggc gcctggacct tcgcccgggt gatcgcccct gctccgcgcc 1200
   cggtgcggcg actcctccgc gggcgcgtgg ggaggaggcg cgaggcgggg agccaggacc 1260
   ccccgggagc agccgcaggg gacgaggctg ctcacgggtg cactttgttt cttcccccac 1320
   ttcctcttgc cctcctcctt cttgctccct cccccatccc acccactcta ggaagagccg 1380
   cgcttcggaa cgaccccgtt ggccatgctg gcggcgacct gcaacaagat cggcaacacg 1440
   agcccgctga cgacgctgcc agagtcgagc gccttcgcca aaggcggctt tcacccctgg 1500
   aagcgctcct cgtccagctg caacctcggc tccagcctct cgggcttcgc ggtggccacc 1560
   gggggccgtg gctcgggcgg cctggcgggc ggctcgggcg ccgccaacag cgccttctgc 1620
   ctggcctcca cgtcgcccac gtcgtccgcc ttcagcagcg actacggcgg cctcttctcc 1680
   aactcggcgg ctgccgcggc ggcagcggcc ggggtgtccc cgcaggaggc gggtggccag 1740
   tcggccttca tttccaaggt gcacacgacg gcagccgacg ggctgtaccc gcgcgtgggc 1800
   atggcgcacc cgtacgagtc ctggtacaag tcgggcttcc attcgacgct ggcggccggc 1860
   gaggtgacca acggcgcggc gtcgtcgtgg tgggacgtgc acagcagccc gggctcgtgg 1920
   ctggaagtgc agaaccccgc tggggggctc cagagctcgc tgcactcggg cgccccccag 1980
   gcctcgctgc actcgcagct gggcacctac aaccccgact tcagctcgct cacgcactcc 2040
   gccttcagct ccacgggcct cggctcctcc gccgccgccg cctcccacct gctctccacc 2100
   agccagcacc tgctggccca ggacggcttc aagccggtgt tgccctccta ttcggactcc 2160
   agcgccgccg tggcagccgc cgccgccagc gccatgatat cgggcgccgc ggctgccgcc 2220
   gccgggggga gctcggcacg ctctgcccgc cgctactcgg gccgcgccac ctgcgactgc 2280
   cccaactgcc aggaggcgga gcggctgggc ccggccgggg cgagcctgcg gcgcaagggc 2340
   ctgcacagct gccacattcc gggctgcggc aaggtgtacg ggaagacgtc gcacctgaag 2400
   gcgcacctgc gctggcacac gggcgagcgg cccttcgtgt gcaactggct cttctgcggc 2460
   aagcgcttca cgcgctcgga cgagctgcag cggcatctgc ggactcacac gggcgagaag 2520
   cgcttcgcct gtccggtgtg caacaagcgc ttcatgcgca gcgaccacct gagcaaacac 2580
   attaagacgc acaacggggg cggcgggggc aaaaagggca gcgacagtga cacggacgcc 2640
   agcaacctgg agacgccccg ttccgaatcc cccgacctca tcctgcatga ctccggcgtc 2700
   agtgccgccc gggcggcggc agcggcggcg gcggcagcgg cggcggcggc ggcggcggcc 2760
   tccgcgggag gcaaggaagc agcgtctggc cccaacgact cttagaggcc gggcgagagg   2820
   cgcgagcaca caagcgagta gagacaccga gaacgaacga gaggttcgga gggcgagcga   2880
   gcgggaggcg ggagggcagg ggcttcagtg acgcccccag ggcccgggct gggcgcgagg   2940
   tggagccgct cagggctccc gggctgcggt tcgcccgctg tgcgaggagc tcccctctgc   3000
   cttccgcgcc cggataagaa tcgaacgcgt ggtccggaaa caaaagcgaa ccatcctccg   3060
   acacaaacac tttaaaaact gtactcccag acgtacacat acaccggaga cctacaacca   3120
   caagcacgca cactcgcgcg cgcacacaca taccactcgc ccaaacttct cgagcg       3176
<210> 44
   <211> 2139
   <212> DNA
   <213> Homo sapiens
<400> 44
   cggcgggaat ggggtggatc atccgcaggg cctcggtgag cgtggtgggg ctgtcggcca      60
   gggccggcca ctcctcgggc gtggccccat ttcccggggt ggaggacgac tcactgccag     120
   agagggagga ggacaggggc tggttatttt taagcaagga ttcacctcgg ctggctagcg     180
   aggggcgtgc gcacaggcgg ctgctagcca gccggacctt acccgggacc gtccaggggc     240
   agcggccgca gccagcgccg cccctcccgg ccgggcgggc cccaaaagcc ctttctgtca     300
   ccgcaccagg gcgcgaccgg gtgatgcatt tccacaccag cccgcccaaa cctccatggt     360
   tttggagctc ccgggcaggc ggtggaaact tggcgcaccg tgcccactct ccggcgccgc     420
   tccgacagcc cgacgggtcc cgcggccagg aagccactcg gcgcccctcg ccgtcactcg     480
   acccccggcc cctttcggac tccgatcctc ccgtccccag gccacacggc gcggaaaggg     540
   gatgccgagc gggacgcgca cgaccagggc gcccaggacg agggcgctgg aggagactcc     600
   gggcagggac cggggtccca ggggcccggg ccggggctca acacccaccc gatggggtgc     660
   gggcccgacg gggcccgggg gtgggagtag gggcggcggg ggcccgcgga ggaggagtgg     720
   ggataggccg cgcagggggt gcccgggacc ccgggcgcaa gctgggaaag aggcacgcgg     780
   gggcggcgcg ccggggccgg gacaggcgcc cgtcctcacc tgccgggcag gtgtcccgcc     840
   gccgagtcgc gcgcgtcgct ttccgaggtg gaactgtcgt ggtccacggc gcaggcggcg     900
   ctgaaggcag cggccagcag ctccataggg tcggcggcgg ggcgggtgcg ggggccgggt     960
   cgggggccac gccggggccc tgggctgggt cgggggcgac tagcgggctg cggagcgggg    1020
   tcgcagcgcg cgcggctcga ccggagctgc agccgccgcc gccaccgctg ccgagcactc    1080
   ccgcgggcgg gcgcgcgcct ggaggctccc ggggttgcca cggcaacgag ggaggcggga    1140
   ggggcgcgcg cggcgggggc gaggccgggc ggcggcgggg cgggggcgcc cgggctgggc    1200
   gcgacccggg ccccgcgagc gccgactgcg ggctggggcc tgccgcgggc tcgcgccttc    1260
   ctcccccgcg ccgccgtgcc ccgacccgga gaggggcaga gctgcggagg ccctggagct   1320
   gcgcctttgc ctgccccgcc cactcctcac ggcgggggaa gcctggaagg ggcgggcgag   1380
   gccaccctcg gctccccagt gcccgcagac ctccgccttg cccacgctgg gcaccccctg   1440
   cggtctcccg cagggtcgca gctcggcgac ccgctctgaa gctccgagcc cggcctcggg   1500
   gaatgtgtcc ccgctggagg gggtctccgt cttgtctctg ctccaggcgg agactggggt   1560
   ctcaggggct ccttgcaagc ccgctcctga gcctcgcggt ggggaggggg ccggcgcgga   1620
   ggtctttccc gggcgagtct ggtcgcagga cgagccacgc tcagctacgc acgaccctgg   1680
   cgggtctggg atgggttcgg gggcaccaga ggaggagaaa ccaagtgccc gaaacctctc   1740
   ctgggttgcc ctgcccggtg acatcgactc ctggactctg cgccttcgcg ttagctgggt   1800
   cagtccgctc gcggatcaga aagagcagaa aggacagtcg gaagagcgag gcctggaggt   1860
   gcctacgggc cgagccaagc cgccctctgc tcagggatca tgctgtctgc tcaggtgtgg   1920
   cgaggatatc tagcaagatg ccccccgcct accctcgcga aggcccctgg cgcgctgccc   1980
   ggtgtccttt tccggaggcc agccggctca cctgggtggg cccttgggtg accgcggcct   2040
   gcgcctgcag ccgggcctgt cctttttacg gccctgagga aaccccaggg gcaggggtgg   2100
   agtgcggagc atagtccccc gcacgctcca acccacccg                         2139
<210> 45
   <211> 975
   <212> DNA
   <213> Homo sapiens
<400> 45
   cgccctcccc gcatagtcac gctcaggttg ctggaggcct gggtcgcccc ctcctgcaag      60
   ccccgagagt cgccagacgc gcggctaggt gccctccgtg agcggggcgg ggccgcctct     120
   gcggcgcggg gcgggggcta tctctggccc cgcccctgcc ccgcccgctc cctctccgcg     180
   agcctcgcgc cgcgcgggtt gcctggccca gaccgccgct gctgtctgcg gggtctggtg     240
   ccggggcctg agtctctgct ggctaagccg acgcctcagc cgcctcagtc gcctcaatct     300
   cgccttccgc cctcgctctc cctccgcgcc accggacccc gtagccccgc gcgcccccag     360
   acctttaagc cagatgatga acttcctgcg gcgccggctg tcggacagca gcttcatcgc     420
   caatctgccc aacggctaca tgaccgacct gcagcggccc gagccccagc aagcgccgac     480
   gccgccgccc cccggcccgg gcgccgcctc ggcctaggcg gcgcccccga ccgcctcgcc     540
   gggaccggag cggacgccgc cggccgccta ggcgcccgcg ccgtaagacg cgccgaagac     600
   cgcgcccccc cccccgcgcg aacaccgtcg gtgggcagca gcttattcag ctcgctgtcc     660
   caagcggtaa agcagacggc cgcctcggct ggcctggtgg acgcgcccgg tgccgcgccg     720
   gcagccgcca ggaaggccaa ggtgctgctg gtggtcgacg agccgcacgc cgactggtag     780
   gtgccgggcg ccttcgccct cgggggtcgg ggtccgccgg cagccgcagg ccaggagagg     840
   acggtcccag gtcgccgagg aaacgtttca gaccaataaa aaggagggtc cccgaggtcc     900
   cgctgggagg aggtgcgggc tgagcgcgcg cgcgtttgcg gggtgaggga gggcatccat     960
   cagcccggaa aggcg                                                      975
<210> 46
   <211> 858
   <212> DNA
   <213> Homo sapiens
<400> 46
   cgaatacccg ttattttggg ggcacgcacg ggctcgtgct ctgagttcct ggaaggaggc      60
   ctcggggagt gacgagaaac caggggggtc tgcaggactt ggaccgccga ccgttcctcg     120
   ctccccgggg cgagcggtct ggaccgcccg ggaagtgcct gcgccggcgg tcgtggggcc     180
   agttcccgcg tggcagctgg gcgcgacaca ggcgcgccct cctcgtccct cccgggcagc     240
   gtcggccgcc cgagcccggg gagacccgcc ccgccccgcg ccgtcacccg ggccccgttc     300
   cgcaggggtg gctcgcggcg ccccacgtcc ctgcgagaag cccgggatcg cttcgcgggg     360
   cgcaccgacg agccgccgct cgcgagctcg ccgcctacct ggagggagct caggcccgcg     420
   tcgaccgcgc gctgccggtg tccgctgggc gctcagcagc ccctggagcg cggagccggc     480
   gtggagagcg cagctcacag ccgagaccag agccgccggc cacacccagt cccgcacctc     540
   ccagcagcca actccgcggc gcgccggagc cggggcgggg acgtggctgg aggcgcgagg     600
   cgcgaggcac gaggcgcgcg ggcccggcgg ggacgtgccg gggacgcgca gaccctcgga     660
   gcgcgcgcag cccgggcggg gggcgaaggg agcgggcgcc gcgcgcagct tctgcttcct     720
   attttctctc tcgtttcctg ccagaaggag aagaaaaaca ttcattcccg ggcagtttcg     780
   ttttcttgtt ggtgtggcgc cggggcacgc tgctaagtag cgcccgtcta agtggcgtgg     840
   ccagtttcct ttccggcg                                                   858
<210> 47
   <211> 1523
   <212> DNA
   <213> Homo sapiens
<400> 47
   cgcggagttc gacgccgcgc gcagcaccaa acacttcccc agaagggcgc agcctggagg      60
   cggccgcgcg caggacgcac gcccaaggcg cctgggccgc tagggaccga ccggagcgct     120
   caaacccaca gggtatctat caggcgcgcg tcagcacgta agcagagcgc gagaacgccc     180
   gagacggcgg cgcagccaat cccagagcag cgctccccaa cggccggccg gagggcgggg     240
   agagaggggg gctcggccag agccacgctt cccattggtt gagacagcac cgcccagcca     300
   aagccccctt gtcctcgcgc gggtgcgccg cctggactcc caccctggcc agtcccgggc     360
   ccaccaccac tctggcatcc ccagcctgtc cccgcggctc ctcctccttc cccggccctg 420
   tggcctgcct gctgtccagc attcttcccc agggcgctgt ggcgcggagg gaggactccc 480
   caaaccccgg gaacagagcc ttgcggagga ccccggagga ggaggaggag gagggacgcg 540
   gacgggactg ggagattcgg tcgggaaggg gacagggcct agagcccggg gctagaaggc 600
   aggacacctg gcttcggtct gggctcggcc tttgactacg acgtgacctt gaggacggtt 660
   ccttcccttc gtggggcctg ggtccccttc agtaagatgc gccggcagga aacgatgagt 720
   cgtttcacct gggagctgca gggccgcgtg tttaacccag ggcctcctcc tgcctggcac 780
   attattccga ataataaatg ccagatcatg ctgctggaga cgccggcaga aaagccgaga 840
   gggagactcc ctggggtctg ggcgcgatcg gccatcctac ccttaccccg ctgcggccgc 900
   agggctcggc ctgtagggcg cgagcccccc ttccccaccc gtatcccggc ttcggagcta 960
   aaggaacacg tgaggccgaa ccaaaccgag tagagggcgc cagggaatcc cgcccgagct 1020
   gcagagacgc ggacccctcc tcttacccaa aaccaaatgg gcagggggct ccgctgcaga 1080
   gggctcgccg aggctctcgc cttcacccgg cgccgcgcag gtgcccgctc tcctgcgggg 1140
   agagccgagg gggggcgctg ccgcagggtc tttctggaag cccctccacc agcactcctg 1200
   cggcctgcgg cctccgccac ccggacgctt ctacacgcga agcgcgcgct ggggctacgc 1260
   tgggcggggc tcacggcggc gtggctgtcg gggggccgcc gagccgcctc ctccgcgctg 1320
   ggtgcccggc tggcgctcct cccgcccaag gtgccctgat cgctaggctc ccggtgcggc 1380
   cgtctcgccc ctaagccggg cgccagatcc cccgccgcct tctcgctccc cgaccgctgg 1440
   gcgggggcga gggccaggga gatggagcca ggtgcggaaa gggttgcagc ctcgcgggct 1500
   gaggccggag ggtgaaggcg ccg 1523
<210> 48
   <211> 1386
   <212> DNA
   <213> Homo sapiens
<400> 48
   cgttccgcgt cttccggagg cgcacagcaa gggagtctcc tggtgtcatc tcgggccatt      60
   acggcgctta gtcgatcatt aggtctgtgg gtgactgtag gcagagccgg gagttgggtg     120
   cttggcgcgg agggtgaact cgcgggtctt gagctgcaac tagaagctgc tcctagcaag     180
   gggtcgggtg gagatgaaaa gactgtgggg aaggggaggt ggttgtatgc ccagagatat     240
   cccggcgtaa ctacggagtc caggcccaga gtggcggtgt gtggcgaagg cgcagcaggc     300
   ctgcgcctgt cggaggacgg cagatctgga cgcgaccgcg cgggcgtgcc tgggtcccgc     360
   ttcctgccca tcgagggcga cttccttcgg gctcagctgt gagctccagg aactctgcaa     420
   cctcccgacg ggcgccacgc tccggcttgg gccggcccgg gagttggcgg agctgcgcgg     480
   cccggccggg cggccggtga ggctttcgag aggcaccggg ctcgctcagc gtccgcgcct     540
   ctctaccccg ccgcgccccc tccgcgccca cgggcccatt ttttcctagt cttctgcctc     600
   tcttccttct tcctccctcc cctcctcctc cacacctttg tcttcttcct cctccctcct     660
   cctgcttctc ctccgcagtt tggatcctac gggcccccta cgtgccgcgc ccttcggtcc     720
   ccggaaccgc ccgggctggg ggtgctgctg cgcggtgaga cttccgcggg cggcgggcgg     780
   agagcgggat ttgactgcga aagccaaggg ggcggggaag gggaagcccc ggccctcggg     840
   ggcgcgcagt aaactgctgc acacgtgccc gcgctcccga ggggggcgcc cagtgggctc     900
   gggatttcct ggctgcgcca ccgcgcgcct cgcccccgac gcccgccacc tgtgccctgg     960
   gccgccttcc ctgacgccag cggccggagg tcgcgcgcgc cgggtgctgc ccagcaacga    1020
   aaggccatgc ggggttcgtc cctcagatgc cggtgacctt gattgaagtc acctgccttc    1080
   tctagcttcc agtcctttaa aacgagggtt tggacccggt gccttctgaa cccggtgctt    1140
   cactgagtga gcaagtgaag ggtgtgtgtg tgtgtttggg gtgggggcgg gggcgagggg    1200
   agggaggggc agcgttgccg gctccccaga gccaggtcca ggcagaggtc gcgcccagca    1260
   ctgaccgtcg gtcccaatcc gcagactccc gagggcgatc caaaccgcct aagcatccca    1320
   agcgtccagc acccgcgcct gcgttcctga gtaatccagc gccctgcagc gtttttcatg    1380
   cacacg                                                               1386
<210> 49
   <211> 285
   <212> DNA
   <213> Homo sapiens
<400> 49
   cgcttttatg tcgcgccaga ctcctccggt gaggagcccg cagagctgct gaaaggggct 60
   ttgagaggcc cgggcgcgcg gctgctgcga gctgcgcgcc ccccgccggc ctgagtggca 120
   ccgcgtgccc ctgccggggc gcggctcctg cactgcgggc gcgcaggcgg cggcgggctc 180
   tgccggcgcc ccttattagc atgcaccctg ggtgcgcctg gcctgctccc gctgcttccg 240
   ggcctctctg cgcccctccg agtctctgcc atccgcctgg gtgcg 285
<210> 50
   <211> 1931
   <212> DNA
   <213> Homo sapiens
<400> 50
   cgccgtggag agaacatgcg cgggggaaag ccccggtcct ccggctggcg aaatggacct 60
   ctggaaggaa tggccacacg gtgtgaaatc actggtgttt attggctgtg attccatccg 120
   gagagaacac acgcaggggc cccgacatgc aggaggaggc gcaggcgcag gacagacgga 180
   cagaggacac cacggtctaa gctaagctcg cggcccgggg cgccatgcgc tgggaacggg    240
   gtgcgcaggt tctacgagag gacgccctgt ctgctcagag ctggctttgt aaggtgtgaa    300
   aacaggagtt tttaaaagac acgacccggg agaagtcagt gagagggcac agggcgagca    360
   ggacggacag cgacgtcccc gcgggccgcg tcgctggggc gcagaggggc gcggtggtct    420
   ctgcccggag gggcgtcggt cgttagtatt gcagtctaac gttatggctt ctctaaagct    480
   atgtaaggtc atgaaggtca atgccaagcc acgccctggc ccgaaacacg tggagacttg    540
   atgcattttt gatgtggacg aaagggcccg ggggcgaggc ggcgcctgtc aagataaaac    600
   tcattaaatg caaagacctc atttacctga gattcaacaa attgtgatgc aaattaaaca    660
   tgaatggagg agaaacaggg gctcggatgc cgccccgcag ggccaccagg tggattaggc    720
   cacacacgcg ccagctgcgc gcagggaacc ccgcaggccc cacccgagga gctgcccacg    780
   gaggaggtgc tgggcagggg cgcagggtct ccagcgtccg gtgcctcggg cctctgcggc    840
   tcctgtggag ggtgcagtgt tcaatggccg agggcagggg tcctccccag ggagaagcag    900
   cagccgcgtg ggcggagagg ctaggaggcc gggcgggggg cgaggacttg ggaagagcgg    960
   ggtgacgggg gtgggggctg ggcgtccccc aaacctattg ctttgtttcc tttagtttag   1020
   aagtgaacac ggccgtggcg ttcgtaagaa gcaaaacctt ccagagagga gaggaaagga   1080
   cgcggacaga gacggatgga caggggcgcg agggggccag gccggggggc ggagagcggc    1140
   cgcgtgcgga cgcaaatact cacggcggag cgatccgaac cccggagtcc gcgtgcagga   1200
   gggggaccca ggccccgccg ccctcgcccc gcccgcagcc ccccgtctgc tatggctgtg   1260
   ctcctgagtg cgggcccgac aggcgcccac cgccgcgaac gcgcgcacag gggtggtcgc   1320
   ggggctgccc ttcggcggcc ctggcctcgg gcgctgctgg gtgggcggta aacagcaaga   1380
   gaaaggggcg tcccaggcga tggggctcca gccacccgcg ggcagaggag cacggggcgg   1440
   actggggggg tcctgggggc gcgcgcccct tgccgggtga gggaggcggc gggcgcgggc    1500
   agggcgggcc tggggctatg gcttatatcg gcgtgggggc tgcgctctgc tcgtccctca   1560
   ggggcctccg cggaggatct gtagggggag gggcgggggc tccgcggggc cgctgtcccg   1620
   cgcgcggcta cttcttgagc atgtcctgca gcggcccggg caggtacttg atgacggtgt   1680
   ccaggatgct ctcgtcctcc tcctccacct cgtccccgca gcccggcggg atggccttct   1740
   tgggccgcgt caagctcccc tcggagttgg cctccatggc ggcctgggcc tcggcctcgc   1800
   gctcctcctt cttcttgatg ccgtactgcg ggggaggcgg gggtcagggc gggggtcccg   1860
   gcggctcccg ggcgggccgc agcctccctg cgccagggac tggcccagga acccccgaag   1920
   gcgtgggtgc g                                                        1931
<210> 51
   <211> 923
   <212> DNA
   <213> Homo sapiens
<400> 51
   gcgctttcgt gcgcatcctc gggtgccagt gccgcggccg cggccgccgc cgacgccgcc      60
   gccgccgtcg cctgggcggc tgcgcctaca cctaccggcc gtggacgcgc ggcggctcgc     120
   tggagcgctc gcagtcgcgc aaggactcgc tggacgacag cggcagctgc ctgagcggca     180
   gccagcggac cctgccctcg gcctcgccga gcccgggcta cctgggccgc ggcgcgccac     240
   cgccagtcga gctgtgcgcc ttccccgagt ggaaggcgcc cggcgccctc ctgagcctgc     300
   ccgcgcctga gccccccggc cgccgcggcc gccacgactc gggcccgctc ttcaccttca      360
   agctcctgac cgagcccgag agccccggga ccgacggcgg cgccagcaac ggaggctgcg     420
   aggccgcggc cgacgtggcc aacgggcagc cgggcttcaa aagcaacatg cccctggcgc     480
   ccgggcagtt ttagggcccc cgtgcgcagc tttctttccc tggggaggaa aacatcgtgg     540
   gggggagggg agggcggggc ggagggggga ggggagcgtc cacgccgggt agacgcgcgc      600
   cccaagggga accgggggga gggccgggga gaggggcagc tgcttttctg gcaggggcat     660
   gggtgccagg taccacgcgg aaagccgggc cgagcatgct gagagcctgg gggacccgac     720
   gccgccggga tttacctctc tctctccctc tgtgtatata taaacgagtc cctctctact     780
   tgtatttaac cgtgggtgca cgtgagtgtg actgtgcggt gtgcgtgtgt tccgcttgtg     840
   tgtgtgcgtg gggccgccct gtgagggcgc ggcgactgtg cgcccaggag gcaaccgggg     900
   gcgttgtgtg tgtcgtgact tcg                                             923
<210> 52
   <211> 457
   <212> DNA
   <213> Homo sapiens
<400> 52
   cgtgcggggc tggctgcggg tgggggtgcg agggggtccg ccggcggccc ccccgcgggg 60
   agggaacggt tcttacagtc actgctcagc gcgaagggcg cgcggcggcg gccgagggag 120
   cggtcttcgg cgggcgcctc gggaggcctc ttaccggcgg gcagcgggaa ggcggacgcg 180
   gtgtggagca gcaccaggca gacagccacg acatcccata acttcatctt aaagtcccgt 240
   ccggcggcgg cacctgcgcg ggcaggcggg aggtggggga gagaaccgca gaatgcacgt 300
   taagcctggg ctccctgcgg gtccctgcgc ccctctccaa cctcgtcacc gccctcctcc 360
   ggcaccgctg caggccagcg gctcctcggg cactcctgcc ctccttgcaa gtccccagtt 420
   cgcttctccc tcctcagcgc gccccaggag cagctcg 457
<210> 53
   <211> 615
   <212> DNA
   <213> Homo sapiens
<400> 53
   cgcaggaagc ggacctggcg aaaaagagca gcgaatcgga actgagcgtg ggggcagtcc 60
   ggggagatcg cgccggtcct tctagaaggc ttccctcctc tgaaacccgc tgcagtccac 120
   agccccccag gtttggcccc cgccccgctt aggctccatc cccgaactca ccggcccgcc 180
   ccgtcccgga ccagtcgaat agcaggtacg gaagaatcgc gaggcgcgga aggatcgcgg 240
   ggctggcggc cgctgccatt gtagaccagg agcgcccggg acacaaggcg cagtcgatgt 300
   ggagaccagc gtatgaatca cttcggccct cgcatgccag ctagtgattg agtgtgctcg 360
   gccccgtccg gtcccgtccc gccccgggat ccaggggagc ctacgcctgc ccacgtttgg 420
   agggatgctc cgccctatcc cgccccgtcc cgcccttccc cgccccaccc caggctggga 480
   cccggggagc ctatgcctgc tgtggctccg cttcgtcccg ccccgggctc gtggcctgac 540
   agcggccccc tctggccaac ccttaccctc caccccaggc tccgcgcggg ccaggacacg 600
   attccttctt gaccg 615
<210> 54
   <211> 2068
   <212> DNA
   <213> Homo sapiens
<400> 54
   cggttgccgc gcgtggactt acaagcccac gaagccatcg ttcgtcacag cctggaaaag     60
   ggagggggca tcgatctggt ttatgtttgg acctcgaggc tccaccagcg ctggcgcagg    120
   caggaaggag gtgagcgccg cccgcgtgca cacaaggcgc gcggccccca ggcagccgcg    180
   cttccacagc gggcaggagc gcgacccccg gcgcgggcag ctctgcgcga aggtgggcga    240
   cggtgggggc acaagaacag cagccggggg cgaaggaatc agccccgcag cggctcctaa    300
   gcctccccgc tggccgcggc tgcgccgctg gagacccggc gagtgacttc cccggtgcgg    360
   ccgggtcccg gcgccacacg cccttttcct ttctcaggat ccgcggccgt gcagagaaca    420
   ggaagacggg ctccaacccc acctggttcg tgtcgggggg tcgttttgcg gtggggggag    480
   tccgaaccgc gccacgggcc cgcgagacgc ccacctctcg cgtgcgtgga gcccggggcc    540
   gaggccaggc ccgctgcgag cagcggaggc atgtgctgat ttgcatgaga acgggcgggt    600
   cgcgtggctc agctggcggc cccggcgcag atgacggctc ggcagcgggc ccccgccccc    660
   tcctctgcgc cgcggctcct tcccctggaa cccgcggatc atctgctggg gctgtcccgc    720
   gccccatctc catgacaaag cgcgtgttta cctgcggccg gagcaccggc gggcacgcct    780
   ttgtcccggg atcagattac cgcagcagct gctgcgggcc cgccccgtgc gaaccccgga    840
   gctgcccctc cgaactttgt gccgactgaa cgcgccggga cccggggctg gggggccggg    900
   tgttgggggg ttagaaggag gccgcgtgtc ccagggaggg gctgttttca gtgcgatgat    960
   tgccggacgc tgagatccgc gacggggagt gagcgcacct gagggcttcc tggaggtggt   1020
   ggcggcgcca ggcagcatta cgaggggcac cagtgcaagg cccaaagcgc gagggcacag   1080
   gcggcgcggc cgtgcggcgg gtcatccctg ggccactgcg agggcggagg gcggcgccag   1140
   ggccctcccg gtggacccca gtcgccgtcg tgacggcccc acgcagcctc gcgcccggcc   1200
   gccgcctcgt gcgggactcg tgcccgcatt cccacctcac cgggtgcagc cggcccggcc   1260
   ccgggctgtg cgaggcgacg gcgcacacct cggagccctg gggccggggg tcggcgggcg    1320
   gggccggggc gcgccctttg tgcgctctta gcggaaaccg cgcgttccca gaggagcgcg   1380
   gcccgccgag accccggatc ccgacgccag gaggtgctgc aggagcccgc gcggggtggg   1440
   ggtctccggc ccaggcccct cggtacgggc cccctgcggc cgccaccctc cgcctccgcg   1500
   cggcggctcc tttccggctg catccggggg gtcgaggcgt cgcacctgga aatcgcagcc   1560
   tgtgactttg taaaaaagtg aatggggaag aaaaggcgct gagaggggcg atgggcggac   1620
   gggccgtgcc caccacaggg aggtcgcggc acctgtcgct cctggttccc agggcggcct   1680
   gtcaatcacc cacgctgggc tcagcttgcc gggagcgggg gcacgcgggg ggcttcaagg   1740
   cccagagctc agaggctcgc aaacaataaa ctcagaaacg tggcgcgcac ccgcagggcc   1800
   tgacacagcc tctggagtgg cccctgggcc tgtctggctg cagggttggt tggccgtggg   1860
   cgtgtgcggc tcagggtttc ctccacgccc tttcacgaat gtccccacgg gaccccgcgt   1920
   ccccccaggc tgctccagag gagtcccgga gagcgcggcc gtggaagctt tcgaacgcgc   1980
   cgtgcttccc cgccgccccc gcctcccttt ctgtgagcat ctgccgggga gaacagatgt   2040
   gggcacctgc ggtgaccggc tgcgtgcg                                      2068
<210> 55
   <211> 723
   <212> DNA
   <213> Homo sapiens
<400> 55
   cgggcaactc ggcgctggtg ctgctggcgg tgcgcgaccc gcgcctgcac acgcccatgt 60
   actacttcct ctgccacctg gccttggtag acgcgggctt cactactagc gtggtgccgc 120
   cgctgctggc caacctgcgc ggaccagcgc tctggctgcc gcgcagccac tgcacggccc 180
   agctgtgcgc atcgctggct ctgggttccg ccgaatgcgt cctcctggcg gtgatggctc 240
   tggaccgcgc ggccgcagtg tgccgcccgc tgcgctatgc ggggctcgtc tccccgcgcc 300
   tatgtcgcac gctggccagc gcctcctggc taagcggcct caccaactcg gttgcgcaaa 360
   ccgcgctcct ggctgagcgg ccgctgtgcg cgccccgcct gctggaccac ttcatctgtg 420
   agctgccggc gttgctcaag ctggcctgcg gaggcgacgg agacactacc gagaaccaga 480
   tgttcgccgc ccgcgtggtc atcctgctgc tgccgtttgc cgtcatcctg gcctcctacg 540
   gtgccgtggc ccgagctgtc tgttgcatgc ggttcagcgg aggccggagg agggcggtgg 600
   gcacgtgtgg gtcccacctg acagccgtct gcctgttcta cggctcggcc atctacacct 660
   acctgcagcc cgcgcagcgc tacaaccagg cacggggcaa gttcgtatcg ctcttctaca 720
   ccg 723
<210> 56
   <211> 545
   <212> DNA
   <213> Homo sapiens
<400> 56
   cgcttcgacg gcctcccggc ggttctgggc ttcccgggct tctcctccgg gcgccaccgc 60
   tggcaggttg acctgcagct gggcgacggc ggcggctgca cggtgggggt ggccggggag 120
   ggggtgagga ggaagggaga gatgggactc agcgccgagg acggcgtctg ggccgtgatc 180
   atctcgcacc agcagtgctg ggccagcacc tccccgggca ccgacctgcc gctgagcgag 240
   atcccgcgcg gcgtgagagt cgccctggac tacgaggcgg ggcaggtgac cctccacaac 300
   gcccagaccc aggagcccat cttcaccttc actgcctctt tctccggcaa agtcttccct 360
   ttctttgccg tctggaaaaa aggttcctgc cttacgctga aaggctgaag tggggcgcgc 420
   gaagggcggc gaagcggaga cggcggctct ccgggatcca gctccgcccc tggccagtgt 480
   gcggcccggg ggctccctgt gcccgcgtga ggcgagagaa caggggactt gagtctcgaa 540
   cagcg 545
<210> 57
   <211> 1057
   <212> DNA
   <213> Homo sapiens
<400> 57
   cgccgcgccg ctcccacggg ctatgaagag ttctacttcc ctctggtgga ggccacccgg 60
   ctccgctgtg tcaccaaatg cacgtcgggc gtggacaacg ccatcgactg tcaccagggc 120
   cagtgcgttc tagagacgag cggtcccgcg tgtcggtaag gccccgctca ccatcagcat 180
   cagtcgagcc ccgcccactc attctaggat gaagcctagc ctcacgcgcc gccccggctc 240
   tgcccccagg ccctacagtg gagcctcgtc cccagagtcc cgctccaagc ccatccccgt 300
   tgccctacag tggagccctg ccctggagct ctgctccgtc gccctaaagt gtagccccgc 360
   ctccttgatg gggttgagtc caatcccctg gttctgggat agaccccgcc cactcattct 420
   agggtggggc cccgcccctt cgttctaggg ctgaaccttg cccccttctt ctggggtgga 480
   gccccgcccc cttgttctag ggtggatccc cgccccctcc ttttagggtg aagccctgcc 540
   cacttgatct aaagtggaat cccgccccct cacctagggt agagccccgc cccctcgttc 600
   tagggtggag acccgtccgc ttgttctacg gtggattccg gccgcttgtc tagggtggaa 660
   ccccccagct tgccctaggg tggaaccccc ccgctgccct aggctggagc cccgccccct 720
   cacccgcccc cgcggggccc aggtgcacgc gtggaccccg agcccggagg tgaagagggt 780
   ctgaccctgc gatctcccgc agctgctact ccaccgacac gcactggttc tctggcccgc 840
   gctgcgaggt ggccgtccac tggagggcgc tggtcggggg cctgacggcc ggcgccgcgc 900
   tgctggtgct gctgctgctg gcgctgggcg tccgggcggt gcgctccgga tggtggggcg 960
   gccagcgccg aggccggtga gcgtgcgggg ggcggggccg gggggcgagg gcagccaagg 1020
   ggtcccaggc gggccggctc tgtctgaccg cgcggcg 1057
<210> 58
   <211> 1695
   <212> DNA
   <213> Homo sapiens
<400> 58
   cggagccgga gccggcgaag ccgcggagtt gaggcgcgcc tgggagagcc aaggcccggc 60
   gggctatgca ggtgcatgcc cccccctccg cccaaggagc ggcgccacca gcgcttcccg 120
   gctttgtcct tggaccctgg caccggctcg ctccagccgc gggggaaggc gcttcatcgc 180
   caaagtgcgc tccggcggcc ccggcccgct ctgcgggccg ccgccggagg gagtgcgggg 240
   gcgccccgaa gccgcccagg ccgcactcgc tggatgctgc gctccctctc ccggcgtcgg 300
   ccgctccggg ccggcggcag ctgattgcgg tcgtgagctc gcggagcggc gagagttaag 360
   aggaggtgtt cgcggcgcct tcgccctccc cagacacaca ccccctttct cctcgcctct 420
   tctcggaggg tggaagagac aaaaacgggg ggtcagttct ccagtccgcc ggctgccggc 480
   acgcggggcg gcgctccgtc cggcccccga ggaccagcgg cgtggcggct gcgctcggac 540
   ccggtccgcc ggccggagga gcgcgcggcg cccgcggtcg cgcccgcacc gcaggcccct 600
   tcagagagtc cgggggcggg cgcgggtccg gcggcggcgg cggccgcagc ccccggcggc 660
   gcgcaaccgc ccgtcgccgc ccgccgctgc ccggcccccg gcgcgccgcc ggctccggct 720
   ttgtgcggag ggagcgagtt cgcggcttcg gctcccggcg ccctcagtgg ccgcgccgcc 780
   agcggactgc tgcagcggcg tgagcagcgg gggtcgcggc tgcgggaaac gctccggagc 840
   ccaggaacat agtccccgct ggctagcggc ggcagcagca gcagcggggc ccctgcgcgc 900
   ggcgcccacc gtctcctcct cgcgccgggc tcgcggtgtt gcaggcggca gccacgcaga 960
   ctgctctctc atccttttgt ccttcagtca gaacgtgaat gtactgctga cgcatactgt 1020
   tctgggagaa gattagcgtg atgcagtgct cttatgtatt agcgccgctc cccctccgtc 1080
   gcctgcctcg cggggttaac gccggcgcct tccagcgccg cgccggccgg cgccgcgcag 1140
   ccccgcgcct ccggccgccc gtcttcctct ttcctccttc cttcctcctc tcttcctctc 1200
   tccccctccc ccctcccccc tccccgggcc gcttcctccc cgccccctct agacgagcgg 1260
   agcggggcca gggcgagcgg gcgagcgagc cggagtgagc gtcaagtgag gggccgcgcg 1320
   caagtcgcag gcgttcgcag ctattttggg ccggtcggag ggagcagttg ggtggccgct 1380
   gataggctgg agcggcgcgg ggctgcggcg ggagagcccg cgccggcccc cgccctccgg 1440
   cccgcacccg gggccccctg gctctgaaga gcggggctca ggggcccggc ggcccgcggc 1500
   ggggctgctg catttggcct gcctctcgcc cggcccgcgt catcctcacc cccgctgact 1560
   gctgacgggg cgcggactcg cgttcggagt tcggaactgc gtggtgggga ctcgggggac 1620
   ggcggccggg gccacgaggc tcccaggacc gagagcccgg gcttcgctgc tgcactgccg 1680
   ccagcccctc cttcg 1695
<210> 59
   <211> 360
   <212> DNA
   <213> Homo sapiens
<400> 59
   cgcttcggcc cccagcgcag ctgcagaggg gcccccctcg acgcatacac tcaagagccc     60
   gaccgcgcgg ctgaaatcgc ggagctcgga gccgcggctg gctgagcgat cgcggttcct    120
   gggctgcgtg cgcgcccctt ggagctgaaa ggagcgccag gatcgggggc gctgcaccgg    180
   gctgggcccc tcaacgctcg cagaccgggc cgggctgcag ctggagatgg cagcaatccc    240
   gggaggtctc cgggcctctt cagggtgcgt ccaggaggcg ggttccgtgc gacgcggcgc    300
   agcccacccc cacgagaccg cttaacttcg cgggggcagc ctcgggcgct cggagacgcg    360
<210> 60
   <211> 358
   <212> DNA
   <213> Homo sapiens
<400> 60
   cgagatggcc tcagcacgcc ctgcaccctc gtccgcgcga ctgccgccgg gtgcccgccc     60
   cagccgctgt gtccctggtg cttcctccgc cagggccacg tgccggggaa gccaaagcgg    120
   gagtctttcg atcttggccc agcccttagc cctcggtcct ttgctctagc atctagattc    180
   tcgtgggtca acaaagtcac tggaaaccca acgaacttcc cgacattttt cgaaaaccgt    240
   atgcgtgtgc ttctgtgcgt ttttcctctg atcgaggtag ggccctgtag ggttctgttt    300
   ggacgggagg ctggagtggt tccttcccgg ctgcggggtc ggaggatgca gagattcg      358
<210> 61
   <211> 948
   <212> DNA
   <213> Homo sapiens
<400> 61
   cgaggcggcg cctggccgag gagctccgca ctgcgccttg ttaacagctg ggtccgggca 60
   aactcgcgcc acctaattct ctgccctcta ccggcttgag atgcttattc ctcagggagg 120
   gcaagaatcg aaacgattga gaccaactat ttacaagtga ggcaaccggg ctacaggtgc 180
   ctcgagggaa aggaggctgg gtctgcgctc ccacaccttg ccgacggctc cacgtccctg 240
   tctgcgcccg aggtccaccg gtctgtgaag cccactttcc ccgcctccct ctcgcgttgt 300
   gccgcttggg gcgcccgcta ggacccaaaa tgcggactgg tgggccgccc atcaagggtt 360
   gcccgcggta gggcgggggc tcccgtgagc tgctcccaaa ccccactcgc aggatgctgc 420
   cctgacgccc agggccaacc cggggagcca aggcccccgg tctctgcaag gccgacccca 480
   gcccgctggg gaccctgcaa gcccgcgcgt ggctcccagc acgccgggcg tccgcggggc 540
   gagctgctca gggtcactcg gggtccctcg ccaatacagg ccgcgcggag agtgtgaggc 600
   gagccccggg cccgcgcccc cacgagcacc taccttccag gtacccaacc ccaggatggg 660
   catcttggcg ccgttgttga gcaggagacg gcttgccatg gctgctgcgc tccccagacc 720
   cccgcccagt acggtgcggc cttggccgcg gcgcgtacct ttaaatagcc cgtgaggtcg 780
   gcagaaaggg cgcctgcggt tggcgcgccg ctgcgcgaag gagccttctg attggttgcg 840
   ctgggggtgc cgcggcggcc ttccccaagg gtcggcgggg atgcttcttc cgcctggtcc 900
   tagtggcagc ggattctttc cgcccccaac gctcgcccgc gccccgcg 948
<210> 62
   <211> 351
   <212> DNA
   <213> Homo sapiens
<400> 62
   cgcgagcatc tccccccgcc cccggctccg cccaatttcc cacctcttcc cggagcgcac 60
   agacgccagc tgagtgggag gggcctagct gcgggcggag cacggaggag gaagggcacg 120
   aggaagaggc ggagcgggcc gaggcgcgcg gagctgggtt tgctgagtgc ttccttcctc 180
   ggccccacct cccgccgggc aggatgccgt ttgatcattg gctggtttaa taaagagggc 240
   gtgtttattt tgcataaata gtgggcgggc acccagggct gctttgtaac tcgagaaccc 300
   ggtgtcccgc ctgtctcccg gggggtatgg gcgggtctcc cgccagcctc g 351
<210> 63
   <211> 1980
   <212> DNA
   <213> Homo sapiens
<400> 63
   cgcttccagg ccggatttgc cttttcttct tcccgcaacg aagattcccg cccctcagca 60
   actttgaaaa aagcatgggg gatcgtaaac tcgaacttcg ccggttaatg ggcttattta 120
   ttggcgctgg cggctgctta ttttggatgc cttacaaaca tccgcgctat ctgcgggcga 180
   gctactttcc ctccctcccc ctccccccgc gtgggccgcg ccgcgcaggc tgggcaggga 240
   ccagggctct gggtcctccc ggccacagga aagagcgcac aggagggggc ctgctcgctg 300
   gtgtcctcgt ccctagtcag ggggagctga ggccagcgcc gaggacgtct tgctgtgggg 360
   cgctaagccg gacatgaatt ttactgcgtc cccacgccca aatattaaaa agcaagttca 420
   caaggtcagc ctgcctgcag cttgggccaa ggccggccgg ctgctgcgcg ggctcctagt 480
   tttctgatcc ttctcctcct tgtgtctgcc tgtctgcccg cctgactgca gccctctgca 540
   gccctgctta cccagggaat ccttctccgg cgaggctttg ctgctctcgg aaggggccgg 600
   ggagagctcc tccgcggccg aggacgacgc gtgcgcctcc tcgtcgccct gcgagccccc 660
   gccgctgccg caagccagcg tggggggcgg cggcgaatcg agggctcgct ccttccgggc 720
   cgcatcggcc gagccggagg ctagcgcggg cgggagatcg aaaccgcgcc ccgggggctg 780
   cgcggggaac gggccagccc cgagttgctg cgcgccgccg ccgccgctgc catagccctt 840
   ggcggtgccg taggcctgag aaaggcggaa gtagccaggc actggcaccc cgctggaggt 900
   gcccagggcg cagccgtcgg gcggcgggcc ccgcgggaag ggagccagtt cggcggcggt 960
   ggccgagact ttggggcatt tgtccgccga gtcgtagagg cagtaggagc tctcttcttt 1020
   gatgttctgc gcgaaagagc acgaggtggc ctgcggcgct ggctggggtg gttgcggcgg 1080
   gggcggcggc tgctgctggg gcggcggcgg cggcccgtca ggcggctcca tccggcaaga 1140
   ccggggcgcg tctagccaca ggtctatggg cgagggcccg tagccgtgcg ccccgggacc 1200
   tagacccccg ccaccgccac cgctgcccgg cgacgctgcc tcattgcgct tgccgcccag 1260
   cgtggggaag agcccgcagc tctgcagccc gtagggcagg tcggcggcgg gcggcaggta 1320
   gaccccgccg tgggcgtagt aaccgccacc gccgccgccc cccgcgccac caccaccgcc 1380
   gcctgcctcg cctctgcccg agctgatgag cgagtcgacc aaaaaagagt tcgcggcggg 1440
   gctctccgag catgacattg ttgtgggata atttggcgaa gggagcagat agccctttct 1500
   ggctgacatt tcttgtgcaa aacatgctga atacgattag caatcccccc gcaccgcggc 1560
   gggcgcccgc agccaatccc gagccagagt ttccgcgcga ccactcccag tttggtttcg 1620
   taggcgcggg gccgctctcc gagggcgccc tcagagcccg cgattgatat aaatatgtaa 1680
   tctgtattga tgggccagga gacgcacccc gacaccttgg cccgaaggcc gggagctgtg 1740
   ggggctgccc caacgtggct ggtggggggc ctggccattg ggctcgcccc gcccctaccc 1800
   ggacgtgagc cccataccgg ggtcccttag aagggccctt gggccccgcg cagttaacaa 1860
   gtggggtgtt tatggtgcgc gcccagtctg ccttgggtgc tcaccatccc tgtcgcagaa 1920
   gctgccacta gtccccggtg tactctaacc actgaagcgg ccgtgtcggg gactcacgcg 1980
<210> 64
   <211> 3043
   <212> DNA
   <213> Homo sapiens
<400> 64
   cgcgcgtgtg gaaggctggg tggggtgcgc acacacgctc acttgtgtac gtacacacac 60
   acacacacac acacacacac aggcacacac acccgagctc agtcacccac attgcttgcc 120
   cagggcgcct ggaagagctg gcgagcccgc ccagctctgt tcacccggcc ccgccccgcc 180
   ccctccggga gggctctccg ggcggcgccc agccccgagc agagcaaagg acggcggcgg 240
   ccacctccgt tctccggccg ctggtttctc gtctttcctc gtcctttcat tgaacccatc 300
   tccgtgcttc gaaaatctga ctctaacccg atctcttcgc gtctccgcct cttctttcta 360
   gagtgagcgc caaaaagggc ctgacagaca gaagcctttg gccccagcac cggcccagcc 420
   cgtctctaga cgattcttgc tcctttcacc ctcacagcct ccagtggtcg cttcatcttc 480
   gcaccctccc ggccaaccct aactctcctc gtctctcctc gcgctgtctc gcgtcctccc 540
   ctcaggatcc ttccgcacat tctcagcgtc cagcgcggtt tcccacaact tcctcacgcc 600
   ccgctcccct cgccctgtcc ccgccctcga caccacctgc gttccccact cgctccaacc 660
   tccctccccc gctatcccca cttgtgggcc tccagctctc tgcccctttc ctggccccca 720
   tccctgacac cccagggacc cttcctccct cctcacgttc tccctccttc caggatcccg 780
   ccccgacact tcggggccct cccgctacgc gcactctttc tcctcaggtc ctgacacctg 840
   ggcgccccct ccctgtcacc caccttcagc tccagccctg actctcgggc gccttgccac 900
   ccttacgctc cccgccccgc cccggtccct cgggcgcccc cactcgccgc ctctacctcc 960
   ctacctgcta cacctggcac ccctgccccc acccctgctc atactcttcc cccgcccccg 1020
   acacctcggg cgccccctca cacgctccta ttctccacac ttccgtcccc gaaaactcgg 1080
   gcgccctctc ccttcacgct cagtctctcc tccctccccg cccggccccg gacccagcct 1140
   ggagatcgcg ctcgggaggg gcggctgccg cccgcggggc gcccgcggtg cccgggcctg 1200
   ggcagcgagg aggtgacgcc gcccccgcgg gatgagcccg ggaggcggag gggcggagga 1260
   ggtgctggcg gcggcactga gcggcggcgg cgcagggcgc gcgggccttc cgcgccgact 1320
   ccatcgaccc aaggggcggc ggcggtggcg gcggctgagc gaccctgggc cgggcgcgtg 1380
   atgaggaggg gccggcgcca gaccccgctg cacgtcggag ctcgcctgga tccgggcgtt 1440
   ggcagccgaa gggccctggc cccgggactc tccgccgcta gcccccgtca tatcttctcc 1500
   gctttcgctt ctccactcta gccgggggtg gggtgggtgg ggttggggtc tccgcggggg 1560
   tttccggccc cgcggcccgc tcccgggtgt gcctggagga gttctccctc tgtggcgcgc 1620
   gggagccctg tgatgcgtca gccggcggga cggatgagtt gcttctccgg gaaaccgtcc 1680
   tcgcttcctc acgaccctct cggctcccgc ctgggtgccc ctcgggccgg cagtactccg 1740
   cctccgggcg ctcgaagcga gttccccggg ggcttgttcg caggcacccc ttcccctccg   1800
   aggcggcgcg cgcgctcccg gccctgaccg cggccggaca cactcgcgcc ccggtccgcc   1860
   tgtcgccctc ccgcctgctc cctccagtca ccccaccctt agctgtcccc gccaccttac   1920
   tccaccaccc tcccccgcct ctccgcgcac tccgcgtccc ggcctccagt tcccctttcc   1980
   cttgaaccgc tcacttcaca gcccttcgcc cccgggaaga agaaacattt cccgaagcgc   2040
   actcctcagc cctccttccc cacgcgctcg ccctcccctc cccctgcttt tcttggggga   2100
   ggggggctgt cgccttggat tgaaggccat tgatttgtat gtatttgtcc cagcgctgga   2160
   ggctgcccca gccgccgcgc cggtgccgcc gctgccagtg gagttgcctc cccgcttccc   2220
   tagggtggtt cggctccacc aaacatgtcg gctcctgtcg ggccccgggg ccgcctggct   2280
   cccatcccgg cggcctctca gccgcctctg cagcccgaga tgcctgacct cagccacctc   2340
   acggaggagg agaggaaaat catcctggcc gtcatggata ggcagaagaa agaagaggag   2400
   aaggagcagt ccgtgctcaa gtaaggacct ggctccatat tcccgcctct ctccctgccc   2460
   tccgccccct cgcccactgc cctgcggccg cctgcgcgcc ccagttcgcc gccctccctc   2520
   ccgctggcgg cgcccaggcc acgagggctg cggccagcgc cggccgcccg ggctgtttta   2580
   ggggtgtctg agagcagggg tgtgtgtcgg ggagggaggg cgccaaggcc ggctgaggtg   2640
   agggtggcga gccttaggcg gtgtgatttt ccttggcgcc tttccggatt tcctcgctgg   2700
   tcatcttggc tccggggccc cagcgggact ggggctgaac ccaggctctg tgcgtaccct   2760
   ctcctttccc gccgcgctga ggcagtgact ggggcacaga atccaatatg gccgtgcaca   2820
   ggtgctccct ggacggaccc gggcgaaggc gcgctggcag gggatgcgga cgccaccctg   2880
   gtcccacgcc tccgcggggc ggctctacca gcaacgcggg acagagcagg gctgcccaca   2940
   gtggctgcga gcagcgggcg gcggcgcggg ctaggggcct aagctctgtc gcggtcgggt   3000
   gggtgtgcgt ccgccgccat cttccagccc ctccccctcg gcg                       3043
<210> 65
   <211> 864
   <212> DNA
   <213> Homo sapiens
<400> 65
   cgccctgcgt ttcttcccca cgtcacttgc cagggagccg ctaaacagca agaccgcgcg 60
   ctctgcggct ccagagtgcg gatttcggtc gcgtgcggct ctgaccgcgt cgccccatcc 120
   ctggcggggc cacgcacgga cgccatggct ggcgccgcgg agccgggcga tgcgcgcgga 180
   ctctcccggg gccctgactg tccctgagtc ctccctgcgg ggggcgtgcg cggcccgccc 240
   cccgcggcgc cacgcggccc ctcctcggcc ggggattggt gcgccgggcg gggcggggcg 300
   gggcgggata aaggcgcggg gtctggctgc gcggggtctg cgggcagctc caactctggg 360
   ttcgtagttt gcgctgggtg cgcaggaagg tcagtgtggg ggtcgcccga catttccccc 420
   ccgcggaggt gggagccgag ccacatcttg gagtggggac tggccgcgga gcgggttgcc 480
   cagggccggc cgaggtcggg gcgagccctg cgcggcgctg gagactctgc attcccgggc 540
   gcgcgcaggg tccccggccg tggtcgcaga gtcaggaggg gcggctccgg agcccggcgc 600
   ggggagggcc caggcgcagt cggggttggc agggcgcgac actcgctccc ctccactttt 660
   gaaagggctt cccacgccga gaagaggggc gggcatggcc ggcccggcga aaccggtttg 720
   tacagacttt gggaagccat cgcctgcgga gggtgggacc ccacagcttg tccacctgcc 780
   caggctgaga cctcgtgtcc tagtcctgga tgccccacgg gtttctcgtc ccgggcagcg 840
   gcgcacggga ggagaagact cccg 864
<210> 66
   <211> 238
   <212> DNA
   <213> Homo sapiens
<400> 66
   cgtctgtccg cttggatgtc ccaccgctgt caggcattta atcaccggcc agtgtcccct 60
   gacccgcgcg acacatggcg catcaaccgc atcgcagagg aagtctgccc cttcctcagc 120
   ccctacggaa gcgcccgggc tgcaaggccc tgccacatgg tacggacagg gcacagaccg 180
   ctcggccaag ctgtcctgag ccgctctgag gcgggtgcac caagggatgc gacacccg 238
<210> 67
   <211> 1247
   <212> DNA
   <213> Homo sapiens
<400> 67
   cgggcgtgtt cagaaggcgg atctcaccaa tccggaggca gctggaagag ttcgcgcgtg      60
   ggaaacctca gccctttctt tcttcccggc acagcgttgt gctgccgcgc gtcggcgcag     120
   gcgggggcgc aaggcccggg tcgtccccct cgcgcggtcg ggaaaacagc tactgagctg     180
   cggcccacga ccctcggccg cgcaggggcg gggcgggggc gctgcggggt ttctctgttg     240
   ctagagccgt gatgtcaccc gcccctccta acccgcggtt ggttgttttg tgtttcagct     300
   ctgcctgcag ttgaacacaa agacctggag gagactccca cctccttcag ggagaagaaa     360
   ggaggcagcg aagcagatta aaggaacttg tggcttgtgg cctttttgtg caagggccgc    420
   ctggaagggg agaaaagtgt atgaaagcca ccgtggcggt taatgagctg tgagatgagg     480
   cgctgctgcg gccgctgctg ctgacactcg ctcccaggct gcacccgccg cccttggcgc     540
   ttcatgtaca tgtgtctatt caggccttgc ggaggcgccc agaagagcat ccaacacggc     600
   tgccggggaa agaccgccca ccatgcccac ggagacccta cagacaggta gcatggtgaa     660
   gccggtcagc cccgcgggca ccttcacctc tgctgtgccc ctgcgcatcc tgaacaaggg     720
   gccagactac ttccgcaggc aggccgagcc caaccccaag aggctcagcg ccgtggagag     780
   gctggaggcc gacaaggcca agtacgtcaa gagccaggag gtgatcaacg ccaagcagga     840
   gcccgtgaag cccgccgtgc tggccaagcc cccggtgtgc ccggctgcca agcgcgcact     900
   gggcagcccc acgctcaaag tgttcggcaa ccacgccaag accgagagcg gcgtgcagag     960
   ggagaacctg aagctggaga tcctgaagaa catcatcaat agctccgagg gctctagctc    1020
   gggctcgggg cacaagcaca gctcccgcaa ctggccgccc caccggtcgg aagccactga    1080
   cctgcaccgt cactccttcg cggagtccct gaaggtctac cccacgcagg gccgcaggag    1140
   cccgcaggag ggcggctccc acgtgggcag gagactgctg gagcagtcag ccgagtcctt    1200
   cctccacgtg tcccacagct cttcggacat ccgcaaggtg accagcg                  1247
<210> 68
   <211> 2087
   <212> DNA
   <213> Homo sapiens
<400> 68
   cgaataccct cagcgttccg cacgccgggc ggacgccctt ccgcagcggc cacgcgtgtt 60
   ctgggttcag cgctcctgcg cgtcccttct cgccgaaccc aaggtgcttt gtcccggcgg 120
   ccacacaagg tcttcggctt tttgttctcc ttttttaaga gactggccgc agtccccagc 180
   gattcagccg gcccaaccaa aggcagtgga tctactgaaa tgaaaaggat cgagataata 240
   cacaggcaga tgaataaggg gggggatgct ggaggtgcag gaagcatcct gggagcggcc 300
   gccacggcca agcccgaact gcttgctgcc ttcgctgggg aaggcgcagg aggcgagccg 360
   gcgctggtgg tcgctccacc tggcaggagg aaatgataac catgataagg aacgtgatga 420
   tgatgatgat gatgatgatg atgatgatga gcatgattta aaaaaaatca gtctccctct 480
   tggtgaacca gagcatgcgt gccggaggga ctgcactgct ggcggcggcg gccgggggaa 540
   ggagagcggc gcaggaggca aaggttgagg aggcagcacc ctcggatgaa taataacacc 600
   gcgccgcgca agttgcggag ccgcctggaa atccagccct tcgcgtgaac tcgcgtcgag 660
   cgcggattgc aggggatgag gaagaagggg gaggggaggg gagggtggag gagggagggg 720
   gaggggtaga ggggaaggca gagagggaca gagagaattg ggagcctttt aatttttcct 780
   taccaagccc taatctcgtg cgccgtcctc tttggctgag gtcgagctcc tttttaaaaa 840
   ttattactcc tttaaaggat tattcaggta aaggaagccg catctgctga cggttccgaa 900
   aacaagagga ttaggagccc gtccacagat ccaggcccgc agacccccac cccctctgca 960
   ccagcacacg tcattctcac tgcccccggg tacctccctc tccttcgtgc tcggggagag 1020
   ctgtgccaag gaaaatgcgg atttggggaa agtgagaagt gggagagctc ggaagagcgg 1080
   gaaagccaag gccggaggtg gcgattcgca cccccacact cacacgcgct tccgtttatc 1140
   cgtttctatc ccgggaaaag ggggcgcctg cccgagccca cgcgcgccgc cgcatctgca 1200
   gcggtcggcg ccccacgctc cctgaacgcc ccccaggcgg caccagtgcc gggcagagtc 1260
   ccctcggcgg ccgcgggcgt caaatcgaca cctgactcca cagctccctt ccctctcctc 1320
   ttcttccacc tccccggggg tccaggcacc gccgtgcgtc cactcccggt ctccacggct 1380
   taggcagacg gagtggggga ctccggggac ccgcgcgtcc tctcctcctc ggccctggcg 1440
   ggaggagccg ggctggggtt tccgcgggcc gcggcgcgtt ttagggctgc cggggactgc 1500
   ggcggatgcg gttgacatca ctgcggcggc tgccgggccg ctgcggcgac ctgagcgccg 1560
   gtgcagcctc ccccaccccg cctgggcggg cgtctgggca ggacagctgc ggggggttgg 1620
   gagaggagtc ggggagaggg ggacgatcgc cccatccccc gcccccccgc agtcaaggtg 1680
   aacatgaagg agagccggcg cgcgtgcccg cgcctgggag gcgcgcggcc ccgcccctcc 1740
   cggagccctc cagcgcgccc gggccagatg gacgcccgag attagaggcg cagaagtgcg 1800
   ctccacgacg ggcccacccg tcccgcggac atcagaccta cttcggggag gcgccttttg 1860
   ttgtggtggc cgtggcggtg tttgtgtctt tccgggagca cagggtgagc ttggccttca 1920
   ggaatgactc agaatgaatc cgcctataag agccaaacta aagacgaccc cccgccccag 1980
   ccccccaacc ataacccaac gcctgccatg agaaaccttc ggactttcca aggggtcagt 2040
   gcctcagcca cgaggggcag ccctgacccg cgcggttcac gcacacg 2087

<210> 69
   <211> 574
   <212> DNA
   <213> Homo sapiens
<400> 69
   cgcacccgcc cctcgcccct ccccgctcta gcttcccgac tttcgtgggg aaggggcacc 60
   ggaacgcagg gcagctgaga aggaccgggc agaggagagc gacggcccct cccggtgtca 120
   gcttcctcgc cgcagtccca ttaatcacag tgccgcagtc cagttgggga tcccgggcgc 180
   ccagggccgc ccgcagcccc agggacaatg ggattatttc cggggagaga ggccgcctcg 240
   ggccggggct caggcggaga cgcctccttc cctcaatccg tccctcggca cccaaggagc 300
   gcctgcggcg gccggagacg agtggcagag cggacgcggg tggcgcgcgg gtggcagggc 360
   ctggccgacc taattcaatt agctgggccg ccgccgcctc cgtgcgactc tgggcccgcc 420
   gggactccag ctctgggcag ctgctctttg tgcacgaaga ccatgcatat ggatgcggtt 480
   cctccccctt cggcgctcac cccacccaca cccacttctg gcacctgcgg cttgccccgc 540
   cctcccactc cttccccgcg agggaaggcg accg 574
<210> 70
   <211> 892
   <212> DNA
   <213> Homo sapiens
<400> 70
   cgcaggaaag ccccgcgtct ctattaaccc ttccatcacc gctgcaccgc gagagggacc      60
   ggagaagtct tagcactgcg cagcagcggc cgccgcagcc cgggggtgaa ggacccggct     120
   gctcaccgtg cgggggccta gccccttcac gagccaccca cgcactcccc atgcacccac     180
   ccccacaccc actagggaca gcgaggtagc gggagcccga gcgagtcggt ggaaggcgaa     240
   cgagggttct aggaccaagg gtctcagggg ccatggactg aggaggggca agtgaagcga     300
   gtacccctgg cccggcagga cccgtaggct ggcgtgcaag gacaaccccc ccctccccgc     360
   ccccaagaag tcctctgcag gatcggaggg gcgcgtgcac gaagcgggaa ggaaggctga     420
   agggaaaacg cccggaggct cccttacctg atttcctctt ggaactgcca tagtccctga     480
   aaaagaagca acaacagata gacatggtta gggccgggcg cgagcgcggg gctgcagctg     540
   caggggcgcc ctcgggctgg ctgcggtcgg tccccggtgc cgctgctcat gtgatgccca     600
   cggccggcta gccgcgcgct ccgcagccgc acgcactgcg cgccgcgccc gcccgcccgc     660
   ccgcccgcgg gccccctgcc agcccggccc ccaggcgcga ttcgcccgcc ccgccccgcc     720
   ccgccccagc ccgccccgct aactggggct caccttccct aagccttggc ggggaagggc     780
   gaggcaggtt ctcctcactt cccaaactgg gaaactgacg ctccgggatg gcgctcgccg     840
   gaacccggcc cctacccagc gctgggagcc cgctctcccc gccaccggag cg             892
<210> 71
   <211> 505
   <212> DNA
   <213> Homo sapiens
<400> 71
   cggagggcga cctggagccg actgacttca caaaggcctc ctgccgcaaa ccttcagcgg      60
   ccaccaaagc cccggctgcc ggcggcggac cacctctgct gccgcgcgcc taccggagcc     120
   gcttggccct agtgctttcc agcggatttc ccctcaggtg cggagccggg tgccggggtc     180
   ccacagccaa ccactaccgg ttcctctttc gtcagccacc ggcgccggca ggacccgcga     240
   atcccgatct ccaggagcct gtaaggaggc cgcccattgg ctcagccgca ctgctgggca     300
   ggtacttcca aagctttgag gattggctga tgctctgggc gccggggcta gttggcgggt     360
   aggatcacgt gcgaggggca ggccccgtct aggccccgcc tccttgctgc tgctgccgcc     420
   gccaatcctg gtccggttgc ccgagttccc ggaggtctct cgcgggacct ctctcaccgc     480
   caccggtggg tccgttcggc ctgcg                                           505
<210> 72
   <211> 1662
   <212> DNA
   <213> Homo sapiens
<400> 72
   cgaccccggg ctgcccgcgg gctccctcct ccctcctgcc ctcgcccgcc agctcgcagt      60
   ctcttaggtc tgcagcgttg gttggtttcc cacgcctgtc ccggtctgac tcgggctgcc     120
   aggcaccggg aagccccgcg gcgccgaccc caacccgcag ccccgggaag gtgccaaccc     180
   cagcgagtgg aggagacgag catttcacgt gggcatctgc cttcactgct gctcgcgcct     240
   ctcctcaacg gccggcgtca gggcccctct gttccaactc atcccccccc cgaagttaat     300
   cagccgtact tgtactttcc agggcgcttt agtttggaaa atacttaaaa gcctcgaaac     360
   cttccgggga gacggccgcc cccagatctc ccagttttgc ctggctaggg ctgtcgggcg     420
   ctcacgaagc tgtggcgtct gtctggaacc cacatcgcag aggcgcagcc ccgctcctcc     480
   ctgacgtcgc caaagttgcc agacgcccct cagcgacccg gtcgcgccag atgcagttgc     540
   ataaccttgc cccccacctg cgaccgcacc gggtccgctc ccggctgcac cggggtgtcc     600
   cggccccggg agccgccgag tttccggcca agccggtttg gggccgagtg tgtgcgcccg     660
   aatcggggtc ggcgcccgtc ccagcggtgc tctgcccgcg gccaagtacg acgggcgcgg     720
   gggcgcggga ggagggcacg cggggctccg cttaccttcc gctgcttaca gataagtcga     780
   ggtcgatgca gcgcctggct cgggcatgtc tcgctgcgga ccctggcaag cctggggcgg     840
   cacggagcac tccggacagg gcatccgcgg ccagactcgc cgcgctcacg cggaggaagc     900
   ctcggctccg gggacttggc ccgctgcgcg cctgctgccc gccccctcca ccgctgccgg     960
   cccggccctg ctggcccggc cggcccccgc gccgcagctc gcgtcgctcg cgtccctccg    1020
   gccccgctta gcgacgcgcg cggggaggca gcggctgggc cagctccggg gacgcccgcg    1080
   cgctcgcttg ctcgcgacgc ggcggagcca gccacagcca cccggcttcg cgctcctccg    1140
   atgtcctcat ttactgcaat tgtcttcggc gagatctcag agccagggcc aggaggcggg    1200
   ggcggggcag aggagggagc aggagggaag tcctttccgc tgctccaggt cccagctact    1260
   ccgcggagcc gggcggccag aggcgagggc gccccaagcc gcgcgcctgc cgcctgtgcg    1320
   cccggctggc accgaggcgg gcggagcccc gggccccgcg gggcggaggt ggggcggggg    1380
   cggcgcgggg ccgggccggg ccgggccgcg tgcgtggggt gggggatagg gagggagtgg    1440
   aggagccccc tcccgccgcc gccagagctg gaggaggagg aggaagagga ggaggaggtg    1500
   gaaggagctg ccgccggctg ccgccggctg ccgcctgcgc gcggggctcc gccacagcgc    1560
   gtcctccggg tttctgggct gcaggcggcg ctgcatggcc cgggaacttg ttgagtctac    1620
   cctggagccg cctccggagc agctggcggg cgggggactc cg                       1662
<210> 73
   <211> 1062
   <212> DNA
   <213> Homo sapiens
<210> 73
   <211> 1578
   <212> DNA
   <213> Homo sapiens
<400> 11
<210> 74
   <211> 449
   <212> DNA
   <213> Homo sapiens
<400> 74
   cgcctttgtt gcgcaggtga ccttcccggc cgggccagtg tcccggctgc tctgagggac      60
   agtcctggct ccgaaagcgg gcgccagcgc tgagccaggg cccgctcgag atcgcatcgt     120
   ctctgctttc agtcgttgtc cgcgcggtgc tggggcctcc cgggacccgg gagccgcggc     180
   ggattaggcc gcccgcccca acctgggctt tgatcttatc tgagacttgt gagtccaaaa     240
   gggcttagca accgcagcca tggcagcccc aacgacgtga acatccgcac ctctgagcct     300
   ccccctgaga agtaccttcg aggtgaggcc tgcgcagccc caggaagagg gtgtgggcgc    360
   aaacctgagg tggggagcaa ggcccgccgg ctacacggtt cctgccatcc tcgctgcgcc     420
   ctttctccca tgctcgcggc ctccatccg                                       449
<210> 75
   <211> 537
   <212> DNA
   <213> Homo sapiens
<400> 75
   cgggcagcgg aaggaaggcg ctgccgggga ttcaccttgg ctggggcgcg cgcggagcac      60
   tggcgccctc cccgggaacc cgagcgcggg cggcgcgggc aggtagcgcg gagcgccctg     120
   gcgcgcacgg ccggctgaaa ttctgctttt cgttctttgg ggtttttctt ccttttgctt     180
   ttattttttt ttttctttgc cttctccaca cggagagcgc gtttgtggcg gcagcccgag     240
   cttagcgggc gtccgggtgt aaggaagaaa cccaagggcc gcgagggagc gaccaggacg     300
   ctgcttcccg ggcgctcccg ggactcccag atacctgctc ggtccccgcg tgggaggtcc     360
   gcaggctccc cctcgccact gcgccccact cgcctccgcg ctgcgagcct aggcgaagcc     420
   gcggccgagc cgccaccggg ggcaggctag tccccggctt ggcttggggg cccggagcac     480
   ccccggtggc cacctccggc ctgccccgga gctttgcgcg cgccgttcgg gccttcg        537
<210> 76
   <211> 1507
   <212> DNA
   <213> Homo sapiens
<400> 76
   cggctccgcc accacctccg aggcccgccg ccgccgcctc tctatccgca tcccggatag      60
   gggcgccacc accccgcgcg tgaccccgct ttcccgcggg tacagataaa aatcccctca     120
   cccttccgta gatgccatgg tctggggaac gccccctcct cttcagcact atccggcagc     180
   tgtgggcgag ggagaaagcg gacgccgggg tgagcgcgaa gtgcggccgg gagcggtatt     240
   taagaggagc ctctgcccgc ccttccaccc gcctccgggg taagcccttt agcgcctcaa     300
   cgggcgcagg aggctcctgc gggcgctaat ccgcgtcccg gctctccacg gcgcgcgacc     360
   cgcgctcccc tccgcccgcg gggatggcgc ttggcgggga gctccggggg ctccgagggt     420
   ggcttccccg agagagcgat gcgcccaggg ctgcaggagg gcgcacgccg gcgatgcgcc     480
   cctgcagccg ctgccacctg tgctttgctg cggggctcgc gggcgcggcg agtggcggga     540
   gcagagagtg gtggcagagg acggtgagcg tgcgtgcgcg tgtgcgggtg tgtgcgcgcc     600
   tggggaggcg gtggaggccg ctgcgctctg gctcggcgcc ggccagggag ggattgcaag     660
   gtttagcccc gccggagctg gggatttgca ggcgatccct ctctattttc gtcgagagct     720
   gacatcaccc gcgccgccgc ctcgggcaac tcctttaacc gccgcccccc gcccccatct     780
   ccagtcgcgg tccctcctct gccatccctc cccccagcct ctcgccataa attagccaaa     840
   taagaaaaga ggccccgccg ctcgtccggg aaggcttggg cagccggtgg caggagccca     900
   ggatctaagg agtagctatt gtttcccctg aagcccgcgt ggccccaggt caccccgtgc     960
   ggggtgaagc gcaggcgacc gcgcggagac ccgggatgcc cctccgtctc cgcctccctg    1020
   gagcagcccc tggcgctccc tcctcactcc ccggagcacc cgcttcagct cacactcacc    1080
   cccaaactca caggcaggca cacccacccc atcctcccac gtctgggggt cccaggtcca    1140
   gcggagtcga gcggctgaaa cacgcgggtg gtgcagccta aacgcggccc agctgcgctc    1200
   cctcccccgc cgaagctcga ataaatctct ggccttcaat tattcagcga gattaatatt    1260
   aatgcagctc cctgggggcg gcgggcgggg tgcaggggag gccgggattt tttttttttt    1320
   tttttaggcc gagagaggtc ggcctcgtgg gtgccggctg agaaggcgcg gagagttggc    1380
   cgaggggtga gtggcggggg acggttctcc acccactcgg cgctgctcgc cgcctgcttc    1440
   cgtgtcccgg gggcggcgca cagagacaga tgggcttgtg caagggagag ttcgagcctt    1500
   ccggacg                                                              1507
<210> 77
   <211> 1309
   <212> DNA
   <213> Homo sapiens
<400> 77
   cgggcagagc agacccgcgc tgagcctggt ccaggccatg ctgttctctg cgtcctgctc     60
   gctgcccccc gccccaaaac agagttagtc ctggagctgg ggggtcgcgc gttgctgctg    120
   gggcaccacg ccaggacccc ccaccccacc cggcctcacc tgcgcttccc caccaggccc    180
   tgtagcagcc gctggagccg cgcgccctcc cgcaggcggc tgagctcgct ggtgaacgtc    240
   ccgtctgagt gtcgccgggc cctgcgggcg gtcgggggtc ggggtcaggg cgcactgggg    300
   gcgggtgggg cccggggggc gggcgggcct ggcgggcggc tcacctgggg ggcgcggggc    360
   gcgcggcgga gcccccgagg agcagcagca gcagcaggag gggccggggg gccatggcgg    420
   ggtcggggcg caggagctga gcgctgcggc cacggccccg ggcccccgca aggccccttt    480
   atggcggcgc ccggaaaggg gacggcccgg ccgcgcccag cgatcccggg aaggtcaggg    540
   ccgccccccc agctgtcgcc ccggcccggc ctcccgcccc ctccgccgcg ctggcgcccc    600
   tcggccggct gggggagggg gacgtcccgg cctggggggc gaacggagcg cgggcctggg    660
   acccgcagag ggaaccgcac cgcgaagcct cggagcctgc gggggcggag gggccggagg    720
   gcttggggga gggtttgggg ggggactggg gggccccggg gaaggggctg cggccggagt    780
   tccggaggcc cctggagcac cttgctgggg cgagttcgtc tccagcttca gcgtggcgtg    840
   actgttgggg ggccggtgtt gccattttat ctgctttgaa gtgcgcgatt caggatcatt    900
   cggtgcatcc atccccacca tccgttctca gactttcccg tcctcccaaa atccgttcac    960
   agacgctccc gtcctcccaa aatccgttca cagacgctcc cgtcctccca aaatccgttc   1020
   acagacgctc ccgtcctccc aaaatcgttc acagacgctc ccgtcctccc aaaatcgttc   1080
   acagacgctc ccgtcctccc aaaatcgttc acagacgctc ccgtcctccc aaaatccgtt   1140
   ctcagactct tccgtcctcc aaaaatccat tctcagactc ttccgtcctc ccaaaatccg   1200
   ttctcagact ctcccgtcct ctcaaaatct gttcacagac gctcccgtcc tcccaaaatc   1260
   cgttcacaga cgctcccgtc ctcccaaaat ccgttcacag acgctcccg               1309
<210> 78
   <211> 2608
   <212> DNA
   <213> Homo sapiens
<400> 78
   cgggggcggg gcgcggaggg aaagaagggg tggccaggcc caggccaggc tgcaggcttt     60
   ggcgacctct cagagctggg agcggggtcc cgaaggcggg tctgagggca gagcagagtc    120
   agaggtgtct atgggcgtcc ccgacctccc aggctgcccc gtggtttccc gccctgcttg    180
   ggggtgagcg ggggttagtg ggcgggttag tctagggccg gggccgccgc acggggcgag    240
   gccctgctta ccagtcccgg tcagggtggg ctgcgggagc ctcaggctgg aaggcagagg    300
   acagtgagag cccgccccca gggtggaccg gggcaggggc gggccgtggg gcggcgcggg    360
   gcacgtaccc acggggaagt agtgggggag ccggctccgg tagatgtcct cgtccttctc    420
   gaggaacacg cagatgatca gccggtccac ctgcgccagg ggctgctcag cctgggccga    480
   gctgccacgc ctggccccag gatcctgtga ctgcctgccc tggtgcaggc tttcacctgc    540
   ctggccctgc gcccttcacg gaggctcacg gctgtgtgcc atcaccccca tctcgccacc    600
   gccaggcgcc cttgcgcagg agaaacaggg aaggaagggg cgggcctggc cctgcgctct    660
   gcaccctgac tccctgggcg atgatggggg ctggtcactc tggccgggat gcggacccca 720
   ccctcgcccg ggcccaggac tcaccttgtc cttgtgctgc tccagccact ctcgcagcgt 780
   ggccagcacg atctcggcgg ccgcctcaca ggggtagcct gaggcgggtg gggcgggagt 840
   gagtcctagg ctctggcccc gcccactccc ctgtccgccc cggcccctcc cggcgtctgg 900
   gtccaccgcc cactcctggt ccttgccttt cttccagact caccaaacac gccggtggag 960
   atgcagggga acgcctgggc ggggaggggt gagagggggt tggaacattg tcactgcgcc 1020
   ccgcctcccg ccctcccccg cgggccgtcc ctcaccaccg agcggagccg gtgctccagc 1080
   agcaggtcca gactgctcag gtagcagctg cggagctcgg cagcctgact ggcgctgggc 1140
   tccccgtagg cgatgggccc cactgtgtgg atgacgtcta cggggggcga cggggtcaga 1200
   ccggcggggg tctacgcggt cctcagctcc ctcgcttccc cctgccggcc tgggcagaag 1260
   ggggcaggaa acacctttcc ccccaagcgc tgagcctcct ccgcgaaggc ccccacccct 1320
   gtgggccccc tcgagcccga atccgcacgc gcacagagcc ccgcgccccc tccccacatc 1380
   tgtatgggcc cggctgagac ccgagcgcga gtgtgggcgc gaaggcgttg ccccccagct 1440
   ccatcctcag cctccacgca cggtctcccc catgtgcttc ctggggtgtg cggggtgggg 1500
   gccgggtctt ggcccttaag gttaagaagg gccccctgag gagttcccca gcactcctgt 1560
   gggggccgca cccctgatgt cccagtgaca cacgggcgcc ctgtctgcgc cccacagctg 1620
   ggggcgcgtc ggggactcac acttggccgg gagccgatag ccgccggtga tcttggcctt 1680
   gccagtctta cagctctgca gggtccggca ctcgtcggta agcagggggc cggcggcccg 1740
   atgaatgcag ccgtccactg cgggaagggc gggcgcgact gagctggcct ggcaaggctg 1800
   cggccccggg gtgagcaccc tcagtcccga gaagcccctc ggcgatgcga ggacccatgc 1860
   ggagccgcgc cccaaccccg tggcctccgg ccgcggccgc tgcccccacc cccgcccgga 1920
   atgtttcaca tctggggaaa ccgaggcgca gagcgggacg gccctgggcc tggcgggaag 1980
   tcgcgcaagg aggccggcag ggggcgcacc cggcccaccc tgtcccttcc aggccgcgcc 2040
   gccagggccc aggcagggcg gctccgggag gcgggggccg ccgggacagg aaccgccgcc 2100
   cgagccgggg tgacggggaa gcttcctccc cgacgcccct ccctagccct gtcccgggcg 2160
   cgctgcaggg cccgcgacca gtcgctgcct ccgcccgggc ctcagcgtcg gtgcgatccc 2220
   aaggcccacc tccccaggcg gcgccccatc acggggcggc ggcaaaccct aggacccatt 2280
   ttgcggatga ggaaaccgag gccaccttgt tacagggtga tcagatctgt agctggagtc 2340
   tgggtcttcc agccgcgcgg ggaggtgagc ccgacgcccg ggcccgagtc tcctggcgcg 2400
   caaaccccag ctggttaatg aaccgcctcg ttagcgcatc cagcttaatt acggccccgc 2460
   atgggcttgt ttatccgagg gcggctgcag ggcctcgcga gcgaccctgg ggacccgggc 2520
   gcagataaac aaccggggcg cagcgggagg catggaggaa acgctccagc agcccaacct 2580
   cgccagggca ccgcgcagga gaggcgcg 2608
<210> 79
   <211> 289
   <212> DNA
   <213> Homo sapiens
<400> 79
   cgcacgagcc ggcagcaggc accagagacg cagggacctg cgcgtcgcat ccagcgcccc 60
   ctccctctcc cgtcccggcg ccggcggtgt gcggcaggca ggagccccca caatcatctg 120
   acgagcccac agtgcgcccg gcggctgcct gggcgtcatc ccgcgcgagc atccgcaggc 180
   tgggctggga gagggcgggg agggggagcg gctggggctc tctctgcatc cccccctcca 240
   gcgtcccccc ggtcccttgc agccttggaa ggtggccggt ggccggtcg 289
<210> 80
   <211> 1959
   <212> DNA
   <213> Homo sapiens
<400> 80
   cgcggaggcg gcggggacgg agggactgcg gctcccgcgg cctcccccgc gcgccgtcag 60
   tgtccggagc cgtacatttt gtcccactcc acgaacgagt ccagttcctt ggcagaggcc 120
   ctagggccca ccttggccag cgccgcctcc aggtccttgt aggagagggg gcgctgcagc 180
   cccgggaggc ccgccccggc cgccgcctgc tggcacagct gccccagctc gcccccagag 240
   aagccctgcg tgccctgcac cagcgccgcc agttcccgct cactgagcgc gcagccctgc 300
   tgggccagcg cccgctgcag gatctgcccg cgggccgggc tgtcgggcag cgccacgtag 360
   aagcggagag agaagcgccg gcgggtcgcc tcgtccagag ccgcgggccg cgaggtggtg 420
   cccacaacca gcacgccgtc agcccccgcg ccgcagcccc cgtccaggca ggccaggagc 480
   ggcacctgca gcgcgccccc tgccgccgcg ccgtcgtccc gggcggggag cagcgcctct 540
   agctcgctga tgaggagtac ggagggtggg cggcagcgcg cggccgcgaa ggcggcctgg 600
   aggaggcgcg cgccctcggc ggcgccgggc gcagccaggg tcgcgccgcg caggcgcaac 660
   agcgtggcgc ccagctgcgt ggcgaggcag cggcccagca gcgctttgcc cgcgccccgc 720
   ggcccaaaga gcaggacggt ccgcggcggg cgcaggctgc ccgggtaggc gggcggcctg 780
   agcaggggcc acaccagctc ctcctccagc gccgccttga gcgcgccctg gcccgccaca 840
   tccgcccact gcaccggggg cccgcagtcc accatcttgc tcgtcaccag ctccagggcc 900
   ccagggtcca cgcctttggg agtctccccc gacggcacgg cgaacccccc acgaggagcc 960
   ggggcccgct ccgggaactt ttcaaagggc tccagttgcg ggccgtagac gggggagccc 1020
   aggaccttga gggggacgcc gccaccgtac ttgcccgacg cctcctccgc ggctcctggc 1080
   ggcttggccc ggaacccgtt gccccgacat tcgccgttgt ccgcggcggg gtaggaggct   1140
   ccgtcagcca cgggggcctt ggcgggctcg tacgcgtact tgcggtagcg gccctcgggc   1200
   ccctcgtcgg cggccttgcg cttcagcgac agcccggatt cggcacccgg cgcggccgtg   1260
   gggaagccat aggcggtggg cggtgccggc gcgggcaggg gcgtgggcgc gggcaggccc   1320
   ggggtcaggt agggggccgg gggtgggcct gggggcggcg ggagcgcgcc atagccgggc   1380
   tgcgctgcgt agccccctgc gggatagttg tacagcggcg ctgagggccc gtaccccgga   1440
   ggcggtgggg gctgcaggag cgcggccggg ggcggcgggg gcagcgcggc gcccgtctgc   1500
   gcgcagtaac ccggcgccag gtaccccccg ccgtagccgg ccgcgtactc gggcgccgcc   1560
   gatgggcccc cgcacgcatt gccggcgtag aggggttcag ggaggttccc ggctaaaact   1620
   ggggagcccc ccagggcccc ggaaccgccg ccaccgcccg atttggttcc tgggaggcct   1680
   tcgtggagtg aggccaaggg gtagggtggc tccggccctg gccagggctc gggatcccct   1740
   ttggcgccgt tgaggaagga ggcgtcgctg tacccgccca gggccggacg ctcgtaggga   1800
   gaatccaaga ccccagagta cttctctgca tagcgcttta ggaggttgga ggcagtgagg   1860
   gctgagatgt cgtcgtgtgc ccaagcgtag tggcagcgtt ggcgaccccc agggggcaac    1920
   tccaacttgt gggccggcga cggggtggtg gaggagacg                          1959
<210> 81
   <211> 329
   <212> DNA
   <213> Homo sapiens
<400> 81
   cgcccagtgg cccgcccgct gccgcccccg gccatcccct ggcccccggc cctcacccgg     60
   cggcgccctc ctcctggggg cccaggcccc gccgctacac ggtgctgagc gtgggtcccg    120
   gaggcctgcg cagcgggagg ctgcccctgc agccccgcgt ccagctggat gagcgcggcc    180
   ggcagcgcgg ggacttctcg ctatggctgc gcccagcccg gcgcgcggac gccggcgagt    240
   accgcgccgc ggtgcacctc agggaccgcg ccctctcctg ccgcctccgt ctgcgcctgg    300
   gccaggcctc gagtatgtgg ggcgggacg                                      329
<210> 82
   <211> 454
   <212> DNA
   <213> Homo sapiens
<400> 82
   cggaggccgg taaatatgta aacgtgggtt tttcggtttc cgtttcgcat gaatacaaaa 60
   taaactgcgg ccctgcccac gctggaaagt tcttccttaa agttaataaa atctgtcaac 120
   ttccataaag gccgagagaa agggggtgca caggtgcaga cgttcgggct gaggctcgag 180
   aagtcgaggc agctaataaa aagccctggg caccgcgcgc ctccttcccc atcaattccc 240
   atcaagcgtt attagagtca tctgccgtct ccgaggtgcg tcccccgcgc tcgccccgca 300
   gaagcgacaa tgacatttgg ctgcgctgca ggtgcggctg tgtgtggccg cgcggccgga 360
   gtggggccgc ctttgtggcg ctcgggtcct ttgatctggg ccacaaaggg ccgagaagct 420
   gcagcccccg gctttctgcg gacgactccg cccg 454
<210> 83
   <211> 227
   <212> DNA
   <213> Homo sapiens
<400> 83
<210> 84
   <211> 2895
   <212> DNA
   <213> Homo sapiens
<400> 84
   cgtgggcggt gagcggccgg gagaggggcg ggccagctga gcgctcaatc acagccccgg 60
   ggctcggcgc ccgccagctg cactcagaac ccccctcacc gtggccctgc cctgtggcgc 120
   ctaattacgt gcccccaggg ctctggagcg gctgctggga ctgcagcgaa gaggggcccg 180
   cgccacctcc gccaggctcc acgcgccggc cttgagtgcg ctcccctcgg ccgccgggaa 240
   acctagaggc gcggtttggc cccaaggtcc ccggtcccgc tcttctttgt ctcggcctag 300
   ttctcggctg tctccgggcc gcgacccacc tctcctggcg gtccttgccg catgcttctg 360
   tgctgcgcca gaatcatcga gtctcagttc tcgctgcctt cccggccagt aggcgtctcc 420
   cccagctggg gagggtcccc caaaccctgt gcttctagga ccgcggggcc ggcaagcggc 480
   aggatctcca agctgcgtct ctgatgcggc taggggaggc gcgcgatgaa ggcccccctc 540
   ttctccgcct tctctctacg ccccaccgct actacacgtc gcgctgcgcc gcgaccccgg 600
   cgctgcctgt tggcgcctta agcggctctt ctgccgcggt gcgatccccg acgccaggag 660
   gactaacccc cacccgccag gcctcagacc ccacgcagat ccagccacac acgcaaccgc 720
   ctgcagatac ccaccacagc cctttaagtt aacccaaagc cccgtacagg cgcactgccc 780
   ctacatgcgc agccccacgg ccccacacac ccaccagccc actcgcaaac aggcccacat 840
   ggccacacaa ctgccctctg gacactcagc cccacgcagg cacaatccca tatacacaca 900
   accgcacaca ggcgcaatcc cacccacgca gtacctcacg gagtcacaca acccccctat 960
   aaacaatcgc acagacacag ccccacgcag cgccacatca ttcagtcaaa ctgagaccca 1020
   gaaacacatt cactagcttg tatccagacc ccccccccca agccccgtga cattcactcg 1080
   taggacagac cagaaatata cagccacatt gatacacaca cggacctaaa cgcacagcca 1140
   agacctcacc cgcaggctcg taagtcactg gaaaaatatt aaagcgttcc ctccctcccc 1200
   tcctctatct ttgacataca cacgcctgcg cgcgcgcgca ctcttacact cacacccccc 1260
   gtccctaagc gcgtcggagc ctcgcagcgg gtgaccccga ggccccgcgg tgagagccaa 1320
   gggagcgtct ccccggcgcc ccctcctccc gctcccttcc cgagctcatc tctcgccccc 1380
   ccgcccgcct cctccttccc tgccccctcc tcctcctccc ttcccccgag tctggggcgc 1440
   gggctgcgcg cagccaagcc ccctcatcgc gcgcccctcc ccgcgcgcgc cccgcgccag 1500
   catcgcgctt accgagtgga tgcggccacg gggccgtctc taattacctc ctgacccccg 1560
   gcggcctgtg aataatggct actgagggcg ctcgcagggg ctgctaagaa ggaaacgcgg 1620
   ggcgcgagag gcgcggcgcg cgcgggagcg cgagggaaag gagcgagcct ggggccgttg 1680
   caagcggctg gcgagagaaa ggattcgtgc gagcgctcac atttgtttct cccaaggatc 1740
   tcatctggcc accgcgttct ggaagaggcg agagggagag cgtgcgcgag aggagagaga 1800
   cggcggggga aagggagacg gcgagacgcg cagcgccggc gcccgggaga cccaggagga 1860
   gcccgcaggt attgttggcg gcggagcttt gtgttttcgg cgggctgctg tgggcgccag 1920
   ggctccccga gcccccggcc cgccgccgcg cgcagacctg cccggagtcc ggggctgcag 1980
   gctggggtcg ccgcccgccg cggacacccc tcccgcggcg ccaggatctt ggctggagtt 2040
   gcggatttgc atgtttgggg gttttccacg ctggttgagg gaatcggtct cagaagaagg 2100
   aatttctttc tttccttttt tttttttttt ttttggccgc ttttggccac agtttggttg 2160
   tgctggggat tcccccctcc cctttcctcc acaaagcatt ggtgagcggt cttgacgaat 2220
   gtggtattgg cggaaggcat ggggctgaga tggcggggct ggtggccacg gatggggagg 2280
   cgagagatgc cagagaagga aggagttctg ttcaagttcg caatctgcgc cccgaagctg 2340
   gtcggaccgg ggagggcgat ccgcgcgtcc gatcctgctc cgggcaaccg atgggaaact 2400
   tcccgagagc gcggcctccc tggggactcg gcgcccctga cgcctccagg cgcttcctcg 2460
   ggccgctgcg gcccctcctc gccgcccgcc agcgcttaag gtcgcgctct ccccaccccc 2520
   gccttcctcc cttcctcggc ggcttccgga tctggacaga gctgcaggag tggcttcaaa 2580
   cacttcccct ggagacccct ccccactccc cgcacgagag cctgcccagt ggcccccctg 2640
   ccctgcgccc cagtctcacg cgcggtcgag tggccaggct gacaatgaaa tctcgcacct 2700
   ctgccggggg gagtggggtg gggaggaggt ccctcctggg gctgctgacc ctgggcctcg 2760
   ggcaggggga gggggctcct gggggagggg cttgttttgg ttagagacgt cgacctggga 2820
   tggcgccggg gcagggaggg ggctgggggg tgggcgcggc gaaggcggca ggcttgggtt 2880
   cgcggccggg ggtcg 2895
<210> 85
   <211> 1202
   <212> DNA
   <213> Homo sapiens
<400> 85
   cgcccaaccc tcgagtctgc acggtgcagt accgtctatt ttgggtccag ggtaacctgg 60
   gtgcaaacgc tcctgcgggc ggaccgcggg gtgggccccg ggcacacagg aggatcagca 120
   gcagccggtc ggtaggcgga gcgctgccag aggcttgggc ctcacaacaa ccctcacagg 180
   accaaaacaa acggcgagac gggctgcgcg cacaatagca tacccggcgg gctagccgag 240
   tcgggggtct cggctcgcgg ctgcgccaag tttcagaacc acctagcccc gtgcctgccg 300
   cccggggccc ggcctcgccc cggccggcgc tcacctggcg gtgccgcccc cggaagacca 360
   cggcgaaggc cccgtgtccc acgagatccc tcttgctgta ctcgaagtca cccaccacct 420
   ccatggccgc gcccccgggg cacacagcgg acgggcgggc ggcgcagtgc ggcgcaggta 480
   tcagcaccgc ggctccgcgg gcccggagcg cgccagcgtg cggcgggtct ggggcagccg 540
   cagccccggg cccgggcgga ctctcatgcc gagagaccgg agcggaaact ggggaagctg 600
   ccgcgcggag ccagggtcag cgaggcccgg cccggcccct gccgctcatg gcccggcctg 660
   ccgccggccg ctggctgtct ggcgaagcgg cctcggcgct gccgggccag gggtgccgtc 720
   accgtcactg tgcgcgccca gagccgcaca cgcgctctga ggcagtgagg ggcccggtac 780
   tctccatccc cagctcgtcg ggcctcctcg gccctccccg ctcggggcgc tgctgcgacg 840
   gcgacggccg ccgccctaga acccgcaccg ccgcggcccc gcgcttcccc gcctcgcggc 900
   ggcgcccaac gccctcgcgc gcgctacccg ctcccgcgcg actggctgcc ctccgcgacg 960
   tccgcgcgcc ttgaaggccg cagcgccagc cggctccctc cgccggggaa aggagtgcgg 1020
   ccagggcttg gcgcccgcag ctgggcagat cctcggccgc tcctgaggcg gggacgaagg 1080
   gcgcgacccg ctgcggccac agcgggttac gggctgcggg ctccgttctg ggcctgggga 1140
   gcagattcga gccgaccacg ccccgggctc caggccgctg ggaggcggac ttcagagaag 1200
   cg 1202
<210> 86
   <211> 1198
   <212> DNA
   <213> Homo sapiens
<400> 86
   cggtgttgct cccacgtacc gatgtcacgt ggggcgggga ggcggggcgg agaacggaga      60
   gcgtcctctc attctccacc cccttcctcc aagtccagcg cagggggagg tggctgcgac    120
   tgactgaggg gctagggaag gctggccagg ggcgtgggcg tggctgggga cggcttgggg    180
   gtggggctgc gcaggaggct ggaggagccc cgcgggaccc agaggcgggg cgtcggcccg    240
   gggaccactg ctcctccggg gcgtggctgc aggaggctgg aggagccccg cgggacccag    300
   aggcggggcg tcggccgggg accgctgctc ctccggggcg tggctgctgc cgagcatctc    360
   ccagctcagc cgagcccgtg cccaggccac gctttgttcc agccgccgcc tcctctaccc    420
   tacggcgtcc ggagccatcc ctcgcctgct cgctctctcc tttcgcccac tccctgcatc    480
   tgggcctgca tcacctttgc caaccgctcc cccgatcctg ccgacactcc tcccccaaac    540
   ttctgaccgg cacccttgcc tggtaccctt ctctccattc ctccccctcc atcttctttc    600
   cccgacccct ctcgggtccc tcttttccca aaacccgggt ctctccgcgt ggccccgcct    660
   ccaggccggg gatgtccccc gcggccccgc gcccatggtc ctgacgctgc ttctctccgc    720
   ctacaagctg tgtcgcttct tcgccatgtc gggcccacgg cggggcgccg agcggctggc    780
   ggtgcctggg ccagatgggg gcggtggcac gggcccatgg tgggctgcgg gtggcccggg    840
   cccgcgaaag tgtcgccggg ggcaggcacc gaggtgcagg acgccctgga gcgcgcgctg    900
   ccggagctgc agcaggcctt gtccgcgctg aagcaggcgg gcggcgcgcg ggccgtgggc    960
   gccggcctgg ccgaggtctt ccaactggtg gaggaggcct ggctgctgcg gccgtgggcc   1020
   gcgaggtagc ccagggtctg tgcgacgcca tccgcctcga tggcggcctc gacctgctgt   1080
   tgcggctgct gcaggcgccg gagttggaga cgcgtgtgca ggccgcgcgc ctgctggagc   1140
   agatcctggt ggctgagaac cggtgagcgc gccaggccgg ggttgggaga gcgccgcg     1198
<210> 87
   <211> 1837
   <212> DNA
   <213> Homo sapiens
<400> 87
   cgattgtgag cgaggccccg cgccgatggt agggaccagg ccacagccct ttccccagga 60
   gccggcgcga ccttccgacc ttccgacctt ccgcccttcc tttccgaagg agatgaggct 120
   cgcgcgcggc tggcaggggc gggagggacc acgcgacgag tgcgcagcgg ccaggcccgg 180
   gtgggcgcgg ggagggcgga gtggggggcg cccggcccgc caggaggaag cggggtcgcc 240
   gtgaagttgg ttattgaacc catgtggagt caaactccca gggccgggag agccagtgtt 300
   cgcggggagc cgaccccagc ccactctgca tcccagctcg gcgatccttg cgccgactct 360
   gcggggaggc gggggcagaa ttccgcctcg ttcctcccat ccgcaagctc ccacccgggg 420
   acccgggccc gggagctgcg agccggggcg ggcgccgggc tcaggtgccc cctgtaagcg 480
   ctgggcggcc gggaggggac ggggaccgcg cgcgggcagt ttcgtttgga cggaggagga 540
   cgcgcgtttc ctcttggtgc ctgttttctc tccctctgtt ttaaaagcag tgacagcatc 600
   ggggaagtat ccggggccgg gccggggagg gcggcagccg cctctgcctg gggagggagc     660
   tcggggccgc cggcgggagt gcgggcgggg accagaagga gcgggcgagg ggaggcaggg     720
   ccggggccgg ggagggcgcc tgggagccgg gcaggggggt cccgggagcg ctgtccgcct     780
   cctcctgcgc ttcgcccgct ccctgtgccc cgcgtcctcc accatccggg cggccgaggc     840
   cggggcgcgg gaggcggggc cggggcgggt cccggcgcgg gcggcgcgtc tgagcgggag     900
   ccgggagccg ccggcccggg ccgcgccccg agcgagcctc ggaaatgccc gcgccggggg     960
   agcggacggc ggctgccccg cgggccccga ggagacgctg aaggtcgccg ggagggaggg    1020
   gagagcgcgc ggtcacctga cccccggggc gcacctaggc ggggcggggc ccatgcgagg    1080
   cggaggccgg gggccggggc gcgggctcag gggccgggtg cggagcgcgc ggagccgggg    1140
   ctgcggggac gcgggcgggc gaggtcgggc accgcgggcg gcctcgccat gtcccagccc    1200
   gcggggagga ggcattgccg aaaggcgggg atccgcgccg ccgtggtgct catcggactc    1260
   ctgcacaaat cccggaggca gaataaagag aaaaggtaaa cccctgcagc gccggccgct    1320
   tccctccgct ctcctgctgc atgccagttt tctttggggt ggacttttcc gatgggtgtt    1380
   tggttccgtg ttggttagaa aaaagcctcc tctccaggta ccgtcgtgcg aaccgagttc    1440
   ggagcttcgc cccggaccct ggctggtgcg aggagcctgg gcaggacctt ggcggaatcg    1500
   gagggccgct gggagggcgc atcggggtgg atccctgacg gagggacccc aagtccgtcg    1560
   cccccagacc ctttgcagac cgcgaccccg ttccccaagc ctgcagccgc ggatgccatt    1620
   gatccagccc aggcggctcc atgcgccggt gttgccattt gcttcctgca acttggctga    1680
   gccctcgagg tgcctgcctg gccgagatcc gcttgctctg cgctcggtgc cggcaggcga    1740
   ggagcaagtt tggaggttgt tcgctcccca cccaccctct cgccccaccg ccatagggtg    1800
   actttgcctc ttcgcctttc tggcgtttgt tctttcg                             1837
<210> 88
   <211> 222
   <212> DNA
   <213> Homo sapiens
<400> 88
   cggctcaccc acgcgtcacc ctcaggttca ccatccagga cctcctggcc cacgccttct      60
   tccgcgagga gcgcggtgtg cacgtggaac tagcggagga ggacgacggc gagaagccgg     120
   gcctcaagct ctggctgcgc atggaggacg cgcggcgcgg ggggcgccca cgggacaacc     180
   aggccatcga gttcctgttc cagctgggcc gggacgcggc cg                        222
<210> 89
   <211> 935
   <212> DNA
   <213> Homo sapiens
<400> 89
   cgggagagag cggcggcggc ggcgccgttc aactgcggca gcagaataag cgcgcgtcgt      60
   tcagcgcagt gtcatcgccg aggcctctag gtccctggat cttggtctgc aggccgcacg     120
   cgcccttggg gcaatggtca ctccagtctc caaagtctcc ccagctcagc ccaggcccct     180
   gcagttcctc gccgtctgaa cagcggaagc gcacgttgtt cgctgctgtg ttgtcaccga     240
   gggtcgtggg tgcctccacg cgaagcgaga aagccactag gtaggcgccg ccgcgacacc     300
   acagcggctc actccattcg ccccagctgc aaggcaaagg gagatgcgtt gcctctgcgg     360
   gccccccatt caccaagccc ttgagacttt ccaagccaag tgctgtgtat atgacctggt     420
   gggctgggtg ctccccgacc tccgccgagg agcggaggag gggtcttcca tccacccttc     480
   gcctccccgg tctatactgc cccgtagctc tggcggagcc agcctgcctg ttgcgacgga     540
   gtatgtaata gagggtaagg gacataccat caccccaagg gatcctcgac ccctgcgccc     600
   caccttccag actgggactc taccacgtgc gtattgccta ggacgttccc gcgcgcgcag     660
   tgcagcctga tcccattcag tgcagtgtcg tcgccaggaa tgccttgggg aggctccacc     720
   tgggtatcga ggagaggggc aagggcgagt cacggaatta tcatccttgg ctctatggtc     780
   ccacgcatct ccgccctctc caggacctac gcctgagccc caaccttgag cgagaacccg     840
   ctggcgaaga atccatcggg acacatctca ggccaggccc agtcgcccca gggacccccg     900
   ctggtcactt cgatgaccgc cgtgtagccg ttccg                                935
<210> 90
   <211> 1079
   <212> DNA
   <213> Homo sapiens
<400> 90
   cgcaggtggg cggcctcctc aggccggggc cttctgggac ccccaagtgc cgtgcaggac     60
   gccacgaagt tgcgcccgag cccgcggggt ccggggagcc gctgcccgtg acagccgcgc    120
   tggcttggga ggctcttcgg gcggccgacg cctcggtggg gccgcgactc ccgtcaggga    180
   agctctcccg gcccggtgcc gcctcctacg cccggcccga gcctcccacg gctgggctca    240
   cggggcatct ggccgccgcc gtcgcgctcc cagctcggac tccagccccg gcgccgcgac    300
   gctgccccgg ccccgacccg gccgtcgcga gctgtcctcc accccacgct ccccggtcac    360
   tcaccccgag gccgggcagc agcagcgtca gcagcagcag cagccgccgg gccggcatgg    420
   cgaggactcg gccctggctc cgggggcggt acggacagag gacggggcgg gggcgccgcg    480
   acgacagcga ggcggtgacc gagcctcagc ggacgcagaa ctgactaggc agcggcggca    540
   cccccattcc cggccctata agcgctgggt gccgcggctt cttccggtcg gggcggagtt    600
   gcgggcgggg acggggcggg gccaatctag gggcgtgttc ggggcggaga tgggggcggg    660
   ccgagggcgt gccccgggcg gggatgtggg aggggctggc tcggggcggg gccgaaggcg    720
   gtgctggcat ctggctggct tcgctcatga ggcgtcggcg gctgtaaagg accaggcctc    780
   ggcatccagc gtctgggggc gctgggggat cgaggcgcga agcttgtctg tggctgggaa    840
   agaccggcaa tgccccacca ggccgtccca ttaaccctgc cgagggacct gccgcgtcag    900
   acttcatcgg ggaaaagggc gggacacggg cggtcccggg tgcctagaag cctccttttc    960
   gtctttccgc cgtggtggaa tcatccgcaa ggtttaccga ggcgacctcc ggagcgaagg   1020
   tttcagcagc taccggaggc ttccagcaaa cacggcgcag ctgccccgcg cggcagtcg    1079
<210> 91
   <211> 885
   <212> DNA
   <213> Homo sapiens
<400> 91
   cgacgccggc gcagagggga cggaagggcc cgcccccagc ccagcgtgca cagaggccat 60
   agccaaggcc ttaaggctca tccaaccggg gactcatatc ccccccaccg gcagcccggc 120
   gccccagcct ctacccgtgc ccgccgagat gctgctgccg cggtcggtgt catcggagcg 180
   ggcccctggg gtgccggagc cggaggagct gtgggaggca gagatggagc ggctgcgcgg 240
   ctctgggacg cccgtgcgcg ggctgcccta tgccatgatg gacaagcgcc tcatctggtg 300
   ggtgccacgc gggcgccaga cggtgcgtgg ggggggtgct gcgaggctgc cccgtcggat 360
   ggtgtgggag caccccgtct gcttggcccg ggtgggcagg gcgggtgact caggggcgcc 420
   cctgtcacca cccccgtcca ggcagctgcg ggagcccgcg ggggtgcaga ccttgcgctg 480
   gcagcggtgg cagcgccggc ggcagacggt ggaaaggcgc ctgcgggagg cagcgcagcg 540
   gctggcccgg ggccttgggc tctgggaggg ggcgctctac gagatcgggg gtaggacccg 600
   cgcgacccgc acctcccctt gccctctctg gagccccact gcccagatcg gaaaaaggag 660
   agtggcatca tcccacctgc cttcacccgg gtcccccgac caatcggccc ctcccccctc 720
   ccaccccttc cgcccgcagg caccgcaaac ctcgggccca cgcagcaccc ccaggtgact 780
   gtcagcggta cctccggact cgggcgggtg ggagcctggc gggcacccca cgccgtccgg 840
   tgggcccgcc cttggactct cagcgcggcc ccagccccgg ccccg 885
<210> 92
   <211> 1485
   <212> DNA
   <213> Homo sapiens
<400> 92
   cggcgggaac tcgcgcggat ccaccggctc ggcacatcct gcctacaagt agggcggcgt 60
   gtgctaggag ccgggccccg ggccgagtcg tccgtgccgc agcggagagc tccgaccggc 120
   gcggccccac catccgctga ccttcgcccg gtccggcctg gagcccggcc gcggcctccc 180
   taggcctctc taggccgggc gccggttcag cgaggaccgg aagcgcccgc cgtccgccct 240
   tccgcctcca ggccgcgggc gccaggccgg gggcgggctg gggacgccct gggagtgccc 300
   cgggggccgc cttcttcccc gcaagcccgg agcgggcgtc gctcccgccc ccgcctcgtc 360
   ttcagttccc ccgtcgcttc cgccgccgtg ggtagaacag acccgagctg ggagccaggc 420
   ctgcggcccc tcgaacacta gccacatctg gcccgccggg cctagcacgg agagaggccc 480
   tggggcttgg cggccggggc cgggagaggt cgcgcggcca aggacacccg agggcctggg 540
   ctgcctcccc gcccttcccc gcccctcccc tcggccctgg aatcaatagt ggccgctgac 600
   ttttcacgac aattgcagct gtctaattca tcagcgaata attaaatcag aggccgccgg 660
   gcctgcctgg agtcggagac gaggggtcgc tcctctcaaa tcctgtcaaa ttggggagcc 720
   agtgggcagc gcggagagcg gacaggcctc gccctgcgcc gaggctgcga gagccgcctg 780
   gacccggcgc cattccgagt gtagggggaa gcaagccccc aggtgtgggg agcaaaagga 840
   aggaggaggg cacaggcggg ggccccgccg aggctggact ccctcgtcag cagggccctc 900
   ctggccggtg cgtggtccgg gtgcccggcg tggggcagcg gccgtggggc ccacagacac 960
   catcctcacg cagacttcgc gaatgtcgtg gaactccatc ctcgggaggc gctttgcctt 1020
   tgaggaagat ggagaggagt cgggagaagc gcctagaaac cgcattgatt tagacatcaa 1080
   tcctggccgg ctccctccgc ctgccgagct gcggggccgc gccgccccgt ccccgagaag 1140
   cggacctagg gtacgccggg ctccgccgag cgggcaaagg gagcggcctg cgccgccacc 1200
   ccgcgcccgc ggcctcgacg ccgcagccgc cacgccaaca ccgattcctc gtggccaaca 1260
   ggtttctcgc agccctccga gtcggcagcg ccgcccgcgt ccctcttcca gccgggccgg 1320
   cgggacgccc tcagagcttg tgctctctcg aggccgccga gcccactgag cccggccggg 1380
   gcgctggttc gcgtgggccg cggggaacag gtcccgcttc tccaacgacc acggccccgg 1440
   cggctgcgaa acggccacct ccgacccttg agtccgccag agtcg 1485
<210> 93
   <211> 3382
   <212> DNA
   <213> Homo sapiens
<400> 93
   cgacatcccg ccttggcctg ccgggcgggc ggccggccct gccccatggc ccgctcaggg     60
   gccagatccg ggctcagctg ggtccggacg gcggccctcg cgcacgtggt tactcccaag    120
   gcgtgcgcaa gcactcgcac accccctggc cctgcggccc cgaaggggac ctaggcgcgc    180
   tcatgcacgt ccctgtctga caccccagcc cggcaactcg gacgtgcgca cgcacataag    240
   cacgctctcg tatagttgct cccacctggg tccgcagcat gcccttgccc ggcgccccca    300
   gtcgggatct caggcaccga aagcttatat gcgcacctag gcctgacatc ccaacgacac    360
   tggctggcgc acttacacct ataacatgcc cctatcgggc accacatggg ggtccctaac 420
   acaagcattc actcccctgc tttccctccg atggagcttt cgagcacagg tactcgagcc 480
   ctgcgcccca acggggagct ccaggtgggt ccgtgagccc gctctggtgc ccacacacat 540
   ggctccaggc ccacgcgggt acacacctcc ccagcctgca cacgcgtgtt tcacatgagt 600
   cacacacctg ccaggccgct cagggcatct ggcttcaaag cggcattcag gttgttgcgg 660
   gagccggcag ggaagggagg cctaattcca cctcccccat ccctctcgga acacccgcct 720
   ccccatatgt ccctggggca caggtgggac atcccagaac tgccgctgcc cagtggcgcc 780
   cctgcatgtg ctatatttat atttgaaatt tgtaccttta tatatacttt ttatatgtaa 840
   gaaccgctcc cccctcttcc cgctccacgt ttgtacttgg attttaccac ccccactagg 900
   cttcgaccca aacctgcaaa aaccatttcg ccaggcaggg atgtaggggg aggcgggagc 960
   agccctcccc ctctccactg cggacggaca tcccacgttc ttaaagaccg gggacccccc 1020
   ctcccccaag ggccggactg cagctccgaa tcccgcagcc ccagaaacac aatcgcgggg 1080
   gtgggtgctg gcggcgagac gttgcttttt ggtgtgtgtt tgctgttacc gcttaaatta 1140
   accctgagtg acggcttgca attcgccaca aagaggccat ttaatgcccc ccaccacccc 1200
   acctcgggag aaatttccac ttgcccccga gtccagctcg ggccgcacgc tacccgccct 1260
   ccaggccttc gctccaggtg cgcccgggcg gtggaggcga gttccctgcg cgcatcatgg 1320
   acttcaggag tgagggtggg tgggttttgg aggggtggga gcagaggcag gggggtgtgc 1380
   cgggactccc cagacgctcc actgcccaat catagctctt tgatatccca gcccggctgg 1440
   agaaagttag atggtactcc agcgtctcct ccgggtcccc ggaagagaga agctgggctg 1500
   caccccatgt tggcacgaac actccctccc cagtgcctct gtgcattagg tcgcgtgcct 1560
   gtacgtaccc tacctgagac cgcccctccg ccaagtgcgc ccctcgacag gtgcccgtcg 1620
   cccacaggct cctttcattg tagcctccct ggaggttctc aaggcgcctg gcatttcctg 1680
   gacccgctgc aactgactca agactggggg gagggggctc atggaggtag aggtggtggg 1740
   gaggtctccc cagggcccag gcaggaggag ggaaggtcgt gtgctttcca tgtcgcgggc 1800
   aaaatgggtg ctgacggtca ctgtcacttg catcgcacac tgaggggtgg gagaggacag 1860
   cacatgcctg gaccgtgcag gaggcccaga accggggcgc gaaggggccg cgggacacga 1920
   gcgggccgtg cgcaaagcct ggcgcgcgcg tgcgcagcga cgaaaaggcg ggaagcggcg 1980
   cttcgaggcc ccggatgtca gacgcgacct gtgtggcatg taccaggctg gctccgacga 2040
   cggaaccgcc atgggtgctg agtggagagt ggggaccgcg gaccccgggg gcaggtgagg 2100
   ggaaactggg agcataggtc tggctttcga ccgtttgtgc cgccagagtc ttctcaatga 2160
   tccctcccag gcaccgtagg ccctctagtg ggtctgggtg gcgtcctccc ggcccctcgc 2220
   tgctcaagcg cggctccctc ttgccggggc gcggcctgcc cctcacagtg acccccccga 2280
   cccacccccc aaaaagtgac cccgcgctgg tgggcatgga ggactgtccc agttcaccat 2340
   ttgctgcgtc aaaggccgcc gccaaaaggg gctccgatgt cggcattttt gccttaaggc 2400
   tagtttgtcc cccaggcgcg cgggggttgg gggttcaggg ccgcctgtgc ctcagtttct 2460
   ctcctcctgc gcccatcctg acatccgacg gaggataatg cgcgttggag ggctttggcc 2520
   gtgaatgcga tgttgatgat tgctgtgatt ctttgggtgc ttgggagagt ttggggttta 2580
   actgccccca aatccgagga cagcttgggg gcgtcccttc ggagaggatc tctccgcgtc 2640
   gcatagtgag cgtcgtcatc tctagactac cccggtttcc gaggagcctc accttcaatc 2700
   cccgagcctt ctcttttggg ggagtaaagg cggggaaatt taccagggga gatttttgtt 2760
   tccagggttg gctgggtttg ggggattttg tcctccagcg agcgcgggga agaaatgtga 2820
   ctaagtggca gaaaatgcgc tttccctttc tctctcttct gccttcgggc cgcggggagt 2880
   tgcgcagact ctaaaaaaaa aacctttaaa aacccaaaac ctcccctcca agtccctttc 2940
   gatctacgcc accttccctc tcagggctga tttcttgaat ttggggcgtc gaggcctccc 3000
   cacccgttgc taccagccct tttgcagcac ttcctgggca ggccagggga gaggacggaa 3060
   aagtgaaaaa agaacgtgtg cgcccagcgg acccgactta acctgttggt ggaggaggaa 3120
   gctggcgtcg ggctgaagtc ccccgagcca cccctggagc cagagcagcc cccgagcatt 3180
   cagagaaagc cgcccgtccc cagtcccctc ggaagccggt cataaacccg acttctcatc 3240
   acccgaatta cttaaatgag ctttgatggt gttaggtcct tttccacgtg cggatgactc 3300
   agcccgctgc agctgcgccc ggggaggtga ccctggggca gcggctgacc ctgctgcgcc 3360
   ctgtaccctg gacccagacg cg 3382
<210> 94
   <211> 246
   <212> DNA
   <213> Homo sapiens
<400> 94
   cgggagtctc ccggggcgtg gggagtaagg ccccgggggt gggggccggg gtgggccggg 60
   cgtgacgcgc ggtcaaagtg caatgatttt tcagttcggt tggctaaaca gggtcagagc 120
   tgagagcgaa gcagaagggg ctccctgtcc ggcccacgtg ccctttccct cgacgacagt 180
   cgagggctcg ggctctgtgg gactgtggga gctagggtct gcggggcgcc tgcccgggcg 240
   aggtcg 246
<210> 95
   <211> 533
   <212> DNA
   <213> Homo sapiens
<400> 95
   cgggtgacgt cacctcctcg gctctgcgcc ccgccgtcca caggcgcgaa gccggagctg      60
   gggttggcga ccgagaacct tcctgcctcc cctccccagc aaggggcacg tcccaccagg     120
   cccggactgc ccgtctgccc ctgcagggcc cctcgctctg gtggcgggct cggtgagctc     180
   tgctgcccgg tctcgggagt ctgggggagc gcggcgtcga cccctgtgga gggaggactc     240
   gtgtttcaag gacagagcct cattaccatg cacgaccagc cgcagctgag gccgcagagg     300
   cctttggcag gggggccggg ccggcggcag ggacggcgtt ggcggtggtg gacgggagtg     360
   ggggtctgtc ctgaacaggt gggggtggtc cctgtgctgg cccccgccat gcgatcgcac     420
   cccaggactc tcctcctctt cctaagcccc agcatgtgcc tggtgccgcg cgggtgctgg     480
   aggcacagcg gcttccacgc agacgcccgc agcaccacag cctgtgcacg gcg            533
<210> 96
   <211> 422
   <212> DNA
   <213> Homo sapiens
<400> 96
   cgagggcccg gcaagccgct gagtccctac gggacagcac ggggcctccg gagagctgtg      60
   gctccctggc ctcacctgca ccccgccagc gtcgggctgc tgctgctggc ggcgcctctg     120
   ctcttccaga agcttcttca cggtttgcgt tgggcagtgg ctgcgctcac ccctgctcac     180
   tgtgcgggag aggagagggg gaagtcatca agggtcctta agtcacctaa atgccgcggg     240
   ggtggcgcgc ggggagtggg tttgggcacc ctcctcgccc tcccgcgcgg aaaaactggc     300
   acatagatgc cgcctgactc ctccaaagcc attagaagat ttggggtggg gcatccgcct     360
   ccgccgtttc cttccttttg ctccgcactt tccctctgac ccaactttcc accaccgtcg     420
   cg                                                                    422
<210> 97
   <211> 592
   <212> DNA
   <213> Homo sapiens
<400> 97
   cgacgccgcg ctccccttca ccaccccagc tcggcccagc gagaggtgca gcacgtgcag 60
   gccgagggcg gggccggcgc ggagagggtg gccctgcatc gtagtaggtc cctcccccgc 120
   agtgctccca gcaggcccgc tcgttctgga gaagggcgtg actcgacctg ggggtggacc 180
   ctaggctgag gggtagggtc ctccggtggg cttggtcccg aaattgaccc gcgcccctcc 240
   ccagttagcc cgcggaccgt ggactgtatt ttcagccccg gatccgaagt gtcccagggg 300
   gcgtggcccg tgcgaggggc ggggcctggg ggcgggacca gagcctgacg tgtgcccgcc 360
   ccggcttcta ggcggatcgt ggaccgcatg gaccgcgcgg gggacggcga cggctgggtg 420
   tcgctggccg agcttcgcgc gtggatcgcg cacacgcagc agcggcacat acgggactcg 480
   gtgagcgcgg cctgggacac gtacgacacg gaccgcgacg ggcgtgtggg ttgggaggag 540
   ctgcgcaacg ccacctatgg ccactacgcg cccggtacgc ggcgagcccc cg 592
<210> 98
   <211> 2019
   <212> DNA
   <213> Homo sapiens
<400> 98
   cggtgggcga ggaggagcgg cgctgcccgc agccagggcc agcgccctcg aggggcagag 60
   gcgggaccgg cggcggcgaa ctcgcggcct cgggcagggg cggcggcggc ggcggcggcg 120
   gcagctgctg ctccctggac gccttggggt ccgcggcgca gccgttaggc acgtggttcc 180
   cagggagttc cgcctgcggg gatgaaggtg ggggctcacc tcccagctta gggagaggct 240
   gaggggtctg ggtcacatct ggccgggggc gcgtgcagag ccgcggtcag gtgtggcaga 300
   gcagttgggg cccacgtagc atccgcgacg gctgcgccgc ctgcggggga gcggaggggc 360
   cttcgagcga gccgcgggcg gcagggccga ggcgcgggca gccggcgggc gcgggctggc 420
   gggcacgcac ctcctcgcgg tgcgccatgc ccgggcgtgc gaccagcgtc tcgccggggc 480
   agcggcccag ctgccggtag tagtcccccg tgctgggctg cttgctgaaa gcgcggggct 540
   tgcggctgga gtcctgcctc cgtagcccgt cgtggccgtc gcaggagctc ggctgcggga 600
   agacagtgac cggtggggct cgggcaagtg cccggtaggc gccccccacg cctccccacc 660
   cagctcttca cctcctggga actcgcggcc agcggccgtc ggggggtgca gcagccgact 720
   tcctagaccc cctgttctca cggtgggcag cgggcgaggt tcatgggggc ctcggtggaa 780
   gggcaggctc cgcccgcctt acagggaggg gttctgggca ccggccaagg ggcacagggt 840
   cccccactga ggccagaagg ggcgggccca ggggcgggcc ggcccagccc cgacgccagg 900
   gggagctaga gaaggggcac ctcccagctt agccttcact aggccctcgg ccgcactccg 960
   ctctcggctg tcagaaggac tgcgggtccc cagggctccg cggagccctg tctttcgggg 1020
   gtcccgggcc ggagggagcc ccctccagag cctgtgctct ccgaggctcc ggcttgcccc 1080
   ggaccccgct tgtccgtcta ggggctgctc caacctgcca cggtgctggt ggtcctgctg 1140
   tgcacctggc ggcgggggcg gcaggacccg acacctgctt tacgtgatac ttcctctcag 1200
   gttacagacg cccgcgcacg gccagcctat gggctccgac ggcctgacat cacccggggc 1260
   ccgccaatcc caggccgaac cccccccagc cgtcgcggat gccacggggg cgccaactac 1320
   tctgccacac ctggccgcgg ctctgcaccc gccccgggcc agatgtgacc ccgccccctg 1380
   cgcctctccc taagctggga gctgagcccc caccttcatc cccgcccgag aggagagagg 1440
   gctgaccgtg ggcagagggg gcctctcata tttggctgcc ggctccgggt cgcgtcccca 1500
   ccgtttccct cctgcatctg gaaaccatcg ccatccacga aagcgacacc gacacccgcg   1560
   ctcaagcctc ggatttcagg ggccgtaagg cggggtcggg tgacagcgcg gcttcccgcc   1620
   ccgtcgcagc tgcccccaac taggcccagc tcagtgaggg agagtgaggc ggccgggcca   1680
   aagactgagt gaccgggtgg gggctgtccc ctgccccact ctccagccca tgcgtccctg   1740
   cggtggcctc agacccttca ccccgcccga cctggctcac gttgcaggaa acgcgacacc   1800
   gcaggattcg ttttctgggc cagcccgccg gctccgcgcc ccctgcagcc cggaggctcc   1860
   gacgccacga ccctgctccc acctgcggtc aggcacccgc gcgggaggcg ccgcggcgac   1920
   acaaagagcc ctttgtagag cttcccggcc cgggccctgg cgtctgcggc ccagcacgca   1980
   cacagccggg agggacgcac acagccggga gggggctcg                          2019
<210> 99
   <211> 459
   <212> DNA
   <213> Homo sapiens
<400> 99
   cgcgcgggga aagatgtgtc tgccacacct tttgcgagcc caggaaactt gaaccgctcc     60
   tgaaaatcct gatatttttt cccgctgcag cccgcacggg tcaagcgcgc gggggctgac    120
   caacactttc caggtcaggt cgagttgatt gctcccagct cacgctccga aatgcggtgc    180
   tgaaccacgg gaaggagggg gtggggagga aggcaggcag ttgcatcctc gcgagtgggg    240
   agtgcgaggc ctcctcagct ccagccagct gggtgaatgg ggcgcgcacg gccccgtaac    300
   gccgagcgtg gataaaatat gctaggactg cgcttctccg gctcatcaag gctgcgggag    360
   cctccccgag ccatcatcct tcgtaattgc aaccgtcaat caacacttta agacaaagtc    420
   agaaccggga agggcgagcc ctggagacct gtctcgtcg                           459
<210> 100
   <211> 4037
   <212> DNA
   <213> Homo sapiens
<400> 100
   cggccagtga gcgcttcctc catccggggc tcgggcggcg agctggctgc aagactgggc      60
   accctggagc cgtgggagcc ttgggagccg aatctgggtt cgaatctgag cagggaagcg     120
   gtgccgaccg cggaggatcc aaggcagaag gcccagcgca ccgccgccgc cagctcctgc     180
   ccaacccggc tccgctgggc ttttcctctg gggtggcgcc ggccccgcgc ccctccccat     240
   ccggggcgcc tctgctagga caccggggtc tcggggtttc tcagggctgc tgcgtttcac     300
   ctcctttaac gcggaggcgc ggagttgcac gtgtgggtct cagtggagcc gccacaggtc     360
   ttattacaca acaaagggca gggagggcaa ggccaggagc ctcgcggggc ggcgcagaga     420
   ccccgcatcg cgggcgcgac ccctgcctcg ggctcaggct gactctgcgg cagctccaag     480
   tcccgttgcc gggcaggact tcggtgctcc ggaagggtgc ggggcagcaa cctggccagg 540
   gcagcgcctc ggtttccttc caattcattc tcgattccca gatactcaga tgagaaatcc 600
   gcactcaggc cgtcaggctc ctgggcccgg gccaccctgc ctgcgctcag cccggcccct 660
   ccgccgcagc gcctcggagt ttgggggcta caggggaaga ggaagcgtct ggtcggctcc 720
   cgtccgagct gctccacctg cgccgctggc ctttccgccc gctacgcacc tgggccccga 780
   gaggccagag gggagtgtcc ccagtggccc gggtccccga aggtggccca gctcgggcgc 840
   tcgcgcagcc gggtactcac cttgggggcg cggcggcagg agagcgcggg cggagaggtg 900
   cgggcgccct gggacgacca gccgaggacg cctggtgttt ggacggctgc ccccgcgctg 960
   tccccccgtg cgaggcgtgt tggcctccgt ggcctcgcga gggaccgagg gcggcgggtc 1020
   cgagtgccgc ccgccggcct gggcagtggc ggagcgcgga gctggcggcg cctccgctgt 1080
   tgtttttgag ccgcgaagcc cgaagcagca gccgttgggt gggggcgaag gggaggaggc 1140
   gggggccggg aggcgcgagg ggggcggtgg ggcccggagg gggcttcgga ggagccgggt 1200
   tctggcgcgc gagccccgac gccccctcca gcgatcgcag ggcggggacg ggggccgagg 1260
   ggagaatgca ggagaaaagc gggccggggc gacggggaac gcaaagcttt taaattagta 1320
   atcaaacgca gtaaaacgga gcaaaaccag cccggactcg cgtccgaact atcggcgcgt 1380
   gtgccccgag atccaaggaa gtcgtgtgga aaaccgcagc cgccgcccca tcttcggccc 1440
   cggctggggc gggagcagca gcgcgacgcg ccgcgacgca ggggcagggt ggggggatag 1500
   tcacctcgcc tccggggcca cctgggtcct tttaaagtgt ccggggtgcg ggccccgggc 1560
   gggagcgccg aacacggaat gttttcttaa agggccagtt ccgagctgcg ctgaaaaggg 1620
   ggggggggag aggggggaga gataagtgag acgaagaccg agaaggccga ggagagcccc 1680
   ccaatcccgc gccgaggcgg cggcggcggc ggcggcggcg cgacgatgag gatgatgaat 1740
   acattgcaaa gtttttttgg ccccggcgag gggtgtcaga ttgagtgctc tgtgcgcatg 1800
   tgcgaaggtg tccaaactga caatgctggg gagatgaaga tagtgtgtgg ctgcttctgg 1860
   actcaaggag gaggagagag attccgcgag ccgacaccat gcgatccaag gcgagggcga 1920
   ggaagctagc caaaagtaag tctcccgcgc tcggccgcgc cgcgccgccg gggcccgggc 1980
   cgccgggccg gggcgcccgg gccaggggtg cgcgtcgggg cgcggccggc gcgcctgcgg 2040
   ctccgggccc ccggctcggc cgcgcggccc ggggggctgc tccgcctccc gcgctccggg 2100
   gcgaccgggc tcggcgcgga ggctcggggc gcccgggccg cgcgctcccc gaaggcgccg 2160
   gccccctccc cgcggaaccc cctccccccc caccagtgtc aggcgctcgg gcccgggaac 2220
   ccgaggcgcg cggtgggggc cggggagggg ggcggagggg gagggccggg ggtggagggg 2280
   agcgaaaagt gaaagttagc gaaaagttga cgaaagggga gagcaacttt ttcctccccg 2340
   ctcgctctct ccctctctct ctccgagtgg ctctcagtcc tggtcaaatc atattccggg 2400
   cttttgaaat agtgggcgct agagcaccgc ctttggcgtc cgccagccgg gcgcagagga 2460
   gggagacccc gagccgggga ggggcggagg agggggcgcg ggccggcgcc cacccggctc 2520
   cgcgggcgca ggggcagggg tggcgacggc gggacagccg cagccacttg gggagcaaaa 2580
   tggagacttt tgcccgggct gggaactgtg gtcgtgaagt ttatccccgg ctgtgccccg 2640
   cgtggtggag ggcgctcctc tgggacgccg gcagcctgcg cgggccgccg gggcgacagt 2700
   gcctccgaca ggagggaccc acccggccac ctcccgaccc gccgctgccg ccctcgggcc 2760
   cgcgtggacc cctctgtacc cgcttgcctg gcacagccgg gcccggcggg gacagagctg 2820
   ccccggccag gctgcccggt cctcggtctt actttctctt tcgctccgtc tcggccgagc 2880
   cccgggctcc gcgcgccccg cctgccgcca gccggtcctt gccgcgggcc gggtgctcca 2940
   gccacccgcg ccctcgcccg ccacagcgcc tccgagcagc ccgcgcgcac cccgcagccc 3000
   cggtcgccgg cgccatccgc ggtgaggcgg gcgggcaggt gaggacaggc ggagccgcgg 3060
   cccggccagc gcggctcccg cagccccgcc gaagccccgg ctgcagcgaa gcggccagcg 3120
   ccgcgcggag cggaatcggc gccgcctgct cccgccgccc cctcctctgc aggccgcccg 3180
   cccgcccacc cgcagaagag gcgcccgggc cgctcccctc gaggtccccg cccgacccac 3240
   gccgttccgt ccgcgcctcc ggggccgggt ccctccaggc cggcctcgca gctcccgggg 3300
   ccgagccccc aaacgtgagc cgggtgcgcg cgggactcgc ggcggttttc cgaagctccg 3360
   ctccctcggc tcggggcccg cccggctcgt cccaacagct gccccgcggc cgtaggcccc 3420
   gcgctctgtg caccagggaa aagcagctcc cgagtttgtg ccggacgctc ctgcgccccg 3480
   ccatcggccg ttcccggccc ctgggtgagg cgcccccttc ccgccgcgct ccgctgccgg 3540
   gtgccctcgg cctcggcctc ccgtgacctc tgacccctct tggtgcgaac aagaccgggc 3600
   gtttcgccgc cgacgcgaag gggctgtctg tgcgcggcgt tgcgggccct ccgcgcgtgg 3660
   ggtgtgcgtg tgcgtgttcg ggttcggttc tgtgtgtgca ccgcgggcct gctcagagtc 3720
   gggaccaccg ggctgcgtgt gggccgccag gtcaggccct cgcccaggat gggccctaag 3780
   ctggaacgag aaaggggaaa ccggccgcgc tgacgccggg gccttcgctg caagcgagag 3840
   gcggactcgg cgcacccgcg cggccctgga aacttccgag aggccccagc tctccgccgg 3900
   ctccacctct gccaggtggg gaaggtggcc cgggaccggg tccgctcagg accagaagtg 3960
   gtgcctggca gccatctggc caggaggccg gagagccggg cccagcggga gcgccgagcg 4020
   ctggcttcct tttcccg 4037
<210> 101
   <211> 2902
   <212> DNA
   <213> Homo sapiens
<400> 101
   cgcctcggcc cgcaggagga agctgaccga catctgggag gagctgaaga gcgagaagga 60
   gcagcgccgg cggctggagg tgaggcgcgg gtcccggcgg gagggggctg acggcgagtg 120
   gccccctgac aaagtctcca cctcactccc gaccaggtcc aggtgatgca ggggacccag 180
   aagtcccaga ccccgcgcgt catccacacg cagacgcaga cctactgagg gtgggcctgg 240
   gaagggaccg cggcctgacc tggctggggc cacccacgtc cttgcacacg actttgggaa 300
   acgcgagaaa gtaaactctg cctagcacgg cgccacgccg gtctggtcgc tgggggcggg 360
   gcctgcgcgg gggcagggcc tgggcctccg cattcctgac ggtccctccc aggcacatct 420
   ggccaacatg tggctcccat taccgttccc aaggcctgcg cgcggctatt tttatccacc 480
   ggatggtgag ggcgggggga gggtgtctcc ttcggactcc agccgcgcac tggagaaatg 540
   gcgggcagga ggggcccagg cggggtcggg ctcttgacgc ccggcctaga ttccggaatc 600
   cagctgtgcg ccggggagga gccgggcggg ccttctcgcc agctcggacc cctcccagct 660
   ggcctccctg tccgctcaga gggactcctg ccctctcgct tgaacgcacc tcggcgcaga 720
   cgtgaagtcc accgtccagc ccaagcacgc atgcttacac cggggctagc agcaccttag 780
   cctcgaggtc agggtcctga ggcctggtgt gtgtgtcatt gcagtgtatg aacccagggc 840
   actgtgggga cagggcgacg tgtgtccagg cgtttgccgg taaggaatgc gttttgaata 900
   aggaatgcgc gcgcgcttgc tctgtgtgtg aggctgggtg caggcctgtg ggcttctgct 960
   tttctgctgg ctgtgaagtg aatgcgggca ggtgtgctgt atcgtcgggt tggtgggtgg 1020
   gggtggtgca gctagggtaa gggagtcaag ctgggcctca gtcctggggc ccctgcgtgg 1080
   caggagcctg ggggtcgctg ggggcaggag taacagaggg agagactgag tcactgagaa 1140
   tgcagctttc tttcatttgg tcacttcacc tcttcatcga ccccagagag ggagtgggga 1200
   ccctgcatgc tgccccctcc ccgcccccgg ggtcttctgg caggactggg gaagggagcc 1260
   tctcaggggg tggcggtcca cgcccagtag cacctgggag ctgtgggggc cgaggcagtc 1320
   cgaaggtgtg gggtagctct gagctcatgt gcaggtccgg taccccgagc gccttgcagc 1380
   gggagccggg gcccatcagt aatagtgcat gcggcccagg gtgcccgtgg ccgtggggct 1440
   gggccccagc gtgcctgtgc ccagcaggtc cggctgcccg gtgcgccaga tctcccgcag 1500
   gatccgttcg cgctcctcca gccgctgcgc ccgctccagc tcctccgccg acgtgaagac 1560
   gttgacgttg aggggcccgt ccggctctgc ggacagctcg gggcccggca gcgtgtcgtc 1620
   ggggcccagc cgagtcaccg tgtcctcctc gtcctcgtcg tcgtcctcgg gctccagggt 1680
   gctgctgcgg gggtcccggc gctgagccgg gccccggggc cgcgggcggg gcgcccacga 1740
   gatgctgatg acgagcaggc agagggtgag cagcaggccg aagcagacgc ccagcacgaa 1800
   gtagaggccg aagctctcgg ggttggctgc ggggcacagg gcgggggtca cggagaggcc 1860
   cggacgggtg ggggttgtcc cacttccgaa agaccccagg ttctaggtgt gggggcgagg 1920
   cctgtaaccc ctactcgaag ggcattaggg aaccgccagc gggcaagagg gcggggagcg 1980
   gggcggaggg gacggacaag gcgctgggga gggaagggga tggtggtcag ggaagggttc 2040
   aggcccccag tcttccggcg cccgccctca ccgcggatgt gcgcgtaggc agccaggctg 2100
   ttgctgagca actccatgtc ccttcgcggg gcatccatgc tgctctgggg ggcagctccc 2160
   ctaccagcct gcgaggtcag caaagtcaga tccttctccc agcctggagc ccaccccgac 2220
   cccttccgcg ggcagctcct ctcccgggag ccgcgggccc aggttccacc ctggagagag 2280
   cgcttcccta acttcgtggc ggcccggggc cctgtcgggg ggaacccctc tcccgggcgc 2340
   ggacgcccct ccagtcctgg gagcagcgcc ggggaccgcg ggaggccccg ccccctggtg 2400
   aggtccccag ctgaggcccg gttccgccgt cccggggcgg gatggaggat agggacgctg 2460
   agcctccacg ccccaggcgg gctccgggaa gggaagctgc ctctctcctc gcccggccgc 2520
   cccgcgggga cccaccggcc cggggccagg cccactcacc gtcagcgccc cgcttcccgg 2580
   ctgcccggcc ccagcgcgtc ccgcggccgc atacactggg tccggccgcc cccgcccgct 2640
   cgggtcccgc cgcccctccg gccccgctgg gctcccaggc cgcggcagca cgggcgggac 2700
   acggaccgag ggaccagccg gcggaaagtt tcctccgagg aaagaggagg gacggggcgg 2760
   ggcggggcgg gcggggaggc ggggaggaaa agggctggga gaggaagggg agaggagcgg 2820
   gcagccggga ggagggagag cccggcccgc gggccgtccg tcccccacag gaaaccgccg 2880
   gggaggccgc ggcagggacc cg 2902
<210> 102
   <211> 2272
   <212> DNA
   <213> Homo sapiens
<400> 102
   cgcggtggca ctggtggctg ccggcagctg tcgtctgcag agtgggtgag accgagtgtg 60
   gcagatggct tggtccgcgg tgctggaggg gactggcgga gcagaagcgc cctggcgccg 120
   ggtccttgct ttcctggagc tggggcttgg ggaatggggt cggtcattgc agggacagag 180
   gagagggcag ggtaatgggt gcgcaggggt cccttccaaa ggctgcgggt ggccaggggt 240
   gtgcgggggc gctgaggtgc ggtcgccgag agattctggc ctccagggtg gtcgggcctg 300
   gcatggactc taggggatgg tgcacacacc ggaccggacg ggcccctccc ttacccccgg 360
   atcccccggc tgggcagcgg ccagggagag gggcgaccta cgggggcggg tgtggggacg 420
   ccggactacg cgtcaggagt cccagatggg cccagacccc cattccccgc cccgggccgc 480
   agtgacgtca gggcccggac gttcccggaa cggcctcttg ggggcgttcc agccccacgg 540
   acccgcaggg agtccccgcc gcaatttgca tggggctcat ttgcatgacc ccgccccgcg 600
   cgggagtcgg gggcgccggc gactggagga gccactgtca atccccaggg gaaagtgggc 660
   gaggcgattt ctgggccgct cacctcactc caccctccag ctcgcgccgc acctggggcc 720
   ctcccccacc tacgccccgc cagggcgggg ccgcgggcgc agacactcgc gggcacacgc 780
   acgacgacgc gcacacgcgg tcgcacgcgg ccccccgagc cccctgcggc gactccgatt 840
   cacccccgcg ggtgcggggc gcggacccgc ccggcccagc tcctgctccc caggcgcttc 900
   ctcctcccgc gacctgcccc ggccgcaccc gcctccgctc cagcacgcga cgctgcccgg 960
   cgcgacccct gctcccccgg cccagcccca ccgctgccct cgactcaatc ccagttgaac 1020
   gcccgcagcc tcagtcccac ccccggccca gccccagcgc ccccagtccc acccccggcc 1080
   ccagcttcag cctcagcgcc cccaggccca gccccagtcc cacccccagt cccaacacct 1140
   tcaggcccac cctcggcccc agcctcagcc accccagccc cagtcccggc ccagccccag 1200
   ccccagcgct cccggccccg ggcccagccc cagccccagc cccagccgag cacccccggc 1260
   cccgcctgcc cggcgttccc ttttgtgcgg cgctctcccg tccgcatccg cccgatcctc 1320
   tggaactccc cgcggacgcc ggggtccctg gaggcggctt cctttgtctc tgctcccgcc 1380
   ctccctccgc ggcgtcttcg cccctcactc acctccccag ccccgcgggg acgaggggag 1440
   ctggagggag cccgggaccc ggcgcggcct ccgcaggcgg cgctttcttc tcgctcccga 1500
   cgcggccgcc cctcctgcct gcccgctcct atcgccgctt ccgcctctgc cggggacggg 1560
   gacagcagac cctcggcgcc cggcccccgc gcacctgccg cgcgcaccgc ctctccggcc 1620
   tgcgcggctc cgggggctcc gggaagaccg agcgctggcg gccagcccgg ggaggctgtg 1680
   ggcagcaggg gtggctgcgg gctcccagtc cctcctccgc ctacctcgga gcccacattt 1740
   gggatttcta ctccgggaga attctgccct cctcccacga tgagcctgga ggactggagc 1800
   cgggacctgg cacccccagg gctgtgactg ggtgaactct gagggcacag ctgctgtccg 1860
   ccgccccagg ggtcccagga tggggcgagc agccctgaag aggcccaagg gcaccccgtg 1920
   aggagccccc acgctctgag agtggggcgc agagccggag ccccgggcca tgcctccgct 1980
   gccgctggcg cgggacaccc ggcagccgcc tggcgcctcc ctgctggtgc gaggcttcat 2040
   ggtgccctgc aacgcctgcc tgatcctgct ggccaccgcc acgctcggct tcgcggtgct 2100
   gctgttcctc aacaactgta ggtggccgcg ggcggggcgt cgcactggcc aagccccaac 2160
   cccgaggccc ctgcacatag aggctggagg ccgcggcggg tccgcggggc tcccggcagc 2220
   ttcccgggga tgggatgggt cactctgcgg acccctcggt ccctggaggc cg 2272
<210> 103
   <211> 299
   <212> DNA
   <213> Homo sapiens
<400> 103
   cgcgcatccg cccggggact tgttggtgga tccatccctc atggcggagc agcaagggga 60
   tcctttagaa aaagcaatgg gcgaagtaac tgaaagagcg acgcagaaag caacagccag 120
   aaacggcggg gacgcgagcg gcccagacag gaagggaggc ggtggcgcag ctctggtgcg 180
   cagcgcgccg cagcgacgga acttctgcaa aagctgcctg cccgcgcgtt atcagcggcg 240
   cgcaggcctg tggttttctc gctctcgcaa ccctgcttta actgccggtt tatttttcg 299
<210> 104
   <211> 275
   <212> DNA
   <213> Homo sapiens
<400> 104
   cggcgcagat ccgcacggtc tccctccgcc tcccgccgca agtccccgct gctcaccgcc 60
   ctcgcgcaag gcgccccagc ccgtaatcca gcagtgcagg ccgggctcga agaagtggga 120
   gcgcgcgggc aggcagacgg ggcgcacggc ggccgagcgc accaccgggt ggtcgagctg 180
   cagcagcgcc acgtcgtagt catggctgtc ctcttcgtgg tacgggtgca ggagcaggcg 240
   gctcaccttg aaggacacct ctccaggcca gcgcg 275
<210> 105
   <211> 2000
   <212> DNA
   <213> Homo sapiens
<400> 105
   cggtccccac ccgcatgcgc cctgctgccc cgcccccctc aggcgatgcc tccgcagccg      60
   gggctgcccg ccgggcctgg agcgctctct gtgcccggac cttgtaatta tgtcccacag     120
   cccgggaaac cgcgggcctc gcagcatctg gctgcggttg cgaacttttg tttcaagatg     180
   cgcgactatt tatagaagga aaactggtag gatagtaatt tcctaggagc aagaggagaa     240
   attggctgcg tctttattaa atgggggtgg gggcgaggcg atgtattagg actgcaaccg     300
   catgggggga gggggtggcg cagggagcgg gaaggcagcg gctgccccct ctggccgcgt     360
   caggcgaccc cggacgtggc ccgcgtgtaa aggaggggga gggggaaggg aagagaaggg     420
   gagggagagc aggctctgag gtttaatcag tagcatccag cttaaattcc cccaaacggc     480
   ggttccataa atgaaacttc tttttaagtc agaggccaga gcatatgcgt cccattagga     540
   attcggcgaa gccaccactg cgcgccccct gggctcactg ggctggactc ggccctgccc     600
   ggagcttctc agagccccgt gggctctgcc ttgccgggca cacacacgcg cgcccacgct     660
   ccgcgctctc ccttggccga gacaactggg cgaacgcggc cgccagcacg caatgcgcgt     720
   cctcctcgcc cccaggtgag gactcggcag gggcagggat caggctaccc agcgcggcag     780
   gaaggcgcga gaagccgcag cgacttcggg gtacagtccg ggcgctgtcc ggtgaagcgc     840
   ggcgtgcacg tgcatgccgg tgtttgcacg agggagcggg tgtgtgagtg cggctctccc 900
   aagtgtggca tggtattgcg cgccgcgtgg gttaatatgg ggaggggtcc cccgctggtc 960
   caggactctg ctgggacgcg cagggaatct cggcgccgct cgcgtgcccg cgggccctcg 1020
   cgctgggcgt gcacagcctg ctctactccc gcagtcgagc cgctctcagg ccatcctttc 1080
   tttgacccca gagccaggac ttctcactgc cgtagctgcc cgccaagtgc tccacctcgg 1140
   cagcttaggc tggcgaggcg acctaaagtc ccccggctcc cgcgcgcgac ggtcacccgg 1200
   tgccccgccc aggggcctcg ctgccgcgtt ccctgcccgc ccggttccag cgctggaggc 1260
   ccggggggag cccacccgcc caccccgcac gccgcccgcg gcactcacaa gtagctgccg 1320
   ctggacagga ggagaaagat ctgcgggcaa tagacggaga cgagcgcgct gcgcggcgac 1380
   agtagaagca tggtgggccg gcgtgcctgg cggggctgaa caggctccgc gggccgggcg 1440
   cctgggccag ctgaggccgg ggtccccgcg gagctccccg atgcagggcg gcggcgctgg 1500
   gcgctagggg ccgggtctcg tgggcccggg cgcagttgtc gcccgtgccc cgccgctagg 1560
   gccgcgcatg gcgctgggtc tggggcagcg gcgggagctg gggctggagt gcggggcggg 1620
   gacccggccg ggggcagcgg gcgcgccttg gctgcggtct ggacctccgt ccgtctgtcg 1680
   gtgcgaaccg gctctgtcgc tcgctctggc tcacttctcc aagtctcccg aggcgcgcag 1740
   ctgccattgg acgggccgcc cctacatccg cctcctcccc cggcgggttt ttttaaaggg 1800
   gaggctgacc ggagcgtgcg gtgggagccg cctccagggc ttgcccaagt tgccttggct 1860
   gggacacccg tacccccacc tttagggagc cacacagctc tggcctggct ccagaaggtg 1920
   cacgtggggg cggctccccg ggggaactgc gcggaggctt gtgctcctct tggggggctg 1980
   tgctcggcaa cgtatgcgcg 2000
<210> 106
   <211> 5759
   <212> DNA
   <213> Homo sapiens
<400> 106
   cgactcggtg aaggatgatg cccggagggt gatctcgacc tttaacattc cacacaccta      60
   cctccacgca ccaatcgccg gaatctccaa cccgcgggcc gcgtgggctt tctaccctgc     120
   accgctgcag ccgcggccac gggaagaggc gcgctcccgg cggcccaagc tgggagccaa     180
   gctctaacgg gtgtggcggg aagtgtggtg gcccgccagc agctgccacg acgctcgctc     240
   caccgacgcc cagagctgtg gccgaggccg ggggctggca cccgctgggc cgccactctc      300
   ggggattttg gtggcaaagc ggaggtcccg ccgaggctgg cgaggtgcgc ggctggctgc     360
   taggaaagag atccagacgg tcgcctggtg cgctggatcc ctgtgccctt tccctgaaac     420
   ccagcctggc ctgactgcaa cctctcccaa cttcagtgcc ggatccccta gacaatcagg      480
   gtgggctccc cgctgcgagc gcgccccaca gccgggtgcc gccaaaggtc tcctgctgtt 540
   agcggtgact cacattccca gtgatttaga aaaactgtgg tgccgagtga aagaaaaaaa 600
   aaaaagcaaa cacccttaga caaaagaaaa aagctccact ctttccgcga gcgggaaaaa 660
   aggtttggtt cagcaagtga tgctatttca gtgaatcaga cgctctgggg ctctgcaagg 720
   aaacgcacgg actgggagaa ggaagtggga gcctagagtt ttgctctcgg caaaacaaga 780
   actcggcttg tcctcccagg ccggcggttt ccgtacggca agacaaggcg cggaaaacat 840
   ttttaccatc tgacgctgta gtctgtcctt tagaagagaa taggtgaaaa gtttaataaa 900
   gaaattaagc gatgtggaag tcgttcccgc tgcttgcaga gggtggggag gcgggcctgg 960
   gcaatttcgg aactaggaga gaccacgtcg gcaaagcctg tggtcccgaa caagggccat 1020
   ggatttccca tttctcagat cccggctgcc cgacttcatt cttgtttctt tcagcacaag 1080
   ccatcctccc ttctccctcc ctccacgtct cttggcagag acagaaaggg acaccgctct 1140
   gcccgcgtcc agcgcgaagg aggtgaccta aaacccatgg gcccttctca cactgcgttc 1200
   tctgcgctcc ggcccagttc cgggaggacc tcccgacggc gcgggagaag gctgtaaggg 1260
   ctcttggccc ccgacgcggc ctgggtctcg ggggacgcat gaacccgcca cctctggccg 1320
   gcttcctttt agtgccccaa atagcagcgc ttagcccgtt gcttcccccg actcttgttt 1380
   ttcaaagtgt tcacagggtc gcgagagcga gcgacgcggg ggcggcgagg ctgcaaggct 1440
   gcacaggaac agacgacaag aaatagggct ttcttctgcg tggttctttt ccggtccctg 1500
   tctgagtcgc ccgaccccgc ggggagcgcg gagcattgcg gcgtgcggcg ggcttcgggc 1560
   tctcagcccg cgcagcaagt tgtgttgctt ttccccgggt gcgacgcggg acggccgcga 1620
   agagcaaagt tcgtccgcgg taggaacttc gaggcccggt taggaccgat ctccggcctc 1680
   cgagtctact tagccgtgtc cacgctcgtg ccttttccct cttgcagcga aataactggg 1740
   cggggcccga cgcgcgtcct ccagcgactc cgggaaccca aggcctcctt cacctcgctc 1800
   aaaattcacc cgaaaatgcg ctgagacttt ggcatggggc tgcgctgtgt cccactgacg 1860
   ttacagaagc gcttatgaat ttaaaaactg ctccaggcgg ccggacggcg ggacaacgcc 1920
   tcctcacttg ctcctctacg gcaaggcccc ttccttctcc ctgccggctg cggtggtgtg 1980
   tagggtggtc cctttacaga gtttccttaa tccatctagc ctgacctcac ctgcgggatg 2040
   ttccatttcc cgatccacct cccacacccc cgctgcagtt gtctagaagc tggggctggg 2100
   gaactgaggc gcgggaggag cagacaggag ggccggggtt ggggcgccag ccacgcgttg 2160
   ggagagcaga cccgggtgca gactcagagg actggagagg cggcatggag tttggtctcc 2220
   tgggaggtag agtgaggtgc tgcggactcc agggaggaaa gggatgggtg gggaggcggg 2280
   aggggaggag ggacggggta ttttgcgctc ccgcaccgcg gcccgaactc agctgttctg 2340
   ccttcactgg cctcagccag tcgagggagc tcctacgagg ttatctgcga gcccagcaaa 2400
   tcttgggggt cctagccaaa tgcagtgcgc aacgttctct ctcacttgga ggccgccatt 2460
   cacctcgttt ttttttttcc taaaaggatt tttttttctg agtgcttttt tcatttccct 2520
   attttacctg ctttgcacat gcctcccgcc ctcacccggg aggcggcgaa gacgcccact 2580
   gggaccgcgc ctggccgttt tctgggcgcg tcctgccccc acggcctctg tctcttaggg 2640
   cgactgggcg cggaagaaaa gctggagagc ccctgcgggg tggcaggagg agggtgcctg 2700
   agtcccgcga gaggcccggg cgaggaagat gcgcagcctg ctgatccgcg tcccgcccgg 2760
   cgccagggac cctcagaggg aggagagctc aaagacctcg ccccgcgcct tcgcgaggac 2820
   caacccagtc cccgcgcctg ccccagagcg gcttagaaac tcagggcaca gtgagagcgc 2880
   agggcgcctc ccgaggcttc acaccgccgg ccgggccagc ggaggctgtg accggagcgc 2940
   cccctctgtg ccgcgcacgc cgcggccgcc gccgggttgg ggtaggtgag cgggaggcta 3000
   gggtacactt cggggtgggg gatgggcgcg cacatggccg ccagcaggca gagttactcc 3060
   ggtaaacacg ccagggacgg cggcgcgcgc gggaggcaac ccagcggggc aacccccgcc 3120
   tttacctgtc cgagcctgca cgcgccggcg gccgcggcgc cgaggggggc ggagcttgcc 3180
   agcccgcgcg ccggggcggg acctagcggg ggcggggtcc gcagtgattg ggcgtaggag 3240
   cggggccgcc agccagagct gggctgcagg gccgcgcagg tttcacttcg ctccgcgcag 3300
   ccgcctggtc tgcagtttgt tggagctctg cgtccagcgc cgctgccgct gccgccgccg 3360
   ccgccgccgc cgccgccgcc gccgccgccg ccactaccac cacttgattc ttgcagccac 3420
   cctgcgaacc ctgccacact gcgatcgcat catcgcggta ttcggttcgc tgcgttcccg 3480
   ccgccaccgc ctcggcgccc tttcttggcc cttgttcccc caaatgtctg actctgactc 3540
   tcggactgag aaacgcaagg taaatacctc caaatcccgg ggcgcgggcc tggcaccgaa 3600
   ggaagactag cgctggagcc cgaacggcac cacctactgc ccgcttcccg tgcacgtgcg 3660
   agcggagacg cttgcatcgg cgccgcttgt cgccggcggg aggtggggat ccgtgtggat 3720
   cgggttccct ctcgggcagg ttctctttaa agaaaagtcg ttcacgcgga acctgcaggc 3780
   tcttggccga agtgcggctt gggacgggat gctgcgcgcg ggcctggtga agggtcgtag 3840
   gtccgggcag gaggagaggc agcgcgtgcc tttcggcgcc ggcccgggag ctgaggacct 3900
   gctcgtagta tcgggctgtg cgcccagctg gaaagtctcg ccgccggctc cggcgccctc 3960
   agagtagcgg ccgcgcagtg tgagggtgtt tcgtgtcttg gtttttgggg gcgcgtgggc 4020
   ctgttgcgga ggatttgcgg aactgcggtg gtgatttaac tgacgagaca cggcttttcc 4080
   ttttactcat tcaggccaag gtttcggggc cactgctgcg ttcgctttct caagcgtacc 4140
   tggggcccct gcttgctctg caggagtcct gcggcctgtg ccatccgcac gattccctgg 4200
   cagcgagaaa tctaggtcct cccctgacca cctggaccgc gcaggggact agtcgcggcc 4260
   ggcagaacct gctcggtccc tgggccgggc tatcttagcg gctcacgcat gcccgagcac 4320
   acccgacccg gttcgcggtg caggctggcg gcgccgtgcc ctctgggacc tgttgcccca 4380
   gggttggcgc tgctggtgga gggcccgacg gacgccaggg cggagggtgt gaatttactc 4440
   gatggtcgcg gacgtgcgcg tccgcggcca ggggtggctc ttcggctttg actcgtgtct 4500
   gcggcgcacc aggagagaga ggaagttgtt ggaggagaat agttgccaca gccagggcgg 4560
   tgacggccgt tagggtctgc ccccgacggg ccaaaacaag gacaagtggc gaggactggg 4620
   cctggggatg gccctgcggg tccgggcgcg gcgcaagtcc tgctttgtct ccaggtgact 4680
   ggctgtccag gacggcggga gctgctaagg cttgtgtccg gaaggagcac agggatgggc 4740
   agggtggccg ggccagccgg ccctcaaatc ctccgacgaa gcggcagccg ccacgaccac 4800
   ggccagagca gcgctcggag gcgagtttgt caacaatcgc ctcgcctttg gcggcctgac 4860
   ccgtaggcgc cgcccccacc gcggctgccg attggctgcg gcgcggagcg accgcgcgcg 4920
   gaccaattgg gaacgcgggc acccggcgcc agcggcgcgg ggaggtcggc ggtgccggcg 4980
   gcggcgggcg cagagcgcga ggggaggagc gggaggaggc ggaggatgtt cccggcggct 5040
   gcggccgggg cagcgcgggc cagaggcgcg gcgtgcggag ccctcggctg cccggcggag 5100
   cgcggcggcg ggctggcggg aaggcgcggg ctttgcgctg cgccggggac tctgaacccg 5160
   agtcccgggc tttgtctcct gcgctgcttt cgtggtgacg gtcagggggc gcccggtcgg 5220
   cgaagggcgc gggccggacg ccgcggggct tggtccctgg cccggacgct gcgctctgaa 5280
   gggaaggcgc ggacgtgagt ttcggtgtga ttgccttctg aggggagggt ctgtgggatg 5340
   aaggcggcgc ggcgcgcacc ggtgtcgcct agcctgcgga cctagttgta gaccttgcag 5400
   gcgttcgctt cgtcgccctc cgccgccccc tccccattta gagatgtgca cctcacggtg 5460
   tgcacctcag agaagttctg tctgattcgg tgcggcgtgc ggtacgggag cgggagggag 5520
   ggagcacggg cggagagagc gccagagccg ggccgagccg cgctggagtt acaaactcta 5580
   ttgtgacgca cttactacga ctgacggccg ctgccagacc ttccccagac ttgctgccct 5640
   cagcattttc ggcaaacaat ggggccggag cagggaacgc ggccagccgc ctgcaatcgc 5700
   tgcatctgcg cccgctcctg tgctccggcg tcctacctaa ccccgggaag tcagagccg 5759
<210> 107
   <211> 1297
   <212> DNA
   <213> Homo sapiens
<400> 107
   cgctcgccga cctcagctgt tggggaccgg gtccctcaat ctccgcgtcc tgagcggtcg      60
   gcggcggtgg ggagagggcg gcgggtgccc ccggtcaccg cgctcggtcg ggctcagtcc     120
   cggcggccga gcgtggggat ccgggggccc ctggggtgga gttgtccgga agcctgggct     180
   ccactcctgc tgcctcacag ccttgccgtg tcccgtctgg gcgcctgcgt tcccaagtga     240
   caaaatgacc tcgctcctcc gaggggtctc ccaacctctg tccagatctt cttccccgct     300
   aagtgccctg tcaccggagg ggatgtccag tgtgaccccg cgggaatgtc ggggtgcttg     360
   ggcccggggg tgggacaggg ctgctgcagg gagggcagcc cccgtaagag gagggaggac     420
   ggcgacaccg ctgagcgcag acggcgcgcg gagccggggc tgggcgctca ggtgcgaggt     480
   tccgtccatc tttctgaacc cacgcaggtg ggcgcgggaa gggacccggc agccgccagg     540
   gcagggtcag ctggcggggc agccccgcac ggtgaccctc cttgggtcgc ggggagccgg     600
   gcacgggagt gggggagcgc gtctttgttg gggaggaggg cgggcgggac agggtaagac     660
   tggggttcca ggcccggggc gcgaacccgg gtgggagggc gcagggggct cccctctgtc     720
   cctgtcacct ccacccatcg ccggccgccg ctaggggttc cggccccgcg ccctcccccg     780
   acccttcccc ggcggaggtc ctggggcagc caagcccacc gcccggccct cctccgcggc     840
   cgtcccgaag acccggagaa gtggaccaca gggggcggcc agtgtcaggg gtcgagtcct     900
   ccgcgctggc cgcggttccg gagatccggg ctttgccgag cgccccgcgc cgtggccggg     960
   gccggggcca ggtccgggtg cggggcctgg cgctgcggcc gcgagctcag cctgtaggct    1020
   gggctagcct ccgtcggcgg cgggcgctca ggagcggggc cgcggcggcc ggaggacaaa    1080
   gggcccgagg taggagcggt ccctccgggc ggcagcgggc ggagcggggg cgcggggctg    1140
   tgcggggctg ctcggggcgg gcactcggcg cccggcgggg aaggtgcgcc ccgccggacg    1200
   cgggcacttc ctggccctgg tcccgggaag gaggtggaag gtgtccggga tcggccctgg    1260
   cagcgtgtag ggacctgggt gctgccgctg ccgtgcg                              1297
<210> 108
   <211> 4027
   <212> DNA
   <213> Homo sapiens
<400> 108
<210> 109
   <211> 976
   <212> DNA
   <213> Homo sapiens
<400> 109
   cggacttgac ggagcaatgc acgtgggcct ttgactcgta atacacccag tcaaagccgg      60
   cctccactgc caagcgcgcc agcagtccat acttattgcg gtcgcggtct gatgtggtga     120
   tgtccaccgc gcggccctca taatgcaggg actcctctga gtggtggccg tcctcgtccc     180
   agccctcggt cacccgcagc ttcacaccgg gccactggtt catcaccgag atagccagcg     240
   agttcaggcg gtccttgcag cgctgggaga ggaatgtgcg cgaaatcaac cagagcgaaa     300
   tcagccggag gagccggccg aggtgcaggt gtaggcgcag cctcgcccag aactctcccg     360
   ccacacccaa gggagctgga agcccgggag ggtgctgtcc ctctccctta ggaacagagg     420
   gccctgaagt gctccctccc tagcatacag ccgcccccgg ggatctggac cgcacggcgg     480
   tatcacaaat ctggaggagg catggaggca agcgccctgg cggcgccttc actggtaggt     540
   ggaggtcccc tctctcctgt gctgtcttca gctccgggta tccatccctc cacagacagg     600
   actacacctt tcagcgcccg gtccctctgt gcctccaagg caggagggcc agccagcggc     660
   cgggagggag gagtggcagc agagccgact gcctgcggcc ggcctgcgag gcgtgcaagg     720
   cccgaggtgc ccagtaggtg tcaccgcagc ctcagggctg aggcctgggg acgacaggct     780
   ctggaggcgc ggcaggtgca gagcgtctcg gtagcttcag ggggtgtgtt ggaggggaat     840
   cgccggcctg cctggatccc tcgagcacca ccgaggattc ccgccgggac cctcttataa     900
   tcttaaaccg gcccacaacc ccgccagaga tgacagggcc agggaaccag aaaccggcgg     960
   attagcgcca gggccg                                                     976
<210> 110
   <211> 539
   <212> DNA
   <213> Homo sapiens
<400> 110
   cgccagcagc ccgcgcggcc ccgcggttcc acggggcctc gtccccgccc ctgccccgct 60
   gtaggccccg cctgctatag gccccgcccc gctgtaggcc ccgcctgcta taggccccgc 120
   tccgctgtag gccccgcctg ctataggccc cgccccgctg taggccccgc ctgctatagg 180
   ccccgcctgc tgtaggcccc gcctgctata ggccccgctc cgctgtaggc cccaccccgc 240
   aggaaacctc gccccgctgg tggcgcgccc tggttgccgt ggcaacctcc tccctccgcc 300
   gccgggttgc cggcttcgct agcctctcag cagcgccgtt gccatagcaa cggcgggtgg 360
   gtcccgcggg gcgagctgag ctagggtgag catcctgtgg ggtagactat ggcggctggg 420
   cgcgtgcggc ctgtgcgggg cctccccggc tgcggtgaga gcggctcgag gggtcccgtg 480
   cccggaccct gaagtgcggc caagccgtgg ggggctccgt gggctcgcgc tgcaccgcg 539
<210> 111
   <211> 467
   <212> DNA
   <213> Homo sapiens
<400> 111
   cggtgcagga cgcagggcgc gctctagagt gaaggagtga cgagcaaagg gaaggaatgg 60
   gcaagcgtag gcgaagagag gagggcagcg gggggtgcaa aggtactcag tttgccctct 120
   gtgagcgggg ggctgcaccc accttcttct ggaaggagcc gggattgccg cggcggatgg 180
   ccagcagcgc ggtgtagtgc agcgtctcgc gcacggtgag gctgctcagc agggtgtcgc 240
   tctgcaggag actcgggcgt cagtgtagcc taagcccccg gggcgggcgg gggggccagg 300
   ggtgtggggg acgcgcccac ctgcaggacg taggagaagc agtcctggaa ctgctcccgg 360
   cgcagcgccc ggccgttcac atacacctcc cccaggaagg tccccgcgcg ccccagcctc 420
   ccggacatgg cgtccagcag cgtggttttc ccggagcctg cggggcg 467
<210> 112
   <211> 239
   <212> DNA
   <213> Homo sapiens
<400> 112
   cgcaacgccc ggctgaggtg ccccttccgg aagggcgcct gcgagatcac ccggaagacc 60
   cggcgacagt gccaggcctg ccgcctgcgc aagtgcctgg agagcggcat gaagaaggag 120
   agtgagcagt gggcgcgcgg gcgggccggc gccggggtgc acggctctga gtaaggacgt 180
   gccgtgggtg tgggcatgct tgtgtggaga tgcgcgccga gtgtgcgcgt gaacacacg 239
<210> 113
   <211> 584
   <212> DNA
   <213> Homo sapiens
<400> 113
   cgcctctgat gggatgcgcc ctccccgccc aggaagtggc ggcagaaagc gagccctgag      60
   aagccagggg caggagcggc ctccgcgcga cactgcggcg ctcctgattc tgcggcctgg     120
   ggccgagcat gcggggcggg cggagcctcg agctaagtcc cctggggtcc cagggccgca     180
   ttcctccgag gtctgcaaag gccactgctt aaaggcgcag aggagcagct gggaacgaga     240
   acaaagcggc caggcccccc tcggaggaag gaaggagaga gccccaggaa acagctgata     300
   gcgctaagct cagcttgttt ttttcctctg ctcaacagtt ctcctgccac ggcaaacaaa     360
   acatgtacat tctgattccc tcttctgttt ggattgtgct gtcgactgga tctggtttgt     420
   gatgagctgg gggaagaggc atccgcgggc gatttctggc tcggcgtgcc agtgtgcttt     480
   tgctgggccg cgccgggatc gcggagcttc ctctccggct cctttctccc cgtctgcgtc     540
   gctaatccag cctggcccgg ccaccccaag ggaagacacg gccg                      584
<210> 114
   <211> 766
   <212> DNA
   <213> Homo sapiens
<400> 114
   cgagttccac cggaattggg ttcgagcgcg ggtgaaaaga gggacccgac agcacccacc      60
   ctccaagtag aggcaaaagt tgaaagtggc gggctggctg cgcttccgaa gaaacacgac     120
   tggcgaaggc tcaccttagt ctctacggtg ggtccctccc tgtagccgac ccgttccttg     180
   aagcgggcca gaaacgccgg ctcggctggc cgcacgtacg atacctggtt ccgcttgctc     240
   atggtagctc tgggtaaaga cagaagacag gttccgcgac ggattcgcga cgatacagag     300
   gcgcttagct gtgacgtcac tgggccacgc ccccgatgcg taggccgggc aacatggcgg     360
   cctccatgcg ggcgtcaacg tccgatccaa gcgccaaatt caaatttgcg gccatcttga     420
   gcgggcggaa ttcagtcgcg cgcggtgcag tcgggaggtg gaggcaccgg ctgcattgtt     480
   ttcgggatcg aggggtgagg gcgctatggc acccggctgc aaaagtaagt ctgggccccc     540
   ggcttcgtta ccctattttt gcccccaaat acagctgtga aaggatggca gcctcggacc     600
   gcccgcaagg ttcttgctag gcatgaactg caggagctga gtgaccggcg gggacgtttg     660
   ggaatcccgc tctgtcgctc aaagacaata cgctagccga aaagctcatc ccttccttca     720
   gtgtcctgtg cgcatgccag gagtctggcg cagttctagg cgctcg                    766
<210> 115
   <211> 737
   <212> DNA
   <213> Homo sapiens
<400> 115
   cgagtggagt gcgacaaggg ataaaccatt ttcgcgcact cttcagcgat ggggcgaaag      60
   taacggacct agtcctcggg agctgtcccc gccgaccccc tctgccgcga cttgacccgc     120
   ggcgactgcg ctgccccttg gctgcccctt ccgctctcgt aggcgcgcgg ggccactact     180
   cacgcgcgca ctgcaggcct ttgcgcacga cgccccagat gaagtcgcca cagaggtcgc     240
   accacgtgtg cgtggcgggc cccgcgggct ggaagcggtg gccacggcca gggaccagct     300
   gccgtgtggg gttgcacgcg gtgccccgcg cgatgcgcag cgcgttggca cgctccagcc     360
   gggtgcggcc cttcccagcg cgcccagcgg gtgccagctc ccgcagctca atgagctcag     420
   gctcccccga catggcccgg ttgggcccgt gcttcgctgg ctttgggcgc tagcaagcgc     480
   gggccgggcg gggccacagg gcgggccccg acttcagcgc ctcccccagg atccagactg     540
   ggcggcggga aggagctgag gagagccgcg caatggaaac ctgggtgcag ggactgtggg     600
   gcccgaaggc ggggctgggc gcgctctcgc agagcccccc ccgccttgcc cttccttccc     660
   tccttcgtcc cctcctcaca ccccaccccg gacggccaca acgacggcga ccgcaaagca     720
   ccacgcggag atacccg                                                    737
<210> 116
   <211> 278
   <212> DNA
   <213> Homo sapiens
<400> 116
   cgctaagcgc tgactcgggg aagggagggc gcagcctttg tcccgcaggt ctcccgcgcc     60
   cgcctcgctg tccccgcgag cgagcttggg ctcgaccccc gttccccctg aacgcacctg    120
   caaacaaagg agagccgttc cttcgccaac ttcctcacct ttgcctcccg gtccttccga    180
   agtcgggcgg ggatgccgcc ccaggtgcta cgacaagccc cagggtgccg gcaccagcca    240
   gcggtgccgt cgctcaagca gccgcgcgca ctgccccg                            278
<210> 117
   <211> 905
   <212> DNA
   <213> Homo sapiens
<400> 117
   cgcctgaagc gctccgactc cgactggcgg gcctgaaggc atcaccgagc ttcaaggacc     60
   cggagtccag gttcatggag ccttggggtt gcgtctcggg cagccatccg acctcgtgat    120
   cttcaaactt agccagcgcc ggatgctgga actccaaggg ctcgggccca gggaggccag    180
   agctggagca ggcctcaccc aggcattccc gcgccggccc accgccccga cccacacgat    240
   ccagctttcc tggctctgag aagcaccact tccgctgctg gttggcgagg ggaccccggc    300
   cggcagttcc tggcgcggga gcccgggagc gcgccggctc cccctccccg cccgggtggc    360
   tgagcgaggc ggagcgcggg tgagtcgccg ggggccgcgc tgggacagtg ggccgcagac    420
   gggcgggcag gctcatgcgg agctccttgc gctggaacga cgtctcccgg agcactcgcc    480
   gctgcgcgcc ctgaagccgc tggcggtagg cggccctcga ggccggcggg ctgggcggct    540
   cggcagcctg cgccgcggcc tccgcctcgg ccgccagcgc gtacagcagc ggggtggcct    600
   gcctgttcag tggtcccggg gtccccggga cctctgttgg ctgcggccca ctgcgggctg    660
   caaccgcggg ccggggccgc ggggatgtgc aaagggcagc gtcgggcggg gcggggcccg    720
   ggccgcccca aaccacacgc acgtagtccc agtctaggta aggaaggagg tctgtccccg    780
   gcgactgcgt gcgcggctcg gggccgccgg aggctgcgga gaaagagctg taggccgagt    840
   ccgcgcgcat ggacagatgc cacaggtcca ggctgctagt ggacgaggcc ggggaggcgc    900
   ggtcg                                                                905
<210> 118
   <211> 674
   <212> DNA
   <213> Homo sapiens
<400> 118
   cggccgccgg gcacagtggc ggcgagtccg agcgcagcgc gtcgtgcgcc ccacgcaggc      60
   ccaggaaagg tgtgctctcg gccgccagcg ccccgtccgc gtcgtccgcg tcgtcgtccg     120
   acgccgaggc tgagccgccg cccgagccgc tgctcagcga cagcgagtcc ctcgccgccc     180
   gtgcgcgctg cgccgccagc ccgttgaggc gcgagcggcg ccagcgccgg ggccccgccg     240
   acgtcctgcg ggacgcaccg cgcgcgcgcg gccgcggcgg cggcgggggc gcgcactcct     300
   gcgtggtctc gtactcgtcg tcctcgggga tgcggaaggg gctggcaggc aggctgccca     360
   ggctgccgcc gagcgcgcag gtcccgcgcc gcggtccggg ccccgccgcg gggtaatagt     420
   agctgtcata gctgcgctgc atgtctgcgc cgggcccggg cccgggcccg ggtccgggtc     480
   ccgggccggg gggcgccggg tgccgcagta acggctgctg ctcggccagg cggtaactga     540
   tgggcgccgc cggcggcagc gacacggcgt gcgccgagtt gggggacgtg atctcgaaag     600
   ttggcacctg cgtggccagc gagtagtgga agtccacggg cgagaggcgc gcgggcgtgg     660
   tcagggccga cacg                                                       674
<210> 119
   <211> 1271
   <212> DNA
   <213> Homo sapiens
<400> 119
   cggacggcac gctcaagtac atggaggtga cgctgcggcc cacagactcg cagagccact      60
   gctacaggac gtgcttttca ccggcctcgg acggcagtga cttcactttt ctaagacccc     120
   tcagcgttca gcagcccaca gctctggcgc tggagcctga cgccatccgg tcccgctcta     180
   atacgctgcg ggagcggagc caggtgaggg gctcggcgcc gccccgggcg acccctgggg     240
   gcggcactgg agaagccgcc cgtcctcata agggattgaa cttgcatcca ctcctctccg     300
   gccggcttgg tcgctggctg cgctccaccc gattctcggg atcattggac cgtttgcgcg     360
   aaaccagagt ggccgattaa gggatggggc tccgagcacc gggggtggtg gcgactgtgg     420
   gcgaggggag gtgggaccga cccccacccc tacactcaaa aaaggccggg gcctccttcg     480
   agcttccggt gaatttcggg cgatttccgc gggtgtcggg ggtcccggga ggaggcagtc     540
   acagatccac ccctgcagcc agcctcctag gcgccggctc cggcacgctt cgccggtctg     600
   tagatttcct cttcgatttc tccccagctc ccagcatctg tgacttcact gttaccctcc     660
   ctatccccgc atcacccaac cgcacctgtc tgcgggactt aggtgtgcgc gcggggctca     720
   tgcgtgtcct ccctgctggc cacccccacg gcccacacaa gttgcacggg ctcgccacgc     780
   cccgccaaca cgtgcgcgga cgcacgcacg cactcctcgc acgtgggctt acgcgaatac     840
   cagctttcac tgccactcgc tcgcggccag attcacaggc ctgttccggt ccactcgcag     900
   ctcccctctg ccgctccctc cgccgggctc aggagtactc gtagctgatt gtgcgcgcct     960
   gagggtccca gatcgcggcc gcccaggacc aggcgaggac tccggagcct cctctcacct    1020
   ctcccacctg cgccccgggc tgggccgggt cgcctggggg gcggcctgag cgaggcgcgg    1080
   ggccaggagc gctggagcga ctgccgctct aagtgccggg cgggcaggac tctacgatcc    1140
   ttgggccaga ggtccggatg gtcccgggac tccgtctcaa gggtcggcga cccctcaacc    1200
   cagaagcctc gagcaggcgg acaggcagag ctgcccagtg gccgaggcgc ggcagggctc    1260
   cgcgttgggc g                                                         1271
<210> 120
   <211> 1364
   <212> DNA
   <213> Homo sapiens
<400> 120
   cggccacctg gcagagcgtg gtctcggagg gtcgcgcggc gccgcttggc aggcacaccg      60
   gctgaacgta aggcgacagg agcgcgcagc tgccgtccgc atcctcctga aggcgcaaca     120
   gagctaaccc gggcggagag gagcgtgagg cggggacgcc ggggccccaa gctctcttcc     180
   cgtccccgcg gggcgccccc acgcacccag gtcgtgctgg tagctgacgg gcgagaaggc     240
   ctcgtgcaag cggtaggagc gcacggccaa cgtctggcac ggctcacagc tgtggttacg     300
   gcgttcctgg ccgagcacca ccgtcagatc ctcgggtgcg ggcctgcggg gggggtaggg     360
   gagaggcagc gctctcagac ctggccacaa gcgttctggg gaagccccct gctcccgaac     420
   cccaatcccg tgttccagct tcctccccgg gagctccgga ggggcgtgga aagaagggtg     480
   gggcgggaga aggtagggca cgggttcggg tgcagcgtgg aagctgcgct gggagacgga     540
   ggagccgcgg cccctggggc ggctctgggc gggcgggtac tcgccggtcc tgcaggcagt     600
   gagcggccgt cagcacccag cagggggcga tgaggctgcc ggcgcagaaa ctgtggcccc     660
   agtacagcgc ggcgatgtag gggtgcgccc cgcgtagcgc caccagcccg ccaacgacgc     720
   gggtcatcga agacagactc ttgcggagcc gctgcccgca gctcagtggg ccgttcctgg     780
   tcagggaagg cggctgctcc cgcttcgccg gcaaggctgt ggaggagcag gggctgagga     840
   cggagagccc gcggccggct ggccggaatc tagctcgccc ggcgccctct cggctcctcc     900
   ttcccccccc cacttcctaa cctcccgggg tctgggactg aggcggggtc cgggtcgtgg     960
   gctgaggctt cggcggtgcc ggctgcgcgg gcatgagtgg gacatgaagc ctaggggaca    1020
   ccggggtcgg aggcgccgcc tgggttgggg tctggcactg tgccaggtcg cagtactccc    1080
   agctcagccg gtcgcggttc agcacgaagc accacgggcg gatgtcgttg tccgggttcc    1140
   tgtagccaca cgacggggcg ccgttagagc gccgggagcc cggagccctg gggcggaggg    1200
   gtcacccagc cccacgcggc gcaccggcag aaggcgtggc cgcccagtcc ccagttccgc    1260
   gcttgctcgg cagtcacgtt ccggtaggtg gcctccgagg cccacggctg acagggcgca    1320
   cccgagagcg tggtcctggc caggccgcgg tagctgagcc cgcg                      1364
<210> 121
   <211> 1010
   <212> DNA
   <213> Homo sapiens
<400> 121
   cgagccggag aagatgcgcg gggcgcgtgt ggctgcggga agagcttctg cattgtgttg      60
   ggtacgctgc ggcgcggcaa tgttcgcaag ctccctggat gcacattgcc tcttttctct     120
   ctttcttttt gtcagcggtt cagcgaggaa gaatgcctcc aacccagccc aatccaggcc     180
   agtacgcact caccaacggg gaccccctca acggccactg ctacctgtcc ggctacatct     240
   cgctgctgct gcgcgccgag ccctacccca cgtcgcgcta cggcagccag tgcatgcagc     300
   ccaacaacat tatgggcatc gagaacatct gcgagctggc cgcgcgcctg ctcttcagcg     360
   ccgtcgagtg ggcccgcaac atccccttct tcccggatct gcagatcacc gaccaggtgt     420
   ccctgctacg cctcacctgg agcgagctgt tcgtgctcaa cgcggcccag tgctctatgc     480
   cgctgcacgt ggcgccgttg ctggccgccg ccggcctgca tgcctcgccc atgtctgccg     540
   accgcgtcgt ggccttcatg gaccacatcc gcatcttcca ggagcaggtg gagaagctca     600
   aggcgctaca cgtcgactca gccgagtaca gctgcctcaa agccatcgtg ctgttcacgt     660
   caggtgaggc tgcggtcgcg gggagggcag gccgcgccgg cagcgagcgc aggccccgcg     720
   ccgcccgggc ctggccttcg gagcctcccg cgcggccggt gcggggcggg ccgggccctc     780
   gctggacact gagcctggcc cgggtctgtg acccccgcgc ggtgaccggc tggccgcgcc     840
   gctgccatct tgggagcgcg gcggtgcggg aaccgaggcg gcgcgggtgc ggccggcggg     900
   cggccgggcc tagtcgcccg gacccctacg tggggccgcg gctgccgagg gtgtgtgtct     960
   gaggcggggg ggcggtgctg tggatccgtc tggctgcgcg cgcgcgcgcg               1010
<210> 122
   <211> 1530
   <212> DNA
   <213> Homo sapiens
<400> 122
   cgggcctccc cttggcgtcc cgcctcggag ccctcccatg gtgatgacca accgcggccc      60
   ggtgccgctg cccatcttca tggagcagca gatcatgcag cagatccgcc cgcccttcat     120
   ccgcgggcct ccgcaccatg cctccaaccc caacagcccc ctgtccaacc ccatgcttcc     180
   cggcatcggg cccccgcccg gtggccccag aaacctgggc cccacttcca gccccatgca     240
   ccggcccatg ctatcgcccc acatccaccc cccgagcacc cccaccatgc ccgggaaccc     300
   cccaggcctg ctgcccccgc cgcctccggg cgccccgctg ccgagtcttc ccttcccgcc     360
   agtgagcatg atgccaaatg gcccgatgcc ggtgccccag atgatgaatt tcgggctgcc     420
   gtcgcttgcc ccgctggtgc cgcccccgac cctgctcgtg ccgtaccccg tgatcgtgcc 480
   cctaccggtg cccatcccca tccccatccc tatccctcac gtcagcgact ccaagccccc 540
   caacgggttc tccagcaacg gggagaactt cattccgaac gcccctggcg actccgcggc 600
   ggcgggcggc aagccaagcg gacactccct gtccccccgg gactccaagc agggctcgtc 660
   caagtccgcg gactcgcccc ccggctgctc gggccaggcc ctgagcctgg cgcccacgcc 720
   cgccgagcat ggccggagcg aggtggtgga cctgacgcgg cgcgccggca gccccccggg 780
   ccccccgggc gcgggcggcc agctcggctt cccaggcgtg ctgcagggcc cgcaggacgg 840
   cgtcatcgac ctgaccgtgg gccaccgagc ccggctgcac aacgtgatcc accgcgcgct 900
   gcacgcgcac gtcaaggcgg agcgcgagcc gagcgccgcg gagcgcagga cctgcggcgg 960
   ctgcagggac ggccactgca gcccgcccgc cgccggcgac ccaggcccgg gcgccccggc 1020
   gggccccgag gcggccgcgg cctgcaacgt catcgtgaac ggcacgcgcg gcgccgccgc 1080
   cgagggcgct aagagcgcgg agccgcctcc cgagcagccg ccgccgccgc cgccgcccgc 1140
   gccccccaag aagctgctgt cgcctgagga accggcggtg agcgagctag agtcggtcaa 1200
   ggagaataac tgtgcttcca actgccacct ggacggggag gcggccaaaa agctgatggg 1260
   cgaggaggcc ctggcggggg gcgacaagtc agacccgaac cttaataacc ccgcggacga 1320
   ggaccatgcc tatgctctgc ggatgctgcc caagaccggc tgcgtgatcc agcctgtgcc 1380
   aaaacccgcg gagaaggctg ccatggcacc gtgcatcatc tcctcgccca tgctcagcgc 1440
   cgggcctgag gacctggagc cgccgctcaa aaggaggtgc ctccgaatta gaaatcagaa 1500
   taagtaaaag gtttgtatgt ccgccgggcg 1530
<210> 123
   <211> 949
   <212> DNA
   <213> Homo sapiens
<400> 123
   cggagggcgt tcgtcctggg actgcacttg ctcccgtcgg gtcgcccggc ttcaccggac 60
   ccgcaggctc ccggggcagg gccggggcca gagctcgcgt gtcggcggga catgcgctgc 120
   gtcgcctcta acctcgggct gtgctctttt tccaggtggc ccgccggttt ctgagccttc 180
   tgccctgcgg ggacacggtc tgcaccctgc ccgcggccac ggaccatgac catgaccctc 240
   cacaccaaag catctgggat ggccctactg catcagatcc aagggaacga gctggagccc 300
   ctgaaccgtc cgcagctcaa gatccccctg gagcggcccc tgggcgaggt gtacctggac 360
   agcagcaagc ccgccgtgta caactacccc gagggcgccg cctacgagtt caacgccgcg 420
   gccgccgcca acgcgcaggt ctacggtcag accggcctcc cctacggccc cgggtctgag 480
   gctgcggcgt tcggctccaa cggcctgggg ggtttccccc cactcaacag cgtgtctccg 540
   agcccgctga tgctactgca cccgccgccg cagctgtcgc ctttcctgca gccccacggc 600
   cagcaggtgc cctactacct ggagaacgag cccagcggct acacggtgcg cgaggccggc 660
   ccgccggcat tctacaggta cccgcgcccg cgccgcccgt cggggtggcc gccgcgcccg 720
   gcaggaggga gggagggagg gagggagaag ggagagccta gggagctgcg ggagccgcgg 780
   gacgcgcgac ccgagggtgc gcgcagggag cccggggcgc gcggcccagc ccgggggttc 840
   tgcgtgcagc ccgcgctgcg ttcagagtca agttctctcg ccgggcagct gaaaaaaacg 900
   tactctccac ccacttaccg tccgtgcgag aggcagaccc gaaagcccg 949
<210> 124
   <211> 671
   <212> DNA
   <213> Homo sapiens
<400> 124
   cgcgcaccag cagcaggcgg cccactggat ccacgcccct ggcccgcagg tcctgcaggt 60
   cttcgggccg cccgtagtgg gcgtacacca gctctccctg gggacacgag gacggtgagg 120
   cgcgctcccc gcgtccctcc ttcgagaccc aggaaagggc tcgtgccgcg ccccatccta 180
   ggagcgggca gggggtgctc accgtgacgt tgccgatggc gctgtagggg cagtagacgt 240
   cagggtcctc cagcggcagc tgctctccga ccttcccggc ctcatcgacc cagtgcaggg 300
   tgttggggtg agccctgggg agcggggctg gtgagcgccc cgagccgcgt ccccctcccc 360
   gcgcgccccc cgcccgcgcg cgcactcacg gatccgggaa ttgcagcccc acgtagtgcg 420
   tgtcggtcca cacgtggtcc agcttctggc gggagagcgc cgcgcgaatg tcctgagtca 480
   gagcggccat cccggccgag cctgccaccc gttcccgaag gctggtttgc ctaagcgggg 540
   agaggtgggg acttttctgg gcgcccggag gagagggagg gaagagggaa ggatgccaga 600
   gacgtggggt cgccaggggc aggggcggcg aggggttcct gagtttagga aaagagcgct 660
   ccccgcggac g 671
<210> 125
   <211> 364
   <212> DNA
   <213> Homo sapiens
<400> 125
   cgagtaactc gtccgcagcg ctcgcgggga gccaggacca gggcagacgc gggaaatact 60
   gccctcgggc ggccgcgctg tgctgaaaag ggaacacagg acaccccaca ggcagcctcg 120
   gtgccacttc ggggtctctc tgtcccacca cggggtgcgc tgtccggacc gcggcctggg 180
   gacgttcttc cgccactaga gggcggccca agccccgagc ccgcggcccc gggaggatag 240
   gatgcccgtg gcgtggaccc gcgcagcaca gcctccccgc gcggcctctc cagccgaaag 300
   agccggcgcc gccacgtgga ggtgtaggtc cggcctggcg cggacacctt cgtgagtggg 360
   cccg 364
<210> 126
   <211> 3722
   <212> DNA
   <213> Homo sapiens
<400> 126
   cgcggtggcc cccgcacccg gagcgtcggc agcacctgga gcggttaggg ctactctgcc 60
   gccgccgccc ccgcggtccc cgccgccgct gtccccgccg cggtccgtgc gcgcgggcgc 120
   cagtgcagcc aggagcgcgt gcgccaggcg gaagggcgcg aaggcccggt gcgcccagct 180
   gtgctcctcg atgaccgcgt agcacgaggc cagcacccgg ttgatgagaa tggtgccctg 240
   ggccgtgagc ggcgcgtagg cgcccgcggc ctcctcgctt agggtcacgc tgtgcacagc 300
   ggcgggcagg agccggcggt ccccgtcacg ctcggccacc acgtacacgc gctggcccgg 360
   gcgcacgcgg ctggcgaaca gcgcccgagg ccccagtgcg cccccggaag gcggccccga 420
   gcccgaggac gcctcgggct ccccggtggc cgagtcgttg tgcggcgcca caaagagcag 480
   gtgcgcggcg gtgagcagca ggcgctcgcg cggctcccgc gtctcgatca cgtagaagac 540
   cttcttggcg ccgtcgtcgc ggtccaggaa agtgaggaag tcgctgtaga gcagccggcc 600
   ctggtcgtcc gccgccagca cgcggtcccc ggggctcagg tccttcacca gcttggtgcc 660
   gccctgctcc aggtgcaccg tggccgagcc cgggaagcag cctcccgatt tggccgccac 720
   cgagttctct gcgggtgagg agaagggaaa gaagagagga cagggcattg agttccgaga 780
   gggaggcgcg tctcggggag gagggcgcac gcttggtgcc cgcgctccta ggccaggggt 840
   gcgcaaggcg cggggcgggg cgattgattc caggcgggtc ccgcacacac ctccctcccc 900
   caaccccact gccccaggga caggattccg acacattcct taacgactct ctaagtctgg 960
   gctggcggtc acagaggttg gctggcgttt cctgcagagg ccggtgacac tctttccttc 1020
   cgccttcctc caaccccacc cccagctccg cacacacccg gcctcgccgc gaatccatcc 1080
   agagggtgtt tgctctgcac ttggattcct caggaagcct ccttgagctc ccgcgggcgc 1140
   cgggcgcacc ctccttccct cccgccccgg cgcggggcgc ggcgcctgct cgcgcactca 1200
   gcacccgggc tgcgcggcgg aaacccctgg cccgcctgcc cgcctgcccg cctgcccgca 1260
   gcaccctgcc gcgcctcggc gtgggcctgc ggaggggcgg gggcggggag tgaaaaatag 1320
   ctgcagtagt tgtagcaact ggacaaacat tccagagatt ggccgaggtg tgaaaatccg 1380
   aggcttgtgt ggattttcta attaaaatcc aataaagggc ccagcattgt cattgtgatt 1440
   tctgggataa acaggagaca ctcttggaag gggacacttc cattttctcc cctccctgtt 1500
   ctcagctcac tctctcccca gctcgcgggc ctccccctcc ctatttgctc aggtgcaacc 1560
   cccccccacc ccgtgtgcaa ttcacaagca ctatttgcac agccaggcct ctcttccaag 1620
   cactcaagat gtgtatttga attttaaatg gcggggcctg ttcggagcgc tggaagcgcc 1680
   gggtctcaag gccccttgac tacccgcttt ctcgagccct ctgtcgctct ctgaggctca 1740
   ctgtccccag gacaataatt gaagttgggg cggagcgcag ggggccgggg aggagggcgg 1800
   ggagggccgt cttctttgca ttccaatctc cgggatcatg ctttttgcaa atagagattg 1860
   ccagaaagaa tcttcctgct ggaattacag ggggctttta atcttctcat cggtttctct 1920
   ggcatgaagt gcggggctgg ccgttggagg ccctccgtag ccccccggaa tgcgaccccg 1980
   ggcgctggcg agcccgagtc gtcgagtcgc acggcccggc ttgacacgct gcgccccgcg 2040
   cccccgcccg tgtgctcaga atgccaatga gcggctcccg cgccgcggct ccgctctccc 2100
   ggcgcgatcc gggggatgga ttagcgctgc tcacttggga ggaggcctcc ggagaccccc 2160
   gctaggggga ccccgcgggc agggagccgg ggaggaggtt ttgcgcaggg ggcgccctgg 2220
   gcccaggggc cgccgccgag cccgcgaggt gggggcgccg cctcctgcct gcctgcagcc 2280
   gggagctctc cgggccgcac acccgcccct gccgcccccg ctccacgcct ttgctttctg 2340
   tggggacgag aagccactct agcctttacc tacaagtgtg ccttgacacc ttaacagtgt 2400
   tttcctacta acatagtgcc attaacatgg aatttaggag acaagtaggg aagcctggaa 2460
   gggggttgag gaaggcagag tttccccgaa attcaccgtg aacggctttg ctaaacgtgc 2520
   caatttatgc gggcggagaa gagcgctgtg cacacagcct cgggccggag gtttgcgtgc 2580
   gcgcgatggc gcctcctcca cggctgttcc tcgccggcaa cagcgtgtgg cgttgcaggc 2640
   ccagagaaga cagcctcgcg ccgactcggg ccggcgtttc ggggcctgcc cgccgcccag 2700
   ggcgtcccgg cccggctccc agtaggaacg caggctccgg gcgcgccgcc tccagatgct 2760
   ggaggtggtt gcccctgggt ttactgccgc ccaccaggcc tctccccaaa ggcagcttag 2820
   gatccacggg cgcccggcgg ggaggagcca ggacgcgggc tccggggcag gatagacacc 2880
   cctgcgcttg ggatagcggg tgggggcggt tggttcttaa gccctaaacg agaggaagga 2940
   gctcgcccag acctggctaa ccgcgcaggg ctgtcttccc tcgcctggaa gggcctagcg 3000
   cccctccccc agtcacgctt tcccaccccc tgcagtgggg gctccagaag cgggcaggaa 3060
   gccacaggac cccatctcgg ggcatggaga accttgggct ctgtggcctc aaaggtaggg 3120
   gtgatttcga ggggccggca cctcacaggg caggttccac cgcggaaacg cagtcatcgc 3180
   ccagcgaccc tgctcctggc cctcagcctc cccccaggtt tctttttctc ttgaatcaag 3240
   ccgaggtgcg ccaatggcct tccttgggtc ggatccgggg ggccagggcc agcttacctg 3300
   ctttcaccga gcagtggata tgtgccttgg actcgtagta cacccagtcg aagccggcct 3360
   ccaccgccag gcgggccagc atgccgtact tgctgcggtc gcggtcagac gtggtgatgt 3420
   ccactgcgcg gccctcgtag tgcagagact cctctgagtg gtggccatct tcgtcccagc 3480
   cctcggtcac ccgcagtttc actcctggcc actggttcat caccgagatg gccaaagcgt 3540
   tcaacttgtc cttacacctc tgcgaagaca aggggacccc caccgacgga cacgttagcc 3600
   tgggcaaccg ccacccctcc cggcccctcc atcagcctgc cctgcccagt ctcaaggcgc 3660
   gagggccatg cggagaggtg gggagacggg ggttggaatg gcccagacac accgaagagc 3720
   cg 3722
<210> 127
   <211> 1995
   <212> DNA
   <213> Homo sapiens
<400> 127
   cggcaccgcg cggcctccgg agctgcaccc ccaaatcccc gtggcgactt catttgaagc      60
   gtggaagaag caaccacgca agtggagagt gggttctgaa agctctggga tgaaccaccc     120
   tctctctttc tgtcgttcct cttttaggac cccggctgcg gcgaggagga aggagccagc     180
   ctagcagctt ctgcgcctgt ggccgcgggt gtcctggagg cctctcggtg tgacgagtgg     240
   gggacccgaa ggctcgtgcg ccacctccag gcctggacgc tgccctccgt cttctgcccc     300
   caataggtgc gccggacctt caggccctgg ggtgaattca gctgctccta catcagcttc     360
   cggaaccacc aaaaattcaa attgggattt tccggagtaa acaagagcct agagcccttt     420
   gctcaatgct ggatttaata cgtatatatt tttaagcgag ttggtttttt cccctttgat     480
   ttttgatctt cgcgacagtt cctcccacgc atattatcgt tgttgccgtc gttttctctc     540
   cccgcgtggc tccttgacct gcgagggaga gagaggacac cgaagccggg agctcgcagg     600
   gaccatgtat cagagcttgg ccatggccgc caaccacggg ccgccccccg gtgcctacga     660
   ggcgggcggc cccggcgcct tcatgcacgg cgcgggcgcc gcgtcctcgc cagtctacgt     720
   gcccacaccg cgggtgccct cctccgtgct gggcctgtcc tacctccagg gcggaggcgc     780
   gggctctgcg tccggaggcg cctcgggcgg cagctccggt ggggccgcgt ctggtgcggg     840
   gcccgggacc cagcagggca gcccgggatg gagccaggcg ggagccgacg gagccgctta     900
   caccccgccg ccggtgtcgc cgcgcttctc cttcccgggg accaccgggt ccctggcggc     960
   cgccgccgcc gctgccgcgg cccgggaagc tgcggcctac agcagtggcg gcggagcggc    1020
   gggtgcgggc ctggcgggcc gcgagcagta cgggcgcgcc ggcttcgcgg gctcctactc    1080
   cagcccctac ccggcttaca tggccgacgt gggcgcgtcc tgggccgcag ccgccgccgc    1140
   ctccgccggc cccttcgaca gcccggtcct gcacagcctg cccggccggg ccaacccggc    1200
   cgcccgacac cccaatctcg gtgagtagga gcgcgagggc tggggcgcgt gagggccggg   1260
   gcaggggccg tcttgagccc tgtcgagggc ctcttgtttt tccaccaacg ccttcgttgg    1320
   gctggggatg gtgcttcact acctcgagtt tctagggaag gcagaagcca gtgcggggct    1380
   ggcgacatca cagccccaga agaccggctt ctgtggaagg ggccgggcct gcccgccggg 1440
   gcctcttctg agatggtgtc agggtcggag tgcggcctcc ccgccatccc agacatcgac 1500
   cgtggccgcg ctgcgctgtg ggtgacgcgg gaggacagcg ggctccctgg agagccgggg 1560
   gcagcggcct gggatttcct cgtggaaggt gctggagatt gctgagtttc tgcgcccctt 1620
   tcctccccgc ccgccctcgg gcctccgcag ggaactgatt acaatggttt ggaccgcaga 1680
   ccttctgggc catttggcgg cccagctgga ggatccctcg gggtagctga tgattttccc 1740
   gtcgggggtc tcacaccgag aacaaaggag ggatggacaa aggagacgcc ggggagatgc 1800
   gcggaacagg agccggcact gtgcgggtgc cacccggccg agcgcgtggg cgcatcatgc 1860
   gggcagcggg ggggggggcg cacacgcccg gtcagtgtcc gggaacatag ggacctcaaa 1920
   cgcgcttgtt catgacaccc gagttaaatg gagactttgc agtcgcttgc acgcgtggag 1980
   cctcctcttc tcgcg 1995
<210> 128
   <211> 4742
   <212> DNA
   <213> Homo sapiens
<400> 128
   cgaatgggga aacgcttctg tcgcgtgccc cggcagagct gcgcgcaggg cagaaccaaa 60
   ggtccctccc cctagattgg aagctccatg gggtagggcc acaaggccgt caccactgcg 120
   ggctggcctt ttgggggact ttgcggggcg ctgggcggga gtccatccct cctcaggtgt 180
   caggtctttg aactcaggcg cccgacaccg cggggccgca gagccacaga gagacgccta 240
   gggggtgggg tggattctgc cgcccgggca gctggagtgc gcggcctatt ccatgctgac 300
   ctgcgtgggg gcgggtacag gggagccgga ggcagcgcac actgttcgct cctcaccagc 360
   gctgcgccgc ccgcactcaa ggcaggtcgt cctccacgtg ccgctggctg catcccgatc 420
   ccgcttgcgt cgggcggggc tcagaggttc ccggtgcgac cttcgcgggt ggatggggcg 480
   ggacgaggag gatccagggt ggggatttga gatcaggtcc ctttcgggtt ttctttttga 540
   agcgcccctc tgcctccgcc cgcgcctccg ccaggctcgc tgcgtcagca cctcaccggc 600
   tttgcacaca ggctgtgcca cgcggccttc acccctgtga ctccccgcag ctcgcgcgga 660
   tgcaccgacg agtcagcttg tcctctggaa gccaatgagt ctccccggcg ccccgtggcc 720
   gaagagctag gtgtaccatg caatccccgc ccgactggtc cccggcgacc gacgccccgc 780
   ggccctcgcc cttcccgccc ccggcgctgc acgccctcca cggcttggcc cgcgcgctgc 840
   tgttcccggc ctactgggcc ctgcaccagc tgcagggtgg ctgaggaggg cggcgggcgg 900
   cgggcggcgc tgctgctgct gctgctggtg gtcggcccgc ccctggcgct gcccggcctg 960
   ccgctctggc tggtgccgca ggtttggcgc cgccccttct gctaccgctc ccctccgctg 1020
   tgctgggcgc cgcccgcgct ctggcgcccg cctgccgaac ccgggcgctg cttcgtcttc 1080
   ctcaccgcca atctgtgcct gctccccgac gggcaagcgc gcttcggcaa cctaccgcac 1140
   agccagcgac cggccgaggc cattggcgcc gcgcggcccg cgctctacag ggctacggac 1200
   tgcagccagc cgtggtccag ggcgccgcgc gggacgctgg tggcctcgct gcccgtgggt 1260
   ctggactttg tgtgcctgca ggaggtattc gatctgcgca gggctcgtcg cttggtgaat 1320
   cgcctggcgc ccaacctggg cccgttgctg tacgacgtgg gcacgctcgg cctacagccc 1380
   gggcggcacc tcaagctgct ggacagcggg ctgctgctgg cctcgcgcta cccgctgctg 1440
   cgcgccacct tccgttgctt cccccacgca cgtggcgagg acgcgctggc ctccaaggga 1500
   ctaccgtccg cacaggtact ggcctatccg gtgcagcccg cagagggggc gggggttcgg 1560
   ccgagaaggc ggagaaagct ggagccgtcc gtggtgttgc aggcgaggct gggcagcctg 1620
   gacgggcgcc gcatcgtggg attcctgcac tgcacgcatt tgcaagcact ggctggtgag 1680
   ccctgcggat gggtcctcgg aacaggtccc gcctctccat agcggctgct gatccctaca 1740
   ttcgcctgca ccccgccccc cacctcccac cctttgttca ctgcccccgg caccggaatt 1800
   cctgagaagt gcctgtcggg gctcacagat caggaactgc ggagacggga gctgactgcc 1860
   caggatgcgg gtccgaatct ggagccaagc ccaccccacc ccccctcccc ccacgcagaa 1920
   gacgggccct tgcgcagcaa acagctgacg ccgctgctgg actgggccga gaagtttggg 1980
   gccgagaagt ttgaggccga cagagaacaa agtggagagg tcgtggcttt cagcgtgctc 2040
   ctgggcgacc taaacttcga taactgctgg aaaggtccag caggagagga gggcgaggag 2100
   gggcggagcc aggaggcccg gccccgccca ccgcgcgccc gcctctctct gcagaccacc 2160
   ggctagagca ggagcacgag ctcttccgct gtttccagga tccctgccgg ctgggcacgc 2220
   gccaaaagca gccctgggcc ctgggtatcg cgcttggggg gagggtaccc ccgccggctg 2280
   ggcacgcgcc aagagcagcc ctgggccctg ggtatcgtgc ttagggggag ggtatcggag 2340
   cgggaagtgg acctggggag cgccgtcggc tgaggctctg gctgatgccg ccctcccccg 2400
   gatcccccag ggaccgcgct gagcacctcc gtgctccacc agtccatggc ctcctccccc 2460
   aagatgccga ggcggtgagt tgcgacctgg atgtaggcac tgcccgcccg aagcgcgcgg 2520
   aggggccctg gccttgatga caccgccccc ctaccagggc cctggagcag gagaaagggc 2580
   gccacctcta cctggccggc cttcccggca gaagccgccg agctaagccc tggagaggtc 2640
   ggcgcctgga ctacatcacg taccgcggag ttcccgggtg gctgggcctg cggcactggg 2700
   acgaccctca acctgactcc cgcccccagg aggtggagca ggtgacgttc agtaccgccc 2760
   tggaggggct cacggaccac cgggcagtgc gcctgcagct ccgagtctca gtgtcctcct 2820
   aaggcaagca cagatgaggg gcgcgcggct ggcgcgcaca gacacgactc ggagcacgaa 2880
   ctaggcgccg tagctgcgtc cccagaaccg ggagacttaa ggcatcttta ttgcgggatc 2940
   ctcacacggc ctcctgggcc cggcgatact catagacgct gccgtgctcg ggaaaggcca 3000
   gtgcttgcgg gggcgacccc ggcggtgggg cggggtcctc cgggtcccca tagccaccgc 3060
   cgccgggcgt gtggagacag aacacatcct gttggcgcgg gggggggcgg ggaggcgggc 3120
   tcagtgcagg cgtggcccct ccctgtcacc tgcgcagtcc cctccccacc gggcagcgtc 3180
   catcacccgc ccccatggac cacggaggcc cggtacgcgc cccctttcct tccggggcca 3240
   aattaaactc ggcctctggt caggtctgca actgttcccc ctctgtggga tggggccggg 3300
   aggggagggg aggggagggg gacaggacag gccgcggccc ttaccccggg gtacacggtc 3360
   accgacgtct tgccgcccag attcaccgtc cggccgtttt tgcggatcag caggtttagg 3420
   ccgcgggcgc caggctcgcc ccctgcggag ggaggcgagg agtccagaga gaccaggggc 3480
   gggggtgggg gcgggggcgg ggagggccgc gagggctggg gaaccgcgca ccgtggagcc 3540
   cgtatggccg gaaggcgcgg cgctcggtca gcactgacag cagcgcctcc tcacgaaaga 3600
   gcagctcgcg ggtgacgccg tcgccgcctc ggaagcggcc tctgcccccc gagccccccg 3660
   ccgcagctcg aagcggcgca ggatgaccgg gtacctgcga gggcgaggac gagggcaaag 3720
   actcagcgtg gcccgagccc cggctcccac ccggccgtgc ccccagcctc cgcgcacgcc 3780
   gctcaccggc tctccaggat ctcagggtcg gtgatgcgtg tgttggtcat gtggctgtgc 3840
   acaccgctgc gcccgtgcca gctgggaccc gcgcccgcgc cgcccgccac cgtctcgtag 3900
   tagcccatgt gggcgttgcc cagggtcacg ttgttcatgc agccctggtg agggcacctg 3960
   gtcgctgcgc tgggctgggg cagggcgtcc ccacctcgcc ctcccgctcc tactccagcc 4020
   tgcccaccca gtccgccccc gagctgcgcc caccccgccc ccgcacctgg gaggcggcgc 4080
   aggccccaaa ggcccccagg atgacatcca ccacgcgctg cgacgtgagc acgttgccgc 4140
   ccaccaccgc cgcctcgggc gacgggtcca ggatggagcc tcggggaatg accacgcgca 4200
   ctggcgccag gcagccctgt gcggggcggg cggctctcag gagctcttgg ggtgggctcc 4260
   gggagaaaca gcaccccacc ccccgcccca gcacccgtgg ggtcagaccg tggctgggcc 4320
   caggaggcgc actcagggtg acctcgggct ggggcccccc ctcgcgcaca cacccctgcg 4380
   aacctggttg agtgggatgt cgcggcccac cagacagcgc aggcagtaga tgagggcgga 4440
   cagggttacg gcccgcggtg cgttgagatt accaaacacc tccggcccag tgccgctgaa 4500
   gtcaaacacg gcgctgccct gcgcgccccg agggaaggga gaggctgtca gcggccgcac 4560
   cgtgcccctg ccgcctagag gcactctccc cacggcccac ctgactcagg ctgatctgca 4620
   cacggaggcg gatgggggaa ccgtcgtcca tgtggtcttc cgaggacacc tccaggggca 4680
   ggccccgggc ctgccgggag gttccaaagg cacgcaacat gtctcgcacg gccagctcag 4740
   cg 4742
<210> 129
   <211> 1267
   <212> DNA
   <213> Homo sapiens
<400> 129
   cgggccaagg cggcgggaaa ggcaccgcta cctgcagccg cacgactcca ccaccatgtc      60
   ctcgtactgc ttgtagacca cattattgcc cgcgtcgatg tatagaatgc tgatgggagt     120
   caatttggtg ggcacgcagc agctgggcgg ggtggagccg gggtccatgg agttcatcag     180
   cgtctggatg atggcgtggt tggtgggctc caggtgcgag cgcagcggga agtcgcatac     240
   accctcgcag tgataggcct cgtactccag gggcgcgata atccagtcgt cccagcccag     300
   ctccttgaag ttcacgtgca ggggcttctt gctgcagcgt agcctggact tcttgccgtg     360
   ccgcttgcca tggcgactgg cgaaggccgt gcgccgccgc cggcggccgg gcgagggcag     420
   ccaaggcctg gcatccgggg cgcccgacgg cggcggccac gacccctcgg cgcccgcgcc     480
   cgggcccgca gcctcggccg agcccagctg ctcgcgcatc tctgcgaaca ggttcttgcg     540
   ctgggatctg gtgaatacca ccagcagggc ccgctcctgg ggaggccgca ccctccggcc     600
   gaagcccaga ctccgcaggt ccgggggcgg cggttgctgg ggtccccgcg cgcgcgcctc     660
   ggcctccccg gcgtccagct cgccccatgc ggcccgcagc tccaagcaca gctgcttcca     720
   gggctggtgg cgcaggccct gccacacgtc gaagacttcc cagccggccg gcggcgcccc     780
   ctgcgggtcc agggtccgcg cgtccagcag taggggcgaa aggcaaggga agagctgcac     840
   gtggagcggc ccggctggtg gcccccaggg cgctgagggc gcctggcgaa agagccgcag     900
   ctccgcgccc accagctctt ctttgtctga gagcatggac acatcaaaca aatacttctg     960
   tctccggaga ggagtgtgcg agagatcgtc tgcgagataa aaaataatta cagtcagttt    1020
   cacttaaggg ggagatcagc ccggtgctct tcggccgccc cgggaggaaa agggcgggga    1080
   gtgggggcag gtcggccggg cagtccagct tgcccggccc agggcctgac caccccggct    1140
   ccccatctgg ctggtgcatg gcgcggggaa gggggcgcgc caggacgggc cccctccttc    1200
   gcgtcagctc cggactctca gggcggaagt cggtggctgc tggcgaggcg gcggcggcgg    1260
   cctaccg                                                               1267
<210> 130
   <211> 270
   <212> DNA
   <213> Homo sapiens
<400> 130
   cggtccgtgg gcgctgggct actggagggg aagcgaggag gcggggaagg ggcgggcccc 60
   aggagcaggc gggcggggct ccccgccctc cagcgcgccc ggaggctacc actccctgca 120
   gatgcgctgg ccccagcccg gggctgccgc tcgccttccc ccggagtctc ctggaccccc 180
   ggagtctcct ggacccccgg agcgggaggc agcggccgcg cggcgctgga ctggagcaga 240
   accccaggac tgcgcccccg ggagcgggcg 270
<210> 131
   <211> 462
   <212> DNA
   <213> Homo sapiens
<400> 131
   cgtctcatgc cagcctcggc gcagctccgg agagcgggag gcggaggctc agagcggtgc 60
   agccccaccg ggccccagcc cgttgcctcc gcccccacct caccccatcc ccagcagcac 120
   cacttccgct caggcctggc tgctggcaaa atctcggcac agagggagga gggggagagg 180
   aaaacgcatg attcctcctc aaaatggagt cagccgaaaa aagcgtgaat gcagagcccg 240
   aagagactcc tgggggaggg gagcccctgc agggccagcc gagggccggc gcaatggctt 300
   atctgaggga caggcagaag gacggacccc cacggtggac cccagctacg caccgtgtcg 360
   tggtggggcg ggaaggcgaa ggtgtactcg tctgccagca gcctgcggta ggcgtagtcc 420
   tcgtgcgcgc ggctcccgta agccccgtag tagtcgtact cg 462
<210> 132
   <211> 569
   <212> DNA
   <213> Homo sapiens
<400> 132
   cgccggaggg gcggggccgg gacccaaggc tgggtcctga gcgccccgag ggggcggccc      60
   tgccaagccg aagagcggag tagcggtgta agcctggcga cggccctcgc gccgattgct     120
   gtctatggcg gcctggagct cccctgggga gctgagcgct cgagtctcgc actcgtacgg     180
   gtacaggtac ttcatgtacc taggggcgga cagcgggcgg tgagtgtatg ggaggctgca     240
   cccagctggt caagtcccac agccggacgc ccgcccgggg accctccccc ctcccgcccc     300
   aggccgaacc cgtgccccct tcccttccgc tcccgcccct ggcctcactg ggtgcgtaga     360
   gtgaaggcgg ccgaggtgat ggtggtgggt aggctgaggc cgcgcgtgac ttcccgccac     420
   actttgcggt tgatgacttc caccaggccg cccttggcgg tcaccaggcg aaacagagcg     480
   tacaggtcga gcacctgctt cgccatgatg ggcacgcggt tcactggcgt ccctggtggg     540
   gagcgggctg ccgtcaggac actgagacg                                       569